(19) Europäisches Patentamt
European Patent Office
Office européen des brevets

(11) EP 1 169 338 B1

(12) EUROPÄISCHE PATENTSCHRIFT

(45) Veröffentlichungstag und Bekanntmachung des Hinweises auf die Patenterteilung:
**03.11.2004 Patentblatt 2004/45**

(21) Anmeldenummer: **00920597.2**

(22) Anmeldetag: **28.03.2000**

(51) Int Cl.[7]: **C07K 5/062**, C07K 5/065, C07K 5/068, C07K 5/072, C07K 5/078, C07D 409/12, A61K 38/55, A61P 7/02, C07K 5/06

(86) Internationale Anmeldenummer:
**PCT/EP2000/002710**

(87) Internationale Veröffentlichungsnummer:
**WO 2000/061608 (19.10.2000 Gazette 2000/42)**

# (54) NIEDERMOLEKULARE INHIBITOREN VON KOMPLEMENTPROTEASEN

LOW-MOLECULAR INHIBITORS OF COMPLEMENT PROTEASES

INHIBITEURS DE FAIBLE POIDS MOLECULAIRE DE PROTEASES DE COMPLEMENT

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **09.04.1999 DE 19915930**

(43) Veröffentlichungstag der Anmeldung:
**09.01.2002 Patentblatt 2002/02**

(73) Patentinhaber: **Abbott GmbH & Co. KG**
**65205 Wiesbaden (DE)**

(72) Erfinder:
- **HILLEN, Heinz**
  **D-67454 Hassloch (DE)**
- **SCHMIDT, Martin**
  **D-64625 Bensheim (DE)**
- **MACK, Helmut**
  **D-67067 Ludwigshafen (DE)**
- **SEITZ, Werner**
  **D-68723 Plankstadt (DE)**
- **HAUPT, Andreas**
  **D-68723 Schwetzingen (DE)**
- **ZECHEL, Johann-Christian**
  **D-69226 Nussloch (DE)**
- **KLING, Andreas**
  **D-68239 Mannheim (DE)**

(74) Vertreter: **Grünecker, Kinkeldey,**
**Stockmair & Schwanhäusser Anwaltssozietät**
**Maximilianstrasse 58**
**80538 München (DE)**

(56) Entgegenhaltungen:
**EP-A- 0 601 459**
**WO-A-94/29336**
**WO-A-95/35309**
**WO-A-96/17860**
**WO-A-96/24609**
**WO-A-96/25426**
**WO-A-97/23499**
**WO-A-98/06740**
**WO-A-98/06741**
**WO-A-98/55471**
**WO-A-99/37611**
**WO-A-99/37668**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 169 338 B1

## Beschreibung

Die vorliegende Erfindung betrifft peptidische Substanzen, deren Herstellung und deren Verwendung als Komplementinhibitoren. Speziell handelt es sich um Substanzen mit einem Guanidin- oder Amidinrest als endständige Gruppe. Insbesondere betrifft die Erfindung Inhibitoren der Komplementproteasen C1s und C1r.

Die Aktivierung des Komplementsystems führt über eine Kaskade von ca. 30 Proteinen letztlich u.a. zur Lyse von Zellen. Gleichzeitig werden Moleküle freigesetzt, die wie z.B. C5a zu einer Entzündungsreaktion führen können. Unter physiologischen Bedingungen dient das Komplementsystem der Abwehr von Fremdkörpern, wie z.B. Viren, Pilzen, Bakterien, Krebszellen. Die Aktivierung auf den verschiedenen Wegen verläuft dabei zunächst über Proteasen. Durch Aktivierung werden diese Proteasen in die Lage versetzt, andere Moleküle des Komplementsystems, die wiederum inaktive Proteasen sein können, zu aktivieren. Unter physiologischen Bedingungen ist dieses System - ähnlich wie die Blutgerinnung - unter der Kontrolle von Regulatorproteinen, die einer überschießenden Aktivierung des Komplementsystems entgegenwirken. In diesen Fällen ist ein Eingriff, um das Komplementsystem zu inhibieren, nicht vorteilhaft.

In einigen Fällen überreagiert das Komplementsystem jedoch und trägt damit zur Pathophysiologie von Krankheiten bei. In diesen Fällen ist ein therapeutischer Eingriff in das Komplementsystem durch Inhibition bzw. Modulation der überschießenden Reaktion wünschenswert. Inhibition des Komplementsystems ist auf verschiedenen Ebenen im Komplementsystem und durch Inhibition verschiedener Effektoren möglich. In der Literatur finden sich Beispiele für Inhibition der Serinproteasen auf C1-Ebene mit Hilfe des C1-Esterase-Inhibitors ebenso wie Inhibition auf der Ebene der C3- bzw. C5-Konvertasen mit Hilfe von löslichem Komplementrezeptor CR1 (sCR1), Inhibition auf der Ebene von C5 mit Hilfe von Antikörpern, Inhibition auf der Ebene von C5a mit Hilfe von Antikörpern oder Antagonisten. Die verwendeten Werkzeuge zur Erreichung der Inhibition sind in den oben angegebenen Beispielen Proteine. In der vorliegenden Erfindung werden niedermolekulare Substanzen beschrieben, die zur Inhibition des Komplementsystems verwendet werden.

Generell ist bei jeder entzündlichen Erkrankung, die mit Einwanderung von neutrophilen Blutzellen einhergeht, mit einer Aktivierung des Komplementsystems zu rechnen. Es wird daher erwartet, daß bei allen diesen Erkrankungen durch Inhibition von Teilen des Komplementsystems eine Verbesserung des pathophysiologischen Status erreicht wird.

Die Aktivierung von Komplement ist mit den folgenden Krankheiten bzw. pathophysiologischen Zuständen assoziiert (Liszewski; M.K., Atkinson, J.P.: Exp. Opin. Invest. Drugs (1998) 7(3): 324-332; Morgan, B.P.: Biochemical Society Transactions 24; 224-9; 1996; Morgan, B.P.: Critical Review in Clinical Laboratory Sciences 32 (3); 265-298 (1995); Hagmann, W.K.; Sindelar, R.D.: Annual reports in medicinal chemistry 27; 199 ff (1992); Lucchesi, B.R.; Kilgore, K.S.: Immunopharmacology 38; 27-42 (1997); Makrides, S.C.: Pharmacological Reviews 50(1) 59-85 (1998))

- Reperfusionsschäden nach Ischämien; Ischämische Zustände treten ein während z.B. Operationen unter Zuhilfenahme von Herz-Lungenmaschinen; Operationen, in denen Blutgefäße generell zur Vermeidung großer Blutungen abgeklemmt werden; Myokardinfarkt; thromboembolischer Hirnschlag; Lungenthrombosen etc.;
- Hyperakute Organabstoßung; speziell bei Xenotransplantationen;
- Organversagen wie z.B. multiples Organversagen oder ARDS (adult respiratory distress syndrome);
- Krankheiten, die auf Trauma (Schädeltrauma) oder Polytrauma beruhen, wie z.B. Thermotrauma (Verbrennungen) und "thermal injury";
- Anaphylaktischer Schock;
- Sepsis; "vascular leak syndrom"": bei Sepsis und nach Behandlung mit biologischen Agenzien, wie Interleukin 2 bzw. nach Transplantation;
- Alzheimer Krankheit sowie andere entzündliche neurologische Krankheiten wie Myastenia graevis, multiple Sklerose, zerebraler Lupus, Guillain-Barre Syndrome; Meningitiden; Encaphilitiden;
- Systemischer Lupus erythematosus (SLE);
- Rheumatoide Arthritis und andere entzündliche Krankheiten des rheumatoiden Krankheitskreises, wie z.B. Behcet's Syndrom; Juvenile rheumatoide Arthritis;
- Nierenentzündungen unterschiedlicher Genese, wie z.B. Glomerulonephritis, Lupus nephriti;
- Pankreatitis;
- Asthma; chronische Bronchitis;
- Komplikationen während Dialyse bei Nierenversagen;
- Vasculitis; Thyroiditis;
- Ulcerative Colitis sowie andere entzündliche Erkrankungen des Magen-Darmtraktes;
- Autoimmunerkrankungen.
- Es besteht die Möglichkeit, daß Komplement bei Spontanen Fehlgeburten, beruhend auf immunologischen Abstoßungsreaktionen, beteiligt ist. (Giacomucci E. Bulletti C. Polli V. Prefetto RA. Flamigni C. Immunologically mediated abortion (IMA). Journal of Steroid Biochemistry & Molecular Biology, 49(2-3):107-21, 1994). Hier ist es möglich, daß durch die Inhibition des Komplementsystems eine Modulation der immunologischen Abstoßungsreaktion erreicht wird und damit die Rate der Fehlgeburten entsprechend reduziert wird.

- Komplementaktivierung spielt eine Rolle bei Nebenwirkungen von Arzneimitteln. Als Beispiel seien hier liposomenbasierte Therapien aufgeführt, die z.B. in der Krebstherapie Anwendung finden. Hypersensitive Reaktionen sind bei Patienten beobachtet worden, die mit Arzneimittelformulierungen auf der Basis von Liposomen behandelt wurden (Transfusion 37; 150; 1997). Auch für andere Hilfsmittel, die in der Arzneimittelformulierung eingesetzt werden, wie z.B. Cremophor EL ist eine Aktivierung des Komplementsystems nachgewiesen worden (Szebeni, J. et al. Journal of the National cancer Institute; 90 (4); 1998). Die Komplementaktivierung kann daher für die in manchen Fällen beobachteten anaphylaktoiden Reaktionen verantwortlich sein. Hemmung des Komplementsystems z.B. mit den hier aufgeführten Cls-Inhibitoren sollte daher die Nebenwirkungen von Medikamenten, die auf Aktivierung des Komplementsystems beruhen, lindern und resultierende Hypersensitivitätsreaktionen herabsetzen.

Bei den vorgenannten Krankheiten ist eine Aktivierung des Komplementsystems gezeigt worden.

Die Synthese von Komplementproteinen in speziellen erkrankten Geweben bzw. Organen deuten auf eine Beteiligung des Komplementsystems in der Pathophysiologie dieser Erkrankungen hin. So konnte bei Myokardinfarkt eine starke Neusynthese vieler Komplementproteine im Myokard nachgewiesen werden (Yasojima, K.; Schwab, C.; McGeer, E.G.; McGeer, P.L.; Circulation Research (1998) 83, 860-869). Ebenso konnte dies bei entzündlichen Erkrankungen des Hirns, wie z.B. Multipler Sclerose, bakteriellen Menigitiden und bei Colitis, nachgewiesen werden.

Der Nachweis einer stattgefundenen Komplementaktivierung kann über den Nachweis des Zellolysekomplexes im Gewebe erfolgen und durch den Nachweis von löslichem SC5b-9 oder anderer Aktivierungsprodukte von Komplement, wie z.B. Faktor Bb, C3a; C4a, C5a; C3b, C3d; etc. im Plasma. Durch dementsprechende Nachweise konnte u.a. eine Beteiligung des Komplementsystems an der Atherosklerose ebenso gezeigt werden wie ein Zusammenhang mit Myokardinfarkt, instabiler Angina pectoris; Organtransplantationen, um nur einige Beispiele zu nennen.

Erhöhte Blutspiegel von Komplementproteinen wie C3 bzw. C4 sind mit verschiedenen cardiovaskulären Erkrankungen, wie z.B. Herzversagen, aber auch Diabetes korreliert worden. In ähnlichem Zusammenhang ist eine Erhöhung von TNF bei Herzversagen festgestellt worden. Erste Studien zur Behandlung von Herzversagen mit TNF-Inhibitoren (löslicher TNF-Rezeptor, Antikörpern) wurden positiv beurteilt. TNF wird z.B. nach Stimulation durch Komplementfaktor C5a ausgeschüttet. Es konnte gezeigt werden, daß Inhibition der C5a-Wirkung eine Freisetzung von TNF verhindert (XVII International Complement Workshop, P. Ward, Abstract 324 in Molecular Immunology 35 (411 6-7), 1998). Dementsprechend ist eine Behandlung von Erkrankungen, bei denen erhöhte Spiegel von Komplementproteinen vorliegen, mit den in dieser Schrift beschriebenen Inhibitoren ebenso möglich wie die Behandlung von Erkrankungen, bei denen erhöhte Spiegel von TNF vorliegen.

Ferner ist bei Atherosklerose die Beteiligung von Komplement nachgewiesen worden (Atherosclerosis 132; 131-138 (1997). Besondere Komplikationen durch schnelle atherosklerotische Prozesse finden sich z.B. in Organen nach Transplantationen. Diese Prozesse stellen einen der häufigsten Gründe für das chronische Versagen der transplantierten Organe in der Klinik dar. Zukünftig ist neben Transplantationen humaner Organe (Allotransplantationen) auch an Anwendungen von Transplantaten anderer Spezies (Xenotransplantaten) im Menschen gedacht.

Dementsprechend ist eine Behandlung der oben erwähnten Krankheiten bzw. pathophysiologischen Zustände mit Komplementinhibitoren wünschenswert, insbesondere die Behandlung mit niedermolekularen Inhibitoren.

FUT und FUT-Derivate sind Amidinophenolester bzw. Amidinonaphtholester und beschrieben als Komplementinhibitoren (z.B. Immunology (1983), 49(4), 685-91).

Serin-Proteasen finden sich im Komplement-System in den drei verschiedenen Wegen der Aktivierung: dem klassischen, alternativen und dem MBL-Weg (Arlaud, G.J.; et al. Advances in Immunology 69; 249 ff; 1998). Sie spielen in ihren jeweiligen Wegen eine entscheidende Rolle am Beginn der Kaskade.

Inhibitoren der entsprechenden Serin-Proteasen können hier sowohl vollkommen inhibierend als auch modulierend (partiell inhibierend) eingreifen, wenn Komplement pathophysiologisch aktiviert ist.

Für die Inhibition des Komplementsystems sind einige Proteasen der verschiedenen Aktivierungswege besonders geeignet. Aus der Klasse der Thrombin-ähnlichen Serinproteasen sind dies die Komplement-Proteasen C1r und C1s im klassischen Weg, Faktor D und Faktor B im alternativen Weg sowie MASP I und MASP II im MBL-Weg. Die Inhibition dieser Proteasen führt dann zu einer Wiederherstellung der physiologischen Kontrolle des Komplementsystems in den oben angegebenen Krankheiten bzw. pathophysiologischen Zuständen führen.

Der klassische Weg des Komplementsystems wird üblicherweise über Antikörper, die an ein Antigen gebunden haben, aktiviert. Unter physiologischen Zuständen hilft dieser Weg des Komplementsystems bei der Abwehr von Fremdkörpern, die über Antikörper erkannt werden. Eine Überreaktion führt jedoch zu Schäden im Gewebe, Organismus. Diese Schäden können durch Inhibition des klassischen Weges verhindert werden. Nach dem Stand des Wissens findet eine Aktivierung des Komplementsystems über Antikörper statt bei der hyperakuten Organabstoßung, speziell

bei Xenotransplantationen; bei Reperfusionsschäden nach Ischämien (möglicherweise über IgM-Antikörper und ein Neoepitop; Literatur: Journal of Exp. Med. 183, 2343-8, 1996; Carroll, XVII International Complement Workshop; Rhodos 1998), wie z.B. bei Myokardinfarkt, anderen thrombotischen Erkrankungen oder längerfristigen Verschlüssen von Gefäßen, wie sie z.B. während operativer Eingriffe üblich sind; bei anaphylaktischem Schock; bei Sepsis; bei SLE; bei Erkrankungen des Umfeldes von rheumatoider Arthritis, Nierenentzündungen unterschiedlicher Genese; Vasculitis, allen Autoimmunerkrankungen sowie Allergien. Generell ist bei jeder Erkrankung, in denen zirkulierende Immunkomplexe vorliegen, mit Schäden in verschiedenen Organen durch Aktivierung des Komplementsystems zu rechnen. Es ist Teil der Erfindung, diese Schäden durch die beschriebenen C1-Inhibitoren zu vermindern.

Eine Aktivierung des Komplementsystems über den klassischen Weg findet unter pathophysiologischen Umständen teilweise unter Umgehung von Antikörpern statt. Beispiele hierfür sind Morbus Alzheimer, sowie die unspezifische Aktivierung dieses Weges durch andere Proteasen, wie sie z.B. bei der Lysetherapie nach Myokardinfarkt auftritt. Auch in diesen Fällen kann mit den beschriebenen C1-Inhibitoren eine Begrenzung des Schadens erreicht werden.

Die Aktivierung des klassischen Weges ist z.B. nachgewiesen worden durch den Nachweis der aktivierten Proteine, wie z.B. C1q im betroffenen Gewebe (z.B. Circulation Research 83; 860; 1998). Deutlicher wird die pathophysiologische Beteiligung des Komplementsystems jedoch durch die Verwendung von Inhibitoren, die im Komplementsystem lediglich den klassischen Weg hemmen. Ein physiologischer Inhibitor hierfür ist der C1-Esterase-Inhibitor (Protein ist beschrieben in The Complement System, Rother, Till, Hänsch eds.; Springer; 1998; Seiten 353 ff). Mit Hilfe dieses Inhibitors ist in Versuchen eine Beteiligung des klassischen Weges sowie die Möglichkeit eines therapeutischen Eingriffes gezeigt worden. Einige Literaturstellen sind im Folgenden näher aufgeführt:


1. Bauernschmitt R. Bohrer H. Hagl S. Rescue therapy with C1-esterase inhibitor concentrate after emergency coronary surgery for failed PTCA.
Intensive Care Medicine. 24(6):635-8, 1998.

2. Khorram-Sefat R. Goldmann C. Radke A. Lennartz A. Mottaghy K. Afify M. Kupper W. Klosterhalfen B.
The therapeutic effect of C1-inhibitor on gut-derived bacterial translocation after thermal injury.
Shock. 9(2):101-8, 1998.

3. Niederau C. Brinsa R. Niederau M. Luthen R. Strohmeyer G. Ferrell LD.
Effects of C1-esterase inhibitor in three models of acute pancreatitis.
International Journal of Pancreatology. 17(2):189-96, 1995.

4. Hack CE. Ogilvie AC. Eisele B. Jansen PM Wagstaff J. Thijs LG.
Initial studies on the administration of C1-esterase inhibitor to patients with septic shock or with a vascular leak syndrome induced by interleukin-2 therapy.
Progress in Clinical & Biological Research. 388:335-57, 1994.

5. Dalmasso AP. Platt JL.
Prevention of complement-mediated activation of xenogeneic endothelial cells in an in vitro model of xenograft hyperacute rejection by C1 inhibitor.
Transplantation. 56(5):1171-6, 1993.

6. Nurnberger W. Michelmann I. Petrik K. Holthausen S. Willers R. Lauermann G. Eisele B. Delvos U. Burdach S. Gobel U.
Activity of Cl esterase inhibitor in patients with vascular leak syndrome after bone marrow transplantation.
Annals of Hematology. 67(1):17-21, 1993.

7. Buerke M. Prufer D. Dahm M. Oelert H. Meyer J. Darius H. Blocking of classical complement pathway inhibits endothelial adhesion molecule expression and preserves ischemic myocardium from reperfusion injury.
Journal of Pharmacology & Experimental Therapeutics. 286(1):429-38, 1998.

8. Nissen MH. Bregenholt S. Nording JA. Claesson MH. C1-esterase inhibitor blocks T lymphocyte proliferation and cytotoxic T lymphocyte generation in vitro.
International Immunology. 10(2):167-73, 1998.

9. Nissen MH. Bregenholt S. Nording JA. Claesson MH. C1-esterase inhibitor blocks T lymphocyte proliferation and cytotoxic T lymphocyte generation in vitro.
International Immunology. 10(2):167-73, 1998.

10. Salvatierra A. Velasco F. Rodriguez M. Alvarez A. Lopez-Pedrera R. Ramirez R. Carracedo J. Lopez-Rubio F. Lopez-Pujol A. Guerrero R.
C1-esterase inhibitor prevents early pulmonary dysfunction after lung transplantation in the dog.
American Journal of Respiratory & Critical Care Medicine. 155(3):1147-54, 1997.

11. Horstick G. Heimann A. Gotze O. Hafner G. Berg O. Boehmer P. Becker P. Darius H. Rupprecht HJ. Loos M. Bhakdi S. Meyer J. Kempski O.
Intracoronary application of C1 esterase inhibitor improves cardiac function and reduces myocardial necrosis in an experimental model of ischemia and reperfusion.

Circulation. 95(3):701-8, 1997.
12. Heckl-Ostreicher B. Wosnik A. Kirschfink M.
Protection of porcine endothelial cells from complement-mediated cytotoxicity by the human complement regulators CD59, C1 inhibitor, and soluble complement receptor type 1. Analysis in a pig-to-human in vitro model relevant to hyperacute xenograft rejection.
Transplantation. 62(11):1693-6, 1996.
13. Niederau C. Brinsa R. Niederau M. Luthen R. Strohmeyer G. Ferrell LD.
Effects of C1-esteras inhibitor in three models of acute pancreatitis.
International Journal of Pancreatology. 17(2):189-96, 1995.
14. Buerke M. Murohara T. Lefer AM.
Cardioprotective effects of a C1 esterase inhibitor in myocardial ischemia and reperfusion Circulation. 91(2):393-402, 1995.
15. Hack CE. Ogilvie AC. Eisele B. Jansen PM. Wagstaff J. Thijs LG.
Initial studies on the administration of C1-esterase inhibitor to patients with septic shock or with a vascular leak syndrome induced by interleukin-2 therapy.
Progress in Clinical & Biological Research. 388:335-57, 1994.
16. Dalmasso AP. Platt JL.
Prevention of complement-mediated activation of xenogeneic endothelial cells in an in vitro model of xenograft hyperacute rejection by C1 inhibitor.
Transplantation. 56(5):1171-6, 1993.
17. Guerrero R. Velasco F. Rodriguez M. Lopez A. Rojas R. Alvarez MA. Villalba R. Rubio V. Torres A. del Castillo D.
Endotoxin-induced pulmonary dysfunction is prevented by C1-esterase inhibitor.
Journal of Clinical Investigation. 91(6):2754-60, 1993 Jun.

Wünschenswert sind Inhibitoren, die C1s und/oder C1r hemmen, aber nicht Faktor D inhibieren. Bevorzugt soll nicht gehemmt werden MASP-I, Lyseenzyme Wie z.B. t-PA, Plasmin.

Eine Erbkrankheit erbliches Angioödem, das auf einer Defizienz von C1-Esterase-Inhibitor beruht, wird üblicherweise durch Gabe von C1-Esterase-Inhibitor behandelt. Behandlung mit den hier beschriebenen Cl-Inhibitoren, unter Umständen als zusätzliche Medikation, ist ebenfalls eine Anwendung dieser Erfindung.

Besonders bevorzugt sind Substanze, die $C_{1s}$ und $C_{1r}$ effektiv hemmen.

Pharmakolgische Beispiele

Beispiel A

Farbsubstrattest für die C1r-Inhibition

Reagentien: C1r aus Humanplasma, aktiviert, Zweikettenform (Reinheit: ca. 95 % nach SDS-Gel). Keine Fremdproteasenaktivität nachweisbar.
Substrat: Cbz-Gly-Arg-S-Bzl Produktnr.: WBASO12, (Fa. PolyPeptide, D-38304 Wolfenbüttel, Deutschland)
Farbeagenz: DTNB (5,5'dinitrobis 2-nitrobenzoic acid)
(No. 43760, Fluka, CH-9470 Buchs, Schweiz)
Puffer: 150 mM Tris/HCl pH = 7,50

Farbsubstrattest für die C1r-Inhibition

Testdurchführung: Der Farbsubstrattest zur Bestimmung der C1s-Aktivität wird in 96-Well-Mikrotiterplatten durchgeführt.
10 µl der Inhibitorlösung in 20%igem DMSO (DMSO verdünnt mit 15 mmolar Tris/HCl pH = 7,50) gelangen zu 140 µl Testpuffer, welcher Cls mit einer Endkonzentration von 0,013 U/ml enthält und DTNB mit mit einer Endkonzentration von 0,27 mM/l. Inkubiert wird 10 Minuten bei 20 bis 25°C.
Gestartet wird der Test durch Zugabe von 50 µl einer 1,5 mmolaren Substratlösung in

30%igem DMSO (Endkonzentration 0,375 mmol/l).
Nach 30 Minuten Inkubationszeit bei 20 bis 25°C wird die Extinktion jedes Wells bei 405 nm in einem Zwei-Strahl-Mikrotiterplattenphotometer gegen einen Leerwert (ohne Enzym) gemessen.

Meßkriterium: $IC_{50}$: Benötigte Inhibitorkonzentration, um die amidolytische C1r-Aktivität auf 50 % herabzusetzen.

Statistische Auswertung: Die Abhängigkeit der Extinktion von der Inhibitorkonzentration dient als Berechnungsgrundlage.

Beispiel B

Material und Methoden:Farbsubstrattest für die C1s-Inhibition

Reagentien: C1s aus Humanplasma, aktiviert, Zweikettenform (Reinheit: ca. 95 % nach SDS-Gel). Keine Fremdproteasenaktivität nachweisbar.
Substrat: Cbz-Gly-Arg-S-Bzl Produktnr.: WBASO12, (Fa. PolyPeptide, D-38304 Wolfenbüttel, Deutschland)
Farbeagenz: DTNB (5,5'dinitrobis 2-nitrobenzoic acid)
(No. 43760, Fluka, CH-9470 Buchs, Schweiz)
Puffer: 150 mM Tris/HCl pH = 7,50

Testdurchführung: Der Farbsubstrattest zur Bestimmung der C1s-Aktivität wird in 96-Well-Mikrotiterplatten durchgeführt.
10 µl der Inhibitorlösung in 20%igem DMSO
(DMSO verdünnt mit 15 mmolar Tris/HCl pH = 7,50) gelangen zu 140 µl Testpuffer, welcher C1s mit einer Endkonzentration von 0,013 U/ml enthält und DTNB mit einer Endkonzentration von 0,27 mM/l. Inkubiert wird 10 Minuten bei 20 bis 25°C. Gestartet wird der Test durch Zugabe von 50 µl einer 1,5 mmolaren Substratlösung in 30%igem DMSO (Endkonzentration 0,375 mmol/l).
Nach 30 Minuten Inkubationszeit bei 20 bis 25°C wird die Extinktion jedes Wells bei 405 nm in einem Zwei-Strahl-Mikrotiterplattenphotometer gegen einen Leerwert (ohne Enzym) gemessen.

Meßkriterium: $IC_{50}$: Benötigte Inhibitorkonzentration, um die amidolytische C1s-Aktivität auf 50 % herabzusetzen.

Statistische Auswertung: Die Abhängigkeit der Extinktion von der Inhibitorkonzentration dient als Berechnungsgrundlage.

Beispiel C

Nachweis der Inhibition von Komplement auf dem klassischen Weg durch hämolytischen Test

Für das Messen von potentiellen Komplement-Inhibitoren wird in Anlehnung an diagnostische Tests ein Test zur Messung des klassischen Weges benutzt (Literatur: Complement, A practical Approach; Oxford University Press; 1997; S. 20 ff). Hierzu wird als Quelle für Komplement Humanserum verwendet. Ein gleichartig aufgebauter Test wird jedoch auch mit verschiedenen Seren anderer Spezies in analoger Weise durchgeführt. Als Indikatorsystem werden Erythrozyten von Schafen verwendet. Die antikörperabhängige Lyse dieser Zellen und das dadurch ausgetretene Hämoglobin sind ein Maß für die Komplementaktivität.

Reagenzien, Biochemikalien:

| | | |
|---|---|---|
| Veronal | Fa. Merck | #2760500 |
| Na-Veronal | Fa. Merck | #500538 |
| NaCl | Fa. Merck | #1.06404 |
| $MgCl_2x6H_2O$ | Fa. Baker | #0162 |
| $CaCl_2x6H_2O$ | Fa. Riedel de Haen | #31307 |
| Gelatine | Fa. Merck | #1.04078.0500 |
| EDTA | Fa. Roth | #8043.2 |
| Alsevers Lsg | Fa. Gibco | #15190-044 |
| Penicillin | Fa. Grünenthal | #P1507 10Mega |
| Ambozeptor | Fa. Behring | #ORLC |

Stammlösungen:

| | |
|---|---|
| VBS-Stammlösung: | 2,875 g/l Veronal; 1,875 g/l Na-Veronal; 42,5 g/l NaCl |
| Ca/Mg-Stammlösung: | 0,15 M Ca++, 1 M Mg++ |
| EDTA-Stammlösung: | 0,1 M pH 7,5 |

Puffer:

| | |
|---|---|
| GVBS-Puffer: | VBS-Stammlösung 1:5 mit Fin Aqua verdünnen; 1 g/L Gelatine mit etwas Puffer heiß Auflösen |
| GVBS++ Puffer: | Ca/Mg-Stammlösung 1:1000 in GVBS-puffer verdünnen |
| GVBS/EDTA-Puffer: | EDTA-Stammlösung 1:10 in GVBS-Puffer verdünnen |

Biogene Komponenten:

- Schafserythrozyten(SRBC): Hammelblut wurde 1+1 (v/v) mit Alsevers-Lösung gemischt, durch Glaswolle filtriert und mit 1/10 Volumen EDTA-Stammlösung +1 Spatelspitze Penicillin versetzt. Humanserum: Nach Abzentrifugieren der geronnenen Anteile bei 4°C wurde der Überstand in Aliquots bei -70°C gelagert. Alle Messungen wurden mit einer Charge durchgeführt. Es ergaben sich keine wesentlichen Abweichungen gegenüber Serum anderer Probanden.

Vorgehen:

1. Sensibilisierung der Erythrozyten

   - SRBC wurden dreimal mit GVBS-Puffer gewaschen. Anschließend wurde die Zellzahl auf 5,00E+08 Zellen/ml in GVBS/EDTA-Puffer eingestellt. Ambozeptor wurde in einer Verdünnung von 1:600 zugegeben und durch Inkubation über 30 Min bei 37°C unter Bewegung die SRBC mit Antikörper sensibilisiert. Anschließend wurden die Zellen dreimal mit GVBS-Puffer bei 4°C gewaschen, anschließend in GVBS++ Puffer aufgenommen und auf eine Zellzahl von 5 x $10^8$ eingestellt.

2. Lyseansatz:

- Inhibitoren wurden in verschiedenen Konzentrationen mit Humanserum oder Serum anderer Spezies in passender Verdünnung (z.B. 1:80 für Humanserum; passend ist eine Verdünnung, bei der ca. 80 % der maximalen Lyse, die durch Serum erzielt werden kann, erreicht ist.) in GVBS++ für 10 Min bei 37°C in einem Volumen von 100 µl vorinkubiert. Anschließend wurden 50 µl sensibilisierte SRBC in GVBS++ zugegeben. Nach Inkubation von 1 Stunde bei 37°C unter Bewegung wurden die SRBC abzentrifugiert (5 Minuten; 2500 Upm 4°C). 130 µl des zellfreien Überstandes wurden in eine 96-well-Platte überführt. Die Auswertung erfolgte durch Messung bei 540 nm gegen GVBS++-Puffer.

Zur Auswertung werden die Absorptionswerte bei 540 nm benutzt.

( 1 ): Background; Zellen ohne Serum
( 3 ): 100 % Lyse; Zellen mit Serum
( x ): gemessene Werte mit Testsubstanzen

Berechnung:

$$\% \text{ Lyse} = \frac{(X) - (1) \times 100\ \%}{(3) - (1)}$$

Beispiel D

Test von Inhibitoren auf Inhibition der Protease Faktor D

Faktor D übt im alternativen Weg des Komplementsystems eine zentrale Funktion aus. Aufgrund der geringen Plasmakonzentration von Faktor D stellt der enzymatische Schritt der Spaltung von Faktor B durch Faktor D den geschwindigkeitsbestimmenden Schritt in dem alternativen Weg der Komplementaktivierung dar. Auf Grund der limitierenden Rolle, die dieses Enzym im alternativen Weg spielt, ist Faktor D ein Target für die Inhibition des Komplementsystems.
Das käufliche Substrat Z-Lys-SBzl*HCl wird von dem Enzym Faktor D umgesetzt (Literatur: Kam, C.M. et al., J. Biol. Chem. 262, 3444-3451, 1987). Die Detektion des gespaltenen Substrates erfolgt durch Umsatz mit Ellmann's Reagenz. Das entstandene Produkt wird spektrophotometrisch detektiert. Die Reaktion kann online verfolgt werden. Hierdurch sind enzymkinetische Messungen möglich.

Material:

| Chemikalien: | | |
|---|---|---|
| Faktor D | Calbiochem | 341273 |
| Ellmann's Reagent | SIGMA | D 8130 |
| Z-Lys-SBzl*HCl (=Substrat) | Bachem | M 1300<br>50 mg/ml<br>(MeOH) |
| NaCl | Riedel-De-Häen | 13423 |
| Triton-X-100 | Aldrich | 23,472-9 |
| Tris (hydroxymethyl)-aminomethan | MERCK | |
| Dimethylformamid (DMF) | | |

Puffer:

50 mM Tris
150 mM NaCl

0,01 % Triton - X - 100

pH 7,6

Stocklösungen:

| | |
|---|---|
| Substrat | 20 mM (8,46 mg/ml = 16,92 µl (50 mg/ml) + 83,1 µl $H_2O$) |
| Ellmann's Reagent | 10 mM (3,963 mg/ml) in DMF |
| Faktor D | 0,1 mg/ml |
| Proben (Inhibitoren) | $10^{-2}$ M in DMSO |

Durchführung:

Ansätze:

| | |
|---|---|
| Leerwert: | 140 µl Puffer + 4,5 µl Substrat (0,6 mM) + 4,5 µl Ellmann's (0,3 mM) |
| Positiv-Kontrolle: | 140 µl Puffer + 4,5 µl Substrat (0,6 mM) + 4,5 µl Ellmann's (0,3 mM) + 5 µl Faktor D |
| Proben-Messung: | 140 µl Puffer + 4,5 µl Substrat (0,6 mM) + 4,5 µl Ellmann's (0,3 mM) + 1,5 µl Proben ($10^{-4}$ M) + 5 µl Faktor D |

Die Ansätze werden in Mikrotiterplatten zusammenpipettiert.
Nach dem Mischen von Puffer, Substrat und Ellmann's (evtl. Inhibitor) wird die Enzymreaktion durch Zugabe von jeweils 5 µl Faktor D gestartet. Inkubation findet bei Raumtemperatur für 60 min. statt.

Messung:

Messen bei 405 nm für 1 Stunde in 3 Minuten Abstand

Auswertung:

Das Ergebnis wird graphisch aufgetragen. Die Änderung der Absorption pro Minute (Delta OD pro Minute; Steigung) ist für den vergleich von Inhibitoren relevant, da sich hieraus $K_i$-Werte von Inhibitoren ermitteln lassen.
Als wirksamer Inhibitor wurde in diesem Test der Serinprotease-Inhibitor FUT-175; Futhan; Fa. Torii; Japan mitgeführt.

Beispiel E

Nachweis der Inhibition von Komplement auf dem alternativen Weg durch hämolytischen Test (Literatur: Complement, A practical Approach; Oxford University Presss; 1997, S. 20 ff.)
Der Test wird in Anlehnung an klinische Tests durchgeführt. Durch zusätzliche Aktivierung mittels z.B. Zymosan oder Cobra Venom Faktor kann der Test modifiziert werden.

Material:

EGTA (Ethylenebis(oxyethylenenitrilo)-tetracetic acid

| | | |
|---|---|---|
| | Boehringer Mannheim | 1093053 |
| $MgCl_2$ * 6 $H_2O$ | MERCK | 5833.0250 |

(fortgesetzt)

| | | |
|---|---|---|
| | Boehringer Mannheim | 1093053 |
| NaCl | MERCK | 1.06404.1000 |
| D - Glucose | Cerestar | |
| Veronal | MERCK | 2760500 |
| Na-Veronal | MERCK | 500538 |
| VBS - Stammlösung ( 5x ) | Gelatine Veronal Puffer PD Dr. Kirschfink; Universität Heidelberg, Inst. F. Immunologie; | |
| Gelatine | MERCK | 1.04078.0500 |
| Tris (hydroxymethyl) aminomethan | MERCK | 1.08382.0100 |
| $CaCl_2$ | MERCK | Art. 2382 |

Humanserum wurde entweder bei verschiedenen Lieferanten (z.B. Sigma) gekauft oder in der Ambulanz der BASF Süd von Probanden gewonnen.
Meerschweinchenblut wurde gewonnen und 2:8 in Citratlösung verdünnt. Es wurden mehrere Chargen ohne offensichtliche Unterschiede verwendet.

Stammlösungen:

| | |
|---|---|
| VBS-Stammlösung: | 2,875 g/l Veronal<br>1,875 g/l Na-Veronal<br>42,5 g/l NaCl |
| GVBS: | VBS Stammlösung 1:5 mit Wasser (Finn Aqua) verdünnen<br>+ 0,1 % Gelatine<br>erhitzen bis Gelatine gelöst und abkühlen |
| 100 mM EGTA: | 38,04 mg EGTA in 500 ml Finn Aqua und mit 10 M NaOH langsam auf pH 7,5 bis gelöst, dann auf 1 l auffüllen |
| Mg - EGTA : | 5 ml 100 mM EGTA<br>3,5 ml 100 mM $MgCl_2$<br>10,4 ml GVBS<br>31,1 ml 5 % Glucoselösung |
| Saline: | 0,9 % NaCl in Wasser (Finn Aqua) |
| GTB: | 0,15 mM $CaCl_2$<br>141 mM NaCl<br>0,5 mM $MgCl_2$* 6 $H_2O$<br>10 mM Tris<br>0,1 % Gelatine<br>pH 7,2 - 7,3 |

Vorgehen:

1. Zellpräparation:
Die Erythrozyten aus dem Meerschweinchenblut wurden mehrfach durch Zentrifugieren (5 Minuten; 1000 Upm) mit GTB gewaschen, bis der Überstand klar war. Die Zellzahl wurde auf 2 * $10^9$ Zellen/ml eingestellt.

2. Durchführung: Die einzelnen Ansätze wurden 30 Minuten bei 37°C unter Bewegung inkubiert. Anschließend wurde mit je 480 µl eiskalter Saline (physikalischer Kochsalzlösung) abgestoppt und die Zellen 5 Minuten mit 5000 Upm abzentrifugiert. 200 µl des Überstandes wurden bei 405 nm gemessen durch Überführen in eine Mikrotiterplatte und Auswertung in einem Mikrotiterplattenphotometer.

| Pipettierschema (Mengenangaben in µl) | | | | | |
|---|---|---|---|---|---|
| | Background (-Serum) | 100 % Lyse | 100 % Lyse + Faktor D | Background + Faktor D (-Serum) | Max. Lyse (Wasser) |
| Zellen | 20 | 20 | 20 | 20 | 20 |
| Serum 1:4 | | 20 | 20 | | |
| Mg - EGTA | 480 | 480 | 480 | 480 | |
| Faktor D | | | 0,5 µg | 0,5 µg | |
| Saline (zum Abstoppen) | 480 | 480 | 480 | 480 | |
| $H_2O$ | | | | | 980 |

Auswertung:

Zur Auswertung werden die OD - Werte benutzt.

( 1 ) : Background; Zellen ohne Serum
( 3 ) : 100 % Lyse + Faktor D; Zellen mit Serum
( x ) : gemessene Werte mit Testsubstanzen

Berechnung:

$$\% \text{ Lyse} = \frac{(X) - (1) * 100}{(3) - (1)}$$

Die vorliegende Erfindung betrifft peptidische und peptidomimetische Substanzen, deren Herstellung und deren Verwendung als Komplementinhibitoren. Insbesondere handelt es sich um Substanzen mit einem Amidin- oder Guanidinrest als endständige Gruppe.

Außerdem betrifft die Erfindung die Verwendung von bekannten amidinhaltigen Substanzen zur Herstellung von Komplentinhibitoren, spezifisch von Inhibitoren von C1s und C1r.

Die Erfindung betrifft die Verwendung von bekannten und neuen Substanzen mit einer Amidin- oder Guanidin-Endgruppe zur Herstellung von Komplementinhibitoren, spezifisch von Inhibitoren von C1s und C1r.

Insbesondere betrifft die Erfindung die Verwendung von chemisch stabilen Substanzen der allgemeinen Formel I, deren Tautomeren, pharmakologisch verträglichen Salzen und Prodrugs zur Herstellung von Arzneimitteln zur Behandlung und Prophylaxe von Krankheiten, die durch teilweise oder vollständige Inhibition, insbesondere selektive Inhibition, von C1s und/oder C1r gelindert oder geheilt werden. Formel I hat die allgemeine Struktur

$$A—B—D—E—G—NH—CH_2—Q^K—L \qquad (I)$$

A steht für
H, $C_{1-6}$-Alkyl, $C_{1-6}$-Alkyl-$SO_2$, $R^{A1}$OCO ($R^{A1}$ gleich H, $C_{1-12}$-Alkyl, $C_{3-8}$-Cycloalkyl, $C_{3-8}$-Cycloalkyl-$C_{1-3}$-Alkyl, $C_{1-3}$-Alkylaryl), $R^{A2}R^{A3}$NCO ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}$OCONR$^{A2}$ ($R^{A4}$ gleich $C_{1-6}$-Alkyl, $C_{1-3}$-Alkylaryl), $R^{A4}$CONR$^{A2}$, $R^{A1}$O, $R^{A2}R^{A3}$N, HO-$SO_2$-, Phenoxy, $R^{A2}R^{A3}$N-$SO_2$, Cl, Br, F, Tetrazolyl, $H_2O_3P$-, $NO_2$, $R^{A1}$N(OH)-CO-, $R^{A1}R^{A2}$NCONR$^{A3}$, wobei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B}-L^B-(CH_2)_{m^B}-$$

mit

$l^B$ = 0, 1, 2;
$m^B$ = 0, 1, 2;
$L^B$ gleich

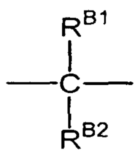 , 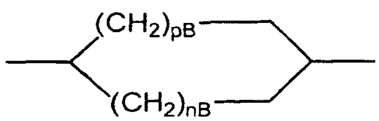 , 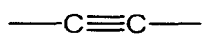 , 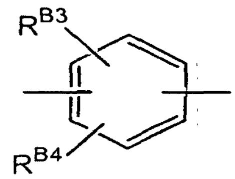

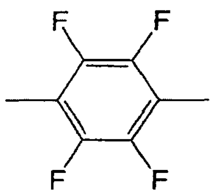 , 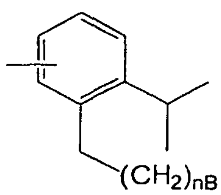

wobei in den vorgenannten Ringsystem jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

$n^B$ = 0, 1;
$p^B$ = 0, 1;
$R^{B1}$ gleich $C_{0-3}$-Alkylaryl, $C_{0-3}$-Alkylheteroaryl, $C_{0-3}$-Alkyl-$C_{3-8}$-Cycloalkyl;
$R^{B2}$ gleich H, $C_{1-6}$-Alkyl, $C_{0-3}$-Alkylaryl, $C_{0-3}$-Alkylheteroaryl;
$R^{B3}$ gleich H, $C_{1-6}$-Alkyl, $R^{B5}OCO$ ($R^{B5}$ gleich H, $C_{1-6}$-Alkyl), $R^{B6}$-O ($R^{B6}$ gleich H, $C_{1-6}$-Alkyl), F, Cl, Br, $NO_2$, $CF_3$;
$R^{B4}$ gleich H, $C_{1-6}$-Alkyl, $R^{B6}$-O, Cl, Br, F, $CF_3$;
$R^{B1}$ und $R^{B2}$ können auch miteinander verbunden sein;
B steht weiterhin für

$$-(CH_2)_{l^B}-L^B-M^B-L^B-(CH_2)_{m^B}-,$$

wobei
$l^B$ und $m^B$ oben angegebene Bedeutung besitzen und die beiden Gruppen $L^B$ unabhängig voneinander für die unter $L^B$ genannten Reste stehen;
$M^B$ bedeutet Einfach-Bindung, O, S, $CH_2$, $CH_2$-$CH_2$, $CH_2$-O, O-$CH_2$, $CH_2$-S, S-$CH_2$, CH=CH, C≡C;
B steht weiterhin für
-adamantyl(1)-$CH_2$-, -adamantyl (2)-$CH_2$-,

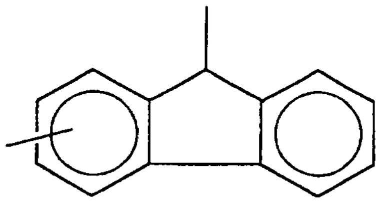, 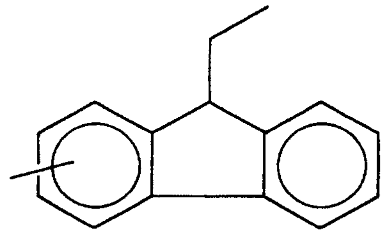,

B kann weiterhin stehen für

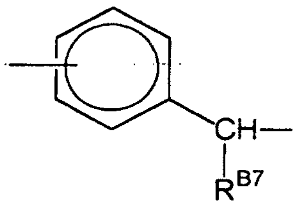, 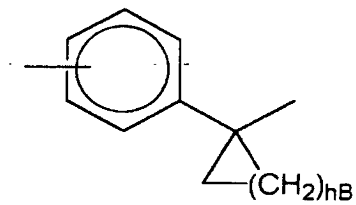

$h^B$ gleich 1, 2, 3, 4;
$R^{B7}$ gleich $C_{1-6}$-Alkyl, $C_{3-8}$-Cycloalkyl;
B kann weiterhin stehen für

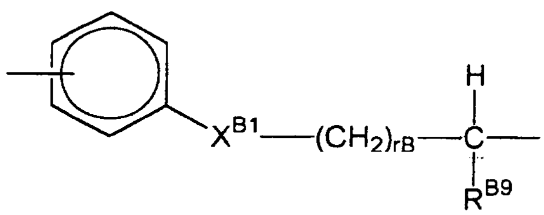

mit $X^{B1}$ gleich einer Bindung, O, S, oder

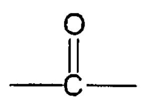 ;

mit $r^B$ gleich 0, 1, 2, 3;
mit $R^{B9}$ gleich H, $C_{1-3}$-Alkyl;
A-B kann stehen für

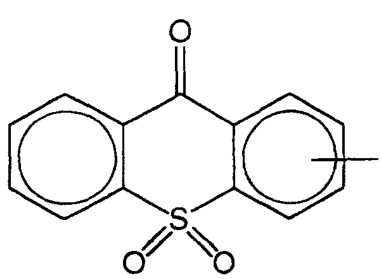, 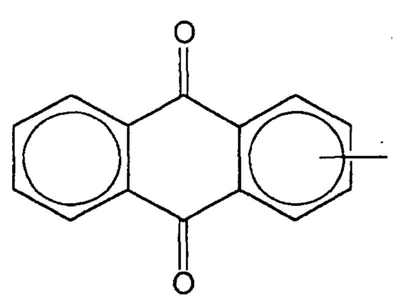

D steht für eine Einfach-Bindung bzw. für CO, OCO, $NR^{D1}$-CO ($R^{D1}$ gleich H, $C_{1-4}$-Alkyl, $C_{0-3}$-Alkylaryl), $SO_2$, $NR^{D1}SO_2$;

E steht für

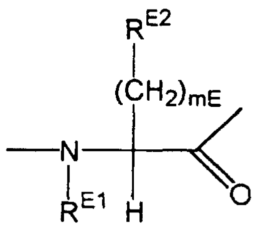

mit

$m^E$ = 0, 1, 2, 3;

$R^{E1}$ bedeutet H, $C_{1-6}$-Alkyl;

$R^{E2}$ bedeutet H, $C_{1-6}$-Alkyl, $C_{3-8}$-Cycloalkyl, wobei die vorgenannten Reste bis zu drei Substituenten der Gruppe $C_{1-6}$-Alkyl, F tragen können, $CH(CH_3)OH$, $CH(CF_3)_2$;

die unter $R^{E1}$ und $R^{E2}$ genannten Gruppen können über eine Bindung miteinander verknüpft sein

E kann auch stehen für D-Asp, D-Glu, D-Lys, D-Orn, D-His, D-Dab, D-Dap, D-Arg;

G bedeutet

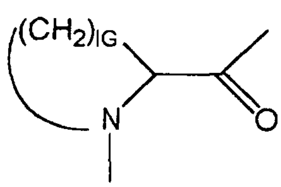
,

mit $l^G$ = 2, 3, wobei eine $CH_2$-Gruppe des Rings durch S, $CHCH_3$ ersetzt sein kann;

G bedeutet weiterhin

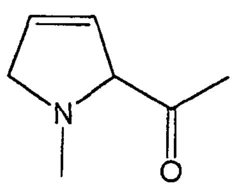
,

$Q^K$ gleich

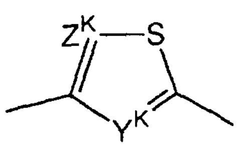
,
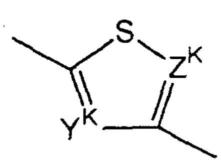
,

$Y^K$ gleich =CH-, =N-;

$Z^K$ gleich =CH-, =N-;

L: 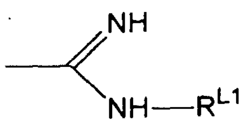 bzw. 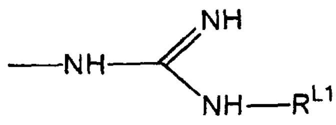

mit

$R^{L1}$ gleich H, OH, CO-$C_{1-6}$-Alkyl, $CO_2$-$C_{1-6}$-Alkyl, $CO_2$-$C_{1-3}$-Alkylaryl.

Der Begriff $C_{1-x}$-Alkyl umfaßt alle geradkettigen und verzweigten Alkylketten mit einem bis $_x$-Kohlenstoffatomen.

Der Begriff $C_{3-8}$-Cycloalkyl steht für carbocyclische gesättigte Reste mit 3- bis 8 Kohlenstoffatomen.

Der Begriff Aryl steht für carbocyclische Aromaten mit 6 bis 14 C-Atomen, insbesondere für Phenyl, 1-Naphthyl, 2-Naphthyl.

Der Begriff Heteroaryl steht für Fünf- und Sechsring-Aromaten mit mindestens einem Heteroatom N, O oder S, insbesondere für Pyridyl, Thienyl, Furyl, Thiazolyl, Imidazolyl; dabei können auch zwei aromatische Ringe kondensiert sein, wie z.B. Indol, N-$C_{1-3}$-Alkylindol, Benzothiophen, Benzothiazol, Benzimidazol, Chinolin, Isochinolin.

Der Begriff $C_{x-y}$-Alkylaryl steht für carbocyclische Aromaten, die über eine Alkylgruppe mit X, x+1, ...y-1 oder y C-Atomen mit dem Gerüst verknüpft sind.

Gegenstand der Erfindung sind weiter die Zwischenprodukte der Formel I, wobei L -CN, -C(=O)$NH_2$ oder -C(=S)$NH_2$ entspricht.

Die neuen Zwischenprodukte dienen zur Herstellung der Verbindungen I und sind wertvolle Bausteine für die Synthese von Serinprotease-Inhibitoren.

Die Verbindungen der Formel I können als solche oder in Form ihrer Salze mit physiologisch verträglichen Säuren vorliegen. Beispiele für solche Säuren sind: Salzsäure, Zitronensäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin.

Die neuen Verbindungen der Formel I sind kompetitive Inhibitoren des Komplementsystems, besonders von $C_{1s}$, sowie weiter von $C_{1r}$.

Die erfindungsgemäßen Verbindungen können in üblicher Weise oral oder parenteral (subkutan, intravenös, intramuskulär, intraperitoneal, rektal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 10 und 2000 mg bei oraler Gabe und zwischen etwa 1 und 200 mg bei parenteraler Gabe. Diese Dosis kann in 2 bis 4 Einzeldosen oder einmalig am Tag als Depotform gegeben werden.

Die Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

Unter Prodrugs werden Verbindungen verstanden, die in vivo (z.B. first pass Metabdisums) in die pharmakologisch aktiven Verbindungen der allgemeinen Formel I umgesetzt werden.

Die Erfindung betrifft außerdem folgende ganz besonders bevorzugten neue Verbindungen, deren Tautomere, physiologisch verträglichen Salze und Prodrugs der Formel A-B-D-E-G-NH-$CH_2$-$Q^K$-L und Arzneimittel, die diese Verbindungen enthalten. Des weiteren eignen sich diese Verbindungen als besonders gute Komplementinhibitoren.

Hierbei gilt:

A steht für
H, $C_{1-6}$-Alkyl, $C_{1-6}$-Alkyl-$SO_2$, $R^{A1}$OCO ($R^{A1}$ gleich H, $C_{1-12}$-Alkyl, $C_{3-8}$-Cycloalkyl, $C_{3-8}$-Cycloalkyl-$C_{1-3}$-Alkyl, $C_{1-3}$-Alkylaryl), $R^{A2}R^{A3}$NCO ($R^{A2}$ gleich H-, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}$OCON$R^2$ ($R^{A4}$ gleich $C_{1-6}$-Alkyl, $C_{1-3}$-Alkylaryl), $R^{A4}$CON$R^{A2}$, $R^{A1}$O, $R^{A2}R^{A3}$N, HO-$SO_2$-, Phenoxy, $R^{A2}R^{A3}$N-$SO_2$, Cl, Br, F, Tetrazolyl, $H_2O_3$P-, $NO_2$, $R^{A1}$-N(OH)-CO-, $R^{A1}R^{A2}$NCON$R^{A3}$, wo-

bei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B} - L^B - (CH_2)_{m^B} -$$

mit

$l^B$ = 0, 1;

$m^B$ = 0, 1, 2;

$L^B$ gleich

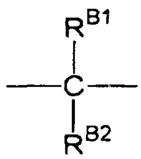
,
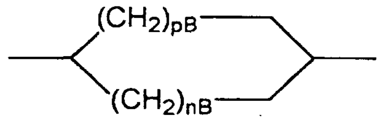
,
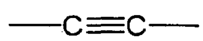
,
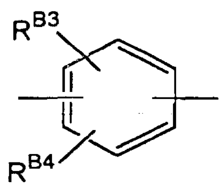

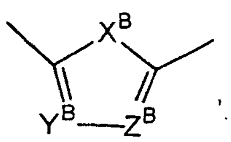
,
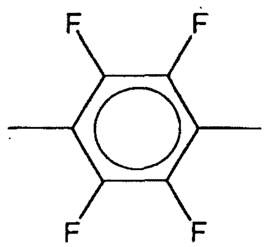
,
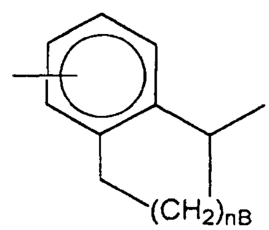

wobei in den vorgenannten Ringsystem jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

$n^B$ = 0, 1;
$p^B$ = 0, 1;
$R^{B1}$ gleich $C_{0-3}$-Alkylaryl, $C_{0-3}$-Alkylheteroaryl, $C_{0-3}$-Alkyl-$C_{3-8}$-Cycloalkyl;
$R^{B2}$ gleich H, $C_{1-6}$-Alkyl, $C_{0-3}$-Alkylaryl, $C_{0-3}$-Alkylheteroaryl;
$R^{B3}$ gleich H, $C_{1-6}$-Alkyl, $R^{B5}OCO$ ($R^{B5}$ gleich H, $C_{1-6}$-Alkyl), $R^{B6}$-O ($R^{B6}$ gleich H, $C_{1-6}$-Alkyl), F, Cl, Br, $NO_2$, $CF_3$;
$R^{B4}$ gleich H, $C_{1-6}$-Alkyl, $R^{B6}$-O, Cl, Br, F, $CF_3$;
$R^{B1}$ und $R^{B2}$ können auch miteinander verbunden sein;
$X^B$ gleich O, S;
$Y^B$ gleich =CH-, =N-;
$Z^B$ gleich =CH-, =N-;
B steht weiterhin für

$$- (CH_2)_{l^B} - L^B - M^B - L^B - (CH_2)_{m^B} -$$

wobei
$l^B$ und $m^B$ oben angegebene Bedeutung besitzen und die beiden Gruppen $L^B$ unabhängig voneinander für die unter

$L^B$ genannten Reste -C≡C-,

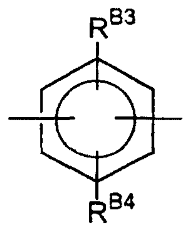
,
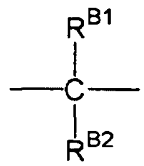

stehen

$M^B$ bedeutet Einfach-Bindung, O, $CH_2$-S, S-$CH_2$, CO, $SO_2$, $CH_2$-O;

B kann weiterhin stehen für

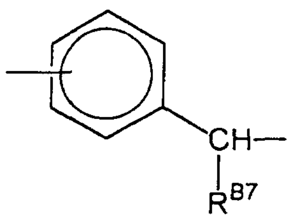
,
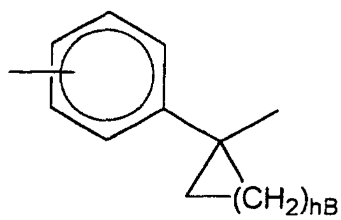

$h^B$ gleich 1, 2, 3, 4;

$R^{B7}$ gleich $C_{1-6}$-Alkyl, $C_{3-8}$-Cycloalkyl

B kann weiterhin stehen für Fluorenyl(1)-, Adamantyl(1)-, Adamantyl(1)-$CH_2$-

A-B kann stehen für Pyridyl(2)-$CH_2$-, Benzthienyl(2)-, Benzthienyl(3)-,

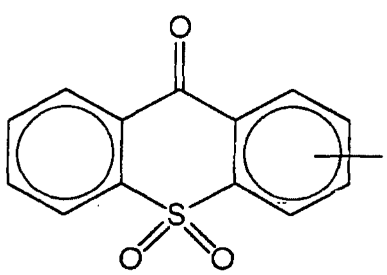

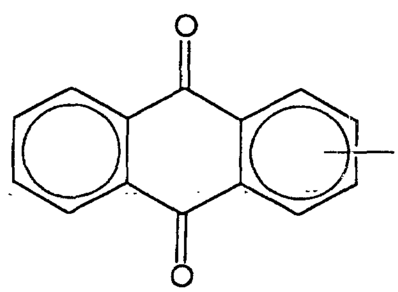

D steht für eine Einfach-Bindung bzw. für CO, $SO_2$;

E steht für

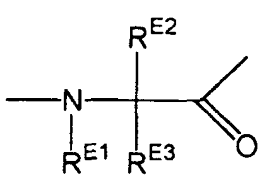

mit

$R^{E1}$ bedeutet H, $CH_3$;
$R^{E2}$ bedeutet H, $C_{1-6}$-Alkyl, $C_{3-8}$-Cycloalkyl, Thienyl, $CH(CH_3)OH$, $CH(CF_3)_2$;
$R^{E3}$ bedeutet H;

die unter $R^{E1}$ und $R^{E2}$ genannten Gruppen können über eine Bindung miteinander verknüpft sein; auch die unter $R^{E2}$ und $R^{E3}$ genannten Gruppen können über eine Bindung miteinander verbunden sein;

E kann auch stehen für D-Lys, D-Orn, D-Dab, D-Dap, D-Arg;
G bedeutet

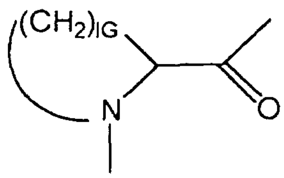,

mit $l^G$ = 2, 3, wobei eine $CH_2$-Gruppe des Rings durch $CHCH_3$ ersetzt sein kann;

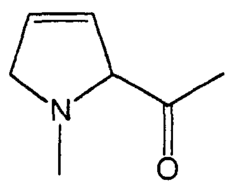.

$Q^K$ gleich

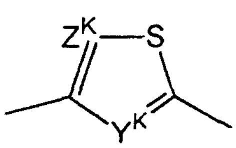, 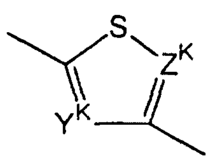, 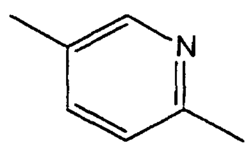

$Y^K$ gleich =CH-, =N-;

$Z^K$ gleich =CH-, =N-;

L:

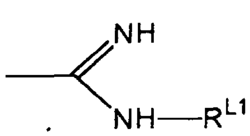 bzw. 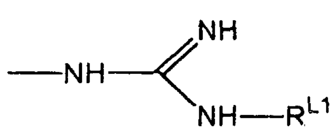

$R^{L1}$ gleich H, OH.

Die Erfindung betrifft außerdem folgende bevorzugten neue Verbindungen, deren Tautomere, physiologische verträglichen Salze und Prodrugs der Formel A-B-D-E-G-NH-$CH_2$-$Q^K$-L und Arzneimittel, die diese Verbindungen enthalten. Des weiteren eignen sich diese Verbindungen als besonders gute Komplementinhibitoren.
Hierbei gilt:

A steht für

H, $C_{1-6}$-Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl), $R^{A2}R^{A3}NCO$ ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^{A2}$, $R^{A4}CONR^{A2}$ ($R^{A4}$ gleich $C_{1-6}$-Alkyl, $C_{1-3}$-Alkylaryl), $R^{A1}O$, Phenoxy, $R^{A2}R^{A3}N$, HO-$SO_2$, $R^{A2}R^{A3}N$-$SO_2$, Cl, Br, F, Tetrazolyl, $H_2O_3P$, $NO_2$, $R^{A1}$-N(OH)-CO, $R^{A1}R^{A2}NCONR^{A3}$, wobei Aryl jeweils mit bis zu 2 gleichen oder verschiedenen Resten aus der Gruppe F, Cl, Br, $OCH_3$, $CH_3$. $CF_3$, $NO_2$ substituiert sein kann;

B steht für

$$-(CH_2)_{l^B}-L^B-(CH_2)_{m^B}-$$

mit

$l^B$ = 0, 1, 2;

$m^B$ = 0, 1, 2;

$L^B$ gleich

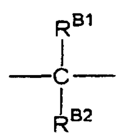 , 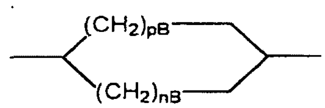 , 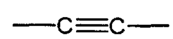 , 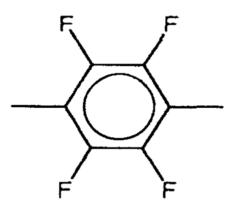 , 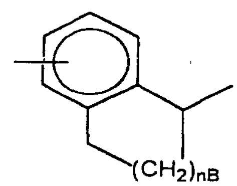

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$ ($R^8$ gleich H, $C_{1-3}$-Alkyl) substituiert sein kann;
mit

$n^B$ = 0, 1, 2;
$p^B$ = 0, 1, 2;
$R^{B1}$ gleich $C_{0-3}$-Alkylaryl, $C_{0-3}$-Alkylheteroaryl, $C_{0-3}$-Alkyl-$C_{3-8}$-Cycloalkyl;
$R^{B2}$ gleich H, $C_{1-6}$-Alkyl, $C_{0-3}$-Alkylaryl, $C_{0-3}$-Alkylheteroaryl,
$R^{B1}$ und $R^{B2}$ können auch miteinander verbunden sein;
B steht weiterhin für -adamantyl(1)-$CH_2$-, -adamantyl(2)-$CH_2$-,

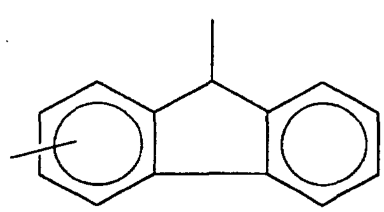 , 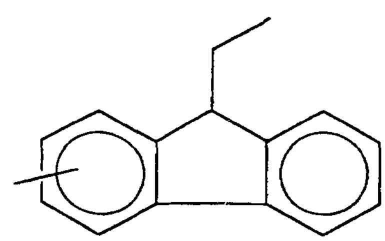 ,

B steht weiterhin

$$-(CH_2)_{l^B} - L^{B1} - M^B - L^{B2} - (CH_2)_{m^B} -$$

wobei
$l^B$ und $m^B$ oben angegebene Bedeutung besitzen und die beiden Gruppen $L^{B1}$ und $L^{B2}$ unabhängig voneinander für folgende Reste stehen:

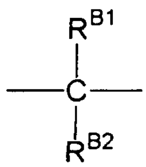 , 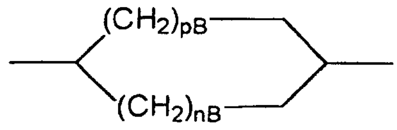 , 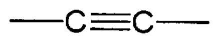 , 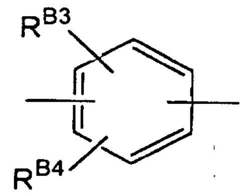

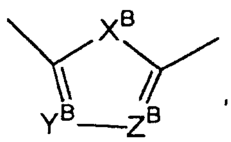 , 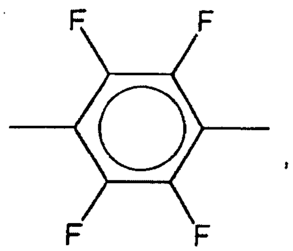 , 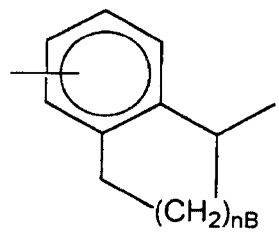

wobei in den vorgenannten Ringsystemen jeweils ein Phenylring ankondensiert sein kann;
mit

| | |
|---|---|
| $n^B$ | = 0, 1, 2; |
| $p^B$ | = 0, 1, 2; |
| $R^{B1}$ | gleich H (nur für $L^{B2}$), $C_{1-6}$-Alkyl (nur für $L^{B2}$), $C_{0-3}$-Alkylaryl, $C_{0-3}$-Alkylheteroaryl, $C_{0-3}$-Alkyl-$C_{3-8}$-Cycloalkyl; |
| $R^{B2}$ | gleich H, $C_{1-6}$-Alkyl, $C_{0-3}$-Alkylaryl, $C_{0-3}$-Alkylheteroaryl; |
| $R^{B3}$ | gleich H, $C_{1-6}$-Alkyl, Aryl, Heteroaryl, $R^{B5}$OCO ($R^{B5}$ gleich H, $C_{1-6}$-Alkyl), $R^{B6}$-O ($R^{B6}$ gleich H, $C_{1-6}$-Alkyl), F, Cl, Br, $NO_2$, $CF_3$; |
| $R^{B4}$ | gleich H, $C_{1-6}$-Alkyl, $R^{B6}$-O, Cl, Br, F, $CF_3$; |
| $X^B$ | gleich O, S; |
| $Y^B$ | gleich =CH-, =N-; |
| $Z^B$ | gleich =CH-, =N-; |
| $R^{B1}$ und $R^{B2}$ | können auch miteinander verbunden sein; |
| $M^B$ | bedeutet Einfach-Bindung, O, S, $CH_2$, $CH_2$-$CH_2$, $CH_2$-O, O-$CH_2$, $CH_2$-S, S-$CH_2$, CO, $SO_2$, CH=CH, C≡C; |
| B | kann weiterhin stehen für |

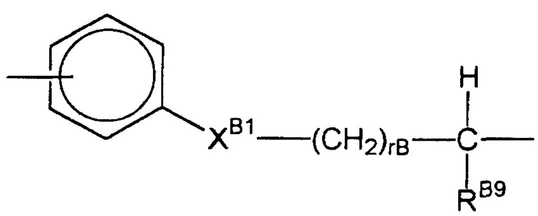

mit $X^{B1}$ gleich eine Bindung O, S oder ;

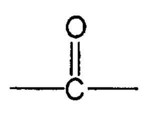

mit $r^B$ gleich 0, 1, 2, 3;
mit $R^{B9}$ gleich H, $C_{1-3}$-Alkyl;
A-B kann stehen für

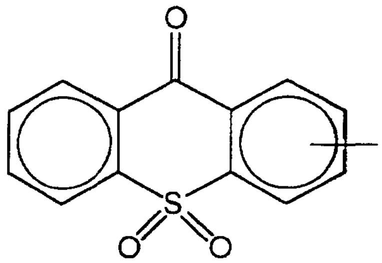
,
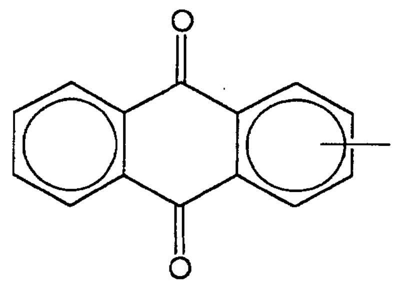

D steht für eine Einfach-Bindung bzw. für CO, OCO, $NR^{D1}$-CO ($R^{D1}$ gleich H, $C_{1-4}$-Alkyl, $C_{0-3}$-Alkyl-aryl), $SO_2$, $NR^{D1}SO_2$;

B-D kann stehen für

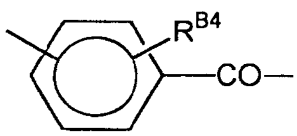

E steht für

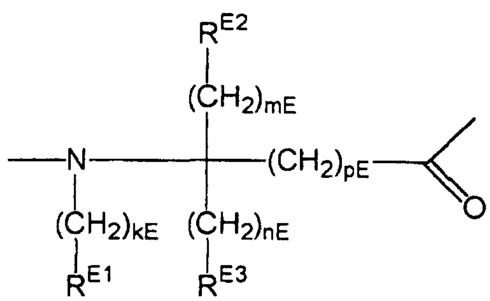

$k^E$ =0, 1;

$m^E$ =0, 1;

$n^E$ = 0, 1;

$p^E$ = 0, 1;

$R^{E1}$ bedeutet H, $C_{1-6}$-Alkyl, $C_{3-8}$-Cycloalkyl, Phenyl, Naphthyl, Pyridyl, Thienyl, $C_{3-8}$-Cycloalkyl mit ankondensiertem Phenylring;

$R^{E2}$ bedeutet H, $C_{1-6}$-Alkyl, $C_{3-8}$-Cycloalkyl, Phenyl, Pyridyl, Thienyl, Furyl, Imidazolyl, Tetrahydropyranyl, Tetrahydrothiopyranyl, $CH(CH_3)OH$, $CH(CF_3)_2$;

$R^{E3}$ bedeutet H, $C_{1-6}$-Alkyl, $C_{3-8}$-Cycloalkyl, Phenyl;

die unter $R^{E1}$ und $R^{E2}$ genannten Gruppen können über eine Bindung miteinander verknüpft sein; auch die unter $R^{E2}$ und $R^{E3}$ genannten Gruppen können über eine Bindung miteinander verbunden sein;

E kann auch stehen für D-Asp, D-Glu, D-Lys, D-Orn, D-His, D-Dab, D-Dap, D-Arg;

G bedeutet

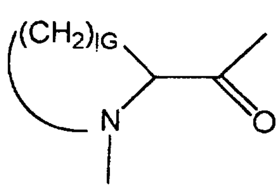
,

Mit $l^G$ = 2, 3, 4, wobei eine $CH_2$-Gruppe des Rings durch $CHCH_3$ ersetzt sein kann

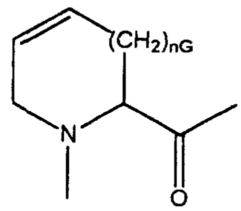

mit
$n^G$ = 0, 1;
weiterhin steht G für

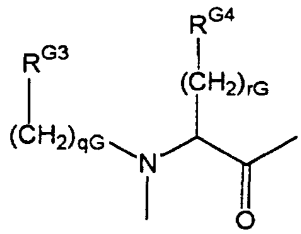

| | |
|---|---|
| qG | 0, 1; |
| $r^G$ | 0, 1; |
| $R^{G3}$ | H, $C_{1-6}$-Alkyl, $C_{3-8}$-Cycloalkyl; |
| $R^{G4}$ | H, $C_{1-6}$-Alkyl, $C_{3-8}$-Cycloalkyl, Phenyl; |
| $Q^K$ | gleich |

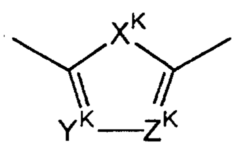

| | |
|---|---|
| $X^K$ | gleich O, S; |
| $Y^K$ | gleich =CH-, =N-; |
| $Z^K$ | gleich =CH-, =N-; |
| L: | |

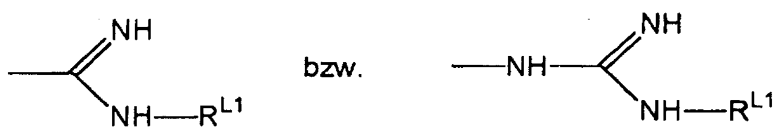

mir

$R^{L1}$ gleich H, OH, CO-$C_{1-6}$-Alkyl, $CO_2$-$C_{1-6}$-Alkyl, $CO_2$-$C_{1-5}$-Alkylaryl.

Die Erfindung betrifft außerdem folgende besonders bevorzugten neue Verbindungen, deren Tautomere, physiologisch verträglichen Salze und Prodrugs der Formel A-B-D-E-G-NH-$CH_2$-$Q^K$-L und Arzneimittel, die diese Verbindungen enthalten. Des weiteren eignen sich diese Verbindungen als besonders gute Komplementinhibitoren.

Hierbei gilt:

A steht für
H, $C_{1-6}$-Alkyl, $C_{1-6}$-Alkyl-$SO_2$, $R^{A1}$OCO ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl), $R^{A2}R^{A3}$NCO ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-

Alkyl, $C_{0-3}$-Alkylaryl), $R^{A4}OCONR^{A2}$, $R^{A4}CONR^{A2}$ ($R^{A4}$ gleich $C_{1-6}$-Alkyl, $C_{1-3}$-Alkylaryl), $R^{A1}O$, Phenoxy, $R^{A2}R^{A3}N$, HO-$SO_2$, $R^{A2}R^{A3}N$-$SO_2$, Cl, Br, F, Tetrazolyl, $H_2O_3P$, $NO_2$, $R^{A1}$-N(OH)-CO, $R^{A1}R^{A2}NCONR^{A3}$, wobei Aryl jeweils mit bis zu 2 gleichen oder verschiedenen Resten aus der Gruppe F, Cl, Br, $OCH_3$, $CH_3$, $CF_3$, $NO_2$ substituiert sein kann;

B steht für

$$-(CH_2)_{l^B}-L^B-(CH_2)_{m^B}-$$

mit

$l^B$ = 0, 1;

$m^B$ = 0, 1, 2;

$L^B$ gleich

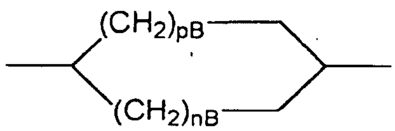
,
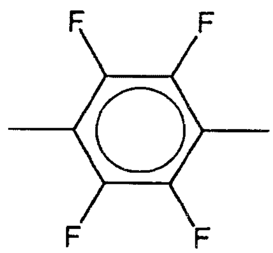
,
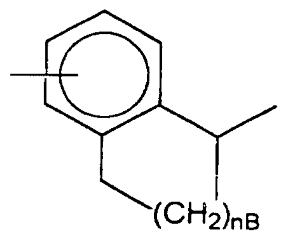

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

$n^B$ = 0, 1;

$p^B$ = 0, 1;

B steht weiterhin für -adamantyl(1)-$CH_2$-, -adamantyl(2)-$CH_2$-,

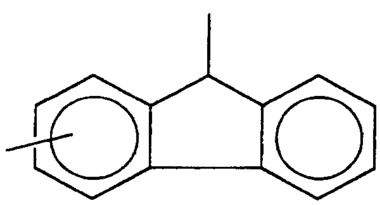

B steht weiterhin für

$$-(CH_2)_{l^B}-L^{B1}-M^B-L^{B2}-(CH_2)_{m^B}-$$

wobei

$l^B$ und $m^B$ die oben angegebene Bedeutung besitzen und die beiden Gruppen $L^{B1}$ und $L^{B2}$ unabhängig voneinander für folgende Reste stehen:

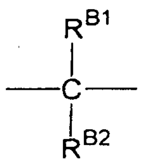 , 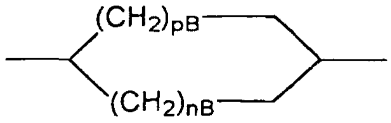 , 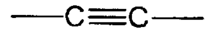 , 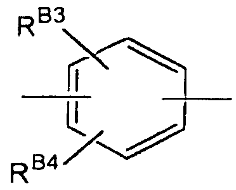 ,

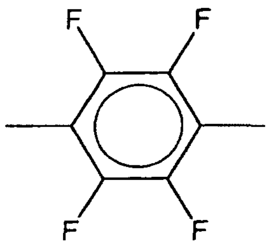 , 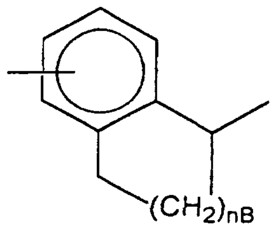

wobei in den vorgenannten Ringsystemen jeweils ein Phenylring ankondensiert sein kann;
mit

| | |
|---|---|
| $n^B$ | = 1; |
| $p^B$ | =0,1; |
| $R^{B1}$ | gleich H (nur für $L^{B2}$), $C_{1-6}$-Alkyl (nur für $L^{B2}$), $C_{0-3}$-Alkylaryl, $C_{0-3}$-Alkylheteroaryl, $C_{0-3}$-Alkyl-$C_{3-8}$-Cycloalkyl; |
| $R^{B2}$ | gleich H, $C_{1-6}$-Alkyl, $C_{0-3}$-Alkylaryl, $C_{0-3}$-Alkylheteroaryl; |
| $R^{B3}$ | gleich H, $C_{1-6}$-Alkyl, $R^{B6}$-O ($R^{B6}$ gleich H, $C_{1-6}$-Alkyl), F, Cl, Br, $NO_2$, $CF_3$; |
| $R^{B4}$ | gleich H, $C_{1-6}$-Alkyl, $R^{B6}$-O, Cl, Br, F, $CF_3$; |
| $R^{B1}$ und $R^{B2}$ | können auch miteinander verbunden sein; |
| $M^B$ | bedeutet Einfach-Bindung, O, S, $CH_2$, $CH_2$-$CH_2$, $CH_2$-O, O-$CH_2$, $CH_2$-S, S-$CH_2$, CO, $SO_2$; |
| A-B | kann stehen für Pyridyl(2)-$CH_2$-, Benzthienyl(2)-, |

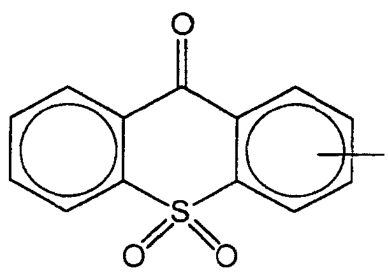 , 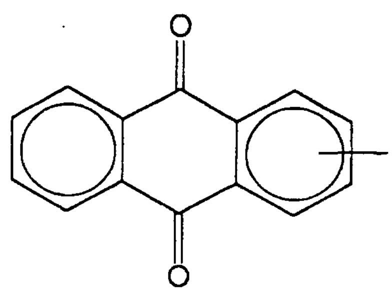

| | |
|---|---|
| D | steht für eine Einfach-Bindung bzw. für CO, $SO_2$; |
| B-D | kann stehen für |

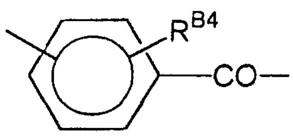

| | |
|---|---|
| E | steht für |

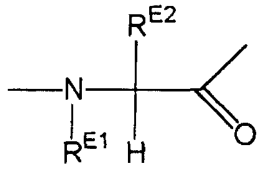

$R^{E1}$ bedeutet H;
$R^{E2}$ bedeutet H, $C_{1-6}$-Alkyl, $C_{3-8}$-Cycloalkyl, Phenyl, Pyridyl, Thienyl, Furyl, Imidazolyl, Tetrahydropyranyl, Tetrahydrothiopyranyl, $CH(CH_3)OH$, $CH(CF_3)_2$;

die unter $R^{E1}$ und $R^{E2}$ genannten Gruppen können über eine Bindung miteinander verknüpft sein;

E kann auch stehen für D-Lys, D-Orn, D-Dab, D-Dap, D-Arg;

G bedeutet

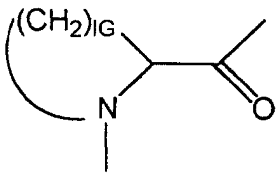

mit $l^G$ = 2, 3, wobei eine $CH_2$-Gruppe des Rings durch $CHCH_2$ ersetzt sein kann

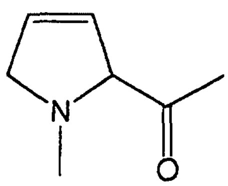
,

$Q^K$ gleich

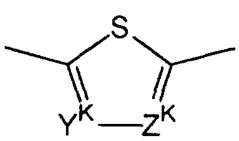

$Y^K$ gleich =CH-, =N-;

$Z^K$ gleich =CH-, =N-;

L:

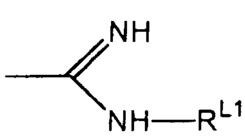
bzw.
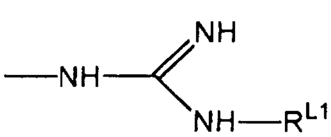

mit

$R^{L1}$ gleich H, OH.

Die Erfindung betrifft außerdem folgende bevorzugten neue Verbindungen, deren Tautomere, physiologisch verträglichen Salze und Prodrugs der Formel A-B-D-E-G-NH-$CH_2$-$Q^K$-L und Arzneimittel, die diese Verbindungen enthalten. Des weiteren eignen sich diese Verbindungen als besonders gute Komplementinhibitoren.

Hierbei gilt:

A steht für
H, $C_{1-6}$-Alkyl, $C_{1-6}$-Alkyl-$SO_2$, $R^{A1}$OCO ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl), $R^{A2}R^{A3}$NCO ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}$OCONR$^{A2}$ ($R^{A4}$ gleich $C_{1-6}$-Alkyl, $C_{1-3}$-Alkylaryl), $R^{A4}$CONR$^{A2}$, $R^{A1}$O, $R^{A2}R^{A3}$N, HO-$SO_2$-, Phenoxy, $R^{A2}R^{A3}$N-$SO_2$, Cl, Br, F, Tetrazolyl, $H_2O_3P$-, $NO_2$, $R^{A1}$-N(OH)-CO-, $R^{A1}R^{A2}$NCONR$^{A3}$, wobei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B} - L^B - (CH_2)_{m^B}-$$

mit

$l^B$ = 0, 1;

$m^B$ = 0, 1, 2;

$L^B$ gleich

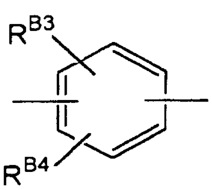 , 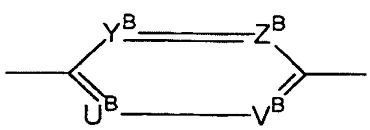 , 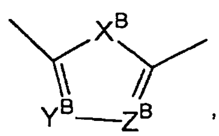 , 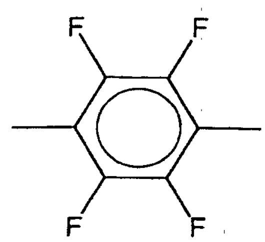

wobei in den vorgenannten Ringsystemen jeweils ein Phenylring ankondensiert sein kann;

$R^{B3}$ gleich H, $C_{1-6}$-Alkyl, Aryl, $R^{B5}$OCO ($R^{B5}$ gleich H, $C_{1-6}$-Alkyl, $C_{1-3}$-Alkylaryl), $R^{B6}$-O ($R^{B6}$ gleich H, $C_{1-6}$-Alkyl), F, Cl, Br, $NO_2$, $CF_3$;

$R^{B4}$ gleich H, $C_{1-6}$-Alkyl, $R^{B6}$-O, Cl, Br, F, $CF_3$;

$X^B$ gleich O, S;

$Y^B$ gleich =CH-, =N-;

$Z^B$ gleich =CH-, =N-;

$U^B$ gleich =CH-, =N-;

$V^B$ gleich =CH-, =N-;

B kann weiterhin stehen für

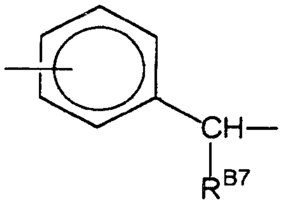
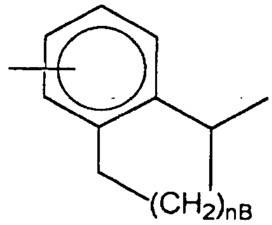

$q^B$ gleich 0, 1, 2;
($R^{B7}$ gleich $C_{1-6}$-Alkyl, $C_{3-8}$-Cycloalkyl)

A-B kann stehen für

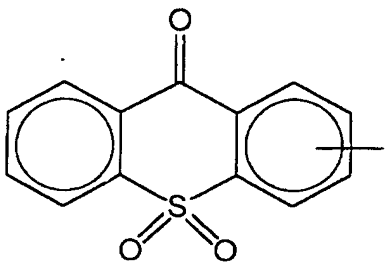
,
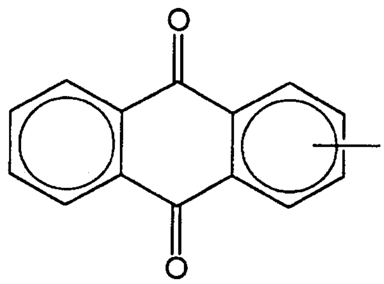

D steht für eine Einfach-Bindung

E steht für

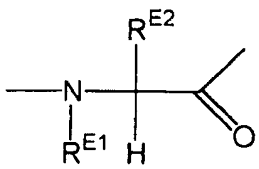

mit

$R^{E1}$ bedeutet H;

$R^{E2}$ bedeutet H, $C_{1-6}$-Alkyl, $C_{3-8}$-Cycloalkyl, Phenyl, Pyridyl, Furyl, Thienyl, Imidazolyl, Tetrahydropyranyl, Tetrahydrothiopyranyl, wobei die vorgenannten Reste bis zu drei gleiche oder verschiedene Substituenten der Gruppe O-$C_{1-6}$-Alkyl, F tragen können, $CH(CH_3)OH$, $CH(CF_3)_2$;

die unter $R^{E1}$ und $R^{E2}$ genannten Gruppen können über eine Bindung miteinander verknüpft sein;

E kann auch stehen für D-Lys, D-Orn, D-Dab, D-Dap, D-Arg;

G bedeutet

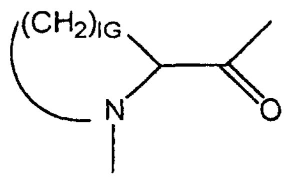

mit $l^G$= 2, 3, wobei eine $CH_2$-Gruppe des Rings durch $CHCH_3$ ersetzt sein kann;

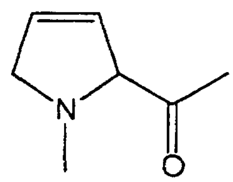
,

$Q^K$ gleich

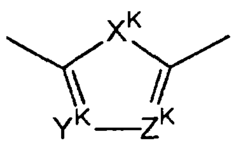

$X^K$ gleich O, S;

$Y^K$ gleich =CH-, =N-;

$Z^K$ gleich =CH-, =N-;

L:

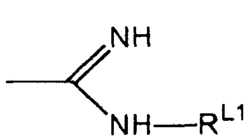

bzw.

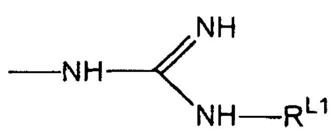

$R^{L1}$ gleich -H, -OH.

Ist bei den Verbindungen der Formel I $R^{L1}$ ungleich Wasserstoff, so handelt es sich bei diesen Substanzen um Prodrugs, aus denen unter in vivo Bedingungen die freien Amidin-/Guanidinverbindungen entstehen. Sind in den Verbindungen der Formel I Esterfunktionen enthalten, so können diese Verbindungen in vivo als Prodrugs wirken, aus welchen die entsprechenden Carbonsäuren entstehen.

Außer den in den Beispielen genannten Substanzen sind folgende Verbindungen ganz besonders bevorzugt und können gemäß der genannten Herstellungsvorschriften hergestellt werden:

| | |
|---|---|
| 1. | $C_6H_5$-C≡C-CO-(D)Cpg-Pyr-NH-$CH_2$-5-(3-am)-thioph |
| 2. | $C_6H_5$-C≡C-CO-(D)Ile-Pyr-NH-$CH_2$-5-(3-am)-thioph |
| 3. | $C_6H_5$-C≡C-CO-(D)allo-Ile-Pyr-NH-$CH_2$-5-(3-am)-thioph |
| 4. | $C_6H_5$-C≡C-CO-(D)Pro-Pyr-NH-$CH_2$-5-(3-am)-thioph |
| 5. | $C_6H_5$-C≡C-CO-(D)(2-(2-thienyl))gly-Pyr-NH-$CH_2$-5-(3-am)-thioph |
| 6. | $C_6H_5$-C≡C-CO-(D)(2-(3-thienyl))gly-Pyr-NH-$CH_2$-5-(3-am)-thioph |

(fortgesetzt)

| | |
|---|---|
| 7. | $C_6H_5$-C≡C-CO-(D)Phg-Pyr-NH-$CH_2$-5-(3-am)-thioph |
| 8. | $C_6H_5$-C≡C-CO-(D)(2-Me)Chg-Pyr-NH-$CH_2$-5-(3-am)-thioph |
| 9. | $C_6H_5$-C≡C-CO-Aib-Pyr-NH-$CH_2$-5-(3-am)-thioph |
| 10. | $C_6H_5$-C≡C-CO-Acpc-Pyr-NH-$CH_2$-5-(3-am)-thioph |
| 11. | $C_6H_5$-C≡5C-CO-Achc-Pyr-NH-$CH_2$-5-(3-am)-thioph |
| 12. | $C_6H_5$-C≡C-CO-(D)(2-(2-furanyl))gly-Pyr-NH-$CH_2$-5-(3-am)-thioph |
| 13. | $C_6H_5$-C≡C-CO-(D)(N-Me)Val-Pyr-NH-$CH_2$-5-(3-am)-thioph |
| 14. | $C_6H_5$-C≡C-CO-(D)Nva-Pyr-NH-$CH_2$-5-(3-am)-thioph |
| 15. | $C_6H_5$-C≡C-CO-(D)Thr-Pyr-NH-$CH_2$-5-(3-am)-thioph |
| 16. | $C_6H_5$-C≡C-CO-(D)(tetrahydro-4-thiopyranyl)gly-Pyr-NH-$CH_2$-5-(3-am)-thioph |
| 17. | 4-HOOC-$C_6H_4$-$CH_2$-(D)Cpg-Pyr-NH-$CH_2$-5-(3-am)-thioph |
| 18. | 4-HOOC-$C_6H_4$-$CH_2$-(D)2-(2-thienyl)gly-Pyr-NH-$CH_2$-5-(3-am)-thioph |
| 19. | 4-HOOC-$C_6H_4$-$CH_2$-(D)2-(3-thienyl)gly-Pyr-NH-$CH_2$-5-(3-am)-thiop |
| 20. | 4-HOOC-$C_6H_4$-$CH_2$-(D)Phg-Pyr-NH-$CH_2$-5-(3-am)-thioph |
| 21. | 4-HOOC-$C_6H_4$-$CH_2$-(D)(2-Me)Chg-Pyr-NH-$CH_2$-5-(3-am)-thioph |
| 22. | 4-HOOC-$C_6H_4$-$CH_2$-Aib-Pyr-NH-$CH_2$-5-(3-am)-thioph |
| 23. | 4-HOOC-$C_6H_4$-$CH_2$-Achc-Pyr-NH-$CH_2$-5-(3-am)-thioph |
| 24. | 4-HOOC-$C_6H_4$-$CH_2$-(D)(2- (2-furanyl))gly-Pyr-NH-$CH_2$-5-(3-am)-thioph |
| 25. | 4-HOOC-$C_6H_4$-$CH_2$-(D)Thr-Pyr-NH-$CH_2$-5-(3-am)-thioph |
| 26. | 4-HOOC-$C_6H_4$-$CH_2$-(D)(tetrahydro-4-thiopyranyl)gly-Pyr-NH-$CH_2$-5-(3-am)-thioph |
| 27. | $C_6H_5$-C≡C-CO-(D)Cpg-Pro-NH-$CH_2$-5-(3-am)-thioph |
| 28. | $C_6H_5$-C≡C-CO-(D)Ile-Pro-NH-$CH_2$-5-(3-am)-thioph |
| 29. | $C_6H_5$-C≡C-CO-(D)allo-Ile-Pro-NH-$CH_2$-5-(3-am)-thioph |
| 30. | $C_6H_5$-C≡C-CO-(D)Pro-Pro-NH-$CH_2$-5-(3-am)-thioph |
| 31. | $C_6H_5$-C≡C-CO-(D)(2-(2-thienyl))gly-Pro-NH-$CH_2$-5-(3-am)-thioph |
| 32. | $C_6H_5$-C≡C-CO-(D)(2-(3-thienyl))gly-Pro-NH-$CH_2$-5-(3-am)-thioph |
| 33. | $C_6H_5$-C≡C-CO-(D)Phg-Pro-NH-$CH_2$-5-(3-am)-thioph |
| 34. | $C_6H_5$-C≡C-CO-(D)(2-Me)Chg-Pro-NH-$CH_2$-5-(3-am)-thioph |
| 35. | $C_6H_5$-C≡C-CO-Aib-Pro-NH-$CH_2$-5-(3-am)-thioph |
| 36. | $C_6H_5$-C≡C-CO-Acpc-Pro-NH-$CH_2$-5-(3-am)-thioph |
| 37. | $C_6H_5$-C≡C-CO-Achc-Pro-NH-$CH_2$-5-(3-am)-thioph |
| 38. | $C_6H_5$-C≡C-CO-(D)(2-(2-furanyl)gly-Pro-NH-$CH_2$-5-(3-am)-thioph |
| 39. | $C_6H_5$-C≡C-CO-(D)(N-Me)Val-Pro-NH-$CH_2$-5-(3-am)-thioph |
| 40. | $C_6H_5$-C≡C-CO-(D)Abu-Pro-NH-$CH_2$-5-(3-am)-thioph |
| 41. | $C_6H_5$-C≡C-CO-(D)Nva-Pro-NH-$CH_2$-5-(3-am)-thioph |
| 42. | $C_6H_5$-C≡C-CO-(D)Thr-Pro-NH-$CH_2$-5-(3-am)-thioph |
| 43. | $C_6H_5$-C≡C-CO-(D)(tetrahydro-4-thiopyranyl)gly-Pro-NH-$CH_2$-5-(3-am)-thioph |
| 44. | $C_6H_5$-C≡C-CO-(D)Cpg-(3S)-3-MePro-NH-$CH_2$-5-(3-am)-thioph |
| 45. | $C_6H_5$-C≡C-CO-(D)Ile-L-(3S)-3-MePro-NH-$CH_2$-5-(3-am)-thioph |

(fortgesetzt)

| | |
|---|---|
| 46. | $C_6H_5$-C≡C-CO-(D)2-(2-thienyl)gly-((3S)-3-Me)Pro-NH-$CH_2$-5-(3-am)-thioph |
| 47. | $C_6H_5$-C≡C-CO-(D)2-(3-thienyl)gly-((3S)-3-Me)Pro-NH-$CH_2$-5-(3-am)-thioph |
| 48. | $C_6H_5$-C≡C-CO-(D)Chg-((3S)-3-Me)Pro-NH-$CH_2$-5-(3-am)-thioph |
| 49. | $C_6H_5$-C≡C-CO- (D)(tetrahydro-4-thiopyranyl)gly-((3S)-3-Me)-Pro-NH-$CH_2$-5-(3-am)-thioph |
| 50. | $C_6H_5$-C≡C-CO-(D)Cpg-(trans-4-F)Pro-NH-$CH_2$-5-(3-am)-thioph |
| 51. | $C_6H_5$-C≡C-CO-(D)Val-(trans-4-F)Pro-NH-$CH_2$-5-(3-am)-thioph |
| 52. | $C_6H_5$-C≡C-CO-(D)2-(2-thienyl)gly-(trans-4-F) Pro-NH-$CH_2$-5-(3-am)-thioph |
| 53. | $C_5H_5$-C≡C-CO-(D)2-(3-thienyl)gly-(trans-4-F)Pro-NH-$CH_2$-5-(3-am)-thioph |
| 54. | $C_6H_5$-C≡C-CO-(D)Chg-(trans-4-F) Pro-NH-$CH_2$-5-(3-am)-thioph |
| 55. | $C_6H_5$-C≡C-CO-(D)Cpg-(cis-4-F)-Pro-NH-$CH_2$-5-(3-am)-thioph |
| 56. | $C_6H_5$-C≡C-CO-(D)Val-(cis-4-F)Pro-NH-$CH_2$-5-(3-am)-thioph |
| 60. | $C_6H_5$-C≡C-CO-(D)Cpg-(5-Me)Pro-NH-$CH_2$-5-(3-am)-thioph |
| 61. | $C_6H_5$-C≡C-CO-(D)Val-(5-Me)Pro-NH-$CH_2$-5-(3-am)-thioph |
| 62. | $C_6H_5$-C≡C-CO-(D)(2-(2-thienyl))gly-(5-Me)Pro-NH-$CH_2$-5-(3-am)-thioph |
| 63. | $C_6H_5$-C≡C-CO-(D)(2-(3-thienyl))gly-(5-Me)Pro-NH-$CH_2$-5-(3-am)-thioph |
| 64. | $C_6H_5$-C≡C-CO-(D)Chg-(5-Me)Pro-NH-$CH_2$-5-(3-am)-thioph |
| 90. | (D)HOOC-CH($CH_2$-$C_6H_5$)-Gly-Pyr-NH-$CH_2$-5-(3-am)-thioph |
| 91. | HOOC-CH($CH_2$-$C_6H_5$)-Gly-Pyr-NH-$CH_2$-5-(3-am)-thioph |
| 92. | (D)HOOC-CH($CH_2$-$C_6H_5$)-(D)Val-Pyr-NH-$CH_2$-5-(3-am)-thioph |
| 93. | HOOC-CH($CH_2$-$C_6H_5$)-(D)Val-Pyr-NH-$CH_2$-5-(3-am)-thioph |
| 94. | (D)HOOC-CH($CH_2$-$C_6H_{10}$)-Gly-Pyr-NH-$CH_2$-5-(3-am)-thioph |
| 95. | HOOC-CH($CH_2$-$C_6H_{10}$)-Gly-Pyr-NH-$CH_2$-5-(3-am)-thioph |
| 96. | (D)HOOC-CH($CH_2$-$C_6H_{10}$)-(D)Val-Pyr-NH-$CH_2$-5-(3-am)-thioph |
| 97. | HOOC-CH($CH_2$-$C_6H_{10}$)-(D)Val-Pyr-NH-$CH_2$-5-(3-am)-thioph |
| 98. | (D)HOOC-CH($CH_2$-$C_6H_5$)-Gly-Pro-NH-$CH_2$-5-(3-am)-thioph |
| 99. | HOOC-CH($CH_2$-$C_6H_5$)-Gly-Pro-NH-$CH_2$-5-(3-am)-thioph |
| 100. | (D)HOOC-CH($CH_2$-$C_6H_5$)-(D)Val-Pro-NH-$CH_2$-5-(3-am)-thioph |
| 101. | HOOC-CH($CH_2$-$C_6H_5$)-(D)Val-Pro-NH-$CH_2$-5-(3-am)-thioph |
| 102. | (D)HOOC-CH($CH_2$-$C_6H_{10}$)-Gly-Pro-NH-$CH_2$-5-(3-am)-thioph |
| 103. | HOOC-CH($CH_2$-$C_6H_{10}$)-Gly-Pro-NH-$CH_2$-5-(3-am)-thioph |
| 104. | (D)HOOC-CH($CH_2$-$C_6H_{10}$)-(D)Val-Pro-NH-$CH_2$-5-(3-am)-thioph |
| 105. | HOOC-CH($CH_2$-$C_6H_{10}$)-(D)Val-Pro-NH-$CH_2$-5-(3-am)-thioph |
| 106. | (D)HOOC-CH($C_6H_5$)-Gly-Pyr-NH-$CH_2$-5-(3-am)-thioph |
| 107. | HOOC-CH($C_6H_5$)-Gly-Pyr-NH-$CH_2$-5-(3-am)-thioph |
| 108. | (D)HOOC-CH($C_6H_{10}$)-Gly-Pyr-NH-$CH_2$-5-(3-am)-thioph |
| 109. | HOOC-CH($C_6H_{10}$)-Gly-Pyr-NH-$CH_2$-5-(3-am)-thioph |
| 110. | (D)HOOC-CH($C_6H_{10}$)-Gly-Pro-NH-$CH_2$-5-(3-am)-thioph |
| 111. | HOOC-CH($C_6H_{10}$)-Gly-Pro-NH-$CH_2$-5-(3-am)-thioph |
| 112. | HOOC-$(CH_2)_5$-(N-$CH_2$-$C_6H_5$)Gly-Pyr-NH-$CH_2$-5-(3-am)-thioph |

(fortgesetzt)

| | |
|---|---|
| 113. | HOOC-$(CH_2)_5$-(N-$CH_2$-$C_6H_{10}$)Gly-Pyr-NH-$CH_2$-5-(3-am)-thioph |
| 114. | HOOC-$(CH_2)_4$-(N-$CH_2$-$C_6H_5$)Gly-Pyr-NH-$CH_2$-5-(3-am)-thioph |
| 115. | HOOC-$(CH_2)_4$-(N-$CH_2$-$C_6H_{10}$)Gly-Pyr-NH-$CH_2$-5-(3-am)-thioph |
| 116. | HOOC-$(CH_2)_5$-(N-$C_6H_5$)Gly-Pyr-NH-$CH_2$-5-(3-am)-thioph |
| 117. | HOOC-$(CH_2)_5$-(N-$C_6H_{10}$)Gly-Pyr-NH-$CH_2$-5-(3-am)-thioph |
| 118. | HOOC-$(CH_2)_4$-(N-$C_6H_5$)Gly-Pyr-NH-$CH_2$-5-(3-am)-thioph |
| 119. | HOOC-$(CH_2)_4$-(N-$C_6H_{10}$)Gly-Pyr-NH-$CH_2$-5-(3-am)-thioph |
| 120. | HOOC-$(CH_2)_4$-$SO_2$-(N-$CH_2$-$C_6H_5$)Gly-Pyr-NH-$CH_2$-5-(3-am)-thioph |
| 121. | HOOC-$(CH_2)_4$-$SO_2$-(N-$CH_2$-$C_6H_{10}$)Gly-Pyr-NH-$CH_2$-5-(3-am)-thioph |
| 122. | HOOC-$(CH_2)_3$-$SO_2$- (N-$CH_2$-$C_6H_5$)Gly-Pyr-NH-$CH_2$-5-(3-am)-thioph |
| 123. | HOOC-$(CH_2)_3$-$SO_2$-(N-$CH_2$-$C_6H_{10}$)Gly-Pyr-NH-$CH_2$-5-(3-am)-thioph |
| 124. | 4-HOOC-$C_6H_4$-$SO_2$-Gly-Pyr-NH-$CH_2$-5-(3-am)-thioph |
| 125. | 3-HOOC-$C_6H_4$-$SO_2$-Gly-Pyr-NH-$CH_2$-5-(3-am)-thioph |
| 126. | 4-HOOC-$C_6H_4$-$SO_2$-D-Val-Pyr-NH-$CH_2$-5-(3-am)-thioph |
| 127. | 3-HOOC-$C_6H_4$-$SO_2$-D-Val-Pyr-NH-$CH_2$-5-(3-am)-thioph |
| 128. | 4-HOOC-$C_6H_4$-$SO_2$-Gly-Pro-NH-$CH_2$-5-(3-am)-thioph |
| 129. | 3-HOOC-$C_6H_4$-$SO_2$-Gly-Pro-NH-$CH_2$-5-(3-am)-thioph |
| 130. | 4-HOOC-$C_6H_4$-$SO_2$-D-Val-Pro-NH-$CH_2$-5-(3-am)-thioph |
| 131. | 3-HOOC-$C_6H_4$-$SO_2$-D-Val-Pro-NH-$CH_2$-5-(3-am)-thioph |
| 132. | MeHNOC-p-$C_6H_4CH_2$-(D)Chg-Pyr-NH-$CH_2$-5-(3-am)-thioph |
| 133. | $H_2NO_2S$-p-$C_6H_4CH_2$-z(D)Chg-Pyr-NH-$CH_2$-5-(3-am)-thioph |
| 134. | $BzHNO_2S$-p-$C_6H_4CH_2$-(D)Chg-Pyr-NH-$CH_2$-5-(3-am)-thioph |
| 135. | 5-Tetrazolyl-p-$C_6H_4CH_2$-(D)Chg-Pyr-NH-$CH_2$-5-(3-am)-thioph |
| 136. | HO-$CH_2$-p-$C_6H_4CH_2$-(D)Chg-Pyr-NH-$CH_2$-5-(3-am)-thioph |
| 137. | HOOC-p-$C_6H_4CH_2$-(D)Chg-Pyr-NH-$CH_2$-5-(4-Me-3-am)-thioph |
| 138. | HOOC-p-$C_6H_4CH_2$-(D)Chg-Pyr-NH-$CH_2$-5-(3-Me-2-am)-thioph |
| 139. | HOOC-p-$C_6H_4CH_2$-(D)Chg-Pyr-NH-3-(6-am)-pico |
| 140. | HOOC-p-$C_6H_4CH_2$-(D)Chg-Pyr-NH-$CH_2$-5-(2-am)-thioph |
| 141. | HOOC-p-$C_6H_4CH_2$-(D)Chg-Pyr-NH-$CH_2$-5-(2-am)-fur |
| 142. | HOOC-p-$C_6H_4CH_2$-(D)Chg-Pyr-NH-$CH_2$-2-(4-am)-thiaz |
| 143. | HOOC-p-$C_6H_4CH_2$-(D)Chg-Pyr-NH-$CH_2$-5-(3-am-4-Cl)-thioph |
| 144. | HOOC-p-$C_6H_4CH_2$-(D)Chg-Pyr-NH-$CH_2$-5-(2-am-3-Cl)-thioph |
| 145. | HOOC-p-$C_6H_4CH_2$-(D)Chg-Pyr-NH-$CH_2$-5-(3-am)-fur |
| 146. | HOOC-m-$C_6H_4CH_2$-(D)Chg-Pyr-NH-$CH_2$-5-(2-am)-thioph |
| 147. | HOOC-m-$C_6H_4CH_2$-(D)Chg-Pyr-NH-$CH_2$-5-(3-am)-thioph |
| 148. | HOOC-m-$C_6H_4CH_2$-(D)Chg-Pyr-NH-3-(6-am)-pico |
| 149. | MeOOC-m-$C_6H_4CH_2$-(D)Chg-Pyr-NH-$CH_2$-2-(4-am)-thiaz |
| 150. | $H_2NCO$-m-$C_6H_4CH_2$-(D)Chg-Pyr-NH-$CH_2$-5-(3-am)-thioph |
| 151. | $HO_3S$-m-$C_6H_4CH_2$-(D)Chg-Pyr-NH-$CH_2$-5-(3-am)-thioph |

(fortgesetzt)

| | |
|---|---|
| 152. | $H_2NO_2S$-m-$C_6H_4CH_2$-(D)Cha-Pyr-NH-$CH_2$-5-(2-am)-thioph |
| 153. | $HO_3S$-m-$C_6H_4CH_2$-(D)Cha-Pyr-NH-$CH_2$-5-(2-am)-thioph |
| 154. | (5-Tetrazolyl)-m-$C_6H_4CH_2$-(D)Chg-Pyr-NH-$CH_2$-5-(3-am)-thioph |
| 155. | trans-(4-HOOC-$C_6H_{10}CH_2$)-(D)Val-Pyr-NH-$CH_2$-5-(3-am)-thioph |
| 156. | HOOC-o-$C_6H_4CH_2$-Gly-Pyr-NH-$CH_2$-5-(3-am)-thioph |
| 157. | 4-Benzyloxyphenyl-NH-C(O)-(D)-Ala-Pyr-NH-$CH_2$-5-(3-am)-thioph |
| 158. | 4-Phenoxyphenyl-NH-C(O)-(D)-Ala-Pyr-NH-$CH_2$-5-(3-am)-thioph |
| 159. | 4-(6'-Methyl-2'-benzothiazolyl)-phenyl-NH-C(O)-(D)-Ala-Pyr-NH-$CH_2$-5-(3-am)-thioph |
| 161. | 4-Benzyloxyphenyl-NH-C(O)-Gly-Pyr-NH-$CH_2$-5-(3-am)-thioph |
| 162. | 4-Phenoxyphenyl-NH-C(O)-Gly-Pyr-NH-$CH_2$-5-(3-am)-thioph |
| 163. | 4-(6'-Methyl-2'-benzothiazolyl)-phenyl-NH-C(O)-Gly-Pro-NH-$CH_2$-5-(3-am)-thioph |
| 165. | 4-Carboxybenzolsulfonyl-(D)-Ala-Pyr-NH-$CH_2$-5-(3-am)-thioph |
| 166. | 3-Carboxybenzolsulfonyl-(D)-Ala-Pyr-NH-$CH_2$-5-(3-am)-thioph |
| 167. | 4-Methyloxycarbonylbenzolsulfonyl-(D)-Ala-Pyr-NH-$CH_2$-5-(3-am)-thioph |
| 168. | 3-Methyloxycarbonylbenzolsulfonyl-(D)-Ala-Pyr-NH-$CH_2$-5-(3-am)-thioph |
| 169. | 4-Acetamidobenzolsulfonyl-(D)-Ala-Pyr-NH-$CH_2$-5-(3-am)-thioph |
| 170. | 3-Acetamidobenzolsulfonyl-(D)-Ala-Pyr-NH-$CH_2$-5-(3-am)-thioph |
| 171. | 4-Phenylbenzolsulfonyl-(D)-Ala-Pro-NH-$CH_2$-5-(3-am)-thioph |
| 172. | 4-Carboxybenzolsulfonyl-(D)-Ala-Pro-NH-$CH_2$-5-(3-am)-thioph |
| 173. | 3-Carboxybenzolsulfonyl-(D)-Ala-Pro-NH-$CH_2$-5-(3-am)-thioph |
| 174. | 4-Methyloxycarbonylbenzolsulfonyl-(D)-Ala-Pro-NH-$CH_2$-5-(3-am)-thioph |
| 175. | 3-Methyloxycarbonylbenzolsulfonyl-(D)-Ala-Pro-NH-$CH_2$-5-(3-am)-thioph |
| 176. | 4-Acetamidobenzolsulfonyl-(D)-Ala-Pro-NH-$CH_2$-5-(3-am)-thioph |
| 177. | 3-Acetamidobenzolsulfonyl-(D)-Ala-Pro-NH-$CH_2$-5-(3-am)-thioph |
| 178. | 4-Carboxybenzolsulfonyl-Ala-Pyr-NH-$CH_2$-5-(3-am)-thioph |
| 179. | 3-Carboxybenzolsulfonyl-Ala-Pyr-NH-$CH_2$-5-(3-am)-thioph |
| 180. | 4-Methyloxycarbonylbenzolsulfonyl-Gly-Pyr-NH-$CH_2$-5-(3-am)-thioph |
| 181. | 3-Methyloxycarbonylbenzolsulfonyl-Gly-Pyr-NH-$CH_2$-5-(3-am)-thioph |
| 182. | 4-Acetamidobenzolsulfonyl-Gly-Pyr-NH-$CH_2$-5-(3-am)-thioph |
| 183. | 3-Acetamidobenzolsulfonyl-Gly-Pyr-NH-$CH_2$-5-(3-am)-thioph |
| 184. | 4-Phenylbenzolsulfonyl-Gly-Pro-NH-$CH_2$-5-(3-am)-thioph |
| 185. | 4-Carboxybenzolsulfonyl-Ala-Pro-NH-$CH_2$-5-(3-am)-thioph |
| 186. | 3-Carboxybenzolsulfonyl-Ala-Pro-NH-$CH_2$-5-(3-am)-thioph |
| 187. | 4-Methyloxycarbonylbenzolsulfonyl-Gly-Pro-NH-$CH_2$-5-(3-am)-thioph |
| 188. | 3-Methyloxycarbonylbenzolsulfonyl-Gly-Pro-NH-$CH_2$-5-(3-am)-thioph |
| 189. | 4-Acetamidobenzolsulfonyl-Gly-Pro-NH-$CH_2$-5-(3-am)-thioph |
| 190. | 3-Acetamidobenzolsulfonyl-Gly-Pro-NH-$CH_2$-5-(3-am)-thioph |
| 191. | 3-Benzoylbenzoyl-(D)-Ala-Pyr-NH-$CH_2$-5-(3-am)-thioph |
| 192. | 4-Phenylbenzoyl-(D)-Ala-Pyr-NH-$CH_2$-5-(3-am)-thioph |

(fortgesetzt)

| | |
|---|---|
| 193. | 4-Phenylphenylacetyl-(D)-Ala-Pyr-NH-$CH_2$-5-(3-am)-thioph |
| 194. | 2-(Benzylthio)-benzoyl-(D)-Ala-Pyr-NH-$CH_2$-5-(3-am)-thioph |
| 195. | 3-Phenylpropionyl-(D)-Ala-Pyr-NH-$CH_2$-5-(3-am)-thioph |
| 196. | 4-Phenylbutyryl-(D)-Ala-Pyr-NH-$CH_2$-5-(3-am)-thioph |
| 197. | 5-Phenylvaleryl-(D)-Ala-Pyr-NH-$CH_2$-5-(3-am)-thioph |
| 198. | (3-phenyl)-acryloyl-(D)-Ala-Pyr-NH-$CH_2$-5-(3-am)-thioph |
| 199. | 3-Benzyloxycarbonylpropionyl-(D)-Ala-Pyr-NH-$CH_2$-5-(3-am)-thioph |
| 200. | 3-(4-methoxycarbonyl(-phenyl)-acryloyl-(D)-Ala-Pyr-NH-$CH_2$-5-(3-am)-thioph |
| 201. | 4-Methoxycarbonylbenzoyl-(D)-Ala-Pyr-NH-$CH_2$-5-(3-am)-thioph |
| 202. | 6-(Acetylamino)-pyridyl-3-carbonyl-(D)-Ala-Pyr-NH-$CH_2$-5-(3-am)-thioph |
| 203. | 3-(3'-Pyridyl)-acryloyl-(D)-Ala-Pyr-NH-$CH_2$-5-(3-am)-thioph |
| 204. | HOOC-p-$C_6H_4$-C≡C-CO-(D)-Ala-Pyr-NH-$CH_2$-5-(3-am)-thioph |
| 205. | HOOC-m-$C_6H_4$-C≡C-CO-(D)-Ala-Pyr-NH-$CH_2$-5-(3-am)-thioph |
| 206. | 4-(4'-Aminophenoxy)-benzoyl-(D)-Ala-Pyr-NH-$CH_2$-5-(3-am)-thioph |
| 207. | 3-(4'-Aminophenoxy)-benzoyl-(D)-Ala-Pyr-NH-$CH_2$-5-(3-am)-thioph |
| 208. | 4-(2'-Chlor-4'-Aminophenoxy)-benzoyl-(D)-Ala-Pyr-NH-$CH_2$-5-(3-am)-thioph |
| 209. | 5-Phenylethinyl-nicotinoyl-(D)-Ala-Pyr-NH-$CH_2$-5-(3-am)-thioph |
| 210. | 4-Phenylethinyl-benzoyl-(D)-Ala-Pyr-NH-$CH_2$-5-(3-am)-thioph |
| 211. | 3-Phenylethinyl-benzoyl-(D)-Ala-Pyr-NH-$CH_2$-5(3-am)-thioph |
| 212. | 3-Benzoylbenzoyl-Ala-Pyr-NH-$CH_2$-5-(3-am)-thioph |
| 213. | 4-Benzoylbenzoyl-Ala-Pyr-NH-$CH_2$-5-(3-am)-thioph |
| 214. | 4-Phenylbenzoyl-Ala-Pyr-NH-$CH_2$-5-(3-am)-thioph |
| 215. | 4-Phenylphenylacetyl-Ala-Pyr-NH-$CH_2$-5-(3-am)-thioph |
| 216. | 2-(Benzylthio)-benzoyl-Ala-Pyr-NH-$CH_2$-5-(3-am)-thioph |
| 217. | 3-Phenylpropionyl-Ala-Pyr-NH-$CH_2$-5-(3-am)-thioph |
| 218. | 4-Phenylbutyryl-Ala-Pyr-NH-$CH_2$-5-(3-am)-thioph |
| 219. | 5-Phenylvaleryl-Ala-Pyr-NH-$CH_2$-5-(3-am)-thioph |
| 220. | Cinnamoyl-Ala-Pyr-NH-$CH_2$-5-(3-am)-thioph |
| 221. | $C_6H_5$-C≡C-CO-Ala-Pyr-NH-$CH_2$-5-(3-am)-thioph |
| 222. | 3-Benzyloxycarbonylpropionyl-Ala-Pyr-NH-$CH_2$-5-(3-am)-thioph |
| 223. | 4-Methoxycarbonylcinnamoyl-Ala-Pyr-NH-$CH_2$-5-(3-am)-thioph |
| 224. | 4-Methoxycarbonylbenzoyl-Ala-Pyr-NH-$CH_2$-5-(3-am)-thioph |
| 225. | 6-(Acetylamino)-pyridyl-3-carbonyl-Ala-Pyr-NH-$CH_2$-5-(3-am)-thioph |
| 226. | 3-(3'-Pyridyl)-acryloyl-Ala-Pyr-NH-$CH_2$-5-(3-am)-thioph |
| 227. | HOOC-p-$C_6H_4$-C≡C-CO-Ala-Pyr-NH-$CH_2$-5-(3-am)-thioph |
| 228. | HOOC-m-$C_6H_4$-C≡C-CO-Ala-Pyr-NH-$CH_2$-5-(3-am)-thioph |
| 229. | 4-(4'-Aminophenoxy)-benzoyl-Ala-Pyr-NH-$CH_2$-5-(3-am)-thioph |
| 230. | 3-(4'-Aminophenoxy)-benzoyl-Ala-Pyr-NH-$CH_2$-5-(3-am)-thioph |
| 231. | 4-(2'-Chlor-4'-Aminophenoxy)-benzoyl-Ala-Pyr-NH-$CH_2$-5-(3-am)-thioph |

(fortgesetzt)

| | |
|---|---|
| 232. | 5-Phenylethinyl-nicotinoyl-Ala-Pyr-NH-$CH_2$-5-(3-am)-thioph |
| 233. | 4-Phenylethinyl-benzoyl-Ala-Pyr-NH-$CH_2$-5-(3-am)-thioph |
| 234. | 3-Phenylethinyl-benzoyl-Ala-Pyr-NH-$CH_2$-5-(3-am)-thioph |
| 235. | 3-Benzoylbenzoyl-(D)-Ala-Pyr-NH-$CH_2$-5-(2-am)-thioph |
| 236. | 4-Phenylbenzoyl-(D)-Ala-Pyr-NH-$CH_2$-5-(2-am)-thioph |
| 237. | 4-Phenylphenylacetyl-(D)-Ala-Pyr-NH-$CH_2$-5- (2-am)-thioph |
| 238. | 4-Phenylphenylacetyl-(D)-Ala-Pro-NH-$CH_2$-5-(2-am)-thioph |
| 239. | 3-Benzoylbenzoyl-(D)-Ala-Pro-NH-$CH_2$-5-(2-am)-thioph |
| 240. | 4-Benzoylbenzoyl-(D)-Ala-Pro-NH-$CH_2$-5-(2-am)-thioph |
| 241. | 4-Phenylbenzoyl-(D)-Ala-Pro-NH-$CH_2$-5-(2-am)-thioph |
| 242. | 3-Benzoylbenzoyl-(D)-Asp-Pyr-NH-$CH_2$-5-(3-am)-thioph |
| 243. | 4-Phenylbenzoyl-(D)-Asp-Pyr-NH-$CH_2$-5-(3-am)-thioph |
| 244. | 4-Phenylphenylacetyl-(D)-Asp-Pyr-NH-$CH_2$-5-(3-am)-thioph |
| 245. | 3-(3'-Pyridyl)-acryloyl-(D)-Asp-Pyr-NH-$CH_2$-5-(3-am)-thioph |
| 246. | 4-(4'-Aminophenoxy)-benzoyl-(D)-Asp-Pyr-NH-$CH_2$-5-(3-am)-thioph |
| 247. | 3-(4'-Aminophenoxy)-benzoyl-(D)-Asp-Pyr-NH-$CH_2$-5-(3-am)-thioph |
| 248. | 4-(2'-Chlor-4'-Aminophenoxy)-benzoyl-(D)-Asp-Pyr-NH-$CH_2$-5-(3-am)-thioph |
| 249. | 3-Benzoylbenzoyl-Asp-Pyr-NH-$CH_2$-5-(3-am)-thioph |
| 250. | 4-Benzoylbenzoyl-Asp-Pyr-NH-$CH_2$-5-(3-am)-thioph |
| 251. | 4-Phenylbenzoyl-Asp-Pyr-NH-$CH_2$-5-(3-am)-thioph |
| 252. | 4-Phenylphenylacetyl-Asp-Pyr-NH-$CH_2$-5-(3-am)-thioph |
| 253. | $C_6H_5$-C≡C-CO-Asp-Pyr-NH-$CH_2$-5-(3-am)-thioph |
| 254. | 3-Benzoylbenzoyl-(D)-Ala-Pyr-NH-3-(6-am)-pico |
| 255. | 4-Benzoylbenzoyl-(D)-Ala-Pyr-NH-3-(6-am)-pico |
| 256. | 4-Phenylbenzoyl-(D)-Ala-Pyr-NH-3-(6-am)-pico |
| 257. | 4-Phenylphenylacetyl-(D)-Ala-Pyr-NH-3-(6-am)-pico |
| 258. | $C_6H_5$-C≡C-CO-(D)-Ala-Pyr-NH-3-(6-am)-pio |
| 259. | 3-Benzoylbenzoyl-(D)-Arg-Pyr-NH-$CH_2$-5-(3-am)-thioph |
| 260. | 4-Phenylphenylacetyl-(D)-Arg-Pyr-NH-$CH_2$-5-(3-am)-thioph |
| 261. | 3-Benzoylbenzoyl-Arg-Pyr-NH-$CH_2$-5-(3-am)-thioph |
| 262. | 4-Benzoylbenzoyl-Arg-Pyr-NH-$CH_2$-5-(3-am)-thioph |
| 263. | 4-Phenylbenzoyl-Arg-Pyr-NH-$CH_2$-5-(3-am)-thioph |
| 264. | 4-Phenylphenylacetyl-Arg-Pyr-NH-$CH_2$-5-(3-am)-thioph |
| 265. | $C_6H_5$-C≡C-CO-Arg-Pyr-NH-$CH_2$-5-(3-am)-thioph |
| 266. | 3-Benzoylbenzoyl-(D)-Val-Pyr-NH-$CH_2$-5-(3-am)-thioph |
| 267. | 4-Phenylbenzoyl-(D)-Val-Pyr-NH-$CH_2$-5-(3-am)-thioph |
| 268. | 4-Phenylphenylacetyl-(D)-Val-Pyr-NH-$CH_2$-5-(3-am)-thioph |
| 269. | 2-(Benzylthio)-benzoyl-(D)-Val-Pyr-NH-$CH_2$-5-(3-am)-thioph |
| 270. | 3-Phenylpropionyl-(D)-Val-Pyr-NH-$CH_2$-5-(3-am)-thioph |

(fortgesetzt)

| | |
|---|---|
| 271. | 4-Phenylbutyryl-(D)-Val-Pyr-NH-$CH_2$-5-(3-am)-thioph |
| 272. | 5-Phenylvaleryl-(D)-Val-Pyr-NH-$CH_2$-5-(3-am)-thioph |
| 273. | Cinnamoyl-(D)-Val-Pyr-NH-$CH_2$-5-(3-am)-thioph |
| 274. | 3-Benzyloxycarbonylpropionyl-(D)-Val-Pyr-NH-$CH_2$-5-(3-am)-thioph |
| 275. | 4-Methoxycarbonylcinnamoyl-(D)-Val-Pyr-NH-$CH_2$-5-(3-am)-thioph |
| 276. | 4-Methoxycarbonylbenzoyl-(D)-Val-Pyr-NH-$CH_2$-5-(3-am)-thioph |
| 277. | 6-(Acetylamino)-pyridyl-3-carbonyl-(D)-Val-Pyr-NH-$CH_2$-5-(3-am)-thioph |
| 278. | 3-(3'-Pyridyl)-acryloyl-(D)-Val-Pyr-NH-$CH_2$-5-(3-am)-thioph |
| 279. | HOOC-p-$C_6H_4$-C≡C-CO-(D)-Val-Pyr-NH-$CH_2$-5-(3-am)-thioph |
| 280. | HOOC-m-$C_6$H4-C≡C-CO-(D)-Val-Pyr-NH-$CH_2$-5-(3-am)-thioph |
| 281. | 4(4'-Aminophenoxy)-benzoyl-(D)-Val-Pyr-NH-$CH_2$-5-(3-am)-thioph |
| 282. | 3-(4'-Aminophenoxy)-benzoyl-(D)-Val-Pyr-NH-$CH_2$-5-(3-am)-thioph |
| 283. | 4-(2'-Chlor-4'-Aminophenoxy)-benzoyl-(D)-Val-Pyr-NH-$CH_2$-5-(3-am)-thioph |
| 284. | 5-Phenylethinyl-nicotinoyl-(D)-Val-Pyr-NH-CH2-5-(3-am)-thioph |
| 285. | 4-Phenylethinyl-benzoyl-(D)-Val-Pyr-NH-$CH_2$-5-(3-am)-thioph |
| 286. | 3-Phenylethinyl-benzoyl-(D)-Val-Pyr-NH-$CH_2$-5-(3-am)-thioph, |
| 287. | 3-Benzoylbenzoyl-(D)-Val-Pyr-NH-$CH_2$-5-(2-am)-thioph |
| 288. | 4-Phenylbenzoyl-(D)-Val-Pyr-NH-$CH_2$-5-(2-am)-thioph |
| 289. | 4-Phenylphenylacetyl-(D)-Val-Pyr-NH-$CH_2$-5-(2-am)-thioph |
| 290. | 4-Phenylphenylacetyl-(D)-Val-Pro-NH-$CH_2$-5-(2-am)-thioph |
| 291. | 3-Benzoylbenzoyl-(D)-Val-Pro-NH-$CH_2$-5-(2-am)-thioph |
| 292. | 4-Benzoylbenzoyl-(D)-Val-Pro-NH-$CH_2$-5-(2-am)-thioph |
| 293. | 4-Phenylbenzoyl-(D)-Val-Pro-NH-$CH_2$-5-(2-am)-thioph |
| 294. | $C_6H_5$-C≡C-CO-(D)-Lys-Pyr-NH-$CH_2$-5-(2-am)-thioph |
| 295. | 3-Benzoylbenzoyl-(D)-Lys-Pyr-NH-$CH_2$-5-(3-am)-thioph |
| 296. | 4-Phenylbenzoyl-(D)-Lys-Pyr-NH-$CH_2$-5-(3-am)-thioph |
| 297. | 4-Phenylphenylacetyl-(D)-Lys-Pyr-NH-$CH_2$-5-(3-am)-thioph |
| 298. | 3-(3'-Pyridyl)-acryloyl-(D)-Lys-Pyr-NH-$CH_2$-5-(3-am)-thioph |
| 299. | 4-(4'-Aminophenoxy)-benzoyl-(D)-Lys-Pyr-NH-$CH_2$-5-(3-am)-thioph |
| 300. | 3-(4'-Aminophenoxy)-benzoyl-(D)-Lys-Pyr-NH-$CH_2$-5-(3-am)-thioph |
| 301. | 4-(2'-Chlor-4'-Aminophenoxy)-benzoyl-(D)-Lys-Pyr-NH-$CH_2$-5-(3-am)-thioph |
| 302. | 3-Benzoylbenzoyl-Gly-Pyr-NH-$CH_2$-5-(3-am)-thioph |
| 303. | 4-Phenylbenzoyl-Gly-Pyr-NH-$CH_2$-5-(3-am)-thioph |
| 304. | 4-Phenylphenylacetyl-Gly-Pyr-NH-$CH_2$-5-(3-am)-thioph |
| 305. | 2-(Benzylthio)-benzoyl-Gly-Pyr-NH-$CH_2$-5-(3-am)-thioph |
| 306. | 3-Phenylpropionyl-Gly-Pyr-NH-$CH_2$-5-(3-am)-thioph |
| 307. | 4-Phenylbutyryl-Gly-Pyr-NH-$CH_2$-5-(3-am)-thioph |
| 308. | 5-Phenylvaleryl-Gly-Pyr-NH-$CH_2$-5-( 3-am)-thioph |
| 309. | (3-phenyl)-acryloyl-Gly-Pyr-NH-$CH_2$-5-(3-am)-thioph |

(fortgesetzt)

| | |
|---|---|
| 310. | 3-Benzyloxycarbonylpropionyl-Gly-Pyr-NH-$CH_2$-5-(3-am)-thioph |
| 311. | 3-(4-methoxycarbonyl-phenyl)-acryloyl-Gly-Pyr-NH-$CH_2$-5-(3-am)-thioph |
| 312. | 4-Methoxycarbonylbenzoyl-Gly-Pyr-NH-$CH_2$-5-(3-am)-thioph |
| 313. | 6-(Acetylamino)-pyridyl-3-carbonyl-Gly-Pyr-NH-$CH_2$-5-(3-am)-thioph |
| 314. | 3-(3'-Pyridyl)-acryloyl-Gly-Pyr-NH-$CH_2$-5-(3-am)-thioph |
| 315. | HOOC-p-$C_6H_4$-C≡C-CO-Gly-Pyr-NH-$CH_2$-5-(3-am)-thioph |
| 316. | HOOC-m-$C_6H_4$-C≡C-CO-Gly-Pyr-NH-$CH_2$-5-(3-am)-thioph |
| 317. | 4-(4'-Aminophenoxy)-benzoyl-Gly-Pyr-NH-$CH_2$-5-(3-am)-thioph |
| 318. | 3-(4'-Aminophenoxy)-benzoyl-Gly-Pyr-NH-$CH_2$-5-(3-am)-thioph |
| 319. | 4-(2'-Chlor-4'-Aminophenoxy)-benzoyl-Gly-Pyr-NH-$CH_2$-5-(3-am)-thioph |
| 320. | 5-Phenylethinyl-nicotinoyl-Gly-Pyr-NH-$CH_2$-5-(3-am)-thioph |
| 321. | 4-Phenylethinyl-benzoyl-Gly-Pyr-NH-$CH_2$-5-(3-am)-thioph |
| 322. | 3-Phenylethinyl-benzoyl-Gly-Pyr-NH-$CH_2$-5-(3-am)-thioph |
| 323. | HOOC-p-$C_6H_4$-C≡C-CO-Gly-Pro-NH-$CH_2$-5-(3-am)-thioph |
| 324. | HOOC-m-$C_6H_4$-C≡C-CO-Gly-Pro-NH-$CH_2$-5-(3-am)-thioph |
| 325. | 5-Phenylethinyl-nicotinoyl-Gly-Pro-NH-$CH_2$-5-(3-am)-thioph |
| 326. | 4-Phenylethinyl-benzoyl-Gly-Pro-NH-$CH_2$-5-(3-am)-thioph |
| 327. | 3-Phenylethinyl-benzoyl-Gly-Pro-NH-$CH_2$-5-(3-am)-thioph |
| 328. | 3-Benzoylbenzoyl-(D)-Val-Pyr-NH-$CH_2$-2-(4-am)-thiaz |
| 329. | 4-Benzoylbenzoyl-(D)-Val-Pyr-NH-$CH_2$-2-(4-am)-thiaz |
| 330. | 4-Phenylbenzoyl-(D)-Val-Pyr-NH-$CH_2$-2-(4-am)-thiaz |
| 331. | 4-Phenylphenylacetyl-(D)-Val-Pyr-NH-$CH_2$-2-(4-am)-thiaz |
| 332. | 3-Benzoylbenzoyl-(D)-Ala-Pyr-NH-$CH_2$-2-(4-am)-thiaz |
| 333. | 4-Benzoylbenzoyl-(D)-Ala-Pyr-NH-$CH_2$-2-(4-am)-thiaz |
| 334. | 4-Phenylbenzoyl-(D)-Ala-Pyr-NH-$CH_2$-2-(4-am)-thiaz |
| 335. | 4-Phenylphenylacetyl-(D)-Ala-Pyr-NH-$CH_2$-2-(4-am)-thiaz |
| 336. | 3-Benzoylbenzoyl-Gly-Pyr-NH-$CH_2$-2-(4-am)-thiaz |
| 337. | 4-Benzoylbenzoyl-Gly-Pyr-NH-$CH_2$-2-(4-am)-thiaz |
| 338. | 4-Phenylbenzoyl-Gly-Pyr-NH-$CH_2$-2-(4-am)-thiaz |
| 339. | 4-Phenylphenylacetyl-Gly-Pyr-NH-$CH_2$-2-(4-am)-thiaz |
| 340. | 3-Benzoylbenzoyl-Val-Pyr-NH-$CH_2$-5-(3-am)-thioph |
| 341. | 4-Benzoylbenzoyl-Val-Pyr-NH-$CH_2$-5-(3-am)-thioph |
| 342. | 4-Phenylbenzoyl-Val-Pyr-NH-$CH_2$-5-(3-am)-thioph |
| 343. | 4-Phenylphenylacetyl-Val-Pyr-NH-$CH_2$-5-(3-am)-thioph |
| 344. | 2-(Benzylthio)-benzoyl-Val-Pyr-NH-$CH_2$-5-(3-am)-thioph |
| 345. | 3-Phenylpropionyl-Val-Pyr-NH-$CH_2$-5-(3-am)-thioph |
| 346. | 4-Phenylbutyryl-Val-Pyr-NH-$CH_2$-5-(3-am)-thioph |
| 347. | 5-Phenylvaleryl-Val-Pyr-NH-$CH_2$-5-(3-am)-thioph |
| 348. | (3-phenyl)-acryloyl-Val-Pyr-NH-$CH_2$-5-(3-am)-thioph |

(fortgesetzt)

| | |
|---|---|
| 349. | $C_6H_5$-C≡C-CO-Val-Pyr-NH-$CH_2$-5-(3-am)-thioph |
| 350. | 3-Benzyloxycarbonylpropionyl-Val-Pyr-NH-$CH_2$-5-(3-am)-thioph |
| 351. | 3-(4-methoxycarbonyl-phenyl)-acryloyl-Val-Pyr-NH-$CH_2$-5-(3-am)-thioph |
| 352. | 4-Methoxycarbonylbenzoyl-Val-Pyr-NH-$CH_2$-5-(3-am)-thioph |
| 353. | 6-(Acetylamino)-pyridin-3-carbonyl-Val-Pyr-NH-$CH_2$-5-(3-am)-thioph |
| 354. | 3-(3'-Pyridyl)-acryloyl-Val-Pyr-NH-$CH_2$-5-(3-am)-thioph |
| 355. | HOOC-p-$C_6H_4$-C≡C-CO-Val-Pyr-NH-$CH_2$-5-(3-am)-thioph |
| 356. | HOOC-m-$C_6H_4$-C≡C-CO-Val-Pyr-NH-$CH_2$-5-(3-am)-thioph |
| 357. | 4-(4'-Aminophenoxy)-benzoyl-Val-Pyr-NH-$CH_2$-5-(3-am)-thioph |
| 358. | 3-(4'-Aminophenoxy)-benzoyl-Val-Pyr-NH-$CH_2$-5-(3-am)-thioph |
| 359. | 4-(2'-Chlor-4'-Aminophenoxy)-benzoyl-Val-Pyr-NH-$CH_2$-5-(3-am)-thioph |
| 360. | 5-Phenylethinyl-nicotinoyl-Val-Pyr-NH-$CH_2$-5-(3-am)-thioph |
| 361. | 4-Phenylethinyl-benzoyl-Val-Pyr-NH-$CH_2$-5-(3-am)-thioph |
| 362. | 3-Phenylethinyl-benzoyl-Val-Pyr-NH-$CH_2$-5-(3-am)-thioph |
| 363. | 3-Benzoylbenzoyl-Sar-Pyr-NH-$CH_2$-5-(3-am)-thioph |
| 364. | 4-Phenylbenzoyl-Sar-Pyr-NH-$CH_2$-5-(3-am)-thioph |
| 365. | 4-Phenylphenylacetyl-Sar-Pyr-NH-$CH_2$-5-(3-am)-thioph |
| 366. | 3-Phenylpropionyl-Sar-Pyr-NH-$CH_2$-5-(3-am)-thioph |
| 367. | 4-Phenylbutyryl-Sar-Pyr-NH-$CH_2$-5-(3-am)-thioph |
| 368. | 5-Phenylvaleryl-Sar-Pyr-NH-$CH_2$-5-(3-am)-thioph |
| 369. | 3-Benzyloxycarbonylpropionyl-Sar-Pyr-NH-$CH_2$-5-(3-am)-thioph |
| 370. | 6-(Acetylamino)-pyridyl-3-carbonyl-Sar-Pyr-NH-$CH_2$-5-(3-am)-thioph |
| 371. | 3-(3'-Pyridyl)-acryloyl-Sar-Pyr-NH-$CH_2$-5-(3-am)-thioph |
| 372. | 4-(4'-Aminophenoxy)-benzoyl-Sar-Pyr-NH-$CH_2$-5-(3-am)-thioph |
| 373. | 3-(4'-Aminophenoxy)-benzoyl-Sar-Pyr-NH-$CH_2$-5-(3-am)-thioph |
| 374. | 4-(2'-Chlor-4'-Aminophenoxy)-benzoyl-Sar-Pyr-NH-$CH_2$-5-(3-am)-thioph |
| 375. | 3-Benzoylbenzoyl-Sar-Pro-NH-$CH_2$-5-(3-am)-thioph |
| 376. | 4-Benzoylbenzoyl-Sar-Pro-NH-$CH_2$-5-(3-am)-thioph |
| 377. | 4-Phenylbenzoyl-Sar-Pro-NH-$CH_2$-5-(3-am)-thioph |
| 378. | 4-Phenylphenylacetyl-Sar-Pro-NH-$CH_2$-5-(3-am)-thioph |
| 379. | 3-Phenylpropionyl-Sar-Pro-NH-$CH_2$-5-(3-am)-thioph |
| 380. | 4-Phenylbutyryl-Sar-Pro-NH-$CH_2$-5-(3-am)-thioph |
| 381. | 5-Phenylvaleryl-Sar-Pro-NH-$CH_2$-5-(3-am)-thioph |
| 382. | $C_6H_5$-C≡C-CO-Sar-Pro-NH-$CH_2$-5-(3-am)-thioph |
| 383. | 3-Benzyloxycarbonylpropionyl-Sar-Pro-NH-$CH_2$-5-(3-am)-thioph |
| 384. | 6-(Acetylamino)-pyridin-3-carbonyl-Sar-Pro-NH-$CH_2$-5-(3-am)-thioph |
| 385. | 3-(3'-Pyridyl)-acryloyl-Sar-Pro-NH-$CH_2$-5-(3-am)-thioph |
| 386. | 4-(4'-Aminophenoxy)-benzoyl-Sar-Pro-NH-$CH_2$-5- (3-am)-thioph |
| 387. | 3-(4'-Aminophenoxy)-benzoyl-Sar-Pro-NH-$CH_2$-5-(3-am)-thioph |

(fortgesetzt)

| | |
|---|---|
| 388. | 4-(2'-Chlor-4'-Aminophenoxy)-benzoyl-Sar-Pro-NH-$CH_2$-5-(3-am)-thioph |
| 389. | 3-Benzoylbenzoyl-(D)-(N-Me)Ala-Pyr-NH-$CH_2$-5-(3-am)-thioph |
| 390. | 4-Benzoylbenzoyl-(D)-(N-Me)Ala-Pyr-NH-$CH_2$-5-(3-am)-thioph |
| 391. | 4-Phenylbenzoyl-(D)-(N-Me)Ala-Pyr-NH-$CH_2$-5-(3-am)-thioph |
| 392. | 4-Phenylphenylacetyl-(D)-(N-Me)Ala-Pyr-NH-$CH_2$-5-(3-am)-thioph |
| 393. | 3-Phenylpropionyl-(D)-(N-Me)Ala-Pyr-NH-$CH_2$-5-(3-am)-thioph |
| 394. | 4-Phenylbutyryl-(D)-(N-Me)Ala-Pyr-NH-$CH_2$-5-(3-am)-thioph |
| 395. | 5-Phenylvaleryl-(D)-(N-Me)Ala-Pyr-NH-$CH_2$-5-(3-am)-thioph |
| 396. | $C_6H_5$-C≡C-CO-(D)-(N-Me)Ala-Pyr-NH-$CH_2$-5-(3-am)-thioph |
| 397. | 3-Benzyloxycarbonylpropionyl-(D)-(N-Me)Ala-Pyr-NH-$CH_2$-5-(3-am)-thioph |
| 398. | 6-(Acetylamino)-pyridyl-3-carbonyl-(D)-(N-Me)Ala-Pyr-NH-$CH_2$-5-(3-am)-thioph |
| 399. | 3-(3'-Pyridyl)-acryloyl-(D)-(N-Me)Ala-Pyr-NH-$CH_2$-5-(3-am)-thioph |
| 400. | 4-(4'-Aminophenoxy)-benzoyl-(D)-(N-Me)Ala-Pyr-NH-$CH_2$-5-(3-am)-thioph |
| 401. | 3-(4'-Aminophenoxy)-benzoyl-(D)-(N-Me)Ala-Pyr-NH-$CH_2$-5-(3-am)-thioph |
| 402. | 4-(2'-Chlor-4'-Aminophenoxy)-benzoyl-(D)-(N-Me)Ala-Pyr-NH-$CH_2$-5-(3-am)-thioph |
| 403. | 3-Benzoylbenzoyl-(D)-(N-Me)Ala-Pro-NH-$CH_2$-5-(3-am)-thioph |
| 404. | 4-Benzoylbenzoyl-(D)-(N-Me)Ala-Pro-NH-$CH_2$-5-(3-am)-thioph |
| 405. | 4-Phenylbenzoyl-(D)-(N-Me)Ala-Pro-NH-$CH_2$-5-(3-am)-thioph |
| 406. | 4-Phenylphenylacetyl-(D)-(N-Me)Ala-Pro-NH-$CH_2$-5-(3-am)-thioph |
| 407. | 3-Phenylpropionyl-(D)-(N-Me)Ala-Pro-NH-$CH_2$-5-(3-am)-thioph |
| 408. | 4-Phenylbutyryl-(D)-(N-Me)Ala-Pro-NH-$CH_2$-5-(3-am)-thioph |
| 409. | 5-Phenylvaleryl-(D)-(N-Me)Ala-Pro-NH-$CH_2$-5-(3-am)-thioph |
| 410. | $C_6H_5$-C≡C-CO-(D)-(N-Me)Ala-Pro-NH-$CH_2$-5-(3-am)-thioph |
| 411. | 3-Benzyloxycarbonylpropionyl-(D)-(N-Me)Ala-Pro-NH-$CH_2$-5-(3-am)-thioph |
| 412. | 6-(Acetylamino)-pyridyl-3-carbonyl-(D)-(N-Me)Ala-Pro-NH-$CH_2$-5-(3-am)-thioph |
| 413. | 3-(3'-Pyridyl)-acryloyl-(D)-(N-Me)Ala-Pro-NH-$CH_2$-5-(3-am)-thioph |
| 414. | 4-(4'-Aminophenoxy)-benzoyl-(D)-(N-Me)Ala-Pro-NH-$CH_2$-5-(3-am)-thioph |
| 415. | 3-(4'-Aminophenoxy)-benzoyl-(D)-(N-Me)Ala-Pro-NH-$CH_2$-5-(3-am)-thioph |
| 416. | 4-(2`-Chlor-4'-Aminophenoxy)-benzoyl-(D)-(N-Me)Ala-Pro-NH-$CH_2$-5-(3-am)-thioph |
| 417. | 3-Benzoylbenzoyl-β-Ala-Pro-NH-$CH_2$-5-(3-am)-thioph |
| 418. | Cinnamoyl-β<-Ala-Pro-NH-$CH_2$-5-(3-am)-thioph |
| 419. | $C_6H_5$-C≡C-CO-β-Ala-Pro-NH-$CH_2$-5-(3-am) -thioph |
| 420. | 3-Benzyloxycarbonylpropionyl-β-Ala-Pro-NH-$CH_2$-5-(3-am)-thioph |
| 421. | 4-Methoxycarbonylcinnamoyl-β-Ala-Pro-NH-$CH_2$-5-(3-am)-thioph |
| 422. | 4-Methoxycarbonylbenzoyl-β-Ala-Pro-NH-$CH_2$-5-(3-am)-thioph |
| 423. | 6-(Acetylamino)-pyridyl-3-carbonyl-β-Ala-Pro-NH-$CH_2$-5-(3-am)-thioph |
| 424. | 3-(3'-Pyridyl)-acryloyl-β-Ala-Pro-NH-$CH_2$-5-(3-am)-thioph |
| 425. | HOOC-p-$C_6H_4$-C≡C-CO-β-Ala-Pro-NH-$CH_2$-5-(3-am)-thioph |
| 426. | HOOC-m-$C_6H_4$-C≡C-CO-β-Ala-Pro-NH-$CH_2$-5-(3-am)-thioph |

(fortgesetzt)

| | |
|---|---|
| 427. | 4-(4'-Aminophenoxy)-benzoyl-β-Ala-Pro-NH-$CH_2$-5-(3-am)-thioph |
| 428. | 3-(4`-Aminaphenoxy)-benzoyl-β-Ala-Pro-NH-$CH_2$-5-(3-am)-thioph |
| 429. | 4-(2'-Chlor-4 '-Aminophenoxy)-benzoyl-β-Ala-Pro-NH-$CH_2$-5-(3-am)-thioph |
| 430. | 5-Phenylethinyl-nicotinoyl-β-Ala-Pro-NH-$CH_2$-5-(3-am)-thioph |
| 431. | 4-Phenylethinyl-benzoyl-β-Ala-Pro-NH-$CH_2$-5-(3-am)-thioph |
| 432. | 3-Phenylethinyl-benzoyl-β-Ala-Pro-NH-$CH_2$-5-(3-am)-thioph |
| 433. | 3-Benzoylbenzoyl-β-Ala-Pyr-NH-$CH_2$-5-(3-am)-thioph |
| 434. | 4-Phenylbenzoyl-β-Ala-Pyr-NH-$CH_2$-5-(3-am)-thioph |
| 435. | 4-Phenylphenylacetyl-β-Ala-Pyr-NH-$CH_2$-5-(3-am)-thioph |
| 436. | 2-(Benzylthio)-benzoyl-β-Ala-Pyr-NH-$CH_2$-5-(3-am)-thioph |
| 437. | 3-Phenylpropionyl-β-Ala-Pyr-NH-$CH_2$-5-(3-am)-thioph |
| 438. | 4-Phenylbutyryl-β-Ala-Pyr-NH-$CH_2$-5-(3-am)-thioph |
| 439. | 5-Phenylvaleryl-β-Ala-Pyr-NH-$CH_2$-5-(3-am)-thioph |
| 440. | 3-Benzyloxycarbonylpropionyl-β-Ala-Pyr-NH-$CH_2$-5-(3-am)-thioph |
| 441. | 4-Methoxycarbonylcinnamoyl-β-Ala-Pyr-NH-$CH_2$-5-(3-am)-thioph |
| 442. | 4-Methoxycarbonylbenzoyl-β-Ala-Pyr-NH-$CH_2$-5-(3-am)-thioph |
| 443. | 6-(Acetylamino)-pyridyl-3-carbonyl-β-Ala-Pyr-NH-$CH_2$-5-(3-am)-thioph |
| 444. | 3-(3'-Pyridyl)-acryloyl-β-Ala-Pyr-NH-$CH_2$-5-(3-am)-thioph |
| 445. | HOOC-p-$C_6H_4$-C≡C-CO-β-Ala-Pyr-NH-$CH_2$-5-(3-am)-thioph |
| 446. | HOOC-m-$C_6H_4$-C≡C-CO-β-Ala-Pyr-MH-$CH_2$-5-(3-am)-thioph |
| 447. | 4-(4'-Aminophenoxy)-benzoyl-β-Ala-Pyr-NH-$CH_2$-5-(3-am)-thioph |
| 448. | 3-(4'-Aminophenoxy)-benzoyl-β-Ala-Pyr-NH-$CH_2$-5-(3-am)-thioph |
| 449. | 4-(2'-Chlor-4'-Aminophenoxy)-benzoyl-β-Ala-Pyr-NH-$CH_2$-5-(3-am)-thioph |
| 450. | 5-phenylethinyl-nicotinoyl-β-Ala-Pyr-NH-$CH_2$-5-(3-am)-thioph |
| 451. | 4-Phenylethinyl-benzoyl-β-Ala-Pyr-NH-$CH_2$-5-(3-am)-thioph |
| 452. | 3-Phenylethinyl-benzoyl-β-Ala-Pyr-NH-$CH_2$-5-(3-am)-thioph, |
| 455. | 4-HOOC-$C_6H_4$-$CH_2$-(D)Cpg-(5-Me)Pro-NH-$CH_2$-5-(3-am)-thioph |
| 458. | 4-HOOC-$C_6H_4$-$CH_2$-(D)Cpg-(3S)(3-Me)Pro-NH-$CH_2$-5-(3-am)-thioph |
| 459. | 4-HOOC-$C_6H_4$-$CH_2$-(D)Cpg-Pyr-NH-$CH_2$-5-(2-am)-thioph |
| 460. | 4-HOOC-$C_6H_4$-CH($CH_3$)-(D)Cpg-Pyr-NH-$CH_2$-5-(3-am)-thioph |
| 461. | 4-HOOC-$C_6H_4$-CO-(D)Cpg-Pyr-NH-$CH_2$-5-(3-am)-thioph |
| 462. | 4-HOOC-$C_6H_4$-CH($CH_3$)-(D)Chg-Pyr-NH-$CH_2$-5-(3-am)-thioph |
| 463. | 4-HOOC-$C_6H_4$-$CH_2$-(N-Me)(D)Chg-Pyr-NH-$CH_2$-5-(3-am)-thioph |
| 464. | 4-HOOC-$C_6H_4$-C$(CH_3)_2$-(D)Chg-Pyr-NH-$CH_2$-5-(3-am)-thioph |
| 465. | 4-HOOC-3-Me-$C_6H_4$-$CH_2$-(D)Chg-Pyr-NH-$CH_2$-5-(3-am)-thioph |
| 466. | 4-HOOC-2-Me-$C_6H_4$-$CH_2$-(D)Chg-Pyr-NH-$CH_2$-5-(3-am)-thioph |
| 467. | 4-HOOC-$CH_2$-$C_6H_4$-$CH_2$-(D)Chg-Pyr-NH-$CH_2$-5-(3-am)-thioph |
| 468. | 3-HOOC-$CH_2$-$C_6H_4$-$CH_2$-(D)Chg-Pyr-NH-$CH_2$-5-(3-am)-thioph |
| 469. | 4-HOOC-$C_6H_4$-CH($CH_3$)-(D)Cpg-Pyr-NH-$CH_2$-5-(3-am)-thioph |

(fortgesetzt)

| | |
|---|---|
| 470. | 4-HOOC-$C_6H_4$-$CH_2$-(N-Me)(D)Cpg-Pyr-NH-$CH_2$-5-(3-am)-thioph |
| 471. | 4-HOOC-$C_6H_4$-$C(CH_3)_2$-(D)Cpg-Pyr-NH-$CH_2$-5-(3-am)-thioph |
| 472. | 4-HOOC-3-Me-$C_6H_4$-$CH_2$-(D)Cpg-Pyr-NH-$CH_2$-5-(3-am)-thioph |
| 473. | 4-HOOC-2-Me-$C_6H_4$-$CH_2$-(D)Cpg-Pyr-NH-$CH_2$-5-(3-am)-thioph |
| 474. | 4-HOOC-$CH_2$-$C_6H_4$-$CH_2$-(D)Cpg-Pyr-NH-$CH_2$-5-(3-am)-thioph |
| 475. | 3-HOOC-$CH_2$-$C_6H_4$-$CH_2$-(D)Cpg-Pyr-NH-$CH_2$-5-(3-am)-thioph |
| 476. | 4-HOOC-$C_6H_4$-$CH(CH_3)$-(D)Chg-Pyr-NH-$CH_2$-5-(2-am)-thioph |
| 477. | 4-HOOC-$C_6H_4$-$CH_2$-(N-Me)(D)Chg-Pyr-NH-$CH_2$-5-(2-am)-thioph |
| 478. | 4-HOOC-$C_6H_4$-$C(CH_3)_2$-(D)Chg-Pyr-NH-$CH_2$-5-(2-am)-thioph |
| 479. | 4-HOOC-3-Me-$C_6H_4$-$CH_2$-(D)Chg-Pyr-NH-$CH_2$-5-(2-am)-thioph |
| 480. | 4-HOOC-2-Me-$C_6H_4$-$CH_2$-(D) Chg-Pyr-NH-$CH_2$-5-(2-am)-thioph |
| 481. | 4-HOOC-$CH_2$-$C_6H_4$-$CH_2$-(D)Chg-Pyr-NH-$CH_2$-5-(2-am)-thioph |
| 482. | 3-2HOOC-$CH_2$-$C_6H_4$-$CH_2$-(D)Chg-Pyr-NH-$CH_2$-5-(2-am)-thioph |
| 483. | 4-HOOC-$C_6H_4$-$CH(CH_3)$-(D)Cpg-Pyr-NH-$CH_2$-5-(2-am)-thioph |
| 484. | 4-HOOC-$C_6H_4$-$CH_2$-(N-Me)(D)Cpg-Pyr-NH-$CH_2$-5-(2-am)-thioph |
| 485. | 4-HOOC-$C_6H_4$-$C(CH_3)_2$-(D)Cpg-Pyr-NH-$CH_2$-5-(2-am)-thioph |
| 486. | 4-HOOC-3-Me-$C_6H_4$-$CH_2$-(D)Cpg-Pyr-NH-$CH_2$-5-(2-am)-thioph |
| 487. | 4-HOOC-2-Me-$C_6H_4$-$CH_2$-(D)Cpg-Pyr-NH-$CH_2$-5-(2-am)-thioph |
| 488. | 4-HOOC-$CH_2$-$C_6H_4$-$CH_2$-(D)Cpg-Pyr-NH-$CH_2$-5-(2-am)-thioph |
| 489. | 3-HOOC-$CH_2$-$C_6H_4$-$CH_2$-(D)Cpg-Pyr-NH-$CH_2$-5-(2-am)-thioph |
| 490. | HOOC-p-$C_6H_4$-$CH(CH_3)$-D-Val-Pyr-NH-$CH_2$-5-(3-am)-thioph |
| 491. | HOOC-p-$C_6H_4$-$(CH_2)_2$-D-Val-Pyr-NH-$CH_2$-5-(3-am)-thioph |
| 492. | HOOC-p-$CH_2$-$C_6H_4$-$CH_2$-D-Val-Pyr-NH-$CH_2$-5-(3-am)-thioph |
| 493. | p-carboxy-tetrafluorbenzyl-D-Val-Pyr-NH-$CH_2$-5-(3-am)-thioph |
| 494. | p-carboxy-2'-F-benzyl-D-Val-Pyr-NH-$CH_2$-5-(3-am)-thioph |
| 495. | p-carboxy-2'-methoxy-benzyl-D-Val-Pyr-NH-$CH_2$-5-(3-am)-thioph |
| 496. | p-carboxy-3'-methoxy-benzyl-D-Val-Pyr-NH-$CH_2$-5-(3-am)-thioph |
| 497. | $H_2O_3P$-p-$C_6H_4$-$CH_2$-D-Val-Pyr-NH-$CH_2$-5-(3-am)-thioph |
| 498. | 5-COOH-indanyl(1)-D-Val-Pyr-NH-5-(3-am)-thioph |
| 499. | 6-COOH-indanyl(1)-D-Val-Pyr-NH-5-(3-am)-thioph |
| 501. | HOOC-p-$C_6H_4$-$CH_2$-D-Val-Pyr-NH-$CH_2$-5-(2-am)-thioph |
| 502. | HOOC-p-$C_6H_4$-$CH_2$-D-Val-Pyr-NH-$CH_2$-4-(2-am)-thioph |
| 503. | HOOC-p-$C_6H_4$-$CH_2$-D-val-Pyr-NH-3-(6-am)pico |
| 504. | HOOC-p-$C_6H_4$-$CH_2$-D-Val-Pyr-NH-$CH_2$-5-(2-am)-fur |
| 505. | HOOC-p-$C_6H_4$-$CH_2$-D-Val-Pyr-NH-$CH_2$-5-(3-am-4-Cl)-thioph |
| 506. | HOOC-p-$C_6H_4$-$CH_2$-D-Val-Pyr-NH-$CH_2$-5-(2-am-3-Cl)-thioph |
| 507. | HOOC-p-$C_6H_4$-$CH_2$-D-Val-Pyr-NH-$CH_2$-2-(4-am)-thiaz |
| 508. | HOOC-p-$C_6H_4$-$CH_2$-D-Val-Pyr-NH-$CH_2$-2-(5-am)-thiaz |
| 509. | HOOC-p-$C_6H_4$-$CH_2$-D-Val-Pyr-NH-$CH_2$-5-(2-am)-thiaz |

(fortgesetzt)

| | |
|---|---|
| 510. | HOOC-p-$C_6H_4$-$CH_2$-D-Val-Pyr-NH-$CH_2$-4-(2-am)-thiaz |
| 511. | HOOC-p-$C_6H_4$-$CH_2$-D-Val-Pyr-NH-$CH_2$-5-(3-am-4-Me)-thioph |
| 512. | HOOC-p-$C_6H_4$-$CH_2$-D-Val-Pyr-NH-$CH_2$-5-(2-am-4-Me)-thioph |
| 513. | HOOC-p-$C_6H_4$-$CH_2$-D-Val-Pyr-NH-$CH_2$-2-(4-guan)-thiaz |
| 514. | HOOC-p-$C_6H_4$-$CH_2$-D-Val-Pyr-NH-$CH_2$-2-(5-guan)-thiaz |
| 515. | HOOC-p-$C_6H_4$-$CH_2$-D-Val-Pyr-NH-$CH_2$-5-(3-guan)-thioph |
| 516. | HOOC-p-$C_6H_4$-$CH_2$-D-Val-Pyr-NH-$CH_2$-5-(2-guan)-thioph |
| 525. | HOOC-p-$C_6H_4$-$CH_2$-D-Val-Pyr-NH-$CH_2$-5-(3-C($NHCH_3$)=$NCH_3$)-thioph |
| 526. | HOOC-p-$C_6H_4$-$CH_2$-D-Val-Pyr-NH-$CH_2$-5-(3-C($NH_2$)=$NCH_3$)-thioph |
| 527. | HOOC-p-$C_6H_4$-$CH_2$-D-Val-Pic-NH-$CH_2$-5-(3-am)-thioph |
| 528. | HOOC-p-$C_6H_4$-$CH_2$-D-Val-Aze-NH-$CH_2$-5-(3-am)-thioph |
| 529. | HOOC-p-$C_6H_4$-$CH_2$-D-Val-N-Me-Ala-NH-$CH_2$-5-(3-am)-thioph |
| 531. | HOOC-p-$C_6H_4$-$CH_2$-D-Val-Thz-4-CO-NH-$CH_2$-5-(3-am)-thioph |
| 532. | HOOC-p-$C_6H_4$-$CH_2$-D-(2-$CF_3$)Gly-Pyr-NH-$CH_2$-5-(3-am)-thioph |
| 533. | HOOC-p-$C_6H_4$-$CH_2$-D-(3-$CF_3$)Ala-Pyr-NH-$CH_2$-5-(3-am)-thioph |
| 534. | HOOC-p-$C_6H_4$-$CH_2$-D-3,3-$(CF_3)_2$-Ala-Pyr-NH-$CH_2$-5-(3-am)-thioph |
| 535. | HOOC-p-$C_6H_4$-$CH_2$-D-2-Methyl-Val-Pyr-NH-$CH_2$-5-(3-am)-thioph |
| 536. | (p-$CH_3$)-benzoyl-D-Val-Pyr-NH-$CH_2$-5-(3-am)-thioph |
| 537. | (p-ethyl)-benzoyl-D-Val-Pyr-NH-$CH_2$-5-(3-am)-thioph |
| 538. | (p-propyl)-benzoyl-D-Val-Pyr-NH-$CH_2$-5-(3-am)-thioph |
| 539. | (p-butyl)-benzoyl-D-Val-Pyr-NH-$CH_2$-5-(3-am)-thioph |
| 540. | (p-isopropyl)benzoyl-D-Val-Pyr-NH-$CH_2$-5-(3-am)-thioph |
| 541. | (p-tBu)benzoyl-D-Val-Pyr-NH-$CH_2$-5-(3-am)-thioph |
| 542. | (p-pentyl)benzoyl-D-Val-Pyr-NH-$CH_2$-5-(3-am)-thioph |
| 543. | (p-hexyl)benzoyl-D-Val-Pyr-NH-$CH_2$-5-(3-am)-thioph |
| 544. | (p-trifluormethyl)benzoyl-D-Val-Pyr-NH-$CH_2$-5-(3-am)-thioph |
| 545. | (o-methyl)benzoyl-D-Val-Pyr-NH-$CH_2$-5-(3-am)-thioph |
| 546. | (o-trifluormethyl)benzoyl-D-Val-Pyr-NH-$CH_2$-5-(3-am)-thioph |
| 547. | (o-methoxy)benzoyl-D-Val-Pyr-NH-$CH_2$-5-(3-am)-thioph |
| 548. | (o-dimethyl)benzoyl-D-Val-Pyr-NH-$CH_2$-5-(3-am)-thioph |
| 549. | (o-dimethoxy)benzoyl-D-Val-Pyr-NH-$CH_2$-5-(3-am)-thioph |
| 550. | (p-methoxy)benzoyl-D-Val-Pyr-NH-$CH_2$-5-(3-am)-thioph |
| 551. | (p-ethoxy)benzoyl-D-Val-Pyr-NH-$CH_2$-5-(3-am)-thioph |
| 552. | (p-propyloxy)benzoyl-D-Val-Pyr-NH-$CH_2$-5-(3-am)-thioph |
| 553. | (p-isopropyloxy)benzoyl-D-Val-Pyr-NH-$CH_2$-5-(3-am)-thioph |
| 554. | (p-butyloxy)benzoyl-D-Val-Pyr-NH-$CH_2$-5-(3-am)-thioph |
| 555. | (p-tert.butoxy)benzoyl-D-Val-Pyr-NH-$CH_2$-5-(3-am)-thioph |
| 556. | (p-aminomethyl)benzoyl-D-Val-Pyr-NH-$CH_2$-5-(3-am)-thioph |
| 557. | 2,6-dichlorophenyl-$CH_2$CO-D-Ala-Pyr-NH-$CH_2$-5-(3-am)-thioph |

(fortgesetzt)

| | |
|---|---|
| 558. | 2,6-dichlorophenyl-$CH_2$CO-D-Ile-Pyr-NH-$CH_2$-5-(3-am)-thioph |
| 559. | 2,6-dichlorophenyl-$CH_2$CO-D-allo-Ile-Pyr-NH-$CH_2$-5-(3-am)-thioph |
| 560. | 2,6-dichlorophenyl-$CH_2$CO-D-tLeu-Pyr-NH-$CH_2$-5-(3-am)-thioph |
| 561. | 2,6-dichlorophenyl-$CH_2$CO-D-hexafluor-Val-Pyr-NH-$CH_2$-5-(3-am)-thioph |
| 562. | 2,6-dichlorophenyl-$CH_2$CO-D-Thr-Pyr-NH-$CF_2$-5-(3-am)-thioph |
| 563. | 2,6-dichlorophenyl-$CH_2$CO-D-Cpg-Pyr-NH-$CH_2$-5-(3-am)-thioph |
| 564. | 2,6-dichlorophenyl-$CH_2$CO-D-2-methyl-Val-Pyr-NH-$CH_2$-5-(3-am)-thioph |
| 566. | 2,6-dichlorophenyl-$CH_2$CO-D-Val-Pyr-NH-$CH_2$-5-(2-am)-thioph |
| 567. | 2,6-dichlorophenyl-$CH_2$CO-D-Val-Pyr-NH-3-(6-am)pico |
| 568. | 2,6-dichlorophenyl-$CH_2$CO-D-Val-Pyr-NH-$CH_2$-5-(2-am)-fur |
| 569. | 2,6-dichlorophenyl-$CH_2$CO-D-Val-Pyr-NH-$CH_2$-5-(3-am-4-Cl)-thioph |
| 570. | 2,6-dichlorophenyl-$CH_2$CO-D-Val-Pyr-NH-$CH_2$-5-(2-am-3-Cl)-thioph |
| 571. | 2,6-dichlorophenyl-$CH_2$CO-D-Val-Pyr-NH-$CH_2$-2-(4-am)-thiaz |
| 572. | 2,6-dichlorophenyl-$CH_2$CO-D-Val-Pyr-NH-$CH_2$-2-(5-am)-thiaz |
| 573. | 2,6-dichlorophenyl-$CH_2$CO-D-Val-Pyr-NH-$CH_2$-5-(2-am)-thiaz |
| 574. | 2,6-dichlorophenyl-$CH_2$CO-D-Val-Pyr-NH-$CH_2$-4-(2-am) -thiaz |
| 575. | 2,6-dichlorophenyl-$CH_2$CO-D-Val-Pyr-N-H-$CH_2$-2-(4-guan)-thiaz |
| 576. | 2,6-dichlorophenyl-$CH_2$CO-D-Val-Pyr-NH-$CH_2$-2-(5-guan)-thiaz |
| 577. | 2,6-dichlorophenyl-$CH_2$CO-D-Val-Pyr-NH-$CH_2$-5-(3-guan)-thioph |
| 578. | 2,6-dichlorophenyl-$CH_2$CO-D-Val-Pyr-NH-$CH_2$-5-(2-guan)-thioph |
| 579. | 2,6-dichlorophenyl-$CH_2$CO-D-Val-Pyr-NH-$CH_2$-4-(2-am)-thioph |
| 588. | 2,6-dichlorophenyl-$CH_2$CO-D-Val-Pyr-NH-$CH_2$-5-(3-C($NHCH_3$)=$NCH_3$)-thioph |
| 589. | 2,6-dichlorophenyl-$CH_2$CO-D-Val-Pyr-NH-$CH_2$-5-(3-C($NH_2$)=$NCH_3$)-thioph |
| 590. | Indanyl(1R)-D-Cpg-Pyr-NH-$CH_2$-5-(3-am)-thioph |
| 591. | Indanyl (1R)-D-Ala-Pyr-NH-$CH_2$-5-(3-am)-thioph |
| 592. | Indanyl(1R)-D-Thr-Pyr-NH-$CH_2$-5-(3-am)-thioph |
| 593. | Indanyl(1R)-D-allo-Ile-Pyr-NH-$CH_2$-5-(3-am)-thioph |
| 594. | Indanyl (1R)-D-tLeu-Pyr-NH-$CH_2$-5-(3-am)-thioph |
| 595. | Indanyl(1R)-D-hexafluor-Val-Pyr-NH-$CH_2$-5-(3-am-)-thioph |
| 596. | Indanyl(1R)-D-2-methyl-Val-Pyr-NH-$CH_2$-5-(3-am)-thioph |
| 597. | Indanyl(1R)-CO-D-Cpg-Pyr-NH-$CH_2$-5-(3-am)-thioph |
| 598. | Indanyl(1R)-CO-D-Ala-Pyr-NH-$CH_2$-5-(3-am)-thioph |
| 599. | Indanyl(1R)-CO-D-allo-Ile-Pyr-NH-$CH_2$-5-(3-am)-thioph |
| 600. | Indanyl(1R)-CO-D-tLeu-Pyr-NH-$CH_2$-5-(3-am)-thioph |
| 601. | Indanyl (1S)-D-Cpg-Pyr-NH-$CH_2$-5-(3-am)-thioph |
| 602. | Indanyl(1S)-D-Ala-Pyr-NH-$CH_2$-5-(3-am)-thioph |
| 603. | Indanyl (1S)-D-Thr-Pyr-NH-$CH_2$-5-(3-am)-thioph |
| 604. | Indanyl(1S)-D-allo-Ile-Pyr-NH-$CH_2$-5-(3-am)-thioph |
| 605. | Indanyl(1S)-D-tLeu-Pyr-NH-$CH_2$-5-(3-am)-thioph |

(fortgesetzt)

| | |
|---|---|
| 606. | Indanyl(1S)-D-hexafluor-Val-Pyr-NH-$CH_2$-5-(3-am)-thioph |
| 607. | Indanyl(1S)-D-2-methyl-Val-Pyr-NH-$CH_2$-5-(3-am)-thioph |
| 608. | Indanyl(1S)-CO-D-Cpg-Pyr-NH-$CH_2$-5-(3-am)-thioph |
| 609. | Indanyl(1S)-CO-D-Ala-Pyr-NH-$CH_2$-5-(3-am)-thioph |
| 610. | Indanyl(1S)-CO-D-allo-Ile-Pyr-NH-$CH_2$-5-(3-am)-thioph |
| 611. | Indanyl(1S)-CO-D-tLeu-Pyr-NH-$CH_2$-5-(3-am)-thioph |
| 612. | (5,6-dimethyl)indanyl(1)-CO-D-Val-Pyr-NH-$CH_2$-5-(3-am)-thioph |
| 613. | (5,7-dimethyl)indanyl(1)-CO-D-Val-Pyr-NH-$CH_2$-5-(3-am)-thioph |
| 614. | (p-aminomethyl)-benzyl-CO-D-Val-Pyr-NH-$CH_2$-5-(3-am)-thioph |
| 615. | (o-carboxy)-benzyl-CO-D-Val-Pyr-NH-$CH_2$-5-(3-am)-thioph |
| 616. | (m-carboxy)-benzyl-CO-D-Val-Pyr-NH-$CH_2$-5-(3-am)-thioph |
| 617. | (p-carboxy)-benzyl-CO-D-Val-Pyr-NH-$CH_2$-5-(3-am)-thioph |
| 618. | (p-carboxy-methyl)-benzyl-CO-D-Val-Pyr-NH-$CH_2$-5-(3-am)-thioph |
| 619. | Indanyl(2)-CO-D-Val-Pyr-NH-$CH_2$-5-(3-am)-thioph |
| 620. | (2,4,6-trimethoxy)-benzyl-CO-D-Val-Pyr-NH-$CH_2$-5-(3-am)-thioph |
| 621. | Tetrahydronaphthyl(1S)-CO-D-Val-Pyr-NH-$CH_2$-5-(3-am)-thioph |
| 622. | Tetrahydronaphthyl(1R)-CO-D-Val-Pyr-NH-$CH_2$-5-(3-am)-thioph |
| 623. | 2,6-dibromphenyl-$CH_2$CO-D-Val-Pyr-NH-$CH_2$-5-(3-am)-thioph |
| 624. | 2,6-ditrifluormethyl-phenyl-$CH_2$CO-D-Val-Pyr-NH-$CH_2$-5-(3-am)-thioph |
| 625. | Indolyl(3)-CO-D-Val-Pyr-NH-$CH_2$-5-(3-am)-thioph |
| 626. | N-methyl-indolyl(3)-CO-D-Val-Pyr-NH-$CH_2$-5-(3-am)-thioph |
| 627. | Benzthienyl(3)CO-D-Val-Pyr-NH-$CH_2$-5-(3-am)-thioph |
| 628. | (5-carboxy)indanyl(1R)-D-Val-Pyr-NH-$CH_2$-5-(3-am)-thioph |
| 629. | (6-carboxy)indanyl(1R)-D-Val-Pyr-NH-$CH_2$-5-(3-am)-thioph |
| 630. | (4-carboxy-2,6-dichlor)benzyl-CO-D-Val-Pyr-NH-$CH_2$-5-(3-am)-thioph |
| 631. | (5-carboxy)indanyl(1S)-D-Val-Pyr-NH-$CH_2$-5-(3-am)-thioph |
| 632. | (6-carboxy)indanyl(1S)-D-Val-Pyr-NH-$CH_2$-5-(3-am)-thioph |
| 633. | (5-carboxy)indanyl (1R)-CO-D-Val-Pyr-NH-$CH_2$-5-(3-am)-thioph |
| 634. | (6-carboxy)indanyl(1R)-CO-D-Val-Pyr-NH-$CH_2$-5-(3-am)-thioph |
| 635. | (5-carboxy)indanyl(1S)-CO-D-Val-Pyr-NH-$CH_2$-5-(3-am)-thioph |
| 636. | (6-carboxy)indanyl(1S)-CO-D-Val-Pyr-NH-$CH_2$-5-(3-am)-thioph |
| 637. | (p-$CH_3$)-benzyl-CO-D-Val-Pyr-NH-$CH_2$-5-(3-am)-thioph |
| 638. | (p-ethyl)-benzyl-CO-D-Val-Pyr-NH-$CH_2$-5-(3-am)-thioph |
| 639. | (p-propyl)-benzyl-CO-D-Val-Pyr-NH-$CH_2$-5-(3-am)-thioph |
| 640. | (p-butyl)-benzyl-CO-D-Val-Pyr-NH-$CH_2$-5-(3-am)-thioph |
| 641. | (p-isopropyl)benzyl-CO-D-Val-Pyr-NH-$CH_2$-5-(3-am)-thioph |
| 642. | (p-tBu)benzyl-CO-D-Val-Pyr-NH-$CH_2$-5-(3-am)-thioph |
| 643. | (p-pentyl)benzyl-CO-D-Val-Pyr-NH-$CH_2$-5-(3-am)-thioph |
| 644. | (p-hexyl)benzyl-CO-D-Val-Pyr-NH-$CH_2$-5-(3-am)-thioph |

(fortgesetzt)

| | |
|---|---|
| 645. | (p-trifluormethyl)benzyl-CO-D-Val-Pyr-NH-$CH_2$-5-(3-am)-thioph |
| 646. | (o-methyl)benzyl-CO-D-Val-Pyr-NH-$CH_2$-5-(3-am)-thioph |
| 647. | (o-trifluormethyl)benzyl-CO-D-Val-Pyr-NH-$CH_2$-5-(3-am)-thioph |
| 648. | (o-methoxy)benzyl-CO-D-Val-Pyr-NH-$CH_2$-5-(3-am)-thioph |
| 649. | (o-dimethyl)benzyl-CO-D-Val-Pyr-NH-$CH_2$-5-(3-am)-thioph |
| 650. | (o-dimethoxy)benzyl-CO-D-Val-Pyr-NH-$CH_2$-5-(3-am)-thioph |
| 651. | (p-methoxy)benzyl-CO-D-Val-Pyr-NH-$CH_2$-5-(3-am)-thioph |
| 652. | (p-ethoxy)benzyl-CO-D-Val-Pyr-NH-$CH_2$-5-(3-am)-thioph |
| 653. | (p-propyloxy)benzyl-CO-Val-Pyr-NH-$CH_2$-5-(3-am)-thioph |
| 654. | (p-isopropyloxy)benzyl-CO-D-Val-Pyr-NH-$CH_2$-5-(3-am)-thioph |
| 655. | (p-butyloxy)benzylCO-D-Val-Pyr-NH-$CH_2$-5-(3-am)-thioph |
| 656. | (p-tert.butoxy)benzyl-CO-D-Val-Pyr-NH-$CH_2$-5-(3-am)-thioph |
| 657. | (p-CN)-benzyl-D-Val-Pyr-NH-$CH_2$-5-(3-am)-thioph |
| 658. | (p-dimethylamino)-benzyl-D-Val-Pyr-NH-$CH_2$-5-(3-am)-thioph |
| 659. | (p-methoxy)-benzyl-D-Val-Pyr-NH-$CH_2$-5-(3-am)-thioph |
| 660. | (p-ethoxy)-benzyl-D-Val-Pyr-NH-$CH_2$-5-(3-am)-thioph |
| 661. | (p-propyloxy)benzyl-D-Val-Pyr-NH-$CH_2$-5-(3-am)-thioph |
| 662. | (p-isopropyloxy)benzyl-D-Val-Pyr-NH-$CH_2$-5-(3-am)-thioph |
| 663. | (p-butyloxy)benzyl-D-Val-Pyr-NH-$CH_2$-5-(3-am)-thioph |
| 664. | (p-tertbutyloxy)benzyl-D-Val-Pyr-NH-$CH_2$-5-(3-am)-thioph |
| 665. | (p-pentyloxy)benzyl-D-Val-Pyr-NH-$CH_2$-5-(3-am)-thioph |
| 666. | (p-trifluormethyl)benzyl-D-Val-Pyr-NH-$CH_2$-5-(3-am)-thioph |
| 667. | (p-ethyl)benzyl-D-Val-Pyr-NH-$CH_2$-5-(3-am)-thioph |
| 668. | (p-propyl)benzyl-D-Val-Pyr-NH-$CH_2$-5-(3-am)-thioph |
| 669. | (p-butyl)benzyl-D-Val-Pyr-NH-$CH_2$-5-(3-am)-thioph |
| 670. | (p-tert.butyl)benzyl-D-Val-Pyr-NH-$CH_2$-5-(3-am)-thioph |
| 671. | (p-pentyl)benzyl-D-Val-Pyr-NH-$CH_2$-5-(3-am)-thioph |
| 672. | (p-hexyl)benzyl-D-Val-Pyr-NH-$CH_2$-5-(3-am)-thioph |
| 673. | (p-$MeSO_2$))benzyl-D-Val-Pyr-NH-$CH_2$-5-(3-am)-thioph |
| 674. | (p-Nitro)benzyl-D-Val-Pyr-NH-$CH_2$-5-(3-am)-thioph |
| 675. | (p-carboxy)benzyl-D-Val-Pyr-NH-$CH_2$-5-(3-ham)-thioph |
| 676. | (p-carboxy)benzyl-D-Ala-Pyr-NH-$CH_2$-5-(3-ham)-thioph |
| 677. | (p-carboxy)benzyl-D-Abu-Pyr-NH-$CH_2$-5-(3-ham)-thioph |
| 678. | (p-carboxy)benzyl-D-Nva-Pyr-MH-$CH_2$-5-(3-ham)-thioph |
| 679. | (p-carboxy)benzyl-D-tLeu-Pyr-NH-$CH_2$-5-(3-ham)-thioph |
| 680. | (p-carboxy)benzyl-D-Ile-Pyr-NH-$CH_2$-5-(3-ham)-thioph |
| 681. | (p-carboxy)benzyl-D-allo-Ile-Pyr-NH-$CH_2$-5-(3-ham)-thioph |
| 682. | (p-carboxy)benzoyl-D-Val-Pyr-NH-$CH_2$-5-(3-ham)-thioph |
| 683. | (p-carboxy)benzyl-D-Cpg-Pyr-NH-$CH_2$-5-(3-ham)-thioph |

(fortgesetzt)

| | |
|---|---|
| 684. | 2,6-dichlorbenzyl-CO-D-Val-Pyr-NH-$CH_2$-5-(3-ham)-thioph |
| 685. | 2,6-dichlorbenzyl-CO-D-Ala-Pyr-NH-$CH_2$-5-(3-ham)-thioph |
| 686. | 2,6-dichlorbenzyl-CO-D-Abu-Pyr-NH-$CH_2$-5-(3-ham)-thioph |
| 687. | 2,6-dichlorbenzyl-CO-D-Nva-Pyr-NH-$CH_2$-5-(3-ham)-thioph |
| 688. | 2,6-dichlorbenzyl-CO-D-tLeu-Pyr-NH-$CH_2$-5-(3-ham)-thioph |
| 689. | 2,6-dichlorbenzyl-CO-D-Ile-Pyr-NH-$CH_2$-5-(3-ham)-thioph |
| 690. | 2,6-dichlorbenzyl-CO-D-allo-Ile-Pyr-NH-$CH_2$-5-(3-ham)-thioph |
| 691. | 2,6-dichlorbenzyl-CO-D-Cpg-Pyr-NH-$CH_2$-5-(3-ham)-thioph |
| 692. | p-benzoyl-benzoyl-D-Val-Pyr-NH-$CH_2$-5-(3-ham)-thioph |
| 693. | (p-Phenyl-NH-CO-NH)benzoyl-D-Val-Pyr-NH-$CH_2$-5-(3-ham)-thioph |
| 694. | 2,4,6-trimethyl-benzyl-CO-D-Val-Pyr-NH-$CH_2$-5-(3-ham)-thioph |
| 695. | Benzhydryl-CO-D-Val-Pyr-NH-$CH_2$-5-(3-ham)-thioph |
| 696. | (p-carboxy)benzyl-CO-D-Val-Pyr-NH-$CH_2$-5-(3-ham)-thioph |
| 697. | (p-COOMe)benzyl-D-Val-Pyr-NH-$CH_2$-5-(3-ham)-thioph |
| 698. | (p-COOEt)benzyl-D-Val-Pyr-NH-$CH_2$-5-(3-ham)-thioph |
| 699. | (p-COOPr) benzyl-D-Val-Pyr-NH-$CH_2$-5-(3-ham)-thioph |
| 700. | (p-COOiPr)benzyl-D-Val-Pyr-NH-$CH_2$-5-(3-ham)-thioph |
| 701. | (p-COOtBu) benzyl-D-Val-Pyr-NH-$CH_2$-5-(3-ham)-thioph |
| 702. | (p-COOCyclohexyl)benzyl-D-Val-Pyr-NH-$CH_2$-5-(3-ham)-thioph |
| 703. | (p-COOCyclopentyl)benzyl-D-Val-Pyr-NH-$CH_2$-5-(3-ham)-thioph |
| 704. | (p-COOMe)benzyl-D-Ala-Pyr-NH-$CH_2$-5-(3-ham)-thioph |
| 705. | (p-COOEt)benzyl-D-Ala-Pyr-NH-$CH_2$-5-(3-ham)-thioph |
| 706. | (p-COOPr)benzyl-D-Ala-Pyr-NH-$CH_2$-5-(3-ham)-thioph |
| 707. | (p-COOiPr)benzyl-D-Ala-Pyr-NH-$CH_2$-5-(3-ham)-thioph |
| 708. | (p-COOtBu)benzyl-D-Ala-Pyr-NH-$CH_2$-5-(3-ham)-thioph |
| 709. | (p-COOCyclohexyl)benzyl-D-Ala-Pyr-NH-$CH_2$-5-(3-ham)-thioph |
| 710. | (p-COOCyclopentyl)benzyl-D-Ala-Pyr-NH-$CH_2$-5-(3-ham)-thioph |
| 711. | (p-COOMe)benzyl-D-Abu-Pyr-NH-$CH_2$-5-(3-ham)-thioph |
| 712. | (p-COOEt) benzyl-D-Abu-Pyr-NH-$CH_2$-5-(3-ham)-thioph |
| 713. | (p-COOPr)benzyl-D-Abu-Pyr-NH-$CH_2$-5-(3-ham)-thioph |
| 714. | (p-COOiPr)benzyl-D-Abu-Pyr-NH-$CH_2$-5-(3-ham)-thioph |
| 715. | (p-COOtBu)benzyl-D-Abu-Pyr-NH-$CH_2$-5-(3-ham)-thioph |
| 716. | (p-COOCyclohexyl)benzyl-D-Abu-Pyr-NH-$CH_2$-5-(3-ham)-thioph |
| 717. | (p-COOCyclopentyl)benzyl-D-Abu-Pyr-NH-$CH_2$-5-(3-ham)-thioph |
| 718. | (p-COOMe)benzyl-D-Ile-Pyr-NH-$CH_2$-5-(3-ham)-thioph |
| 719. | (p-COOEt)benzyl-D-Ile-Pyr-NH-$CH_2$-5-(3-ham)-thioph |
| 720. | (p-COOPr)benzyl-D-Ile-Pyr-NH-$CH_2$-5-(3-ham)-thioph |
| 721. | (p-COOiPr)benzyl-D-Ile-Pyr-NH-$CH_2$-5-(3-ham)-thioph |
| 722. | (p-COOtBu)benzyl-D-Ile-Pyr-NH-$CH_2$-5-(3-ham)-thioph |

(fortgesetzt)

| | |
|---|---|
| 723. | (p-COOCyclohexyl )benzyl-D-Ile-Pyr-NH-$CH_2$-5-(3-ham)-thioph |
| 724. | (p-COOCyclopentyl)benzyl-D-Ile-Pyr-NH-$CH_2$-5-(3-ham)-thioph |
| 725. | (p-COOMe)benzoyl-D-Val-Pyr-NH-$CH_2$-5-(3-ham)-thioph |
| 726. | (p-COOEt)benzoyl-D-Val-Pyr-NH-$CH_2$-5-(3-ham)-thioph |
| 727. | (p-COOPr)benzoyl-D-Val-Pyr-NH-$CH_2$-5-(3-ham)-thioph |
| 728. | (p-COOiPr)benzoyl-D-Val-Pyr-NH-$CH_2$-5-(3-ham)-thioph |
| 729. | (p-COOtBu)benzoyl-D-Val-Pyr-NH-$CH_2$-5-(3-ham)-thioph |
| 730. | (p-COOCyclohexyl)benzoyl-D-Val-Pyr-NH-$CH_2$-5-(3-ham)-thioph |
| 731. | (p-COOCyclopentyl)benzoyl-D-Val-Pyr-NH-$CH_2$-5-(3-ham)-thioph |
| 732. | (p-COOMe)benzoyl-D-Ala-Pyr-NH-$CH_2$-5-(3-ham)-thioph |
| 733. | (p-COOEt)benzoyl-D-Ala-Pyr-NH-$CH_2$-5- (3-ham)-thioph |
| 734. | (p-COOPr)benzoyl-D-Ala-Pyr-NH-$CH_2$-5-(3-ham)-thioph |
| 735. | (p-COOiPr)benzoyl-D-Ala-Pyr-NH-$CH_2$-5- (3-ham)-thioph |
| 736. | (p-COOtBu)benzoyl-D-Ala-Pyr-NH-$CH_2$-5-(3-ham)-thioph |
| 737. | (p-COOCyclohexyl)benzoyl-D-Ala-Pyr-NH-$CH_2$-5-(3-ham)-thioph |
| 738. | (p-COOCyclopentyl)benzoyl-D-Ala-Pyr-NH-$CH_2$-5-(3-ham)-thioph |
| 739. | (p-COOMe)benzoyl-D-Abu-Pyr-NH-$CH_2$-5-(3-ham)-thioph |
| 740. | (p-COOEt)benzoyl-D-Abu-Pyr-NH-$CH_2$-5-(3-ham)-thioph |
| 741. | (p-COOPr)benzoyl-D-Abu-Pyr-NH-$CH_2$-5-(3-ham)-thioph |
| 742. | (p-COOiPr)benzoyl-D-Abu-Pyr-NH-$CH_2$-5-(3-ham)-thioph |
| 743. | (p-COOtBu)benzoyl-D-Abu-Pyr-NH-$CH_2$-5-(3-ham)-thioph |
| 744. | (p-COOCyclohexyl)benzoyl-D-Abu-Pyr-MH-$CH_2$-5-(3-ham)-thioph |
| 745. | (p-COOCyclopentyl) benzoyl-D-Abu-Pyr-NH-$CH_2$-5-(3-ham)-thioph |
| 746. | (p-COOMe)benzoyl-D-Ile-Pyr-NH-$CH_2$-5-(3-ham)-thioph |
| 747. | (p-COOEt)benzoyl-D-Ile-Pyr-NH-$CH_2$-5-(3-ham)-thioph |
| 748. | (p-COOPr)benzoyl-D-Ile-Pyr-NH-$CH_2$-5-(3-ham)-thioph |
| 749. | (p-COOiPr)benzoyl-D-Ile-Pyr-NH-$CH_2$-5-(3-ham)-thioph |
| 750. | (p-COOtBu)benzoyl-D-Ile-Pyr-NH-$CH_2$-5-(3-ham)-thioph |
| 751. | (p-COOCyclohexyl)benzoyl-D-Ile-Pyr-NH-$CH_2$-5-(3-ham)-thioph |
| 752. | (p-COOCyclopentyl benzoyl-D-Ile-Pyr-NH-$CH_2$-5-(3-ham)-thioph |
| 753. | (p-COOMe)benzyl-CO-D-Val-Pyr-NH-$CH_2$-5-(3-ham)-thioph |
| 754. | (p-COOEt)benzyl-CO-D-Val-Pyr-NH-$CH_2$-5-(3-ham)-thioph |
| 755. | (p-COOPr)benzyl-CO-D-Val-Pyr-NH-$CH_2$-5-(3-ham)-thioph |
| 756. | (p-COOiPr)benzyl-CO-D-Val-Pyr-NH-$CH_2$-5-(3-ham)-thioph |
| 757. | (p-COOtBu)benzyl-CO-D-Val-Pyr-NH-$CH_2$-5-(3-ham)-thioph |
| 758. | (p-COOCyclohexyl)benzyl-CO-D-Val-Pyr-NH-$CH_2$-5-(3-ham)-thioph |
| 759. | (p-COOCyclopentyl)benzyl-CO-D-Val-Pyr-NH-$CH_2$-5-(3-ham)-thioph |
| 760. | (p-COOMe)benzyl-CO-D-Ala-Pyr-NH-$CH_2$-5-(3-ham)-thioph |
| 761. | (p-COOEt)benzyl-CO-D-Ala-Pyr-NH-$CH_2$-5-(3-ham)-thioph |

(fortgesetzt)

| | |
|---|---|
| 762. | (p-COOPr)benzyl-CO-D-Ala-Pyr-NH-$CH_2$-5-(3-ham)-thioph |
| 763. | (p-COOiPr)benzyl-CO-D-Ala-Pyr-NH-$CH_2$-5-(3-ham)-thioph |
| 764. | (p-COOtBu)benzyl-CO-D-Ala-Pyr-NH-$CH_2$-5-(3-ham)-thioph |
| 765. | (p-COOCyclohexyl)benzyl-CO-D-Ala-Pyr-NH-$CH_2$-5-(3-ham)-thioph |
| 766. | (p-COOCyclopentylbenzyl-CO-D-Ala-Pyr-NH-$CH_2$-5-(3-ham)-thioph |
| 767. | (p-COOMe)benzyl-CO-D-Abu-Pyr-NH-$CH_2$-5-(3-ham)-thioph |
| 768. | (p-COOEt)benzyl-CO-D-Abu-Pyr-NH-$CH_2$-5-(3-ham)-thioph |
| 769. | (p-COOPr)benzyl-CO-D-Abu-Pyr-NH-$CH_2$-5-(3-ham)-thioph |
| 770. | (p-COOiPr)benzyl-CO-D-Abu-Pyr-NH-$CH_2$-5-(3-ham)-thioph |
| 771. | (p-COOtBu)benzyl-CO-D-Abu-Pyr-NH-$CH_2$-5-( 3-ham)-thioph |
| 772. | (p-COOCyclohexyl)benzyl-CO-D-Abu-Pyr-NH-$CH_2$-5-(3-ham)-thioph |
| 773. | (p-COOCyclopentyl )benzyl-CO-D-Abu-Pyr-NH-$CH_2$-5-(3-ham)-thioph |
| 774. | (p-COOMe)benzyl-CO-D-Ile-Pyr-NH-$CH_2$-5-(3-ham)-thioph |
| 775. | (p-COOEt)benzyl-CO-D-Ile-Pyr-NH-$CH_2$-5-(3-ham)-thioph |
| 776. | (p-COOPr)benzyl-CO-D-Ile-Pyr-NH-$CH_2$-5-( 3-ham)-thioph |
| 777. | (p-COOiPr)benzyl-CO-D-Ile-Pyr-NH-$CH_2$-5-(3-ham)-thioph |
| 778. | (p-COOtBu)benzyl-CO-D-Ile-Pyr-NH-$CH_2$-5-(3-ham)-thioph |
| 779. | (p-COOCyclohexyl)benzyl-CO-D-Ile-Pyr-NH-$CH_2$-5-(3-ham)-thioph |
| 780. | (p-COOCyclopentyl)benzyl-CO-D-Ile-Pyr-NH-$CH_2$-5-(3-ham)-thioph |
| 781. | 5-EtOOC-indanyl(1R)-CO-D-Val-Pyr-NH-$CH_2$-5-(3-ham)-thioph |
| 782. | 6-EtOOC-indanyl(1R)-CO-D-Val-Pyr-NH-$CH_2$-5-(3-ham)-thioph |
| 783. | 5-EtOOC-indanyl(1R)-D-Val-Pyr-NH-$CH_2$-5-(3-ham)-thioph |
| 784. | 6-EtOOC-indanyl(1R)-D-Val-Pyr-NH-$CH_2$-5- (3-ham) -thioph |
| 785. | 5-EtOOC-indc-inyl(1S)-CO-D-Val-Pyr-NH-$CH_2$-5-(3-ham)-thioph |
| 786. | 6-EtOOC-indanyl(1S)-CO-D-Val-Pyr-NH-$CH_2$-5-(3-ham)-thioph |
| 787. | 5-EtOOC-indanyl(1S)-D-Val-Pyr-NH-$CH_2$-5-(3-ham)-thioph |
| 788. | 6-EtOOC-indanyl(1S)-D-Val-Pyr-NH-$CH_2$-5-(3-ham)-thioph |
| 789. | 4-(Benzylamino-methyl)-benzoyl-D-Val-Pyr-NH-$CH_2$-5-(3-am)-thioph |
| 790. | 4-(Cyclohexylmethylamino-methyl)-benzoyl-D-Val-Pyr-NH-$CH_2$-5-(3-am)-thioph |
| 791. | 4-(Isobutylamino-methyl)-benzoyl-D-Val-Pyr-NH-$CH_2$-5-(3-am)-thioph |
| 792. | 4-(Isopropylamino-methyl)-benzoyl-D-Val-Pyr-NH-$CH_2$-5-(3-am)-thioph |
| 793. | 4-(Benzylamino-methyl)-benzoyl-D-Ala-Pyr-NH-$CH_2$-5-(3-am)-thioph |
| 794. | 4-(Cyclohexylmethylamino-methyl)-benzoyl-D-Ala-Pyr-NH-$CH_2$-5-(3-am)-thioph |
| 795. | 4-(Isobutylamino-methyl)-benzoyl-D-Ala-Pyr-NH-$CH_2$-5-(3-am)-thioph |
| 796. | 4-(Isopropylamino-methyl)-benzoyl-D-Ala-Pyr-NH-$CH_2$-5-(3-am)-thioph |
| 797. | 4-(Cyclohexylmethylamino-methyl)-benzoyl-D-Abu-Pyr-NH-$CH_2$-5-(3-am)-thioph |
| 798. | 4-(Benzylamino-methyl)-benzoyl-D-Abu-Pyr-NH-$CH_2$-5-(3-am)-thioph |
| 799. | 3-(Benzylamino-methyl)-benzoyl-D-Val-Pyr-NH-$CH_2$-5-(3-am)-thioph |
| 800. | 3-(Cyclohexylmethylamino-methyl)-benzoyl-D-Val-Pyr-NH-$CH_2$-5-(3-am)-thioph |

(fortgesetzt)

| | |
|---|---|
| 801. | 3-(Isobutylamino-methyl)-benzoyl-D-Val-Pyr-NH-$CH_2$-5-(3-am)-thioph |
| 802. | 3-(Isopropylamino-methyl)-benzoyl-D-Val-Pyr-NH-$CH_2$-5-(3-am)-thioph |
| 803. | 3-(Benzylamino-methyl)-benzoyl-D-Ala-Pyr-NH-$CH_2$-5-(3-am)-thioph |
| 804. | 3-(Cyclohexylmethylamino-methyl)-benzoyl-D-Ala-Pyr-NH-$CH_2$-5-(3-am)-thioph |
| 805. | 3-(Isobutylamino-methyl)-benzoyl-D-Ala-Pyr-NH-$CH_2$-5-(3-am)-thioph |
| 806. | 3-(Isopropylamino-methyl)-benzoyl-D-Ala-Pyr-NH-$CH_2$-5-(3-am)-thioph |
| 807. | 4-(Benzylamino-methyl)-phenylacetyl-D-Val-Pyr-NH-$CH_2$-5-(2-am)-thioph |
| 808. | 4-(Cyclohexylmethylamino-methyl)-phenylacetyl-D-Val-Pyr-NH-$CH_2$-5-(2-am)-thioph |
| 809. | 4-(Isobutylamino-methyl)-phenylacetyl-D-Val-Pyr-NH-$CH_2$-5-(2-am)-thioph |
| 810. | 4-(Isopropylamino-methyl)-phenylacetyl-D-Val-Pyr-NH-$CH_2$-5-(2-am)-thioph |
| 811. | 4-(Benzylamino-methyl)-phenylacetyl-D-Ala-Pyr-NH-$CH_2$-5-(2-am)-thioph |
| 812. | 4-(Cyclohexylmethylamino-methyl)-phenylacetyl-D-Ala-Pyr-NH-$CH_2$-5-(2-am)-thioph |
| 813. | 4-(Isobutylamino-methyl)-phenylacetyl-D-Ala-Pyr-NH-$CH_2$-5-(2-am)-thioph |
| 814. | 4-(Isopropylamino-methyl)-phenylacetyl-D-Ala-Pyr-NH-$CH_2$-5-(2-am)-thioph |
| 815. | 4-(Benzylamino-methyl)-phenylacetyl-D-Abu-Pyr-NH-$CH_2$-5-(2-am)-thioph |
| 816. | 4-(Cyclohexylmethylamino-methyl)-phenylacetyl-D-Abu-Pyr-NH-$CH_2$-5-(2-am)-thioph |
| 817. | 4-(Benzylamino-methyl)-phenylacetyl-D-Val-Pyr-NH-$CH_2$-5-(3-am)-thioph |
| 818. | 4-(Cyclohexylmethylamino-methyl)-phenylacetyl-D-Val-Pyr-NH-$CH_2$-5-(3-am)-thioph |
| 819. | 4-(Isobutylamino-methyl)-phenylacetyl-D-Val-Pyr-NH-$CH_2$-5-(3-am)-thioph |
| 820. | 4-(Isopropylamino-methyl)-phenylacetyl-D-Val-Pyr-NH-$CH_2$-5-(3-am)-thioph |
| 821. | 4-(Benzylamino-methyl)-phenylacetyl-D-Ala-Pyr-NH-$CH_2$-5-(3-am)-thioph |
| 822. | 4-(Cyclohexylmethylamino-methyl)-phenylacetyl-D-Ala-Pyr-NH-$CH_2$-5-(3-am)-thioph |
| 823. | 4-(Isobutylamino-methyl)-phenylacetyl-D-Ala-Pyr-NH-$CH_2$-5-(3-am)-thioph |
| 824. | 4-(Isopropylamino-methyl)-phenylacetyl-D-Ala-Pyr-NH-$CH_2$-5-(3-am)-thioph |
| 825. | 4-(Benzylamino-methyl)-phenylacetyl-D-Abu-Pyr-NH-$CH_2$-5-(3-am)-thioph |
| 826. | 4-(Cyclohexylmethylamino-methyl)-phenylacetyl-D-Abu-Pyr-NH-$CH_2$-5-(3-am)-thioph |
| 827. | 3-[4-(Benzylamino-methyl)-phenyl]-propionyl-D-Val-Pyr-NH-$CH_2$-5-(3-am)-thioph |
| 828. | 3-[4-(Cyclohexylmethylamino-methyl)-phenyl]-propionyl-D-Val-Pyr-NH-$CH_2$-5-(3-am)-thioph |
| 829. | 3-[4-(Isobutylamino-methyl)-phenyl]-propionyl-D-Val-Pyr-NH-$CH_2$-5-(3-am)-thioph |
| 830. | 3-[4-(Isopropylamino-methyl)-phenyl]-propionyl-D-Val-Pyr-NH-$CH_2$-5-(3-am)-thioph |
| 831. | 3-[4-(Benzylamino-methyl)-phenyl]-propionyl-D-Ala-Pyr-NH-$CH_2$-5-(3-am)-thioph |
| 832. | 3-[4-(Cyclohexylmethylamino-methyl)-phenyl]-propionyl-D-Ala-Pyr-NH-$CH_2$-5-(3-am)-thioph |
| 833. | 3-[4-(Isobutylamino-methyl)-phenyl]-propionyl-D-Ala-Pyr-NH-$CH_2$-5-(3-am)-thioph |
| 834. | 3-[4-(Isopropylamino-methyl)-phenyl]-propionyl-D-Ala-Pyr-NH-$CH_2$-5-(3-am)-thioph |
| 835. | 3-[4 -(Benzylamino-methyl)-phenyl]-propionyl-D-Abu-Pyr-NH-$CH_2$-5-(3-am)-thioph |
| 836. | 3-[4-(Isopropylylamino-methyl)-phenyl]-propionyl-D-Abu-Pyr-NH-$CH_2$-5-(3-am)-thioph |
| 837. | 3-[4-(Cyclohexylmethylamino-methyl)-phenyl]-propionyl-D-Abu-Pyr-NH-$CH_2$-5-(3-am)-thioph |
| 838. | 3-[4-(Benzylamino-methyl)-phenyl]-propionyl-D-Abu-Pyr-NH-$CH_2$-5-(2-am)-thioph |
| 839. | 3-[4-(Isopropylylamino-methyl)-phenyl]-propionyl-D-Abu-Pyr-NH-$CH_2$-5-(2-am)-thioph |

(fortgesetzt)

| | |
|---|---|
| 840. | 3-[4-(Cyclohexylmethylamino-methyl)-phenyl]-propionyl-D-Abu-Pyr-NH-$CH_2$-5-(2-am)-thioph |
| 841. | 3-[4-(Benzylamino-methyl)-phenyl]-propionyl-D-Ala-Pyr-NH-$CH_2$-5-(2-am)-thioph |
| 842. | 3-[4-(Isopropylamino-methyl)-phenyl]-propionyl-D-Val-Pyr-NH-$CH_2$-5-(2-am)-thioph |

Abkürzungsliste:

| | |
|---|---|
| Abu: | 2-Aminobuttersäure |
| AIBN: | Azobisisobutyronitril |
| Ac: | Acetyl |
| Acpc: | 1-Aminocyclopentan-1-carbonsäure |
| Achc: | 1-Aminocyclohexan-1-carbonsäure |
| Aib: | 2-Aminoisobuttersäure |
| Ala: | Alanin |
| β-Ala: | β-Alanin (3-Aminopropionsäure) |
| am: | Amidino |
| amb: | amidinobenzyl |
| 4-amb: | 4-amidinobenzyl (p-amidinobenzyl) |
| Arg: | Arginin |
| Asp: | Asparaginsäure |
| Aze: | Azetidin-2-carbonsäure |
| Bn: | Benzyl |
| Boc: | tert.Butyloxycarbonyl |
| Bu: | Butyl |
| Cbz: | Benzyloxycarbonyl |
| Cha: | Cyclohexylalanin |
| Chea: | Cycloheptylalanin |
| Cheg: | Cycloheptylglycin |
| Chg: | Cyclohexylglycin |
| Cpa: | Cyclopentylalanin |
| Cpg: | Cyclopentylglycin |
| d: | Dublett |
| Dab: | 2,4-diaminobuttersäure |
| Dap: | 2,3-diaminopropionsäure |
| DC: | Dünnschichtchromatographie |
| DCC: | Dicyclohexylcarbodiimid |
| Dcha: | Dicyclohexylamin |
| DCM: | Dichlormethan |
| Dhi-1-COOH: | 2,3-Dihydro-1H-isoindol-1-carbonsäure |
| DMF: | Dimethylformamid |
| DIPEA: | Diisopropylethylamin |
| EDC: | N'-(3-Dimethylaminopropyl)-N-ethylcarbodiimid |
| Et: | Ethyl |
| Eq: | Äquivalente |
| Gly: | Glycin |
| Glu: | Glutaminsäure |
| fur: | Furan |
| guan: | Guanidino |
| ham: | Hydroxyamidino |
| HCha | Homocyclohexylalanin, 2-Amino-4-cyclohexylbuttersäure |
| His: | Histidin |
| HOBT: | Hydroxybenzotriazol |
| HOSucc: | Hydroxysuccinimid |

| | |
|---|---|
| HPLC: | Hochleistungsflüssigkeitschromatographie |
| Hyp: | Hydroxyprolin |
| Ind-2-COOH: | Indolin-2-carbonsäure |
| iPr: | iso-Propyl |
| Leu: | Leucin |
| Lsg: | Lösung |
| Lys: | Lysin |
| m: | Multiplett |
| Me: | Methyl |
| MPLC: | Mitteldruckflüssigkeitschromatographie |
| MTBE: | Methyl-tert.-butyl-ether |
| NBS: | N-Bromsuccinimid |
| Nva: | Norvalin |
| Ohi-2-COOH: | Octahydroindol-2-carbonsäure |
| Ohii-1-COOH: | Octahydroisoindol-1-carbonsäure |
| Orn: | Ornithin |
| Oxaz: | Oxazol |
| p-amb: | p-amidinobenzyl |
| Ph: | Phenyl |
| Phe: | Phenylalanin |
| Phg: | Phenylglycin |
| Pic: | Pipecolinsäure |
| pico: | picolyl |
| PPA: | Propylphosphonsäureanhydrid |
| Pro: | Prolin |
| Py: | Pyridin |
| Pyr: | 3,4-Dehydroprolin |
| q: | Quartett |
| RT: | Raumtemperatur |
| RP-18 | Reversed Phase C-18 |
| s: | Singulett |
| Sar: | Sarkosin (N-Methylglycin) |
| sb: | Singulett breit |
| t: | Triplett |
| t: | tertiär |
| tBu: | tertiär-Butyl |
| tert: | tertiär |
| TBAB: | Tetrabutylammoniumbromid |
| TEA: | Trietylamin |
| TFA: | Trifluoressigsäure |
| TFFA: | Trifluoressigsäureanhydrid |
| thiaz: | Thiazol |
| Thz-2-COOH: | 1,3-Thiazolidin-2-carbonsäure |
| Thz-4-COOH: | 1,3-Thiazolidin-4-carbonsäure |
| thioph: | Thiophen |
| 1-Tic: | 1-Tetrahydroisochinolincarbonsäure |
| 3-Tic: | 3-Tetrahydroisochinolincarbonsäure |
| TOTU: | O-(Cyan-ethoxycarbonylmethylen)-amino-]-N,N,N', N'-tetramethyluroniumtetrafluoroborat |
| Z: | Benzyloxycarbonyl |

Experimenteller Teil

Die Verbindungen der Formel I lassen sich entsprechend Schemata I-III darstellen.

Die Bausteine A-B-D, E, G und K (mit K = NH-$CH_2$-$Q^K$) werden vorzugsweise separat aufgebaut und in geeignet geschützter Form eingesetzt (siehe Schemata I-III, Verwendung jeweils orthogonaler, mit der angewandten Synthesemethode kompatibler Schutzgruppen (P oder P*) .

Schema I

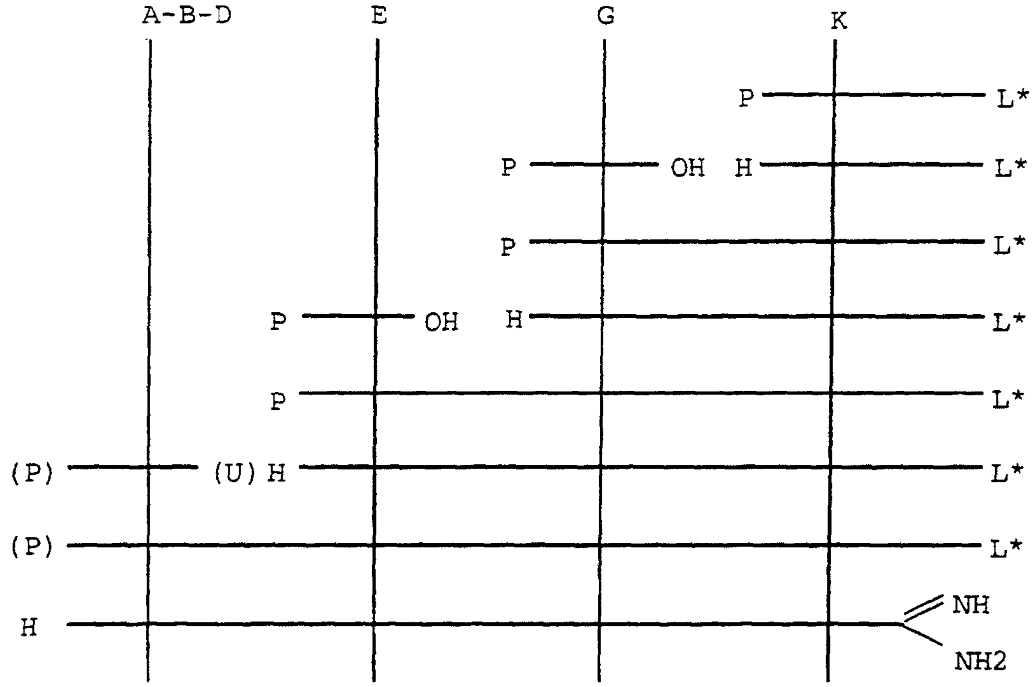

(P = Schutzgruppe, (P) = Schutzgruppe oder H)

Schema 1 beschreibt den linearen Aufbau des Moleküls I durch Schutzgruppenabspaltung von P-K-L* (L* gleich $CONH_2$, $CSNH_2$, CN, C(=NH)NH-COOR*; R* gleich Schutzgruppe oder polymerer Träger mit Spacer (Festphasensynthese)), Kupplung des Amins H-K-L* mit der N-geschützten Aminosäure P-G-OH zu P-G-K-L*, Abspaltung der N-terminalen Schutzgruppe zu H-G-K-L*, Kupplung mit der N-geschützten Aminosäure P-E-OH zu P-E-G-K-L*, Abspaltung der Schutzgruppe P zu H-E-G-K-L*, anschließende Kupplung oder Alkylierung mit dem gegebenenfalls geschützten (P)-A-B-D-U Baustein (U = Abgangsgruppe) oder reduktive Alkylierung mit (P)-A-B-D'-U (U = Aldehyd, Keton) oder Michael-Addition mit einem geeignetem (P)-A-B-D"-C=C-Derivat zu (P)-A-B-D-E-G-K-L*. Ist L* eine Amidfunktion, so kann diese auf den jeweils geschützten Stufen durch Dehydratisierung mit Trifluoressigsäureanhydrid in die entsprechende Nitrilfunktion überführt werden. Amidinsynthesen für die Benzamidin-, Picolylamidin-, Thienylamidin-, Furylamidin- und Thiazolylamidin-Verbindungen des Strukturtyps I ausgehend von den entsprechenden Carbonsäureamiden, Nitrilen, Carbonsäurethioamiden und Hydroxyamidinen sind in einer Reihe von Patentanmeldungen beschrieben (s. z.B. WO 95/35309, WO 96/178860, WO 96/24609, WO 96/25426, WO 98/06741, WO 98/09950). Anschließend werden eventuell noch vorhandene Schutzgruppen abgespalten. Ist L* gleich C(=NH)NH-Spacer-polymerer Träger, so werden diese Verbindungen im letzten Schritt vom polymeren Träger abgespalten und somit die Wirksubstanz freigesetzt.

Schema II

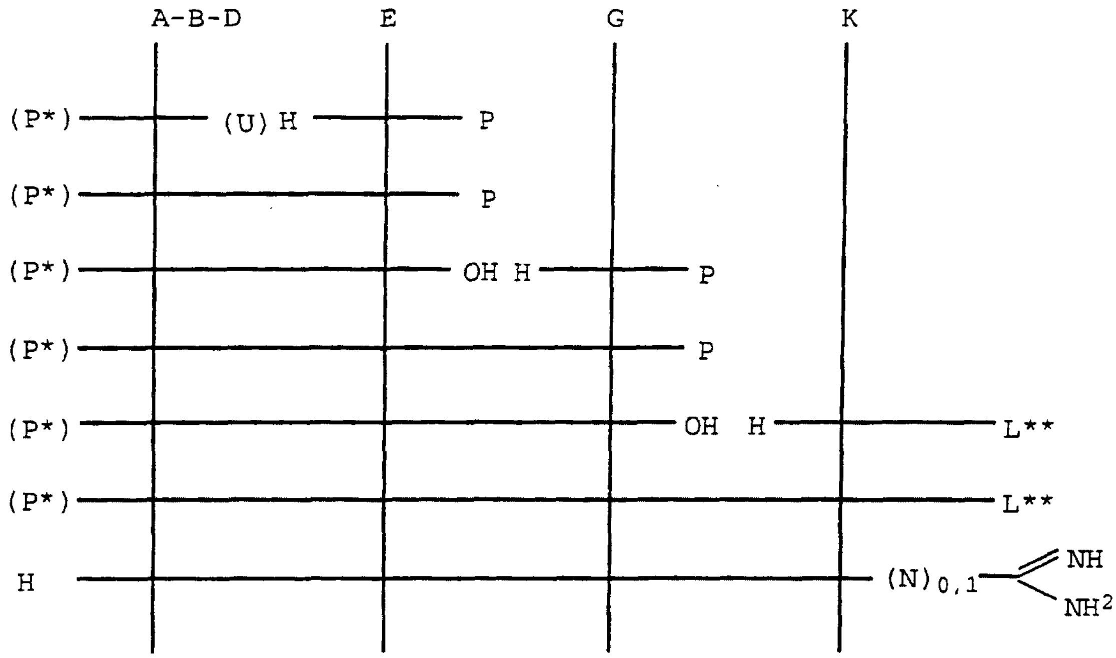


Schema II beschreibt den linearen Aufbau des Moleküls I durch Kupplung, Alkylierung, reduktive Aminierung oder Michael-Addition von H-E-P an entsprechend geeignete gegebenenfalls geschützte (P*)-A-B-D Bausteine [(P*)-A-B-D-U (U = Abgangsgruppe) bzw. (P*)-A-B-D'-U (U = Aldehyd, Keton) bzw. (P*)-A-B-D"-C=C-Derivat] zu (P*)-A-B-D-E-P. Nach Abspaltung der C-terminalen Schutzgruppe zu (P*)-A-B-D-E-OH, Kupplung mit H-G-P zu (P*)-A-B-D-E-G-P, erneute Abspaltung der C-terminalen Schutzgruppe zu (P*)-A-B-D-E-G-OH und Kupplung mit H-K-L** (L** gleich CONH2, CSNH2, CN, NH-C(=NH)$NH_2$, C(=NH)NH-R**; R** gleich Wasserstoffatom oder Schutzgruppe) zu (P*)-A-B-D-E-G-K-L**. Die Umsetzung dieses Zwischenprodukts zum Endprodukt erfolgt analog Schema I. Die Synthesesequenz nach Schema II eignet sich ebenfalls für eine Festphasensynthese, wenn der A-B-D Baustein eine entsprechende Ankerfunktion wie z.B. eine Carbonsäure- oder Aminofunktion aufweist.

Schema III

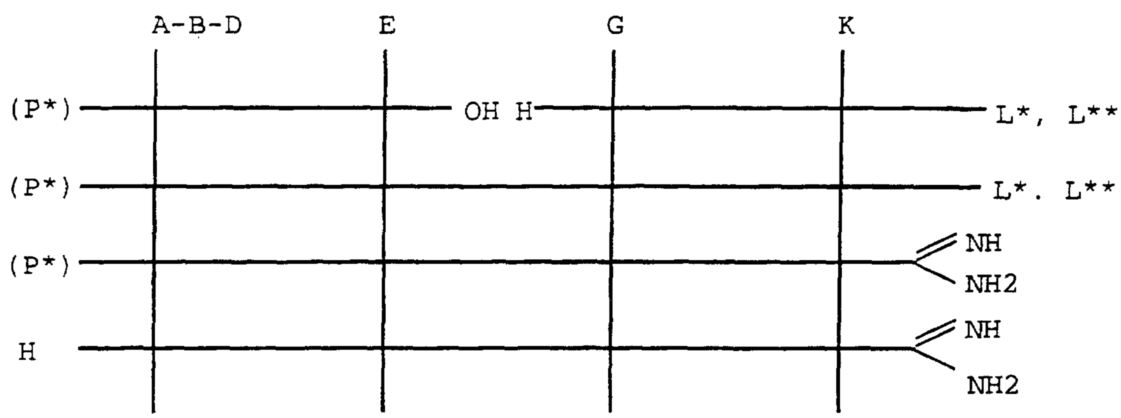


Schema III beschreibt einen sehr effizienten Weg zur Darstellung der Verbindungen I durch eine konvergente Synthese. Die entsprechend geschützten Bausteine (P*)-A-B-D-E-OH und H-G-K-L* bzw. H-G-K-L** werden miteinander gekuppelt und die entstandenen Zwischenprodukte (P*)-A-B-D-E-G-K-L* bzw. (P*)-A-B-D-E-G-K-L** Schema I zum Endprodukt umgesetzt.

Als N-terminale Schutzgruppen werden Boc, Cbz oder Fmoc eingesetzt, C-terminale Schutzgruppen sind Methyl, tert.-Butyl und Benzylester. Amidinschutzgruppen sind vorzugsweise BOC, Cbz und davon abgeleitete Gruppen für die Festphasensynthese. Enthalten die Zwischenprodukte olefinische Doppelbindungen so sind Schutzgruppen, die hydrogenolytisch abgespalten werden, ungeeignet. Die erforderlichen Kupplungsreaktionen sowie die üblichen Reaktionen der Schutzgruppeneinführung und -abspaltung werden nach Standardbedingungen der Peptidchemie durchgeführt (siehe M. Bodanszky, A. Bodanszky "The Practice of Peptide Synthesis", 2. Auflage, Springer Verlag Heidelberg, 1994).

Boc-Schutzgruppen werden mittels Dioxan/HCl oder TFA/DCM, Cbz-Schutzgruppen hydrogenolytisch oder mit HF, Fmoc- Schutzgruppen mit Piperidin abgespalten. Die Verseifung von Esterfunktionen erfolgt mit LiOH in einem alkoholischen Lösungsmittel oder in Dioxan/Wasser. t-Butylester werden mit TFA oder Dioxan/HCl gespalten.

Die Reaktionen wurden mittels DC kontrolliert, wobei üblicherweise folgende Laufmittel benutzt wurden:

| | | |
|---|---|---|
| A. | DCM/MeOH | 95:5 |
| B. | DCM/MeOH | 9:1 |
| C. | DCM/MeOH | 8:2 |
| D. | DCM/MeOH/50%ig HOAc | 40:10:5 |
| E. | DCM/MeOH/50%ig HOAc | 35:15:5 |

Sofern säulenchromatographische Trennungen erwähnt werden, waren dies Trennungen über Kieselgel, für die die oben genannten Laufmittel verwendet wurden.

Reversed phase HPLC Trennungen wurden mit Acetonitril/Wasser und HOAc Puffer durchgeführt.

Die Ausgangsverbindungen lassen sich nach folgenden Methoden herstellen:

A-B-D Bausteine:

Die als A-B-D Bausteine eingesetzten Verbindungen sind größtenteils kommerziell erhältlich wie z.B. α-Bromessigsäure-tert.-butylester, Methylsulfonsäurechlorid, Benzylsulfonsäurechlorid, 4-Chlorsulfonyl-benzoesäure, Zimtsäure, Hydrozimtsäure, 5-Bromvaleriansäure, Phenylpropiolsäure, 4-Phenylbuttersäure, 5-Phenylvaleriansäure, 4-Phenylbenzoesäure, 4-Biphenylessigsäute, etc. Soweit diese Verbindungen mehrere funktionelle Gruppen aufweisen werden an den notwendigen Stellen Schutzgruppen eingeführt. Gegebenenfalls werden funktionelle Gruppen in Reaktiv- oder Abgangsgruppen umgewandelt (z.B. Aktivester, gemischte Anhydride, Sulfonsäurechloride, etc.), um eine entsprechende chemische Verknüpfung mit den anderen Bausteinen zu ermöglichen.

Die Synthese der E-Bausteine wurde wie folgt durchgeführt:

Die als E-Bausteine eingesetzten Verbindungen Glycin, (D) bzw (L)-Alanin, (D) bzw (L)-Valin, (D)-Phenylalanin, (D)-Cyclohexylalanin, (D)-Cycloheptylglycin, etc. sind entweder als freie Aminosäuren, als Boc-geschützte Verbindungen oder als entsprechende Methylester käuflich zu erwerben.

Die Darstellung von Cycloheptylglycin und Cyclopentylglycin erfolgte durch Umsetzung von Cycloheptanon bzw. Cyclopentanon mit Isonitrilessigsäureethylester entsprechend bekannter Vorschriften (H.-J. Prätorius, J. Flossdorf, M.Kula, Chem. Ber. 1985, 108, 3079 oder U. Schöllkopf und R. Meyer, Liebigs Ann. Chem. 1977, 1174).

Die genannten Aminosäuren wurden nach allgemein bekannten Verfahren je nach Bedarf entweder N oder C-terminal mit einer Schutzgruppe versehen.

Die Synthese der G-Bausteine wurde wie folgt durchgeführt:

Die als G-Bausteine eingesetzten Verbindungen (L)-Prolin, (L)-3-Methylprolin, (L)-5-Methylprolin, (L)-3,4-Dehydroprolin, (L), (L)-Thiazolidin-4-carbonsäure und (L)-Azetidincarbonsäure sind entweder als freie Aminosäuren, als Boc-geschützte Verbindungen oder als entsprechende Methylester käuflich zu erwerben (-)-Thiazolidin-2-carbonsäuremethylester wurde nach R.L. Johnson, E.E. Smissman, J. Med. Chem. 21, 165 (1978) dargestellt.

Die Synthese der K-Bausteine wurde wie folgt durchgeführt:

3-(6-Cyano)-picolylamin

Die Darstellung dieses Bausteins wurde wie in WO 96/25426 bzw. WO 96/24609 beschrieben, durchgeführt.

5-Aminomethyl-2-cyanothiophen

Die Darstellung dieses Bausteins wurde wie in WO 95/23609 beschrieben, durchgeführt.

5-Aminomethyl-3-cyanothiophen

Die Darstellung dieses Bausteins wurde wie in WO 96/17860 beschrieben, durchgeführt.

2-Aminomethyl-thiazol-4-thiocarboxamid

Die Darstellung erfolgte entsprechend G. Videnov, D. Kaier, C. Kempter und G. Jung Angew. Chemie (1996) 108, 1604, wobei die dort beschriebene N-Boc-geschützte Verbindung mit etherischer Salzsäure in Methylenchlorid entschützt wurde.

5-Aminomethyl-2-cyanofuran

Die Darstellung dieses Bausteins wurde wie in WO 96/17860 beschrieben, durchgeführt.

5-Aminomethyl-3-cyanofuran

Die Darstellung dieses Bausteins wurde wie in WO 96/17860 beschrieben, durchgeführt

5-Aminomethyl-3-methylthiophen-2-carbonitril

a) 5-Formyl-3-methylthiophen-2-carbonitril:
Zu einer auf -78°C gekühlten Lösung von 25,1 ml (179 mmol) Diisopropylamin in 400 ml Tetrahydrofuran gab man innerhalb von 20 min 112 ml (179 mmol) einer 1,6 molaren Lösung von n-Butyllithium in n-Hexan. Die Lösung ließ man auf -35°C kommen, kühlte erneut auf -78°C und tropfte bei dieser Temperatur langsam eine Lösung von 20,0 g (162 mmol) 2-Cyano-3-methylthiophen in 80 ml Tetrahydrofuran hinzu. Die Lösung färbte sich dabei dunkelrot. Man ließ 45 min nachrühren, tropfte langsam 63 ml (811 mmol) Dimethylformamid hinzu und ließ erneut 30 min rühren. Zur Aufarbeitung versetzte man bei -70°C mit einer Lösung von 27 g Zitronensäure in 160 ml Wasser. Man engte am Rotationsverdampfer ein, versetzte mit 540 ml gesättigter Natriumchloridlösung und extrahierte dreimal mit je 250 ml Diethylether. Die vereinigten organischen Extrakte wurden über Magnesiumsulfat getrocknet.
Nach dem Abfiltrieren des Trockenmittels wurde das Lösungsmittel im Wasserstrahlvakuum abdestilliert und der Rückstand säulenchromatographisch gereinigt (Laufmittel Hexan/Essigester 4/1). Man erhielt 23 g (94 %) der Titelverbindung. $^1$H-NMR (270 MHz, DMSO-$d_6$): $\delta$ = 2,4 (s, 3H), 8,0 (s, 1H), 9,8 (s, 1H).

b) 5-Hydroxymethyl-3-methylthiophen-2-carbonitril:
Zu einer Lösung von 23 g (152 mmol) 5-Formyl-3-methylthiophen-2-carbonitril in 300 ml absolutem Ethanol wurden bei Raumtemperatur portionsweise 5,75 g (152 mmol) Natriumborhydrid gegeben. Man rührte 5 Minuten, engte das Reaktionsgemisch im Wasserstrahlvakuum ein, nahm in Essigester auf, extrahierte mit 5%iger Zitronensäurelösung und mit gesättigter Natriumchloridlösung, trocknete die organische Phase über Magnesiumsulfat, filtrierte das Trockenmittel ab und destillierte das Lösungsmittel im Wasserstrahlvakuum bei Raumtemperatur ab. Man erhielt auf diese Weise 24 g der Titelverbindung als dunkelrotes Öl, das noch Lösungsmittel enthielt und ohne weitere Reinigung in die folgenden Umsetzungen eingesetzt wurde $^1$H-NMR (270 MHz, DMSO-$d_6$): $\delta$ = 2,4 (s, 3H), 4,7 (m, 2H), 5,9 (m, 1H), 7,0 (s, 1H).

c) 5-Brommethyl-3-methylthiophen-2-carbonitril:
Zu einer Lösung von 24 g (152 mmol) 5-Hydroxymethyl-3-methylthiophen-2-carbonitril in 180 ml Tetrahydrofuran wurden 44 g (167 mmol) Triphenylphosphin gegeben. Man gab dann eine Lösung von 55 g (167 mmol) Tetrabrommethan in 100 ml Tetrahydrofuran hinzu. Man ließ 90 min lang bei Raumtemperatur rühren. Die Reaktionsmischung wurde am Rotationsverdampfer im Wasserstrahlvakuum eingeengt und der Rückstand säulenchromatographisch gereinigt (Laufmittel Hexan: Essigester 8:2). Man erhielt 34 g der Titelverbindung, die noch ein wenig Lösungsmittel enthielt. $^1$H-NMR (270 MHz, DMSO-$d_6$): $\delta$ = 2,4 (s, 3H), 5,0 (s, 2H), 7,3 (s, 1H).

d) 5-N,N-Bis(tert.-butoxycarbonyl)aminomethyl-3-methylthiophen-2-carbonitril:
Eine auf 0°C gekühlte Lösung von 33,8 g (152 mmol) 5-Bromomethyl-3-methylthiophen-2-carbonitril in 255 ml Tetrahydrofuran wurde portionsweise mit 5,0 g (167 mmol) Natriumhydrid (80%ige Suspension in Mineralöl) versetzt. Anschließend wurde eine Lösung von 36,4 g (167 mmol) Di-tert.-butyl-iminodicarboxylat in 255 ml Tetrahydrofuran hinzugetropft, wobei die Temperatur 5°C nicht überstieg. Man ließ auf Raumtemperatur kommen und über Nacht rühren. Man erwärmte zur Vervollständigung des Umsatzes noch drei Stunden lang auf 35°C, ließ danach auf Raumtemperatur abkühlen und versetzte langsam mit 510 ml einer gesättigten Ammoniumchloridlösung. Das Lösungsmittel wurde im Wasserstrahlvakuum abdestilliert, der Rückstand mehrere Male mit Essigsäureethylester extrahiert, die vereinigten organischen Phasen mit gesättigter Natriumchloridlösung gewaschen, über

Magnesiumsulfat getrocknet und am Rotationsverdampfer eingeengt. Man erhielt 57,6 g eines öligen Rückstandes, der noch Di-tert.-butyl-iminodicarboxylat enthielt und als Rohprodukt in die folgende Umsetzung eingesetzt wurde. $^1$H-NMR (270 MHz, DMSO-$d_6$): δ = 1,45 (s, 18H), 2,35 (s, 3H), 4,85 (s, 2H), 7,05 (s, 1H).

e) 5-Aminomethyl-3-methylthiophen-2-carbonitril Hydrochlorid:
52,6 g 5-N,N-Bis(tert.-butoxycarbonyl)aminomethyl-3-methylthiophen-2-carbonitril (Rohprodukt aus d), maximal 139 mmol) wurden in 950 ml Essigsäureethylester gelöst und auf 0°C gekühlt. Man sättigte mit Chlorwasserstoffgas, wobei nach 10 min ein weißer Niederschlag ausfiel. Man rührte zwei Stunden lang bei Raumtemperatur, eine Stunde lang bei 30°C, engte die entstandene Suspension anschließend am RotationsVerdampfer ein, rührte den Rückstand mit Diethylether aus, filtrierte vom Lösungsmittel ab und trocknete den festen Rückstand bei Raumtemperatur im Vakuum. Man erhielt 24,7 g (94 %) der Titelverbindung als weißes Pulver. $^1$H-NMR (270 MHz, DMSO-$d_6$): δ = 2,4 (s, 3H), 4,25 (s, 2H), 7,3 (s, 1H), 8,8-9,0 0 (bs, 3H). $^{13}$C-NMR (DMSO-$d_6$): 15,0 ($CH_3$), 36,4 ($CH_2$), 104,8 (C-2), 113,8 (CN), 131,5 (C-4), 142,8 (C-5), 149,6 (C-3).

5-Aminomethyl-3-chlorthiophen-2-carbonitril-Hydrochlorid

Die Darstellung dieser Verbindung erfolgte analog 5-Aminomethyl-3-methylthiophen-2-carbonitril, wobei das eingesetzte 3-Chlor-2-cyanothiophen durch Dehydratisierung von 3-Chlorthiophen-2-carboxamid (Substanz ist käuflich zu erwerben) mit Trifluoressigsäureanhydrid hergestellt wurde.

5-Aminomethyl-4-methylthiophen-3-thiocarboxamid

a) 2-Amino-3-cyan-4-methylthiophen-5-carbonsäureethylester
2-Amino-3-cyan-4-methylthiophen-5-carbonsäureethylester wurde nach "Organikum", 19. Aufl., Dt. Verlag der Wissenschaften, Leipzig, Heidelberg, Berlin, 1993, Kap. 6, S. 374-375, ausgehend von 130 g (1,0 mol) Acetessigsäureethylester, 66 g (1,0 mol) Malonsäuredinitril, 32 g (1,0 mol) Schwefel und 80 g (0,92 mol) Morpholin synthetisiert. $^1$H-NMR (270 MHz, DMSO-$d_6$): δ = 1,25 (t, 3H), 2,3 (s, 3H), 4,2 (q, 2H), 7,9 (bs, 2H).

b) 4-Cyan-3-methylthiophen-2-carbonsäureethylester
Eine Lösung von 20,5 g (97,5 mmol) 2-Amino-3-cyan-4-methylthiophen-5-carbonsäureethylester in 600 ml einer 1:1-Mischung aus Acetonitril und Dimethylformamid wurde auf 5°C gekühlt und tropfenweise mit 15,7 g (146 mmol) tert.-Butylnitrit versetzt, wobei sich das Reaktionsgemisch erwärmte und eine heftige Gasentwicklung einsetzte. Man rührte sieben Stunden lang bei Raumtemperatur, engte am Rotationsverdampfer und im Hochvakuum ein, reinigte den Rückstand säulenchromatographisch (Laufmittel Dichlormethan) und erhielt 9,1 g (48 %) der gewünschten Verbindung als gelbes Öl. $^1$H-NMR (270 MHz, DMSO-$d_6$): δ = 1,3 (t, 3H), 2,55 (s, 3H), 4,3 (q, 2H), 8,8 (s, 1H).

c) 5-Hydroxymethyl-4-methylthiophen-3-carbonitril:
Zu einer Lösung von 25,1 g (129 mmol) 3-Cyan-4-methylthiophen-5-carbonsäureethylester in 400 ml Tetrahydrofuran wurden bei 0°C portionsweise 2,44 g (64 mmol) Lithiumaluminiumhydrid gegeben. Man rührte fünf Stunden lang bei Raumtemperatur, vernichtete überschüssiges Reduktionsmittel durch Zugabe von 0,5 n Salzsäure, engte das Reaktionsgemisch im Wasserstrahlvakuum ein, verdünnte mit Wasser und extrahierte dreimal mit Essigester. Die vereinigten organischen Phasen wurden dann je einmal mit 0,5 n Salzsäure und gesättigter Natriumchloridlösung gewaschen. Man trocknete die organische Phase über Magnesiumsulfat, filtrierte das Trockenmittel ab und destillierte das Lösungsmittel im Wasserstrahlvakuum bei Raumtemperatur ab. Man reinigte den Rückstand säulenchromatographisch (Laufmittel Dichlormethan/Methanol 95:5) und erhielt 16.1 g (83 %) der gewünschten Verbindung als leicht gelbes Öl. $^1$H-NMR (270 MHz, DMSO-$d_6$) : δ = 2,2 (s, 3H), 4,6 (d, 2H), 5,7 (m, 1H), 8,35 (s, 1H).

d) 5-Brommethyl-4-methylthiophen-3-carbonitril:
Zu einer Lösung von 16 g (104 mmol) 5-Hydroxymethyl-4-methylthiophen-3-carbonitril in 300 ml Tetrahydrofuran wurden bei 5°C 30 g (115 mmol) Triphenylphosphin gegeben. Man gab dann eine Lösung von 38g (115 mmol) Tetrabrommethan in 100 ml Tetrahydrofuran hinzu. Man ließ über Nacht bei Raumtemperatur rühren. Die Reaktionsmischung wurde am Rotationsverdampfer im Wasserstrahlvakuum eingeengt und der Rückstand säulenchromatographisch gereinigt (Laufmittel Petrolether: Dichlormethan 1:1). Man erhielt 17 g (76 %) der Titelverbindung als gelbes Öl. $^1$H-NMR (270 MHz, DMSO-$d_6$): δ = 2,25 (s, 3H), 5,0 (s, 2H), 8,5 (s, 1H).

e) 5-N,N-Bis(tert.-butoxycarbonyl)aminomethyl-4-methylthiophen-3-carbonitril:

Eine auf 0°C gekühlte Lösung von 17,2 g (79,5 mmol) 5-Bromomethyl-4-methylthiophen-3-carbonitril in 250 ml Tetrahydrofuran wurde portionsweise mit 3.5 g (103 mmol) Natriumhydrid (ölfrei) versetzt. Anschließend wurde eine Lösung von 22,5 g (103 mmol) Di-tert.-butyl-iminodicarboxylat in 100 ml Tetrahydrofuran hinzugetropft, wobei die Temperatur 5°C nicht überstieg. Man ließ auf Raumtemperatur erwärmen und zwei Stunden lang rühren. Man versetzte langsam mit 400 ml einer gesättigten Ammoniumchloridlösung. Das Lösungsmittel wurde im Wasserstrahlvakuum abdestilliert, der Rückstand mit wenig Wasser verdünnt und dreimal mit Essigsäureethylester extrahiert. Die vereinigten organischen Phasen wurden mit gesättigter Ammoniumchloridlösung und mit gesättigter Natriumchloridlösung gewaschen, über Magnesiumsulfat getrocknet und am Rotationsverdampfer eingeengt. Man erhielt 28 g eines Öls, das noch Di-tert.-butyl-iminodicarboxylat enthielt und als Rohprodukt in die folgende Umsetzung eingesetzt wurde. $^1$H-NMR (270 MHz, DMSO-$d_6$): $\delta$ = 1,4 (s, 9H), 1,45 (s, 9H), 2,3 (s, 3H), 4,8 (s, 2H), 8,4 (s, 1H).

f) 5-N,N-Bis(tert.-butoxycarbonyl)aminomethyl-4-methylthiophen-3-thiocarboxamid

Das aus e) erhaltene Rohprodukt (max. 79 mmol) wurde in 280 ml Pyridin und 140 ml Triethylamin gelöst und bei Raumtemperatur mit Schwefelwasserstoff gesättigt. Die zuvor gelbe Lösung färbte sich grün. Man rührte über Nacht bei Raumtemperatur. Zur Vervollständigung des Umsatzes wurde nochmals 15 min Schwefelwasserstoff eingeleitet und zwei Stunden bei Raumtemperatur nachgerührt. Man trieb überschüssigen Schwefelwasserstoff mit Hilfe eines Stickstoffstromes über einen Waschturm aus. Danach wurde das Reaktionsgemisch am Rotationsverdampfer eingeengt, in Essigester aufgenommen, mehrmals mit 20%iger Natriumhydrogensulfatlösung gewaschen, über Magnesiumsulfat getrocknet und am Rotationsverdampfer eingeengt. Dabei wurden 27 g eines hellgelben festen Schaumes erhalten, der ohne weitere Reinigung in die folgende Umsetzung eingesetzt wurde. $^1$H-NMR (270 MHz, DMSO-$d_6$): $\delta$ = 1,4 (s, 18H), 2,15 (s, 3H), 4,8 (s, 2H), 7,5 (s, 1H), 9,3 (bs, 1H), 9,75 (bs, 1H).

g) 5-Aminomethyl-4-methylthiophen-3-thiocarboxamid Hydrochlorid

27 g 5-N,N-Bis(tert.-butoxycarbonyl)aminomethyl-4-methylthiophen-3-thiocarboxamid (Rohprodukt aus f), maximal 70 mmol) wurden in 400 ml Essigsäureethylester gelöst und auf 0°C gekühlt. Man sättigte mit Chlorwasserstoffgas, wobei nach 10 min ein weißer Niederschlag ausfiel. Man rührte zwei Stunden lang bei Raumtemperatur, filtrierte den Niederschlag ab, wusch ihn mit Essigsäureethylester und trocknete den festen Rückstand bei Raumtemperatur im Vakuum. Man erhielt 13,6 g (87 %) der Titelverbindung als weißes Pulver. EI-MS: $M^+$ = 186.

5-Aminomethyl-4-chlorthiophen-3-thiocarboxamid

a) 5-Formyl-4-chlorthiophen-3-carbonitril:

Eine Lösung von 53,0 g (250 mmol) 2-Amino-4-chlor-5-formylthiophen-3-carbonitril (die Darstellung dieser Verbindung ist im Patent DB 3738910 beschrieben) in 600 ml einer 1:1-Mischung aus Acetonitril und Dimethylformamid wurde bei Raumtemperatur tropfenweise mit 35 g (325 mmol) tert.-Butylnitrit versetzt, wobei sich das Reaktionsgemisch von 20°C auf 37°C erwärmte und eine kräftige Gasentwicklung einsetzte. Man kühlte auf 25°C, rührte sieben Stunden bei Raumtemperatur, engte die schwarze Lösung am Rotationsverdampfer und im Hochvakuum ein, reinigte den Rückstand säulenchromatographisch (Laufmittel Dichlormethan) und erhielt 29 g (68 %) der gewünschten Verbindung als gelbes Öl. $^1$H-NMR (270 MHz, DMSO-$d_6$) : $\delta$ = 9,1 (s, 1H), 10,0 (s, 1H).

b) 5-Hydroxymethyl-4-chlorthiophen-3-carbonitril:

Zu einer Lösung von 28,5 g (166 mmol) 5-Formyl-4-chlorthiophen-3-carbonitril in 400 ml absolutem Methanol wurden bei 5°C portionsweise 6,3 g (166 mmol) Natriumborhydrid gegeben. Das Reaktionsgemisch erwärmte sich leicht und färbte sich dunkelrot. Man beobachtete eine starke Gasentwicklung. Nach zehn Minuten engte man das Reaktionsgemisch im Wasserstrahlvakuum ein, nahm in 200 ml Essigester auf, extrahierte mit 200 ml 1 m Salzsäure, wusch zweimal mit je 250 ml Wasser und mit gesättigter Natriumchloridlösung, trocknete die organische Phase über Magnesiumsulfat, filtrierte das Trockenmittel ab und destillierte das Lösungsmittel im Wasserstrahlvakuum bei Raumtemperatur ab. Man erhielt 22 g (76 %) der Titelverbindung als dunkelrotes Öl, das ohne weitere Reinigung in die folgenden Umsetzungen eingesetzt wurde. $^1$H-NMR (270 MHz, DMSO-$d_6$): $\delta$ = 4,65 (bs, 1H), 5,95 (t, 2H), 8,6 (s, 1H).

c) 5-Brommethyl-4-chlorthiophen-3-carbonitril:

Zu einer Lösung von 21,7 g (125 mmol) 5-Hydroxymethyl-4-chlorthiophen-3-carbonitril in 250 ml Tetrahydrofuran

wurden bei 5°C 36,1 g (137 mmol) Triphenylphosphin gegeben. Man gab dann eine Lösung von 45,6 g (137 mmol) Tetrabrommethan in 100 ml Tetrahydrofuran hinzu. Man ließ über Nacht bei Raumtemperatur rühren. Man filtrierte vom Niederschlag ab, engte das Filtrat am Rotationsverdampfer im Wasserstrahlvakuum ein und reinigte den Rückstand säulenchromatographisch (Laufmittel Petrolether: Dichlormethan 1:1). Man erhielt 26,0 g (88 %) der Titelverbindung als Öl. $^1$H-NMR (270 MHz, DMSO-$d_6$): δ = 4,95 (s, 2H), 8,8 (s, 1H).

d) 5-N,N-Bis(tert.-butoxycarbonyl)aminomethyl-4-chlorthiophen-3-carbonitril:
Eine auf 0°C gekühlte Lösung von 25,0 g (106 mmol) 5-Bromomethyl-4-chlorthiophen-3-carbonitril in 300 ml Tetrahydrofuran wurde portionsweise mit 6,9 g (159 mmol) Natriumhydrid (ölfrei) versetzt. Anschließend wurde eine Lösung von 34,4 g (159 mmol) Di-tert.-butyl-iminodicarboxylat in 100 ml Tetrahydrofuran hinzugetropft, wobei die Temperatur 5°C nicht überstieg. Man ließ auf Raumtemperatur erwärmen und zwei Stunden lang rühren. Man versetzte langsam mit 300 ml einer gesättigten Ammoniumchloridlösung. Das Lösungsmittel wurde im Wasserstrahlvakuum abdestilliert, der Rückstand mit wenig Wasser verdünnt und dreimal mit Essigsäureethylester extrahiert. Die vereinigten organischen Phasen wurden mit gesättigter Ammoniumchloridlösung und mit gesättigter Natriumchloridlösung gewaschen, über Magnesiumsulfat getrocknet und am Rotationsverdampfer eingeengt. Man erhielt 51,3 g eines Öls, das noch Di-tert.-butyl-iminodicarboxylat und Lösungsmittelreste enthielt und als Rohprodukt in die folgende Umsetzung eingesetzt wurde. $^1$H-NMR (270 MHz, DMSO-$d_6$) : δ = 1,4 (s, 9H), 1,45 (s, 9H), 4,8 (s, 2H), 8,65 (s, 1H).

e) 5-N,N-Bis(tert.-butoxycarbonyl)aminomethyl-4-methylthiophen-3-thiocarboxamid
Ein Teil des aus d) erhaltenen Rohprodukts (39,4 g, max. 106 mmol) wurde in 400 ml Pyridin und 40 ml Triethylamin gelöst und bei Raumtemperatur mit Schwefelwasserstoff gesättigt. Die zuvor gelbe Lösung färbte sich grün. Man rührte über Nacht bei Raumtemperatur. Man trieb überschüssigen Schwefelwasserstoff mit Hilfe eines Stickstoffstromes über einen Waschturm aus. Danach wurde das Reaktionsgemisch in eisgekühlte, 20%ige Natriumhydrogensulfatlösung gegossen und dreimal mit Essigsäureethylester extrahiert. Die organische Phase wurde anschließend mehrmals mit 20%iger Natriumhydrogensulfatlösung gewaschen, über Magnesiumsulfat getrocknet und am Rotationsverdampfer eingeengt. Dabei wurden 49,0 g eines lösungsmittelhaltigen Rückstandes erhalten, der ohne weitere Reinigung in die folgende Umsetzung eingesetzt wurde. $^1$H-NMR (270 MHz, DMSO-$d_6$): δ = 1,4, 1,45 (s, 18H), 4,8 (s, 2H), 7,75 (s, 1H), 9,4 (bs, 1H), 10,0 (bs, 1H).

f) 5-Aminomethyl-4-chlorthiophen-3-thiocarboxamid Hydrochlorid
38,0 g des Rohprodukts aus e), maximal 93 mmol, wurden in 400 ml Essigsäureethylester gelöst und auf 0°C gekühlt. Man sättigte mit Chlorwasserstoffgas, wobei nach 10 min ein weißer Niederschlag ausfiel. Da der Umsatz noch nicht vollständig war, gab man 200 ml Essigsäureethylester hinzu, sättigte erneut mit Chlorwasserstoffgas und rührte über Nacht bei Raumtemperatur. Der Niederschlag wurde abfiltriert, mit Petrolether nachgewaschen und bei Raumtemperatur im Vakuum getrocknet. Man erhielt 21,1 g der Titelverbindung als weißes Pulver, das Ammoniumchlorid als Verunreinigung enthielt. EI-MS: $M^+$ = 206.

5-Aminomethyl-2-guanidino-thiazol-Bishydrochlorid

a) N-Phthaloyl-5-aminomethyl-2-guanidino-thiazol
Eine Lösung von 31 g (130 mmol) N-Phthaloyl-3-amino-2-chlorpropionaldehyd (S. Marchais et al., Tetrahedron Letters 39 (1998), 8085-8088) und 15,4 g (130 mmol) Amidinothioharnstoff in 200 ml Butanol wurde unter Stickstoffatmosphäre 75 min auf 110°C erhitzt, danach das Reaktionsgemisch im Vakuum (1 mbar, Badtemperatur bis 50°C) eingeengt und der Rückstand mit Methylenchlorid und konz. Ammoniak versetzt. Dabei fiel ein Teil des Produktes aus Wasser aus. Dieses wurde zusammen mit dem aus der Methylenchloridphase nach dem Trocknen und Einengen gewonnen Teil säulenchromatographisch gereinigt (Kieselgel; Fließmittel: Methylenchlorid mit von 0 bis 5 % ansteigendem Methanolgehalt). Anschließend wurden die überwiegend sauberen Fraktionen aus Aceton kristallisiert, wobei 12,3 g der Titelverbindung erhalten wurden.

b) 5-Aminomethyl-2-guanidino-thiazol-Bishydrochlorid
Eine Lösung von 5 g (16,6 mmol)N-Phthaloyl-5-Aminomethyl-2-guanidino-thiazol und 4,15 g (83 mmol) Hydrazinhydrat in 100 ml Methanol wurden unter Stickstoffatmosphäre eine Stunde bei Raumtemperatur gerührt, danach das Reaktionsgemisch im Vakuum (1 mbar, Badtemperatur bis 50°C) eingeengt, der Rückstand mit 70 ml Wasser und 20%iger Salzsäure bis pH 1 versetzt, wobei Phthalylhydrazid ausfiel, welches abfiltriert wurde. Das Filtrat wurde im Vakuum eingeengt, dreimal mit Methanol kodestilliert, bei 50°C im Vakuum getrocknet und anschließend

aus Ethanol umkristallisiert. Dabei wurden 3,7 g der Titelverbindung erhalten.

5-Amino-3-amidino-thiophen-Bishydrochlorid

Die Synthese dieser Verbindung erfolgte ausgehend von 5-Aminomethyl-3-cyanothiophen (WO 96/17860) durch Umsetzung mit $(Boc)_2O$ zu 5-t-Butyloxycarbonyl-aminomethyl-3-cyanothiophen, Umwandlung der Nitrilfunktion in das entsprechende Thioamid durch Addition von Schwefelwasserstoff, Methylierung der Thioamidfunktion mit Methyliodid, Umsetzung mit Ammoniumacetat zum entsprechenden Amidin und anschließende Schutzgruppenabspaltung mit Salzsäure in Isopropanol zum 5-Aminomethyl-3-amidino-thiophen-Bishydrochlorid.

3-Amidino-5-[*N*-1-(4,4-Dimethyl-2,6-dioxocyclohexyliden)ethyl]-aminomethylthiophen-hydrochlorid

3-Amidino-5-aminomethylthiophenbishydrochlorid (1,3 g, 5,7 mmol) wurde in DMF (15 ml) vorgelegt und mit N,N- Diisopropylethylamin (0,884 g, 6,84 mmol) versetzt. Nach 5 min Rühren bei Raumtemperatur wurden 2-Acetyldimedon (1,25 g, 6,84 mmol) und Orthoameisensäuretrimethylester (3,02 g, 28,49 mmol) zugegeben. Nach 2,5 h Rühren bei Raumtemperatur wurde das DMF im Hochvakuum entfernt und der Rückstand mit DCM (5 ml) und Petrolether (20 ml) ausgerührt. Das Lösungsmittel wurde vom leicht gelblichen Produkt abdekantiert und der Feststoff im Vakuum bei 40°C getrocknet. Ausbeute: 1,84 g (5,2 mmol, 91 %).
$^1$H-NMR (400 MHz, $[D_6]$DMSO, 25°C, TMS): $\delta$= 0,97 (s, 6H); 2,30 (s, 4H); 2,60 (s, 4H); 4,96 (d, J = 7 Hz, 2H); 7,63 (s, 1H); 8,60 (s, 1H); 9,07 (sbr, 2H); 9,37 (sbr, 1H).

Bausteinsynthesen:

A-B-D-E-OH (in entsprechend geschützter Form):

Die E-Bausteine wurden teilweise in die entsprechenden Benzylester (bzw. Methylester) überführt und mit den entsprechend geschützten A-B-D-U Bausteinen (U = Abgangsgruppe) verknüpft. Bei Verbindungen mit noch freier N-H-Funktion wurde diese anschließend mit einer Boc-Gruppe geschützt, die Benzylestergruppe abhydriert (bzw. die entsprechende Methylestergruppe hydrolisiert) und der Baustein A-B-D-E-OH durch Kristallisation, Salzfällung bzw. Säulenchromatographie gereinigt. Dieser Weg ist exemplarisch für tBuOOC-$CH_2$-(BOC) (D)Cha-OH in WO 98/06741 beschrieben.

A-B-D-E-G-OH (in entsprechend geschützter Form):

Die Darstellung des A-B-D-E-G-OH Bausteins in entsprechend geschützter Form ist exemplarisch für N-Boc-N-(tert. butyloxycarbonylmethylen)-(D)-cyclohexylalanyl-3,4-dehydroprolin in WO 98/06741 beschrieben.

H-G-K-CN:

Die Darstellung des H-G-K-CN Bausteins ist exemplarisch für Prolyl-4-cyanobenzylamid in WO 95/35309, für 3,4-Dehydroprolyl-4-cyanobenzylamid in WO 98/06740 und für 3,4-Dehydroprolyl-5-(2-cyano)-thienylmethylamid in WO 98/06741 beschrieben.

In den folgenden Beispielen werden Komplementinhibitoren aufgeführt:

Beispiel 1

$CF_3$-$CH_2$-$SO_2$-(D)Phe-Pro-NH-p-amb·$CH_3COOH$ (WO 96/17860 Bsp. 13)

Beispiel 2

n-Octyl-$SO_2$-(D)Phe-Pro-NH-p-amb·$CH_3COOH$ (WO 96/17860 Bsp. 14)

Beispiel 3

3-Py-$SO_2$-(D)Phe-Pro-NH-p-amb·$CH_3COOH$ (WO 96/17860 Bsp. 4)

Beispiel 4

$CH_3$-$SO_2$-(D)Cha-Pyr-NH-p-amb·$CH_3COOH$ (Darstellung analog WO 96/17860 Bsp. 1) FAB-MS: $(M+H^+) = 476$

Beispiel 5

H-(D)Val-Pro-NH-p-amb·2HCl (WO 95/35309 Bsp. 151)

Beispiel 6

Boc-(D)Asp(OBn)-Pro-NH-p-amb·$CH_3COOH$ (WO 95/35309 Vorstufe von Bsp. 179) FAB-MS: $(M+H^+) = 552$

Beispiel 7

2-$C_6H_{10}$-$CH_2$-Gly-Pro-NH-p-amb·2HCl (Darstellung analog WO 95/35309 Bsp. 166) FAB-MS: $(M+H^+) = 444$

Beispiel 8

$C_6H_5$-$CH_2$-$CH_2$-CO-Gly-Pro-NH-p-amb·HI (Darstellung analog WO 95/35309 Bsp. 6) FAB-MS: $(M+H^+) = 436$

Beispiel 9

$C_6H_5$-$(CH_2)_3$-CO-Gly-Pro-NH-p-amb·HI (Darstellung analog WO 95/35309 Bsp. 6) FAB-MS: $(M+H^+) = 450$

Beispiel 10

(D)(4-Me)Pic-Pro-NH-p-amb·2$CH_3COOH$ (Darstellung analog WO 95/35309 Bsp. 112) FAB-MS: $(M+H^+) = 372$

Beispiel 11

H-(D)3-Tic-Pro-NH-p-amb·2$CH_3COOH$ (WO 95/35309 Bsp. 112)

Beispiel 12

$HO_3S$-$(CH_2)_3$-(D)Phe-Pro-NH-p-amb·HCl (Die Darstellung dieser Verbindung erfolgte durch Alkylierung von H-(D)Phe-Pro-NH-$CH_2$-p$C_6H_4$-CN mit

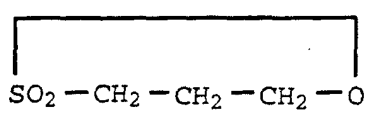 .

Die Nitrilfunktion wurde durch Hydrierung der Hydroxyamidinzwischenstufe in die Amidingruppe überführt.) FAB-MS: $(M+H^+) = 516$

Beispiel 13

$CH_3$-$SO_2$-(D)Cha-Pyr-NH-3-(6-am)-pico·$CH_3COOH$ (WO 96/24609 Bsp. 8)

Beispiel 14

$CH_3$-$SO_2$-(D)Chg-Pro-NH-3-(6-am)-pico·$CH_3COOH$

(WO 96/24609 Bsp. 6)

Beispiel 15

$C_6H_5$-$CH_2$-$SO_2$-(D)Cha-Pyr-NH-3-(6-am)-pico·$CH_3COOH$
(Darstellung analog WO 96/24609 Bsp. 8) FAB-MS: $(M+H^+)$ = 553

Beispiel 16

HOOC-$CH_2$-$SO_2$-(D)Chg-Pro-NH-3-(6-am)-pico·$CH_3COOH$
(WO 96/24609 Bsp. 10)

Beispiel 17

$CH_3OOC$-$CH_2$-$SO_2$-(D)Chg-Pro-NH-3-(6-am)-pico·$CH_3COOH$
(WO 96/24609 Zwischenprodukt bei der Darstellung von Bsp. 10)
FAB-MS: $(M+H^+)$ = 523

Beispiel 18

HOOC-$CH_2$-(D)Chg-Pyr-NH-3-(6-am)-pico·$CH_3COOH$
(Darstellung analog WO 96/25426 Bsp. 93; als Nebenprodukt in der Synthese von Bsp. 95 (WO 96/25426) beschrieben)
FAB-MS: $(M+H^+)$ = 443

Beispiel 19

HOOC-$CH_2$-HCha-Pyr-NH-3-(6-am)-pico
(Darstellung analog WO 96/25426 Bsp. 93) FAB-MS: $(M+H^+)$ = 471

Beispiel 20

Boc-NH-p-$C_6H_4CH_2$-$SO_2$-(D)Cha-Pyr-NH-3-(6-am)-pico-$CH_3COOH$

a) N-(4-Nitrobenzylsulfonyl)-(D)-cyclohexylalanin-methylester
Zu einer Lösung von 5,53 g (25 mmol) (D)-Cyclohexylalaninmethylester-hydrochlorid in 150 ml Methylenchlorid und 10 ml Acetonitril wurden bei 0°C unter Rühren 2,6 g (25 mmol) Triethylamin, 2,6 g (25 mmol) N-Methylmorpholin und eine Lösung von 5,9 g (25 mmol) p-Nitrobenzylsulfonylchlorid (J.E. Macor u.a., THL 1992, 33, 8011) in 50 ml Methylenchlorid getropft. Nach 30minütigem Nachrühren wurde die gelbe Reaktionslösung mit Wasser, 5%iger Zitronensäurelösung, 5%iger $NaHCO_3$-Lösun und nochmals mit Wasser gewaschen, über $Na_2SO_4$ getrocknet und das Lösungsmittel im Vakuum abdestilliert. Es verblieben 10 g eines leicht gelblichen Öls.

b) N-(4-Aminobenzylsulfonyl)-(D)-cylohexylalanin-methylester
Das vorstehende Öl wurde in 250 ml Methanol gelöst, mit 1,5 g 10%iger Pd/C versetzt und bei Raumtemperatur mit Wasserstoff hydriert. Nach Absaugen des Katalysators wurde das Methanol im Vakuum abdestilliert, wobei gegen Ende Kristallisation einsetzte. Der methanolfeuchte Rückstand wurde durch Lösen in Methylenchlorid und erneutes Einengen weitgehend von Methanol befreit und nach Digerieren mit Toluol-n-Hexan (1:4) abgesaugt. Man isolierte 8 g der Titelverbindung (90 % d.Th.), bezogen auf D-Cyclohexylalanin-methylester-hydrochlorid) als schwach gelbliche Kristalle, Fp. 134-136°C, DC: $CH_2Cl_2$/Aceton (9:1).

c) N-(4-t-Butoxycarbonylamino-benzylsulfonyl)-(D)-cyclohexylalanin-methylester
Eine Lösung von 7,95 g (22,45 mmol) der vorstehenden Verbindung und 5,4 g (24,7 mmol) $Boc_2O$ in 80 ml THF wurde 10 Stunden unter Stickstoff am Rückfluß gekocht. Der nach Abziehen des Lösungsmittels verbleibende dunkelbraune Rückstand wurde über eine Kieselgelsäule gereinigt (Eluent: $CH_2Cl_2$/Aceton, 50:2,5). Aus den einheitlichen Fraktionen wurden nach Behandeln mit n-Hexan 8,85 g der Titelverbindung (86,7 % d.Th.) als weiße Kristalle (Fp. 143-144°C, DC: $CH_2Cl_2$/Aceton, 47:3) isoliert.

d) N-(4-t-Butoxycarbonylamino-benzylsulfonyl)-(D)-cyclohexylalanin
Zu einer Lösung von 8,85 g (19,5 mmol) des vorstehenden Esters in 70 ml Dioxan tropfte man bei 5°C unter

Rühren 40 ml 1 n LiOH und ließ 20 Stunden bei Raumtemperatur nachrühren. Gemäß DC ($CH_2Cl_2$/Aceton, 9:1) waren noch Spuren von Ester nachweisbar. Durch Zutropfen von 1 n HCl wurde ein pH-Wert von 8 eingestellt, das Dioxan weitgehend abdestilliert und der Rückstand mit 1 Liter Wasser verdünnt. Durch Zugabe von $KHSO_4$-Lösun wurde die Wasserphase auf pH 2 eingestellt, mit 500 ml Essigester überschichtet und 2 Stunden gerührt. Die organische Phase wurde abgetrennt, mit Wasser gewaschen und mit $Na_2SO_4$ getrocknet. Der nach Abdestillieren des Lösungsmittels erhaltene Rückstand wurde zur Entfernung von Esterspuren in der Wärme mit 1,2-Dichlorethan digeriert. Nach Absaugen und Nachwaschen mit n-Hexan isolierte man 7,1 g der Titelverbindung als weiße Kristalle (Fp. 186-187°C (Zers.), DC: $CH_2Cl_2$/Aceton/Eisessig, 20:5:1).

e) BocNH-p-$C_6H_4CH_2$-$SO_2$-(D)Cha-Pyr-NH-3-(6-CN)-pico
Zu einer Suspension von 4,4 g (10 mmol) N-(4-t-Butoxycarbonylamino-benzylsulfonyl)-(D)-cyclphexylalanin und 2,7 g (10 mmol) 3,4-Dehydroprolyl-(3-(6-cyano)picolyl)-amid (hergestellt aus Boc-3,4-Dehydroprolyl(3-(6-carboxyamido)picolylamid (WO 96/25426) durch Dehydratisierung mittels Trifluoressigsäureanhydrid und anschließende Abspaltung der Boc-Gruppe) in 70 ml Methylenchlorid tropfte man bei 0°C 5,8 g Diisopropylamin gefolgt von 11 ml (15 mmol) einer 50%igen Lösung von Propanphosphorsäureanhydrid in Essigester und ließ 3 Stunden bei 0°C nachrühren.
Die organische Phase wurde mit Wasser 5%iger $NaHCO_3$- und 5%iger Zitronensäurelösung gewaschen, über $Na_2SO_4$ getrocknet und zur Trockene eingeengt. Der verbliebene ölige Rückstand wurde säulenchromatographisch gereinigt (Eluent: $CH_2Cl_2$/Aceton/Methanol, 45:5:4). Der nach Abziehen des Eluenten verbliebene Rückstand konnte durch Behandeln mit Ether in 5 g weißes Pulver, Fp. 175-180°C (Zers.), überführt werden.

f) Boc-NH-p-$C_6H_4CH_2$-$SO_2$-(D)Cha-Pyr-NH-3-(6-am)·pico-$CH_3COOH$
Eine Lösung von 3,12 g (4,8 mmol) BOC-NH-p-$C_6H_4$-$CH_2$-$SO_2$-(D)Cha-Pyr-NH-3-(6-CN)-pico und 0,94 g (5,8 mmol) L-Acetylcystein in 6 ml Methanol wurde unter Einleitung von Ammoniak 4 Stunden auf 50°C erwärmt.
Zur Entfernung des Ammoniaks wurde das Methanol abdestilliert, der Rückstand erneut in 50 ml Methanol aufgenommen und mittels eines Ionenaustauschers (Acetat auf polymeren Träger, Fluka 00402) ins Acetat überführt. Nach Abziehen des Methanols wurde der Rückstand säulenchromatographisch (Eluent: $CH_2Cl_2$/MeOH/50%ige Essigsäure, 43:7:1,5) gereinigt. Durch Behandeln des reinen Acetats mit Essigester wurden 2,25 g der Titelverbindung als schwach gelbliches Pulver erhalten, FAB-MS: 668 ($M+H^+$).

Beispiel 21

$H_2N$-p-$C_6H_4CH_2$-$SO_2$-(D)Cha-Pyr-NH-3-(6-am)-pico-HCl

1,7 g (2,3 mmol) der Verbindung von Bsp. 20 wurden in 10 ml Isopropanol und 4,5 ml 4 n Salzsäure in Dioxan gelöst und 3 Stunden auf 50°C erwärmt. Nach Abziehen des Lösungsmittels wurde der Rückstand mit Ether behandelt und das abgeschiedene amorphe Hydrochlorid abgesaugt. Dieses wurde in 200 ml Isopropanol unter Zusatz von etwas Wasser in der Wärme gelöst, die Lösung nach Zusatz von Aktivkohle filtriert und auf ein Volumen von ca. 40 ml eingeengt. Das ausgefallene Hydrochlorid der Titelverbindung wurde abgesaugt, 1,65 g schwach gelbliche Kristalle; DC: $CH_2Cl_2$/MeOH/50%ige Essigsäure, 43:7:2; FAB-MS: ($M+H^+$) = 568.

Beispiel 22

Boc-NH-p-$C_6H_4$-$CH_2$-$SO_2$-(D)Chg-Pyr-NH-3-(6-am)-pico·$CH_3COOH$ (Die Darstellung erfolgte analog Bsp. 20) FAB-MS ($M+H^+$) = 654

Beispiel 23

$H_2N$-p-$C_6H_4$-$CH_2$-$SO_2$-(D)Chg-Pyr-NH-$CH_2$-3-(6-am)-pico·HCl (Die Darstellung erfolgt ausgehend von Bsp. 22 analog Bsp. 21) FAB-MS: ($M+H^+$) = 554

Beispiel 24

HOOC-$(CH_2)_5$-(D)Chg-Pro-NH-3-(6-am)-pico·$CH_3COOH$ (Darstellung analog WO 95/35309 Bsp. 221) FAB-MS: ($M+H^+$) = 501

Beispiel 25

$C_2H_5OOC-(CH_2)_5$-(D)Chg-Pro-NH-3-(6-am)-pico·$CH_3COOH$ (Darstellung analog WO 95/35309 Bsp. 221) FAB-MS: $(M+H^+)$ = 529

Beispiel 26

HOOC-$(CH_2)_4$-(D)Chg-Pro-NH-3-(6-am)-pico·$CH_3COOH$ (Darstellung analog WO 95/35309 Bsp. 221) FAB-MS: (M#H$^+$) = 487

Beispiel 27

t-BuOOC-$(CH_2)_3$-(D)Chg-Pro-NH-3-(6-am)-pico·$CH_3COOH$ (Darstellung analog WO 95/35309 Bsp. 221 Stufe c) FAB-MS: $(M+H^+)$ = 529

Beispiel 28

$(C_6H_5-CH_2)_2$Gly-Pyr-NH-3-(6-am)-pico·$CH_3COOH$ (Darstellung analog WO 96/25426 Bsp. 33 aus $(C_6H_5-CH_2)_2$-Gly-OH und H-Pyr-NH-$CH_2$-3-(6-CN-pico) FAB-MS: $(M+H^+)$ = 483

Beispiel 29

HOOC-$CH_2$-(D)Chg-Pyr-NH-$CH_2$-5-(2-am)-thiph·$CH_3COOH$ (WO 98/06741 Bsp. 3)

Beispiel 30

HOOC-$CH_2$-$CH_2$-(D)Cha-Pro-NH-$CH_2$-5-(2-am)-tioph·$CH_3COOH$ (Darstellung analog WO 98/06741 Bsp. 1) FAB-MS: $(M+H^+)$ = 479

Beispiel 31

HOOC-$CH_2$-(D)Chg-Aze-NH-$CH_2$-5-(2-am)-thioph (Darstellung analog WO 98/06741 Bsp. 3) FAB-MS: $(M+H^+)$ = 436

Beispiel 32

HOOC-$CH_2$-(D)Cha-Pyr-NH-$CH_2$-5-(2-am)-thioph·$CH_3COOH$ (WO 98/06741 Bsp. 1)

Beispiel 33

HOOC-$CH_2$-(D)Cha-Thz-4-CO-NH-$CH_2$-5-(2-am)-thioph·2HCl (Darstellung analog WO 98/06741 Bsp. 1) FAB-MS: $(M+H^+)$ = 482

Beispiel 34

HOOC-$CH_2$-(D)Cha-Pro-NH-$CH_2$-5-(3-am)-fur·$CH_3COOH$ (Darstellung analog WO 98/06741 Bsp. 10) FAB-MS: $(M+H^+)$ = 448

Beispiel 35

HOOC-$CH_2$-(D)Chg-Pyr-NH-$CH_2$-2-(4-am)-thiaz·2HCl (WO 98/06741 Bsp. 22)

Beispiel 36

HOOC-$CH_2$-(D)Chg-Pyr-NH-$CH_2$-5-(2-am-3-Cl)-thioph·2HCl (Darstellung analog WO 98/06741 Bsp. 3) FAB-MS: $(M+H^+)$ = 482

Beispiel 37

HOOC-$CH_2$-(D)Cha-Pyr-NH-$CH_2$-5-(2-am-3-Cl)-thioph·2HCl (Darstellung analog WO 98/06741 Bsp. 1) FAB-MS: $(M+H^+)$ = 496

Beispiel 38

HOOC-$CH_2$-(D)Cha-Pyr-NH-$CH_2$-5-(3-am)-thioph·$CH_3COOH$ (WO 98/06741 Bsp. 5)

Beispiel 39

HOOC-$CH_2$-(D)Chg-Aze-NH-$CH_2$-5-(3-am)-thioph (Darstellung analog WO 98/06741 Bsp. 8) FAB-MS: $(M+H^+)$ = 436

Beispiel 40

HOOC-$CH_2$(D)Chg-Pyr-NH-$CH_2$-5-(3-am)-thioph·$CH_3COOH$ (WO 98/06741 Bsp. 8)

Beispiel 41

HOOC-$CH_2$-Cheg-Pyr-NH-$CH_2$-5-(3-am)-thioph·$CH_3COOH$ (Darstellung analog WO 98/06741 Bsp. 8) FAB-MS: $(M+H^+)$ = 462

Beispiel 42

HOOC-$CH_2$-Cpg-Pyr-NH-$CH_2$-5-(3-c-m)-thioph·$CH_3COOH$ (Darstellung analog WO 98/06741 Bsp. 8) FAB-MS: $(M+H^+)$ = 434

Beispiel 43

HOOC-$CH_2$-(D)Chg-Pro-NH-$CH_2$-5-(3-am).thioph·2HCl (Darstellung analog WO 98/06741 Bsp. 8) FAB-MS: $(M+H^+)$ = 450

Beispiel 44

HOOC-$CH_2$-(D)Cha-Pyr-NH-$CH_2$-5-(3-am)-fur·$CH_3COOH$ (WO 98/0671 Bsp. 13)

Beispiel 45

HOOC-$CH_2$-(D)Chg-Thz-2-CO-NH-$CH_2$-5-(3-am)-thioph (Darstellung analog WO 98/06741 Bsp. 5) FAB-MS: $(M+H^+)$ = 468

Beispiel 46

HOOC-$CH_2$-(D)Cha-Thz-2-CO-NH-$CH_2$-5-(3-am)-thioph·2HCl (Darstellung analog WO 98/06741 Bsp. 8) FAB-MS: $(M+H^+)$ = 482

Beispiel 49

HOOC-$CH_2$-(D)Chg-Pyr-NH-$CH_2$-5-(4-Cl-3-am)-thioph·$CH_3COOH$

(Darstellung analog WO 98/06741 Bsp. 5) FAB-MS: $(M+H^+)$ = 482

Beispiel 50

HOOC-$CH_2$(D)Cha-Pyr-NH-$CH_2$-5-(4-Cl-3-am)-thioph·$CH_3COOH$ (Darstellung analog WO 98/06741 Bsp. 8) FAB-MS: $(M+H^+)$ = 496

Beispiel 51

HOOC-$CH_2$-(D)Chg-Pyr-NH-$CH_2$-5-(4-Me-3-am)-thioph·$CH_3COOH$ (Darstellung analog WO 98/06741 Bsp. 5) FAB-MS: $(M+H^+)$ = 462

Beispiel 52

HOOC-$CH_2$-(D,L)Cpg-Pyr-NH-$CH_2$-5-(3-Me-3-am)-thioph·$CH_3COOH$ (Darstellung analog WO 98/06741 Bsp. 5) FAB-MS: $(M+H^+)$ = 448

Beispiel 53

HOOC-$CH_2$-(D)Cha-Pyr-NH-$CH_2$-5-(3-Me-2-am)-thioph·$CH_3COOH$ (Darstellung analog WO 98/06741 Bsp. 8) FAB-MS:.(M+H+) - 462

Beispiel 54

N-(Hydroxycarbonyl-methylen)-(D)-cyclohexylalanyl-3,4-dehydroprolyl-[5-(2-guanidino)-thiazolylmethyl] amid Bishydrochlorid:

a) N-(tert.Butoxycarbonyl-methylen)-(N-Boc)-(D)-cyclohexylalanyl-3,4-dehydroprolyl-[5-(2-guanidino)-thiazolmethyl]amid
7,28 g (15,15 mmol) N-(t-$BuO_2C$-$CH_2$)-(N-Boc)-(D)-Cha-Pyr-OH, 3,7 g (15,15 mmol) 5-Aminomethyl-2-guanidinothiazol-Bishydrochlorid und 7,8 g (10,3 ml 60,6 mmol) Diisopropylethylamin wurden in 90 ml Dichlormethan und 6 ml DMF vorgelegt und portionsweise mit 6,46 g (19,7 mmol) TOTU versetzt, wobei die Temperatur bei 20°C gehalten wurde. Nach 90 min, die DC Kontrolle zeigte einen vollständigen Umsatz, wurde die Reaktionsmischung im Vakuum schonend eingeengt, der Rückstand in Essigester aufgenommen, nacheinander mit Wasser, verd. Salzsäure (pH 1,5), ges. Kochsalzlösung (dreimal) extrahiert, die organische Phase über Magnesiumsulfat getrocknet und im Vakuum eingeengt. Das Rohprodukt (9,3 g) wurde säulenchromatagraphisch gereinigt (Kieselgel; Fließmittel: Methylenchlorid mit von 0 bis 5 % ansteigendem Methanolgehalt). Die fast sauberen Fraktionen (3,2 g) wurden durch Kristallisation aus einem Hexan/Ethergemisch weiter aufgereinigt, wobei 2,7 g der Titelverbindung erhalten wurden.

b) N-(Hydroxycarbonyl-methylen)-(D)-cyclohexylalanyl-3,4-dehydroprolyl-[5-(2-guanidino)-thiazolylmethyl]amid Bishydrochlorid
2,7 g (4,03 mmol) N-(tert.Butoxycarbonyl-methylen)-(N-Boc)-(D)-cyclohexylalanyl-3,4-dehydroprolyl-[5-(2-guanidino)-thiazolylmethyl]amid wurden in 190 ml Dichlormethan und 50 ml 5 M Salzsäurelösung in Ether 17 h bei Raumtemperatur gerührt, wobei ein Niederschlag ausfiel. Das Reaktionsgemisch wurde im Vakuum eingeengt, mehrfach mit Dichlormethan kodestilliert und am Schluß aus Ether/Dichlormethan 1:1 ausgerührt, wobei 2,2 g der Titelverbindung erhalten wurden.
FAB-MS $(M+H^+)$ : 478.

Beispiel 55

HOOC-p-$C_6H_4CH_2$-(D)Cha-Pyr-NH-$CH_2$-5-(3-am)-thioph

Ausgehend von D-Cyclohexylalanin-methylester-hydrochlorid wurde die Verbindung analog Beispiel 56 hergestellt.
Weißes, amorphes Pulver, FAB-MS $(M-H^+)$ = 538.
Die Zwischenstufe N-(t-Butoxycarbonyl)-N-(4-t-butoxycarbonylbenzyl)-D-cyclohexylamin konnte in kristalliner Form, Fp. 119°C, erhalten werden.

Beispiel 56

HOOC-p-$C_6H_4$-$CH_2$-(D)Chg-Pyr-NH-$CH_2$-5-(3-am)-thioph

a) N-(4-t-Butoxycarbonylbenzyl)-D-cyclohexylglycin-methylester
Eine Lösung von 10 g (48,2 mmol) D-Cyclohexylglycin-methylester-hydrochlorid, 13,1 g (38,3 mmol) 4-Brommethyl-benzoesäure-t-butylester (A. Rosowsky u.a. J. Med. Chem. 1989, 32, 709) und 15,6 g (121 mmol) Diisopropylethylamin in 50 ml Dimethylformamid wurde 16 h bei Raumtemperatur gerührt. Nach Zugabe von 300 ml Wasser wurde mit Methyl-t-butylether (MTBE) extrahiert, die organische Phase mit 5%iger Zitronensäurelösung und Wasser gewaschen, über $MgSO_4$ getrocknet und zur Trockene eingeengt. Der ölige Rückstand wurde säulenchromatographisch gereinigt (Eluent: $CH_2Cl_2$/MTBE, 50:1) und ergab 11,5 g (66 % d.Th.) der Titelverbindung als farbloses Öl.

b) N-(t-Butoxycarbonyl)-N-(4-t-butoxycarbonylbenzyl)-D-cyclohexylglycin-methylester
Eine Lösung von 11,5 g (31,8 mmol) der vorstehenden Verbindung, 10,4 g (47,7 mmol) Di-t-butyldicarbonat und 1,5 ml Diisopropylethylamin wurden unter Stickstoff 40 h bei Raumtemperatur gerührt. Das Acetonitril wurde abdestilliert, der Rückstand in MTBE aufgenommen, mit 5%iger Zitronensäurelösung und Wasser gewaschen, über $MgSO_4$ getrocknet und zur Trockene eingeengt. Der Rückstand ergab nach säulenchromatographischer Reinigung (Eluent: $CH_2Cl_2$/Aceton, 99:2) 14 g (95 % d.Th.) der Titelverbindung als farbloses Öl.

c) N-(t-Butoxycarbonyl)-N-(4-t-butoxycarbonylbenzyl)-D-cyclohexylglycin
Zu einer Lösung von 14 g (30,3 mmol) der vorstehenden Verbindung in 100 ml Dioxan tropfte man bei 10°C 60 ml 1 n Natronlauge und ließ 20 h bei 40°C nachrühren. Durch Zugabe von Zitronensäure wurde der pH-Wert der Reaktionslösung auf ca. 8 eingestellt, das Dioxan abdestilliert, die wäßrige Phase mit MTBE extrahiert, durch weitere Zugabe von Zitronensäure sauergestellt und mehrmals mit MTBE extrahiert. Die vereinigten MTBE-Extrakte wurden über $MgSO_4$ getrocknet, das Lösungsmittel abdestilliert und der Rückstand durch Behandeln mit wassergesättigtem n-Hexan kristallisiert. Ausbeute: 7,2 g der Titelverbindung (53 % d.Th.), Fp. 154°C, $R_f$ 0,39 ($CH_2Cl_2$/Methanol, 95:5).

d) N-(t-Butoxycarbonyl)-N-(4-t-butoxycarbonylbenzyl)-D-cyclohexylglycyl-3,4-dehydroprolin
Zu einer Suspension von 4,1 g (9 mmol) der vorstehenden Verbindung und 1,5 g (9 mmol) 3,4-Dehydroprolin-methylester-hydrochlorid in 40 ml $CH_2Cl_2$ tropfte man bei 0°C 5,3 g (40,5 mmol) Diisopropylethylamin gefolgt von 10 ml einer 50%igen Lösung von Propanphosphonsäureanhydrid in Essigester und ließ 2 h bei 0°C und 12 h bei Raumtemperatur nachrühren. Die Aufarbeitung erfolgte analog Bsp. 20 Stufe e). Nach säulenchromatographischer Reinigung (Eluent: $CH_2Cl_2$/Ether, 50:3) wurden 2,1 g (41,2 % d.Th.) eines schwach gelblichen, amorphen Pulvers isoliert. Die Verseifung zur Säure wurde analog Stufe c) durchgeführt, wobei eine Reaktionszeit von 3 h und eine Reaktionstemperatur von 10°C ausreichte. Es wurden 1,8 g der Titelverbindung als weißes amorphes Pulver isoliert, DC: Ether/Eisessig, 50:1.

e) N-Boc-N-(t-BuOOC-p-$C_6H_4CH_2$)-(D)Chg-Pyr-NH-$CH_2$-5-(3-am)-thiophacetat
Eine Suspension von 1,8 g (3,3 mmol) der vorstehenden Säure und 0,75 g (3,3 mmol) 5-Aminomethyl-3-amidino-thiophendihydrochlorid in 12 ml DMF wurde unter Stickstoff bei 0°C mit 0,68 g (6,6 mmol) N-Methylmorpholin versetzt. Nach portionsweiser Zugabe von 1,9 g (5,8 mmol) O-[Cyano(ethoxycarbonyl)methylenamino]-N,N,N',N'-tetramethyl-uroniumtetrafluoroborat (TOTU) trat eine klare Lösung auf, die 3 h nachgerührt wurde. Die gelbe Reaktionslösung wurde im Vakuum bei 35 bis 40°C eingeengt, der Rückstand dreimal mit Diisopropylether digeriert und nach Auflösen in Methanol mittels eines Ionenaustauschers (Acetat auf polymeren Träger, Fluka 00402) ins Acetat überführt. Nach Einengen des Eluenten wurde das Rohacetat säulenchromatographisch (Eluent: $CH_2Cl_2$/MeOH/50%ige Essigsäure, 40:10:0,5) gereinigt. Es wurden 1,8 g der Titelverbindung als weißes amorphes Pulver isoliert, FAB-MS ($M-H^+$) = 580.

f) HOOC-p-$C_6H_4CH_2$-(D)Chg-Pyr-NH-$CH_2$-5-(3-am)-thioph
1,8 g der vorstehenden Amidinverbindung wurden in 12 ml Eisessig gelöst, mit 12 ml 4 n HCl in Dioxan und 0,5 ml Wasser versetzt und 2,5 h bei Raumtemperatur stehen gelassen.
Nach Abziehen des Lösungsmittels wurde der Rückstand mit Acetonitril behandelt, wobei sich das Dihydrochlorid abschied. Dieses wurde zur Überführung in ein Monohydrochlorid in Wasser gelöst und mit einem schwach basischen Ionenaustauscher (3-X4 Resin, BioRad) auf pH 4,5 eingestellt. Die wäßrige Lösung wurde nach Behandeln

mit Aktivkohle lyophilisiert. Man erhielt 1,0 g der Titelverbindung als Lyophilisat, das durch Behandeln mit Isopropanol in einen kristallinen Zustand überführt wurde, Fp. 230-233°C (Zers.), FAB-MS ($M+H^+$) = 524.

Beispiel 57

MeOOC-p-$C_6H_4CH_2$-(D)Chg-Pyr-NH-$CH_2$-5-(3-am)-thioph·HCl

In eine Lösung von 1,1 g (2 mmol) der in Bsp. 56 beschriebenen Verbindung in 70 ml Methanol wurden 0,75 g (20 mmol) Salzsäure eingeleitet und anschließend 8 h unter Rückfluß gekocht.

Die erkaltete Lösung wurde mit einem schwach basischen Ionenaustauscher (3-X4 Resin, BioRad) auf pH 6 eingestellt, das Methanol abdestilliert und der zähe, ölige Rückstand durch Behandeln mit Acetonitril in ein absaugbares, leicht gelbliches Monohydrochlorid umgewandelt. Durch Lösen in Methanol, Behandeln mit Aktivkohle, Abdestillieren des Methanols zum Schluß unter Zusatz von Acetonitril wurden 1,9 g der Titelverbindung als weiße Kristalle isoliert, Fp. 215-220°C (Zers.), FAB-MS ($M+H^+$) = 538; DC: $CH_2Cl_2$/MeOH/50%ige Essigsäure, 20:5:1.

Beispiel 58

$H_2N$-CO-p-$C_6H_4CH_2$-(D)Chg-Pyr-NH-$CH_2$-5-(3-am)-thioph·HCl

0,6 g der vorstehenden Verbindung (Bsp. 57) wurden in 40 ml Methanol gelöst und 4 Tage unter Einleiten von Ammoniak auf ca. 45°C erwärmt. Nach Abziehen des Lösungsmittels wurde säulenchromatographisch (Eluent: $CH_2Cl_2$/MeOH/50%ige Essigsäure, 35:15:2,5) gereinigt. Der Rückstand wurde in Wasser gelöst, mit 1 n Salzsäure auf pH 2 eingestellt, zur Trockene eingeengt, der Rückstand erneut in Wasser aufgenommen, mit einem schwach basischen Ionenaustauscher auf pH 6 eingestellt und nach Behandeln mit Aktivkohle lyophilisiert. Man erhielt 0,28 g der Titelverbindung als weißes, amorphes Pulver, FAB-MS ($M-H^+$) = 523.

Beispiel 59

HOOC-m-$C_6H_4CH_2$-D(Chg)-Pyr-NH-$CH_2$-5-(3-am)-thioph

Analog Bsp. 56 wurde ausgehend von 3-Brommethyl-benzoesäure-t-butylester (N. Shirai u.a., J. Org. Chem. 1990, 55, 2767) die Titelverbindung erhalten. Weißes, amorphes Pulver, FAB-MS ($M+H^+$) = 524.

Beispiel 60

HOOC-p-$C_6H_4CH_2$-(D)Cha-Pyr-NH-3-(6-am)-pico·HCl

Die Herstellung erfolgte durch Umsetzung von N-(t-Butoxycarbonyl)-N-(4-t-butoxycarbonylbenzyl)-D-cyclohexylalanin (Bsp. 55) mit 3,4-Dehydroprolyl-(3-(6-cyano)picolyl)-amid (Bsp. 20, Stufe e), nachfolgender Amidinbildung (Bsp. 20 Stufe f) und Abspaltung der Schutzgruppen (Bsp. 56, Stufe f). Farbloses, amorphes Pulver, FAB-MS ($M+H^+$) = 533.

Beispiel 61

HOOC-p-$C_6H_4CH_2$-(D)Chg-Pyr-NH-3-(6-am)-pico·HCl

Die Herstellung erfolgte analog Bsp. 60. Das Startmaterial N-(t-Butoxycarbonyl)-N-(t-butoxycarbonylbenzyl) -D-cyclohexylglycin ist in Bsp. 56 Stufe a) bis c) beschrieben. Farbloses, amorphes Pulver, FAB-MS ($M+H^+$) = 519.

Beispiel 62

N-(4-Hydroxycarbonyl-phenylsulfonyl)-(D)-cyclohexylglycyl-3,4-dehydroprolyl-[5-(3-amidino)-thienylmethyl]amid:

Die Darstellung dieser Verbindung erfolgte durch Kupplung (PPA, Dichlormethan) von H-Pyr-NH-$CH_2$-5-(3-CN)-thioph mit BOC(D)Chg-OH zu BOC(D)Chg-Pyr-NH-$CH_2$-5-(3-CN)-thioph, Schutzgruppenabspaltung (HCl in Isopropanol)und anschließender Umsetzung (Dichlormethan, DIPEA)mit 4-HOOC-$C_6H_4$-$SO_2$Cl zu 4-HOOC-$C_6H_4$-$SO_2$-(D)Chg-Pyr-NH-$CH_2$-5-(3-CN)-thioph. Nach Umsetzung der Nitril- zur Amidinfunktion und Reinigung über MPL-Chromatographie wurde die Titelverbindung als weißes amorphes Pulver erhalten.

FAB-MS ($M+H^+$) : 574.

Beispiel 63

N-(3-Hydroxycarbonyl-phenylsulfonyl)-(D)-cyclohexylglycyl-3,4-dehydroprolyl-[5-(3-amidino)-thienylmethyl]amid:

Die Darstellung dieser Verbindung erfolgte durch Kupplung (PPA, Dichlormethan) von H-Pyr-NH-$CH_2$-5-(3-CN)-thioph mit BOC(D)Chg-OH zu BOC(D)Chg-Pyr-NH-$CH_2$-5-(3-CN)-thioph, Schutzgruppenabspaltung (HCl in Isopropanol)und anschließender Umsetzung (Dichlormethan, DIPEA)mit 3-HOOC-$C_6H_4$-$SO_2Cl$ zu 3-HOOC-$C_6H_4$-$SO_2$-(D)Chg-Pyr-NH-$CH_2$-5-(3-CN)-thioph. Nach Umsetzung der Nitril- zur Amidinfunktion und Reinigung über MPL-Chromatographie wurde die Titelverbindung als weißes amorphes Pulver erhalten.
FAB-MS ($M+H^+$): 574

Beispiel 64

t-BuOOC-p-$C_6H_4CH_2$-(D) Chg-Pyr-NH-$CH_2$-5-(3-am)-thiop-acetat

a) N-(4-t-Butocycarbonylbenzyl)-D-cyclohexylglycin
Zu einer Lösung von 29 g (80 mmol) N-(4-t-Butoxycarbonylbenzyl)-D-cyclohexylglycin-methylester (Beispiel 56, Stufe a) tropfte man bei 10°C 96,3 ml (96,3 mmol) 1 n Natronlauge und ließ 48 Stunden bei Raumtemperatur nachrühren. Nach Zugabe von weiteren 0,3 Äquivalenten 1 n NAOH wurden weitere 10 Stunden bei 50° nachgerührt. Durch Zugabe von 5 %iger Zitronensäurelösung wurde der pH-Wert der Lösung auf ca.8 eingestellt, das Dioxan abdestilliert, die wässrige Phase mit MTBE extrahiert und durch weitere von Zitronensäure sauer gestellt. Die ausgefallene Säure wurde in Essigester aufgenommen, die Wasserphase mehrmals mit Essigester nachextrahiert, die vereinigten Essigesterextrakte mit $MgSO_4$ getrocknet und anschließend das Lösungsmittel abdestilliert wobei gegen Ende die Säure auskristallisierte. Ausbeute: 17,5 g weiße Kristalle (63 % d.Th.), Fp > 225°C (Z.).

b) N-(t-Butoxycarbonyl)-3,4-dehydroprolyl-[2-(4-hydroxyamidino)-thienylmethyl]amid
Eine Suspension von 15,6 g (224,5 mmol) Hydroxylaminhydrochlorid in 300 ml Ethanol wurde mit 8 g konz. Ammoniak versetzt, 30 Minuten nachgerührt, das ausgefallene $NH_4Cl$ abgesaugt, anschließend 30 g (90 mmol) N-(t-Butoxycarbonyl)-3,4-dehydroprolyl-[2-(4-cyano)-thienylmethyl]amid (WO 98/06741, Beispiel 1 und 5) zugegeben und über Nacht bei Raumtemperatur nachgerührt. Danach war kein Ausgangsmaterial mehr nachweisbar (DC, Fließmittel: $CH_2CL_2$/MeOH,9/1 bzw.
$CH_2Cl_2$/MeOH/konz. Ammoniak, 4,5/5/0,3).
Nach Abdestillieren des Lösungsmittels wurde der Rückstand in 300 ml Methylenchlorid aufgenommen, mit Wasser und wässriger $NaHCO_3$-Lösung gewaschen und über $Na_2SO_4$ getrocknet. Nach Einengen verblieben 31,5 g (95,5 % d.Th.) amorpher Rückstand, RF 0,32 ($CH_2Cl_2$/MeOH,)/1, FAB-MS: 366 ($M^+$).

c) N-(t-Butoxycarbonyl)-3,4-dehydroprolyl-[2-(4-hydroxyamidino)-thienylmethyl]amid
31,5 g (86 mmol) der vorstehenden Hydroxyamidin-verbindung wurden unter Stickstoff in 300 ml Eisessig gelöst, bei 40 bis 50°C portionsweise mit 17 g Zinkstaub (<10μ) versetzt und 6 Stunden bei 40°C nachgerührt. Danach war kein Ausgangsmaterial mehr nachweisbar (DC, Fließmittel: $CH_2Cl_2$/Methanol,9/1).
Nach Absaugen der Feststoffe und Nachwaschen mit Eisessig wurde die Essigsäure-gegen Ende unter Zusatz von Toluol-weitgehend abdestilliert. Der Rückstand wurde in 350 ml Wasser aufgenommen, mit 1n Natronlauge auf pH 7 eingestellt und einmal mit 180 ml MTBE extrahiert. Die Wasserphase wurde nach Zugabe von 200 ml $CH_2Cl_2$ auf pH 12 eingestellt, nach Abtrennen der $CH_2Cl_2$-Phase nochmals nachextrahiert und die vereinigten $CH_2Cl_2$-Phasen über $Na_2SO_4$ getrocknet. Nach Abdestillieren verblieben 28,4 g (94 % d.Th.) amorpher Rückstand, RF 0,35 ($CH_2Cl_2$/MeOH/50 %ige Essigsäure, 12/3/1, FAB-MS: 350 ($M^+$).

c) 3,4-Dehydroprolyl-[2-(4-amidino)-thienylmethyl]amid-dihydrochlorid
28,4 g (81 mmol) des vorstehenden Amidins wurden in 450 ml Isopropanol suspendiert und unter Rühren mit 1215 ml 4n HCL in Dioxan versetzt, wobei kurzfristig eine klare Lösung auftrat aus der das Dihydrochlorid langsam ausfiel. Das Reaktionsgemisch wurde 3 Stunden bei Raumtemperatur nachgerührt, das Kristallisat abgesaugt und mit kaltem Isopropanol zuletzt MTBE gut nachgewaschen. Nach Trocknen verblieben 19,5 g (74,4 % d.Th.) hygroskopischer Dihydrochlorid,
RF 0,53 ($CH_2Cl_2$/MeOH/$H_2O$ $CF_3COOH$, 24/9/1/0,5,

FAB-MS: 250 ($M^+$), Fp 220-223°C (Z).

d) t-BuOOC-p-$C_6H_4CH_2$-(D)Chg-Pyr-NH-$CH_2$-5-(3-am)-thioph-acetat
N-(4-t-Butoxycarbonylbenzyl)-D-cyclohexylglycin (Stufe a) und 3,4-Dehydroprolyl-[2-(4-amidino)-thienylmethyl] amid-dihydrochlorid wurden analog Beispiel 56 Stufe e zum Endprodukt gekuppelt. Weißer amorpher Pulver, FAB-MS: 579 ($M^+$) .

Beispiel 65

HOOC-p-$C_6H_4CH_2$-(D)Val-Pyr-NH-$CH_2$-5-(3-am)-thioph-HCL

a) N-(4-t-Butoxycarbonylbenzyl)-D-valinmethylester
Hergestellt durch Umsetzung von D-Valinmethylesterhydrochlorid und 4-Brommethyl-benzolsäure-t-butylester analog Beispiel 56 Stufe a. Die Verbindung wurde nach chromatographischer Reinigung in 74 %iger Ausbeute erhalten,
FAB-MS: 321 ($M^+$).

b) N-(4-t-Butoxycarbonylbenzyl)-D-valin
Die Verseifung erfolgte analog Beispiel 64 Stufe a. Weiße Kristalle, Fp 224-226°C (Z), FAB-MS: 307 ($M^+$).

c) t-BuOOC-p-$C_6H_4CH_2$-(D)Val-Pyr-NH-$CH_2$-5-(3-am)-thioph-acetat
N-(4-t-Butoxycarbonylbenzyl)-D-valin und 3,4-Dehydroprolyl-[2-(4-amidino)-thienylmethyl]amid-dihydrochlorid (Beispiel 64 Stufe c) wurden analog Beispiel 56 Stufe e gekuppelt. Nach säulenchromatsgrapischer Reinigung (Eluent: $CH_2Cl_2$/MeOH/50 %ige $CH_3COOH$, 20/5/1) wurden 3,1 g weißes amorphes Pulver isoliert, FAB-MS: 539 ($M^+$).

d) HOOC-p-$C_6H_4CH_2$-(D)Val-Pyr-NH-$CH_2$-5-(3-am)-thioph-HCL
Die Verseifung des t-Butylesters erfolgt analog Beispiel 56 Stufe f. Nach Gefriertrocknung wurden 1,6 g Lyophilisat isoliert, FAB-MS: 483 ($M^+$).
Analog Beispiel 56 und 64 wurden die folgenden Verbindungen erhalten:

Beispiel 66

HOOC-m-$C_6H_4CH_2$-(D)Val-Pyr-NH-$CH_2$-5-(3-am)-thioph-HCL
Weißes amorphes Pulver, FAB-MS: 483 ($M^+$).

Beispiel 67

HOOC-P-$C_6H_4CH_2$-(D)tBu-Ala-Pyr-NH-$CH_2$-5-(3-am)-thioph-Acetat
Weißes amorphes Pulver, FAB-MS: 511 ($M^+$).

Beispiel 68

HOOC-p-$C_6H_4CH_2$-(D)tBu-Gly-Pyr-NH-$CH_2$-5-(3-am)-thioph-HCL
Weißes amorphes Pulver, FAB-MS: 497 ($M^+$).

Beispiel 69

HOOC-$_P$-$C_6H_4CH_2$-Pyr-NH-$CH_2$-5-(3-am)-thioph-HCL
Weißes amorphes Pulver, FAB-MS: 441 ($M^+$).

Beispiel 70

HOOC-m-$C_6H_4CH_2$-Gly-Pyr-NH-$CH_2$-5-(3-am)-thioph-HCL
Weißes amorphes Pulver, FAB-MS: 441 ($M^+$).

Beispiel 71

$H_2N$-p-$C_6H_4CH_2$-$SO_2$-(D)CHa-Pyr-NH-$CH_2$-(3-am)-thioph-HCL

N-(4-t-Butoxycarbonylamino-benzylsulfonyl)-D-cyclohexylcelanin (Herstellung Beispiel 20, Stufe d) und 3,4-Dehydroprolyl-[2-(4-amidino)-thienylmethyl]amid-dihydrochlorid (Beispiel 64, Stufe c) wurden analog. Beispiel 56 Stufe c gekuppelt und anschließend die t-Butoxycarbonyl-schutzgruppe analog Beispiel 21 abgespalten. Weißes, amorphes Pulver, FAB-MS: 572 ($M^+$).

Beispiel 72

$H_2N$-p-$C_6H_4CH_2$-$SO_2$-(D)CHg-Pyr-NH-$CH_2$-(3-am)-thioph-HCL
Herstellung analog Beispiel 20 und 21. Die Vorstufen N-(4-Nitrobenzylsulfonyl)-bzw. N-(4-Aminobenzylsulfonyl)-(D)-cyclohexylglycin-methylester konnten als schwach gelbliche Kristalle, Fp 137°C bzw. 181°C, erhalten werden.
Weißes, amorphes Pulver, FAB-MS: 558 ($M^+$).

Beispiel 73

$H_2N$-p-$C_6H_4CH_2$-(D)Val-Pyr-NH-$CH_2$-5-(3-am)-thioph-2HCL
Die Herstellung erfolgte analog Beispiel 20 und 21.
Vorstufen:
N-(4-Nitrobenzylsulfonyl)-(D)-valin-methylester, schwach gelbliche Kristalle, Fp 98-100°C, FAB-MS: 330 ($M^+$) ; N-(4-Aminobenzylsulfonyl)-(D)-valin-methylester schwach gelbliche Kristalle, Fp 96-98°C, FAB-MS: 300 ($M^+$) ; N-(4-t-Butoxycarbonylamino-benzylsulfonyl)-D-valinmethylester. Weiße Kristalle, Fp 150-152°C, (i-Propanol) ; N-(4-t-Butoxycarbonylamino-benzylsulfonyl)-D-valin, farblose Kristalle, Fp 177-180°C (Z.), FAB-MS: 386 ($M^+$) .
Das Endprodukt wurde als Lyophilisat isoliert, FAB-MS: 558 ($M^+$) .

Beispiel 74

$H_2N$-$SO_2$-p-$C_6H_4CH_2$-(D)Chg-Pyr-NH-$CH_2$-5-(3-am)-thioph-HCL

a) N-(4-Sulfonamidobenzyl)-D-cyclohexylglycin-mehtylester.
Zu einer Lösung von 5,2 g (25 mmol) D-Cyclohexylglycinmethylester-hydrochlorid und 5,5 g (22 mmol) 4-Brommethylbenzolsulfonsäure-amid (F. Amer. Chem. Soc. 79, 1957, 4232) in 30 ml DMF tropfte man bei Raumtemperatur 7,3 g Diisopropylethylamin, wobei die Temperatur auf 26°C anstieg. Die farblose Lösung blieb über Nacht bei Raumtemperatur stehen. Danach war kein Ausgangsmaterial mehr nachweisbar (DC, $CH_2Cl_2$/ether, 5/2).
Nach Verdünnen mit 100 ml Eiswasser wurde der ausgefallene weiße Niederschlag abgesaugt, mit Wasser nachgewaschen und in Essigester gelöst. Die Essigesterphase wurde mehrmals mit Kochsalzlösung gewaschen, über $Na_2SO_4$ getrocknet und das Lösungsmittel abdestilliert. Der Rückstand wurde aus 50 ml i-Propanol umkristallisiert. Man erhielt 4,8 g (64 % d.Th.) weiße Kristalle, Fp 113-114°C, FAB-MS: 340 ($M^+$).

b) N-(4-Sulfonamidobenzyl)-D-cyclohexylglycin
4,0 g (11,8 mmol) des vorstehenden Esters wurden in 50 ml Wasser suspendiert, durch Zugabe von 35 ml 1n NaOH in Lösung gebracht und über Nacht bei Raumtemperatur stehen gelassen. Durch Zutropfen von 10 %iger Salzsäure wurde ein pH-Wert von 5 eingestellt, wobei sich ein feiner Niederschlag abschied. Durch kurzfristiges Erwärmen auf 80°, langsames Abkühlen auf Raumtemperatur und 30 minütigem Rühren unter Eisbadkühlung wurde eine gut absaugbare Struktur erhalten. Der Niederschlag wurde nach absaugen mit kaltem Wasser chloridfrei gewaschen, anschließend mit 50 ml Aceton digeriert, nach erneutem absaugen mehrmals mit einem Aceton-Ether-Gemisch nachgewaschen und getrocknet. Es verblieben 3,6 g (93,5 % d.Th.) weißes pulvriges Material, das äußerst schwerlöslich ist.

c) $H_2N$-$SO_2$-p-$C_6H_4CH_2$-(D)Chg-Pyr-NH-$CH_2$-5-(3-am)-thioph-HCL
Die Kupplung zum Endprodukt wurde analog Beispiel 56 Stufe e durchgeführt. Es wurde 1 g Lyophilisat erhalten, FAB MS: 558 ($M^+$).

Beispiel 75

$HO_3S$-p-$C_6H_4CH_2$-(D)Chg-Pyr-NH-$CH_2$-5-(3-am)-thioph

Analog Beispiel 74 wurde 4-Brommethyl-benzol-sulfonsäure (F. Med.Chem.33, 1990, 2437) mit D-Cyclohexylglycin-methylester-hydrochlorid umgesetzt, das Reaktionsprodukt verseift und anschließend mit 3,4-Dehydroprolyl-[2-(4-amidino)-thienylmethyl]amid-dihydrochlorid gekuppelt.
Weißes, amorphes Pulver, FAB-MS: 559 ($M^+$).

Beispiel 76

HO-p-$C_6H_4CH_2$-(D)Chg-Pyr-NH-$CH_2$-5-(3-am)-thioph-2HCL

a) N-(4-t-Butoxybenzyl)-D-cyclohexylglycin-methylester
5,2 g (25 mMol) D-Cyclohexylglycin-methylester-hydrochlorid wurden in 200 ml Toluol unter leichtem Erwärmen gelöst, mit 2,6 g (25,7 mmol) Triethylamin versetzt und 1 Stunde nachgerührt. Nach Absaugen des Triethylaminhydrochlorids. Auswaschen mit Toluol wurde das Filtrat auf 70 ml eingeengt, mit 4,5 g (25 mmol) p-t-Butoxybenzaldehyd und 0,1 ml Eisessig versetzt und 2,5 Stunden am Wasserabscheider zum Rückfluß erhitzt. Das Toluol wurde im Vakuum abdestilliert, der Rückstand in 50 ml Methanol gelöst, mit 1,5 g (25 mmol) Eisessig versetzt und bei 5°C portionsweise 0,9 g Natriumcyanoborhydrid eingetragen (DC-Kontrolle: $CH_zCl_2/E_2O$, 25/1). Das Methanol wurde abdestilliert, der Rückstand mit überschüssiger 5 %iger $NaHCO_3$-Lösung versetzt und mit Ether extrahiert. Nach Waschen der Etherphase mit Kochsalzlösung, Trocknen über $Na_2SO_4$ und Abdestillieren des Ethers wurde der ölige Rückstand säulenchromatographisch gereinigt (Eluent: $CH_2Cl_2/E_2O$, 25/1).
Ausbeute: 4,3 g (51 % d.Th.) farbloses Oel; FAB-MS: 333 ($M^+$).
Analog: Beispiel 74 wurde der vorstehende Ester verseift, mit 3,4-Dehydroprolyl-[2-(4-amidino)-thienylmethyl] amiddihydrochlorid gekuppelt und die t-Butylgruppe durch Salzsäure abgespalten. Amorphes, weißes Pulver, FAB-MS: 495 ($M^+$).

Beispiel 77

HO-p-$C_6H_4CH_2$-(D)Val-Pyr-NH-$CH_2$-5-(3-am)-thioph-2HCL

Die Darstellung erfolgte analog Beispiel 76.
Weißes, amorphes Pulver, FAB-MS: 455 ($M^+$).

Beispiel 78

$HOCH_2$-p-$C_6H_4CH_2$-(D)Val-Pyr-NH-$CH_2$-5-(3-am)-thioph-HCl

Die Darstellung erfolgte ausgehend von 4-(Hydroxymethyl)-benzylchlorid (J. Org.Chem. 61, 1996, 449) analog Beispiel 76.
Weißes, amorphes Pulver, FAB-MS: 469 ($M^+$).

Beispiel 79

$O_2N$-p-$C_6H_4CH_2$-(D)Val-Pyr-NH-$CH_2$-5-(3-am)-thioph-HCL

Die Darstellung erfolgte analog Beispiel 76.
Schwach gelbliches, amorphes Pulver, FAB-MS: 484 ($M^+$).

Beispiel 80

HOOC-p-$C_6H_4CH_2$-(D)Val-Pyr-NH-$CH_2$-5-(3-am)-thioph-HCL

4-(t-Butoxycarbonyl)benzylsulfonylchlorid
Eine Suspension von 15 g (55 mol) 4-Brommethyl-benzoesäure-t-butylester und 6,95 g (55 mol) Natriumsulfit in 28,5

ml Wasser und 13,5 ml DMF wurde nach Zusatz von 0,4 g Adogen® unter Rühren 4 Stdn. auf 80-90° erhitzt. Nach Abkühlung auf RT wurden 100 ml Wasser zugesetzt, zweimal mit je 100 ml MTBE extrahiert, die Wasserphase mit 250 ml MeOH versetzt, ausgefallene Salze abgesaugt und das Filtrat - gegen Ende unter Oelpumpenvakuum - eingeengt. Der Rückstand wurde mit 200 ml MeOH digeriert, unlösliche Festbestandteile abgesaugt und das Methanol - gegen Ende nach mehrmaligem Zusatz von Ethanol/Toluol - abdestilliert. Der Rückstand (16,1 g) wurde in 200 ml $CH_2Cl_2$ suspendiert, 0,8 g Tetraethyl-benzylammonium-chlorid zugesetzt, bei 0°C 15 g Oxalsäuredichlorid zugetropft und 30 Minuten am Rückfluß gekocht. Ungelöste Anteile wurden abgesaugt, die $CH_2Cl_2$-Phase mit 5%iger $NaHCO_3$-Lösung gewaschen, über $Na_2SO_4$ getrocknet und abdestilliert. Durch Behandeln mit n-Hexan wurden 6,6 g fast weiße Kristalle isoliert, Fp 82-83°C (Z.).

Analog Beispiel 76 wurde mit D-Valinmethylesterhydrochlorid umgesetzt, zur Säure verseift, mit 3,4-Dehydroprolyl-[2-(4-amidino)-thienylmethyl]amid-dihydrochlorid gekuppelt und die t-Butylestergruppe gespalten.

Weiße, amorphe Pulver, FAB-MS: 547 ($M^+$).

Beispiel 81

HOOC-p-$C_6H_4CH_2$-$SO_2$-(D)CHg-Pyr-NH-$CH_2$-5-(3-am)-thioph-HCl

Die Herstellung erfolgte analog Beispiel 80 bzw. 76.
Weiße, amorphe Pulver, FAB-MS: 587 ($M^+$).

Beispiel 82

trans-HOOC-4-Cyclohexylmethyl-Gly-Pyr-NH-$CH_2$-5-(3-am)-tioph-2HCL

a) trans-4-[N-(o-Nitrophenylsulfonyl)]aminomethyl-cyclohexancarbonsäure
Zu einer Lösung von 14,13 g (0,09 Mol) trans-4-(Aminomethyl)-cyclohexancarbonsäure in einem Zweiphasen-System aus 90 ml 1nNaOH und 90 ml Dioxan tropfte man bei 4°C (Eisbad) simultan eine Lösung von 29,9 g (0,135 Mol) o-Nitrobenzolsulfonsäurechlorid in 150 ml Dioxan und 150 ml 1nNaOH. Nach Abklingen der leicht exothermen Reaktion wurde 30 Minuten bei Raumtemperatur nachgerührt, der ausgefallene Niederschlag abgesaugt, mit wenig Eiswasser nachgewaschen und das Filtrat im Vakuum eingeengt, wobei es zu einer weiteren Salzabscheidung kam. Die vereinigten Salzmengen wurden mit Ether digeriert, in Wasser suspendiert, mit 1m$KHSO_4$-Lösung angesäuert und mit Essigester extrahiert. Die Essigesterphase wurde mit Kochsalzlösung gewaschen, über $Na_2SO_4$ getrocknet und im Vakuum eingeengt. Der Rückstand wurde aus Acetonitril umkristallisiert. Ausbeute: 27,4 g (89 % d. Th.), Fp 179°C.

b) trans-4-[N-(o-Nitrophenylsulfonyl)]aminomethyl-cyclohexancarbonsäure-t-butylester.
Zu einer Lösung von 20,4 g (60 mmol) der vorstehenden Verbindung und 0,1 ml DMF in 350 ml $CH_2Cl_2$ tropfte man bei 0° 11,3 g (90 mMol) Oxalsäuredichlorid und erwärmte anschliessend bis zur Beendigung der Gasentwicklung. Nach Abdestillieren des Methylenchlorids - gegen Ende unter Zusatz von Toluol- wurde der Rückstand in 20 ml Methylenchlorid gelöst und unter Eiskühlung zu einer Lösung von 6,1 g (83 mmol) t-Butanol und 9,4 g (119 mmol) Pyriden in 60 ml $Ch_2Cl_2$ getropft. Das Reaktionsgemisch blieb 24 Std. bei Raumtemperatur stehen, wurde anschliessend mit 1n $KHSO_4$-Lösung, Wasser und $NaHCO_3$-Lösung gewaschen, über $Na_2SO_4$ getrocknet und abdestilliert. Der Rückstand wurde aus Cyclohexan/Essigester (95/5) umkristallisiert und ergab 9,3 g leicht gelbliche Kristalle, Fp 114°C.

c) trans-4-[N-(o-Nitrophenylsulfonyl)-N-(methoxycarbonylmethyl)]aminomethyl-cyclohexancarbonsäure-t-butylester
Eine Lösung von 2,68 g (6,7 mmol) der vorstehenden Verbindung und 1.23 g (7,6 mmol) Bromessigsäuremethylester in 50 ml DMF wurde unter Zusatz von 1,85 g (13,4 mmol) $K_2CO_3$-Pulver über Nacht bei Raumtemperatur gerührt (DC: Essigester/n-Hesan, 1/1). Das Reaktionsgemisch wurde mit 100 ml Wasser versetzt, mehrmals mit Essigester extrahiert, die vereinigten Essigesterextrakte mit Kochsalzlösung gewaschen, über $Na_2SO_4$ getrocknet und abdestilliert. Nach säulenchromatographischer Reinigung (Eluent: Essigester/n-Hexan, 1/1) und Kristallisation aus Ether/n-Hexan wurden 2,6 g (82,3 % d. Th) gelbliche Kristalle, Fp 123-124°C erhalten.

d) trans-4-[N-(o-Nitrophenylsulfonyl)-N-(hydroxycarbonylmethyl)]aminomethyl-cyclohexancarbonsäure-t-butylester

Analog Beispiel 20 Stufe d wurde die Methylestergruppe der vorstehenden Verbindung verseift. Zäher gelbes Oel, FAB-MS: 456 ($M^+$), DC: Essigester7n-Hexan/Eisessig, 34/15/1,5.

e) trans-t-Bu00C-4-cyclohexylmethyl-(o-$NO_2$-$C_6H_4SO_2$)Gly-Pyr-NH-$CH_2$-5-(3-CN)-thioph
Analog Beispiel 20 Stufe e wurde die vorstehende Säure mit 3,4-Dehydroprolyl-[2-(4-cyano)thienylmethyl]amidhydrochlorid gekuppelt. Amorpher, gelblicher Rückstand, FAB-MS: 671 ($M^+$), DC: $CH_2Cl_2$/Aceton/Methanol, 45/5/1.

f) trans-t-Bu00C-4-cyclohexylmethyl-Gly-Pyr-NH-$CH_2$-5-(3-N)-thioph
Eine Lösung von 3,5 g (5,5 mmol) der vorstehenden Verbindung und 0,7 g (6,35 mmol) Thiophenol in 10 ml DMF wurden unter Zusatz von 2,5 g (18.1 mmol) $K_2CO_3$-Pulver über Nacht bei Raumtemperatur gerührt. Das gelbe Reaktionsgemisch wurde mit 100 ml Eiswasser versetzt, 4 x mit je 35 ml Essigester extrahiert, die Essigesterextrakte mit Kochsalzlösung gewaschen, über $Na_2SO_2$ getrocknet und das nach Abdestillieren des Lösungsmittels erhaltene zähe gelbe Oel säulenchromatographisch gereinigt (Eluent: $CH_2Cl_2$/Methanol, 50/4). 2,3 g gelblich amorpher Rückstand, FAB-MS: 486 ($M^+$).

g) trans-HOOC-4-Cyclohexylmethyl-Gly-Pyr-NH-5-(3-am)-thioph-2 HCl
Die Amidinbildung erfolgte analog Beispiel 64 Stufe b und c. Die Verseifung des t-Butylesters wurde mit 4n Salzsäure in Dioxan durchgeführt. 1.1 g Lyophilisat, FAB-MS: 447 ($M^+$), DC: $CH_2Cl_2$/MeOH/50 % iger Eisessig, 35/15/6.

Beispiel 83

trans-HOOC-4-Cyclohexylmethyl-(D)Chg-Pyr-NH-$CH_2$-5-(3-am)-thioph-2HCL

Zu einer Lösung von 1,72 g (10 mmol) S-Hexahydromandelsäuremethylester tropfte man bei -8°C unter Rühren 1,9 ml (11 mmol) Trifluormethansulfonsäureanhydrid und anschliessend 1,2 g (11 mmol) 2,6-Lutidin. Nach 20 minütigem Nachrühren bei 0°C (DC: $Et_2O$/n-Hexan, 3/2) wurde eine Lösung von 5,3 g (24,9 mmol) trans-4-(Aminomethyl)-cyclohexancarbonsäure-t-butylester und 2,6 g (20 mmol) Diisopropylethylamin in 20 ml $CH_2Cl_2$ zugetropft, weiter 2 Std. bei 0° und über Nacht bei RT nachgerührt (DC: $CH_2Cl_2$/Ether, 25/3).

Die Reaktionslösung wurde mit Wasser, 2x mit je 10 ml 1n Salzsäure, 5%iger $NaHCO_3$-Lösung gewaschen, über $Na_2SO_4$ getrocknet, abdestilliert und der Rückstand säulenchromatographisch gereinigt (Eluent: $CH_2Cl_2$/Ether, 10/1). Es wurden 2,7 g eines leicht gelblichen Oels isoliert, das analog Beispiel 56 Stufe c zur Säure verseift und anschliessend analog Stufe e mit 3.4-Dehydroprolyl[2-[4-amidino)thienylmethyl]-amid-dihydrochlorid gekuppelt wurde. Nach Hydrolyse der t-Butylestergruppe mit 4n Salzsäure in Dioxan wurde der Rückstand gefriergetrocknet, leicht gelbliches amorphes Pulver, FAB-MS: 529 ($M^+$), DC: $CH_2Cl_2$/MeOH/50%ige Essigsäure, 35/15/3.

Beispiel 84

4-Benzoylbenzoyl-Ala-Pro-5-(3-am)-thioph

a) 3 g (1,62 mmol) p-Nitrophenylcarbonat-Wang-Harz (Novabiochem, Substitution 0,54 mmol/g) wurden in 20 mL DMF suspendiert und mit 1,15 g (3,24 mmol) 4-Amidino-2-[*N*-1-(4,4-Dimethyl-2,6-dioxocyclohexyliden)ethyl]-aminomethylthiophen-hydrochlorid und 4,48 ml (32,4 mmol) Triethylamin 4 Tage bei Raumtemperatur geschüttelt. Es wurde abgesaugt und mit DMF, $CH_2Cl_2$, Methanol und $CH_2Cl_2$ gewaschen. Anschließend wurde das Harz mit 0,5M $NH_4OAc$-Lösung in Methanol behandelt (3*10 min), mit Methanol, DMF und $CH_2Cl_2$ gewaschen und im Vakuum bei Raumtemperatur getrocknet. Zur Abspaltung der Dde-Schutzgruppe wurde das Harz mit 20 ml einer 2%igen Lösung von Hydrazinhydrat in DMF bei Raumtemperatur 5 Minuten behandelt. Es wurde abgesaugt und mit DMF gewaschen. Die Abspaltung wurde zweimal wiederholt. Anschließend wurde das Harz mit DMF, $CH_2Cl_2$, Methanol und $CH_2Cl_2$ gewaschen und im Vakuum bei Raumtemperatur getrocknet. (Auswaage: 2,84 g).

b) 0,044 mmol Harz aus a), 0,088 mmol Fmoc-Pro-OH und 0,088 mmol N,N,-Diisopropylethylamin in 1,5 ml Dimethylformamid wurden bei Raumtemperatur mit einer Lösung von 0,088 mmol 2(1H-Benzotriazol-1-yl-)1,1,3,3-tetramethyluronium-tetrafluoroborat in 0,5 ml Dimethylformamid versetzt und 2 h bei Raumtemperatur gerührt. Dann wurde abgesaugt und mit Dimethylformamid, $CH_2Cl_2$, Methanol und $CH_2Cl_2$ gewaschen. Die Abspaltung der Fmoc-Schutzgruppe erfolgte mit 2 mL einer Lösung von 10 % (1,8-Diazabicyclo-5.4.0.-undec-7-en), 2 % Piperidin und 88 % Dimethylformamid (3 min). Anschließend wurde das Harz abgesaugt und mit Dimethylformamid, $CH_2Cl_2$,

Methanol und $CH_2Cl_2$ gewaschen.

c) Das Harz aus b) wurde in einer Lösung von 0,088 mmol Fmoc-Ala-OH und 0,088 mmol N,N,-Diisopropylethylamin in 1,5 ml Dimethylformamid suspendiert, mit einer Lösung von 0,088 mmol 2(1H-Benzotriazol-1-yl-)1,1,3,3-tetramethyluronium-tetrafluoroborat in 0,5 ml Dimethylformamid versetzt und 2 h bei Raumtemperatur gerührt. Dann wurde abgesaugt und mit Dimethylformamid, $CH_2Cl_2$, Methanol und $CH_2Cl_2$ gewaschen. Die Abspaltung der Fmoc-Schutzgruppe erfolgte mit 2 ml einer Lösung von 10 % (1,8-Diazabicyclo-5.4.0.-undec-7-en), 2 % Piperidin und 88 % Dimethylformamid (3 min). Anschließend wurde das Harz abgesaugt und mit Dimethylformamid, $CH_2Cl_2$, Methanol und $CH_2Cl_2$ gewaschen.

d) Das Harz aus c) wurde in einer Lösung von 0,088 mmol 4-Benzoylbenzoesäuresäure in 1 ml $CH_2Cl_2$ suspendiert und mit 0,088 mmol Diisopropylcarbodiimid in 0,5 ml $CH_2Cl_2$ versetzt. Nach 2 h Rühren bei Raumtemperatur wurde abgesaugt und mit Dimethylformamid, $CH_2Cl_2$, Methanol und $CH_2Cl_2$ gewaschen. Die Abspaltung des Produktes vom Träger erfolgte durch Behandlung mit Trifluoressigsäure-Wasser 95:5 (1 h/Raumtemperatur). Ausbeute: 13 mg. HPLC-MS: $M+H^+$ 532 (berechnet: 532).

Analog Bsp. 84 wurden die folgenden Beispiele hergestellt, wobei z.B. auch anstelle der letzten Kupplung reduktive Aminierungen am Harz durchgeführt werden können mit z.B. 4-carboxy-Benzaldehyd oder anderen Aldehyden unter Standardbedingungen mit Natriumcyanoborhydrid in 1 % AcOH/DMF.

Beispiel 85

3-Benzoylbenzoyl-Gly-Pro-NH-$CH_2$-5-(3-am)-thioph
ESI-MS $[M+H]^+$ 518

Beispiel 86

4-Benzoylbenzoyl-Gly-Pro-NH-$CH_2$-5-(3-am)-thioph
ESI-MS $[M+H]^+$ 518

Beispiel 87

4-Phenylbenzoyl-Gly-Pro-NH-$CH_2$-5- (3-am)-thioph
ESI-MS $[M+H]^+$ 490

Beispiel 88

4-Phenylphenylacetyl-Gly-Pro-NH-$CH_2$-5-(3-am)-thioph
ESI-MS $[M+H]^+$ 504

Beispiel 89

2-(Benzylthio)-benzoyl-Gly-Pro-NH-$CH_2$-5-(3-am)-thioph
ESI-MS $[M+H]^+$ 536

Beispiel 90

3-Phenylpropionyl-Gly-Pro-NH-$CH_2$-5-(3-am)-thioph
ESI-MS $[M+H]^+$ 442

Beispiel 91

4-Phenylbutyryl-Gly-Pro-NH-$CH_2$-5-(3-am)-thioph
ESI-MS $[M+H]^+$ 456

Beispiel 92

5-Phenylvaleryl-Gly-Pro-NH-$CH_2$-5-(3-am)-thioph

ESI-MS $[M+H]^+$ 470

Beispiel 93

Cinnamoyl-Gly-Pro-NH-$CH_2$-5-(3-am)-thioph
ESI-MS $[M+H]^+$ 440

Beispiel 94

$C_6H_5$-C≡C-CO-Gly-Pro-NH-$CH_2$-5-(3-am)-thioph
ESI-MS $[M+H]^+$ 438

Beispiel 95

9-Fluorenon-4-carbonyl-Gly-Pro-NH-$CH_2$-5-(3-am)-thioph
ESI-MS $[M+H]^+$ 516

Beispiel 96

3-Benzyloxycarbonylpropionyl-Gly-Pro-NH-$CH_2$-5-(3-am)-thioph
ESI-MS $[M+H]^+$ 500

Beispiel 97

4-Methoxycarbonylcinnamoyl-Gly-Pro-NH-$CH_2$-5-(3-am)-thioph
ESI-MS $[M+H]^+$ 498

Beispiel 98

4-Methoxycarbonylbenzoyl-Gly-Pro-NH-$CH_2$-5-(3-am)-thioph
ESI-MS $[M+H]^+$ 472

Beispiel 99

2-(4'-Chlor-3'-Nitrobenzoyl)-benzoyl-Gly-Pro-NH-$CH_2$-5-(3-am)-thioph
ESI-MS $[M+H]^+$ 597

Beispiel 100

6-(Acetylamino)-pyridyl-3-carbonyl-Gly-Pro-NH-$CH_2$-5-(3-am)-thioph
ESI-MS $[M+H]^+$ 472

Beispiel 101

3-(3'-Pyridyl)-acryloyl-Gly-Pro-NH-$CH_2$-5-(3-am)-thioph
ESI-MS $[M+H]^+$ 441

Beispiel 102

4-Acetylaminobenzoyl-Gly-Pro-NH-$CH_2$-5-(3-am)-thioph
ESI-MS $[M+H]^+$ 471

Beispiel 103

4-(4'-Aminophenoxy)-benzoyl-Gly-Pro-NH-$CH_2$-5-(3-am)-thioph
ESI-MS $[M+H]^+$ 521

Beispiel 104

4-(2'-Chlor-4'-Aminophenoxy)-benzoyl-Gly-Pro-NH-$CH_2$-5-(3-am)-thioph
ESI-MS $[M+H]^+$ 555

Beispiel 105

4-Aminobenzoyl-Gly-Pro-NH-$CH_2$-5-(3-am)-thioph
ESI-MS $[M+H]^+$ 486

Beispiel 106

(4-Aminophenyl)acetyl-Gly-Pro-NH-$CH_2$-5-(3-am)-thioph
ESI-MS $[M+H]^+$ 443

Beispiel 107

(4-Aminophenylthio)-acetyl-Gly-Pro-NH-$CH_2$-5-(3-am)-thioph
ESI-MS $[M+H]^+$ 475

Beispiel 108

2-(Pyrid-3-yl)-acetyl-Gly-Pro-NH-$CH_2$-5-(3-am)-thioph
ESI-MS $[M+H]^+$ 429

Beispiel 109

3-(4'-Aminobenzoyl)-butyryl-Gly-Pro-NH-$CH_2$-5-(3-am)-thioph
ESI-MS $[M+H]^+$ 499

Beispiel 110

4-Benzoylbenzoyl-(D)-Val-Pro-NH-$CH_2$-5-(3-am)-thioph
ESI-MS $[M+H]^+$ 560

Beispiel 111

4-Phenylphenylacetyl-(D)-Val-Pro-NH-$CH_2$-5-(3-am)-thioph
ESI-MS $[M+H]^+$ 546

Beispiel 112

4-Phenylphenylacetyl-(D)-Ala-Pro-NH-$CH_2$-5-(3-am)-thioph
ESI-MS $[M+H]^+$ 518

Beispiel 113

4-Benzoylbenzoyl-β-Ala-Pro-NH-$CH_2$-5-(3-am)-thioph
ESI-MS $[M+H]^+$ 532

Beispiel 114

4-Benzoylbenzoyl-(D)-Ala-Pro-NH-$CH_2$-5-(3-am)-thioph
ESI-MS $[M+H]^+$ 532

Beispiel 115

2-(Benzylthio)-benzoyl-(D)-Ala-Pro-NH-$CH_2$-5-(3-am)-thioph

ESI-MS $[M+H]^+$ 550

Beispiel 116

5-Phenylvaleryl-(D)-Val-Pro-NH-$CH_2$-5-(3-am)-thioph
ESI-MS $[M+H]^+$ 512

Beispiel 117

5-Phenylvaleryl-(D)-Ala-Pro-NH-$CH_2$-5-(3-am)-thioph
ESI-MS $[M+H]^+$ 484

Beispiel 118

5-Phenylvaleryl-Ala-Pro-NH-$CH_2$-5-(3-am)-thioph
ESI-MS $[M+H]^+$ 484

Beispiel 119

3-Phenylpropionyl-(D)-Val-Pro-NH-$CH_2$-5-(3-am)-thioph
ESI-MS $[M+H]^+$ 484

Beispiel 120

4-Phenylbutyryl-(D)-Val-Pro-NH-$CH_2$-5-(3-am)-thioph
ESI-MS $[M+H]^+$ 498

Beispiel 121

4-Phenylbutyryl-(D)-Ala-Pro-NH-$CH_2$-5-(3-am)-thioph
ESI-MS $[M+H]^+$ 470

Beispiel 122

4-Phenylbenzoyl-(D)-Val-Pro-NH-$CH_2$-5-(3-am)-thioph
ESI-MS $[M+H]^+$ 532

Beispiel 123

4-Phenylbenzoyl-Ala-Pro-NH-$CH_2$-5-(3-am)-thioph
ESI-MS $[M+H]^+$ 504

Beispiel 124

4-Phenylbenzoyl-Val-Pro-NH-$CH_2$-5-(3-am)-thioph
ESI-MS $[M+H]^+$ 532

Beispiel 125

3-Phenylpropionyl-(D)-Ala-Pro-NH-$CH_2$-5-(3-am)-thioph
ESI-MS $[M+H]^+$ 456

Beispiel 126

2-(Benzylthio)-benzoyl-(D)-Val-Pro-NH-$CH_2$-5-(3-am)-thioph
ESI-MS $[M+H]^+$ 578

Beispiel 127

5-Phenylvaleryl-Val-Pro-NH-$CH_2$-5-(3-am)-thioph
ESI-MS $[M+H]^+$ 512

Beispiel 128

4-Phenylphenylacetyl-β-Ala-Pro-NH-$CH_2$-5-(3-am)-thioph
ESI-MS $[M+H]^+$ 518

Beispiel 129

4-Phenylbenzoyl-(D)-Ala-Pro-NH-$CH_2$-5-(3-am)-thioph
ESI-MS $[M+H]^+$ 504

Beispiel 130

4-Phenylphenylacetyl-Val-Pro-NH-$CH_2$-5-(3-am)-thioph
ESI-MS $[M+H]^+$ 546

Beispiel 131

4-Phenylphenylacetyl-Ala-Pro-NH-$CH_2$-5-(3-am)-thioph
ESI-MS $[M+H]^+$ 518

Beispiel 132

3-Phenylpropionyl-Ala-Pro-NH-$CH_2$-5-(3-am)-thioph
ESI-MS $[M+H]^+$ 456

Beispiel 133

3-Phenylpropionyl-β-Ala-Pro-NH-$CH_2$-5-(3-am)-thioph
ESI-MS $[M+H]^+$ 456

Beispiel 134

4-Phenylbutyryl-β-Ala-Pro-NH-$CH_2$-5-(3-am)-thioph
ESI-MS $[M+H]^+$ 470

Beispiel 135

5-Phenylvaleryl-β-Ala-Pro-NH-$CH_2$-5-(3-am)-thioph
ESI-MS $[M+H]^+$ 484

Beispiel 136

4-Benzoylbenzoyl-Val-Pro-NH-$CH_2$-5-(3-am)-thioph
ESI-MS $[M+H]^+$ 560

Beispiel 137

4-Phenylbenzoyl-β-Ala-Pro-NH-$CH_2$-5-(3-am)-thioph
ESI-MS $[M+H]^+$ 504

Beispiel 138

3-Phenylpropionyl-Val-Pro-NH-$CH_2$-5-(3-am)-thioph

ESI-MS $[M+H]^+$ 484

Beispiel 139

4-Phenylbutyryl-Val-Pro-NH-$CH_2$-5-(3-am)-thioph
ESI-MS $[M+H]^+$ 498

Beispiel 140

2-(Benzylthio)-benzoyl-Val-Pro-NH-$CH_2$-5-(3-am)-thioph
ESI-MS $[M+H]^+$ 578

Beispiel 141

2-(Benzylthio)-benzoyl-Ala-Pro-NH-$CH_2$-5-(3-am)-thioph
ESI-MS $[M+H]^+$ 550

Beispiel 142

4-Benzoylbenzoyl-(D)-Ala-Pyr-NH-$CH_2$-5-(3-am)-thioph
ESI-MS $[M+H]^+$ 530

Beispiel 143

4-Benzoylbenzoyl-(D)-Val-Pyr-NH-$CH_2$-5-(3-am)-thioph
ESI-MS $[M+H]^+$ 558

Beispiel 144

4-Benzoylbenzoyl-Sar-Pyr-NH-$CH_2$-5-(3-am)-thioph
ESI-MS $[M+H]^+$ 530

Beispiel 145

$C_6H_5$-C≡C-CO-Gly-Pyr-NH-$CH_2$-5-(3-am)-thioph
ESI-MS $[M+H]^+$ 436

Beispiel 146

$C_6H_5$-C≡C-CO-Sar-Pyr-NH-$CH_2$-5-(3-am)-thioph
ESI-MS $[M+H]^+$ 450

Beispiel 147

$C_6H_5$-C≡C-CO-(D)-Val-Pyr-NH-$CH_2$-5-(3-am)-thioph
ESI-MS $[M+H]^+$ 478

Beispiel 148

$C_6H_5$-C≡C-CO-(D)-Ala-Pyr-NH-$CH_2$-5-(3-am)-thioph
ESI-MS $[M+H]^+$ 450

Beispiel 149

4-Phenylbutyryl-Ala-Pro-NH-$CH_2$-5-(3-am)-thioph
ESI-MS $[M+H]^+$ 470

Beispiel 150

MeOC(O)-$(CH_2)_5$-NHC(O)-Gly-Pro-NH-$CH_2$-5-(3-am)-thioph

a) 0,044 mmol Harz aus Beispiel 84/Abschnitt b), wurden in einer Lösung von 0,088 mmol Fmoc-Gly-OH und 0,088 mMol N,N,-Diisopropylethylamin in 1,5 ml Dimethylformamid suspendiert, mit einer Lösung von 0,088 mmol 2 (1H-Benzotriazol-1-yl-)1,1,3,3-tetramethyluronium-tetrafluoroborat in 0,5 ml Dimethylformamid versetzt und 2 h bei Raumtemperatur gerührt. Dann wurde das Harz abgesaugt und mit Dimethylformamid, $CH_2Cl_2$, Methanol und $CH_2Cl_2$ gewaschen. Die Abspaltung der Fmoc-Schutzgruppe erfolgte mit 2 ml einer Lösung von 10 % (1,8-Diazabicyclo-5.4.0.-undec-7-en), 2 % Piperidin und 88 % Dimethylformamid (3 min). Anschließend wurde das Harz abgesaugt und Dimethylformamid, $CH_2Cl_2$, Methanol und $CH_2Cl_2$ gewaschen.

b) Das Harz wurde in 1 ml $CH_2Cl_2$ suspendiert und mit 0,088 mmol 6-Isocyanatocapronsäuremethylester in 0,5 ml $CH_2Cl_2$ versetzt. Nach 2 Stunden Rühren bei Raumtemperatur wurde abgesaugt und mit Dimethylformamid, $CH_2Cl_2$, Methanol und $CH_2Cl_2$ gewaschen. Die Abspaltung des Produktes vom Träger erfolgt durch Behandlung mit Trifluoressigsäure-Wasser 95:5 (1 h/Raumtemperatur).
Ausbeute: 18 mg. HPLC-MS: $M+H^+$ 481 (berechnet: 481).

Beispiel 151

Phenylsulfonyl-Gly-Pro-NH-$CH_2$-5-(3-am)-thioph

0,01 mmol Harz aus Beispiel 150/Abschnitt a) wurden in 0,2 ml $CH_2Cl_2$/DMF (1:1) suspendiert, mit 10,4 µl (0,06 mmol) N,N,-Diisopropylethylamin und anschließend mit einer Lösung von 2,5 µl (0,02 mmol) Benzolsulfonsäurechlorid in 200 µl $CH_2Cl_2$/DMF (1:1) versetzt. Nach 2 h Rühren bei Raumtemperatur wurde abgesaugt und mit Dimethylformamid, $CH_2Cl_2$, Methanol und $CH_2Cl_2$ gewaschen. Die Abspaltung des Produktes vom Träger erfolgt durch Behandlung mit Trifluoressigsäure-Wasser 95:5 (1 h/Raumtemperatur).
Ausbeute: 4,6 mg. HPLC-MS: $M+H^+$ 450 (berechnet: 450).

Beispiel 152

3-[4-(2,5-Dichlor-benzyloxy)-phenyl]propionyl(-D-Val-Pyr-NH-$CH_2$-5-(3-am)-thioph

a) 0,2 mMol 3-(4-Hydroxyphenyl)-propionsäure-2-Chlortritylharz wurden in einer Lösung von 262 mg (1 mMol) Triphenylphosphin in 2 ml THF suspendiert. Nach Zugabe einer Lösung von 2 mMol 2,5-Dichlor-Benzylalkohol in 2 ml THF wurde unter Rühren eine Lösung von 408 µl (2 mMol) Diisopropylazodicarboxylat in 200 µl THF portionsweise innerhalb 30 Minuten zugegeben. Nach 20 Stunden Inkubation wurde das Harz abgesaugt und mit THF gewaschen. Anschließend wurde Schritt a) wiederholt.

b) Zur Aufarbeitung wurde das Harz abgesaugt, mit THF und anschließend mit Methanol und Dichlormethan gewaschen. Die Spaltung des Produktes vom Träger erfolgte mit Trifluorethanol, Essigsäure, Dichlormethan (1:1:3) in 45 Minuten. Nach Eindampfen im Vakuum wurde der Rückstand mit Essigsäure gelöst und gefriergetrocknet. Ausbeute 31 mg.

Literatur:

Krchnak, V., Flegelova, Z., Weichsel, A.S., and Lebl, M. (1995). Tetrahedron Lett., 36, 6193.

c) Die Säurekomponente wurde entsprechend wie für Beispiel 84 beschrieben mit TBTU an polymergebundenes H-D-Val-Pyr-NH-$CH_2$-5-(3-am)-thioph gekuppelt. Nach Abspaltung mit TFA-Wasser (95:5) (1 h bei Raumtemperatur) wurde das Produkt erhalten (ESI-MS $[M+H]^+$656.

Analog wie die vorgehenden Beispiele wurden folgende Verbindungen hergestellt:

| | |
|---|---|
| 153. | 4-(2,5-Dichlor-benzyloxy)-benzoyl-D-Val-Pyr-NH-$CH_2$-5-(3-am)-thioph<br>ESI-MS $[M+H]^+$ 628 |
| 154. | 4-(2-Chlor-benzyloxy)-benzoyl-D-Val-Pyr-NH-$CH_2$-5-(3-am)-thioph<br>ESI-MS $[M+H]^+$ 594 |
| 155. | 3-[4-(2-Chlor-benzyloxy)-phenyl]-propionyl-D-Val-Pyr-NH-$CH_2$-5-(3-am)-thioph<br>ESI-MS $[M+H]^+$ 622 |
| 156. | 3-[4-(4-Nitro-benzyloxy)-phenyl]-propionyl-D-Val-Pyr-NH-$CH_2$-5-(3-am)-thioph<br>ESI-MS $[M+H]^+$ 633 |
| 157. | 3-[4-(4-Methoxycarbonyl-benzyloxy)-phenyl]-propionyl-D-Val-Pyr-NH-$CH_2$-5-(3-am)-thioph<br>ESI-MS $[M+H]^+$ 646 |
| 158. | 3-[4-(4-Fluor-3-Trifluormethyl-benzyloxy)-phenyl]-propionyl-D-Val-Pyr-NH-$CH_2$-5-(3-am)-thioph<br>ESI-MS $[M+H]^+$ 674 |
| 159. | 3-[4-(2-Chlor-3-Isopropyl-benzyloxy)-phenyl]-propionyl-D-Val-Pyr-NH-$CH_2$-5-(3-am)-thioph<br>ESI-MS $[M+H]^+$ 664 |
| 160. | 4-(2,5-Dichlor-benzyloxy)-phenylacetyl-D-Val-Pyr-NH-$CH_2$-5-(3-am)-thioph<br>ESI-MS $[M+H]^+$ 642 |
| 161. | 4-(4-Chlor-3-Nitro-benzyloxy)-phenylacetyl-D-Val-Pyr-NH-$CH_2$-5-(3-am)-thioph<br>ESI-MS $[M+H]^+$ 653 |
| 162. | 4-(4-Nitro-benzyloxy)-phenylacetyl-D-Val-Pyr-NH-$CH_2$-5-(3-am)-thioph<br>ESI-MS $[M+H]^+$ 619 |
| 163. | 4-(4-Methoxycarbonyl-benzyloxy)-phenylacetyl-D-Val-Pyr-NH-$CH_2$-5-(3-am)-thioph<br>ESI-MS $[M+H]^+$ 632 |
| 164. | 4-(4-Fluor-3-Trifluormethyl-benzyloxy)-phenylacetyl-D-Val-Pyr-NH-$CH_2$-5-(3-am)-thioph<br>ESI-MS $[M+H]^+$ 660 |
| 165. | 4-(2-Chlor-3-Isopropyl-benzyloxy)-phenylacetyl-D-Val-Pyr-NH-$CH_2$-5-(3-am)-thioph<br>ESI-MS $[M+H]^+$ 650 |
| 166. | 4-(4-Chlor-benzyloxy)-phenylacetyl-D-Val-Pyr-NH-$CH_2$-5-(3-am)-thioph<br>ESI-MS $[M+H]^+$ 608 |
| 167. | 5-[4-(2,5-Dichlor-benzyloxy)-phenyl)-5-oxo-pentanoyl-D-Val-Pyr-NH-$CH_2$-5-(3-am)-thioph<br>ESI-MS $[M+H]^+$ 698 |
| 168. | 5-[4-(4-Chlor-3-Nitro-benzyloxy)-phenyl]-5-oxo-pentanoyl-D-Val-Pyr-NH-$CH_2$-5-(3-am)-thioph<br>ESI-MS $[M+H]^+$ 709 |
| 169. | 5-[4-(4-Nitro-benzyloxy)-phenyl]-5-oxo-pentanoyl-D-Val-Pyr-NH-$CH_2$-5-(3-am)-thioph<br>ESI-MS $[M+H]^+$ 675 |
| 170. | 5-[4-(4-Methoxycarbonyl-benzyloxy)-phenyl]-5-oxo-pentanoyl-D-Val-Pyr-NH-$CH_2$-5-(3-am)-thioph<br>ESI-MS $[M+H]^+$ 688 |
| 171. | 5-[4-(4-Fluor-3-Trifluormethyl-benzyloxy)-phenyl]-5-oxo-pentanoyl-D-Val-Pyr-NH-$CH_2$-5-(3-am)-thioph<br>ESI-MS $[M+H]^+$ 715 |
| 172. | 5-[4-(2-Chlor-3-Isopropyl-benzyloxy)-phenyl]-5-oxo-pentanoyl-D-Val-Pyr-NH-$CH_2$-5-(3-am)-thioph<br>ESI-MS $[M+H]^+$ 706 |
| 173. | 5-(4-Benzyloxy-phenyl)-5-oxo-pentanoyl-D-Val-Pyr-NH-$CH_2$-5-(3-am)-thioph<br>ESI-MS $[M+H]^+$ 630 |
| 174. | 5-[4-(4-Chlor-benzyloxy)-phenyl]-5-oxo-pentanoyl-D-Val-Pyr-NH-$CH_2$-5-(3-am)-thioph<br>ESI-MS $[M+H]^+$ 664 |
| 175. | 2-[4-(2,5-Dichlor-benzyloxy)-phenoxy]-propionyl-D-Val-Pyr-NH-$CH_2$-5-(3-am)-thioph<br>ESI-MS $[M+H]^+$ 672 |

(fortgesetzt)

| | |
|---|---|
| 176. | 2-[4-(4-Chlor-3-Nitro-benzyloxy)-phenoxy]-propionyl-D-Val-Pyr-NH-$CH_2$-5-(3-am)-thioph<br>ESI-MS $[M+H]^+$ 683 |
| 177. | 2-[4-(2-Chlor-benzyloxy)-phenoxy]-propionyl-D-Val-Pyr-NH-$CH_2$-5-(3-am)-thioph<br>ESI-MS $[M+H]^+$ 638 |
| 178. | 2-[4-(4-Nitro-benzyloxy)-phenoxy]-propionyl-D-Val-Pyr-NH-$CH_2$-5-(3-am)-thioph<br>ESI-MS $[M+H]^+$ 649 |
| 179. | 2-[4-(4-Methoxycarbonyl-benzyloxy)-phenoxy]-propionyl-D-Val-Pyr-NH-$CH_2$-5-(3-am)-thioph<br>ESI-MS $[M+H]^+$ 662 |
| 180. | 2-[4-(4-Fluor-3-Trifluormethyl-benzyloxy)-phenoxy]-propionyl-D-Val-Pyr-NH-$CH_2$-5-(3-am)-thioph<br>ESI-MS $[M+H]^+$ 690 |
| 181. | 2-[4-(2-Chlor-3-Isopropyl-benzyloxy)-phenoxy]-propionyl-D-Val-Pyr-NH-$CH_2$-5-(3-am)-thioph<br>ESI-MS $[M+H]^+$ 680 |
| 182. | 2-(4-Benzyloxy-phenoxy)-propionyl-D-Val-Pyr-NH-$CH_2$-5-(3-am)-thioph<br>ESI-MS $[M+H]^+$ 604 |
| 183. | 2-[4-(4-Chlor-benzyloxy)-phenoxy]-propionyl-D-Val-Pyr-NH-$CH_2$-5-(3-am)-thioph<br>ESI-MS $[M+H]^+$ 638 |
| 184. | 2-[4-(2,5-Dichlor-benzyloxy)-phenyl]-3-methyl-butyryl-Pyr-NH-$CH_2$-5-(3-am)-thioph<br>ESI-MS $[M+H]^+$ 585 |
| 185. | 2-[4-(2,5-Dichlor-benzyloxy)-phenyl]-3-methyl-butyryl-D-Val-Pyr-NH-$CH_2$-5-(3-am)-thioph<br>ESI-MS $[M+H]^+$ 684 |
| 186. | 2-[4-(4-Chlor-3-Nitro-benzyloxy)-phenyl]-3-methyl-butyryl-D-Val-Pyr-NH-$CH_2$-5-(3-am)-thioph<br>ESI-MS $[M+H]^+$ 695 |
| 187. | 2-[4-(4-Nitro-benzyloxy)-phenyl]-3-methyl-butyryl-D-Val-Pyr-NH-$CH_2$-5-(3-am)-thioph<br>ESI-MS $[M+H]^+$ 661 |
| 188. | 2-[4-(4-Methoxycarbonyl-benzyloxy)-phenyl]-3-methyl-butyryl-D-Val-Pyr-NH-$CH_2$-5-(3-am)-thioph<br>ESI-MS $[M+H]^+$ 674 |
| 189. | 2-[4-(4-Fluor-3-Trifluormethyl-benzyloxy)-phenyl]-3-methyl-butyryl-D-Val-Pyr-NH-$CH_2$-5-(3-am)-thioph<br>ESI-MS $[M+H]^+$ 702 |
| 190. | 2-(4-benzyloxy-phenyl)-3-methyl-butyryl-D-Val-Pyr-NH-$CH_2$-5-(3-am)-thioph<br>ESI-MS $[M+H]^+$ 616 |
| 191. | 2-[4-(4-Chlor-benzyloxy)-phenyl]-3-methyl-butyryl-D-Val-Pyr-NH-$CH_2$-5-(3-am)-thioph<br>ESI-MS $[M+H]^+$ 650 |
| 192. | 2-[4-(2,5-Dichlor-benzyloxy)-phenoxy]-propionyl-Pyr-NH-$CH_2$-5-(3-am)-thioph<br>ESI-MS $[M+H]^+$ 573 |
| 193. | 2-[4-(4-Nitro-benzyloxy)-phenoxy]-propionyl-Pyr-NH-$CH_2$-5-(3-am)-thioph<br>ESI-MS $[M+H]^+$ 550 |
| 194. | 2-[4-(4-Methoxycarbonyl-benzyloxy)-phenoxy]-propionyl-Pyr-NH-$CH_2$-5-(3-am)-thioph<br>ESI-MS $[M+H]^+$ 563 |
| 195. | 2-[4-(2-Chlor-3-Isopropyl-benzyloxy)-phenoxy]-propionyl-Pyr-NH-$CH_2$-5-(3-am)-thioph<br>ESI-MS $[M+H]^+$ 581 |
| 196. | 2-(4-benzyloxy-phenoxy)-propionyl-Pyr-NH-$CH_2$-5-(3-am)-thioph<br>ESI-MS $[M+H]^+$ 505 |
| 197. | 2-[4-(4-Chlor-benzyloxy)-phenoxy]-propionyl-Pyr-NH-$CH_2$-5-(3-am)-thioph<br>ESI-MS $[M+H]^+$ 539 |
| 198. | 2-[4-(4-Chlor-3-Nitro-benzyloxy)-phenoxy]-propionyl-Pyr-NH-$CH_2$-5-(3-am)-thioph<br>ESI-MS $[M+H]^+$ 596 |

(fortgesetzt)

| | |
|---|---|
| 199. | 3-(2,5-Dichlor-benzyloxy)-benzoyl-D-Val-Pyr-NH-$CH_2$-5-(3-am)-thioph ESI-MS $[M+H]^+$ 628 |
| 200. | 3-(4-Chlor-3-Nitro-benzyloxy)-benzoyl-D-Val-Pyr-NH-$CH_2$-5-(3-am)-thioph ESI-MS $[M+H]^+$ 639 |
| 201. | 3-(2-Naphthylmethoxy)-benzoyl-D-Val-Pyr-NH-$CH_2$-5-(3-am)-thioph ESI-MS $[M+H]^+$ 610 |
| 202. | 3-(4-Methyl-3-Nitro-benzyloxy)-benzoyl-D-Val-Pyr-NH-$CH_2$-5-(3-am)-thioph ESI-MS $[M+H]^+$ 619 |
| 203. | 3-(4-Nitro-benzyloxy)-benzoyl-D-Val-Pyr-NH-$CH_2$-5-(3-am)-thioph ESI-MS $[M+H]^+$ 605 |
| 204. | 3-(4-Fluor-3-Trifluormethyl)-benzyloxy)-benzoyl-D-Val-Pyr-NH-$CH_2$-5-(3-am)-thioph ESI-MS $[M+H]^+$ 646 |
| 205. | 3-(2-Chlor-3-Isopropyl-benzyloxy)-benzoyl-D-Val-Pyr-NH-$CH_2$-5-(3-am)-thioph ESI-MS $[M+H]^+$ 636 |
| 206. | 3-Benzyloxybenzoyl-D-Val-Pyr-NH-$CH_2$-5-(3-am)-thioph ESI-MS $[M+H]^+$ 560 |
| 207. | 3-(4-Chlorbenzyloxy)-benzoyl-D-Val-Pyr-NH-$CH_2$-5-(3-am)-thioph ESI-MS $[M+H]^+$ 594 |
| 208. | 3-(2,5-Dichlor-benzyloxy)-phenylacetyl-D-Val-Pyr-NH-$CH_2$-5-(3-am)-thioph ESI-MS $[M+H]^+$ 642 |
| 209. | 3-(4-Chlor-3-Nitro-benzyloxy)-phenylacetyl-D-Val-Pyr-NH-$CH_2$-5-(3-am)-thioph ESI-MS $[M+H]^+$ 653 |
| 210. | 3-(4-Methyl-3-Nitro-benzyloxy)-phenylacetyl-D-Val-Pyr-NH-$CH_2$-5-(3-am)-thioph ESI-MS $[M+H]^+$ 633 |
| 211. | 3-(4-Nitro-benzyloxy)-phenylacetyl-D-Val-Pyr-NH-$CH_2$-5-(3-am)-thioph ESI-MS $[M+H]^+$ 619 |
| 212. | 3-(4-Fluor-3-Trifluormethyl-benzyloxy)-phenylacetyl-D-Val-Pyr-NH-$CH_2$-5-(3-am)-thioph ESI-MS $[M+H]^+$ 660 |
| 213. | 3-(2-Chlor-3-Isopropyl-benzyloxy)-phenylacetyl-D-Val-Pyr-NH-$CH_2$-5-(3-am)-thioph ESI-MS $[M+H]^+$ 650 |
| 214. | 3-Benzyloxy-phenylacetyl-D-Val-Pyr-NH-$CH_2$-5-(3-am)-thioph ESI-MS $[M+H]^+$ 574 |
| 215. | 3-(4-Chlor-benzyloxy)-phenylacetyl-D-Val-Pyr-NH-$CH_2$-5-(3-am)-thioph ESI-MS $[M+H]^+$ 608 |
| 216. | 3-[3-(2,5-Dichlor-benzyloxy)-phenyl]-acryloyl-D-Val-Pyr-NH-$CH_2$-5-(3-am)-thioph ESI-MS $[M+H]^+$ 654 |
| 217. | 3-[3-(4-Chlor-3-Nitro-benzyloxy)-phenyl]-acryloyl-D-Val-Pyr-NH-$CH_2$-5-(3-am)-thioph ESI-MS $[M+H]^+$ 665 |
| 218. | 3-[3-(4-Methyl-3-Nitro-benzyloxy)-phenyl]-acryloyl-D-Val-Pyr-NH-$CH_2$-5-(3-am)-thioph ESI-MS $[M+H]^+$ 645 |
| 219. | 3-[3-(4-Nitro-benzyloxy)-phenyl]-acryloyl-D-Val-Pyr-NH-$CH_2$-5-(3-am)-thioph ESI-MS $[M+H]^+$ 631 |
| 220. | 3-[3-(4-Fluor-3-Trifluormethyl-benzyloxy)-phenyl]-acryloyl-D-Val-Pyr-NH-$CH_2$-5-(3-am)-thioph ESI-MS $[M+H]^+$ 672 |
| 221. | 3-[3-(2-Chlor-3-Isopropyl-benzyloxy)-phenyl]-acryloyl-D-Val-Pyr-NH-$CH_2$-5-(3-am)-thioph ESI-MS $[M+H]^+$ 662 |

(fortgesetzt)

| | |
|---|---|
| 222. | 3-(3-Benzyloxy-phenyl)-acryloyl-D-Val-Pyr-NH-$CH_2$-5-(3-am)-thioph<br>ESI-MS $[M+H]^+$ 586 |
| 223. | 4-Phenylbenzolsulfonyl-β-Ala-Pyr-NH-$CH_2$-5-(3-am)-thioph<br>ESI-MS $[M+H]^+$ 538 |
| 224. | 4-Phenylbenzolsulfonyl-D-Ala-Pyr-NH-$CH_2$-5-(3-am)-thioph<br>ESI-MS $[M+H]^+$ 538 |
| 225. | 4-Phenylbenzolsulfonyl-Sar-Pyr-NH-$CH_2$-5-(3-am)-thioph<br>ESI-MS $[M+H)^+$ 538 |
| 226. | 4-Phenylbenzolsulfonyl-Gly-Pyr-NH-$CH_2$-5-(3-am)-thioph<br>ESI-MS $[M+H]^+$ 524 |
| 227. | $C_6H_5$-C≡C-CO-β-Ala-Pyr-NH-$CH_2$-5-(3-am)-thioph<br>ESI-MS $[M+H^+$ 450 |
| 228. | $C_6H_5$-C≡C-CO-D-Asp-Pyr-NH-$CH_2$-5-(3-am)-thioph<br>ESI-MS $[M+H]^+$ 494 |
| 229. | $C_6H_5$-C≡C-CO-D-Arg-Pyr-NH-$CH_2$-5-(3-am)-thioph<br>ESI-MS $[M+H]^+$ 535 |
| 230. | 4-Benzoylbenzoyl-β-Ala-Pyr-NH-$CH_2$-5-(3-am)-thioph<br>ESI-MS $[M+H]^+$ 530 |
| 231. | 4-Benzoylbenzoyl-D-Asp-Pyr-NH-$CH_2$-5-(3-am)-thioph<br>ESI-MS $[M+H]^+$ 574 |
| 232. | 4-Benzoylbenzoyl-D-Arg-Pyr-NH-$CH_2$-5-(3-am)-thioph<br>ESI-MS $[M+H]^+$ 615 |
| 233. | $C_6H_5$-C≡C-CO-Gly-Pyr-NH-$CH_2$-5-(2-am)-thioph<br>ESI-MS $[M+H]^+$ 436 |
| 234. | $C_6H_5$-C≡C-CO-β-Ala-Pyr-NH-$CH_2$-5-(2-am)-thioph<br>ESI-MS $[M+H]^+$ 450 |
| 235. | $C_6H_5$-C≡C-CO-D-Ala-Pyr-NH-$CH_2$-5-(2-am)-thioph<br>ESI-MS $[M+H]^+$ 450 |
| 236. | $C_6H_5$-C≡C-CO-D-Val-Pyr-NH-$CH_2$-5-(2-am)-thioph<br>ESI-MS $[M+H]^+$ 478 |
| 237. | 4-Benzoylbenzoyl-Gly-Pyr-NH-$CH_2$-5-(2-am)-thioph<br>ESI-MS $[M+H]^+$ 516 |
| 238. | 4-Benzoylbenzoyl-β-Ala-Pyr-NH-$CH_2$-5-(2-am)-thioph<br>ESI-MS $[M+H]^+$ 530 |
| 239. | 4-Benzoylbenzoyl-D-Ala-Pyr-NH-$CH_2$-5-(2-am)-thioph<br>ESI-MS $[M+H]^+$ 530 |
| 240. | 4-Benzoylbenzoyl-D-Val-Pyr-NH-$CH_2$-5-(2-am)-thioph<br>ESI-MS $[M+H]^+$ 558 |
| 241. | 4-Benzoylbenzoyl-D-Lys-Pyr-NH-$CH_2$-5-(3-am)-thioph<br>ESI-MS $[M+H]^+$ 587 |
| 242. | 4-Benzoylbenzoyl-D-Orn-Pyr-NH-$CH_2$-5-(3-am)-thioph<br>ESI-MS $[M+H]^+$ 573 |
| 243. | 4-Benzoylbenzoyl-D-His-Pyr-NH-$CH_2$-5-(3-am)-thioph<br>ESI-MS $[M+H]^+$ 596 |
| 244. | 4-Benzoylbenzoyl-D-Dab-Pyr-NH-$CH_2$-5-(3-am)-thioph<br>ESI-MS $[M+H]^+$ 559 |

(fortgesetzt)

| | |
|---|---|
| 245. | 4-Benzoylbenzoyl-D-Dap-Pyr-NH-$CH_2$-5-(3-am)-thioph<br>ESI-MS $[M+H]^+$ 545 |
| 246. | 4-Benzoylbenzoyl-D-Arg-Pyr-NH-$CH_2$-5-(2-am)-thioph<br>ESI-MS $[M+H]^+$ 615 |
| 247. | 4-Benzoylbenzoyl-D-Lys-Pyr-NH-$CH_2$-5-(2-am)-thioph<br>ESI-MS $[M+H]^+$ 587 |
| 248. | 4-Benzoylbenzoyl-D-Orn-Pyr-NH-$CH_2$-5-(2-am)-thioph<br>ESI-MS $[M+H]^+$ 573 |
| 249. | 4-Benzoylbenzoyl-D-His-Pyr-NH-$CH_2$-5-(2-am)-thioph<br>ESI-MS $[M+H]^+$ 596 |
| 250. | 4-Benzoylbenzoyl-D-Dab-Pyr-NH-$CH_2$-5-(2-am)-thioph<br>ESI-MS $[M+H]^+$ 559 |
| 251. | 4-Benzoylbenzoyl-D-Dap-Pyr-NH-$CH_2$-5-(2-am)-thioph<br>ESI-MS $[M+H]^+$ 545 |
| 252. | 9,10,10-Trioxo-9,10-dihydro-10*1*$^{6}$-Thioxanthen-3-carbonyl-D-Ala-Pyr-NH-$CH_2$-5-(3-am)-thioph<br>ESI-MS $[M+H]^+$ 592 |
| 253. | 9,10,10-Trioxo-9,10-dihydro-10*1*$^{6}$-Thioxanthen-3-carbonyl-Gly-Pyr-NH-$CH_2$-5-(3-am)-thioph<br>ESI-MS $[M+H]^+$ 578 |
| 254. | 9, 10,10-Trioxo-9,10-dihydro-10*1*$^{6}$-Thioxanthen-3-carbonyl-D-Val-Pyr-NH-$CH_2$-5-(3-am)-thioph<br>ESI-MS $[M+H]^+$ 620 |
| 255. | 9,10-Dioxo-9,10-dihydro-anthracen-2-carbonyl-D-Ala-Pyr-NH-$CH_2$-5-(3-am)-thioph<br>ESI-MS $[M+H]^+$ 556 |
| 256. | 9,10-Dioxo-9,10-dihydro-anthracen-2-carbonyl-Gly-Pyr-NH-$CH_2$-5-(3-am)-thioph<br>ESI-MS $[M+H]^+$ 542 |
| 257. | 9,10-Dioxo-9,10-dihydro-anthracen-2-carbonyl-D-Val-Pyr-NH-$CH_2$-5-(3-am)-thioph<br>ESI-MS $[M+H)^+$ 584 |
| 258. | 4-Benzoylbenzoyl-D-Ser-Pyr-NH-$CH_2$-5-(3-am)-thioph<br>ESI-MS $[M+H]^+$ 546 |
| 259. | 4-Aminobenzoyl-D-Ala-Pyr-NH-$CH_2$-5-(3-am)-thioph<br>ESI-MS $[M+H]^+$ 441 |
| 260. | 4-Methylaminobenzoyl-D-Ala-Pyr-NH-$CH_2$-5-(3-am)-thioph<br>ESI-MS $[M+H]^+$ 455 |
| 261. | 4-Aminobenzoyl-D-Val-Pyr-NH-$CH_2$-5-(3-am)-thioph<br>ESI-MS $[M+H]^+$ 469 |
| 262. | 4-Methylaminobenzoyl-D-Val-Pyr-NH-$CH_2$-5-(3-am)-thioph<br>ESI-MS $[M+H]^+$ 483 |
| 263. | 3-Aminobenzoyl-D-Val-Pyr-NH-$CH_2$-5-(3-am)-thioph<br>ESI-MS $[M+H]^+$ 469 |
| 264. | 4-(4-HOOC-Benzoyl)-benzoyl-D-Val-Pyr-NH-$CH_2$-5-(3-am)-thioph<br>ESI-MS $[M+H]^+$ 602 |
| 265. | 4-(3-Phenyl-ureido)-benzoyl-D-Ala-Pyr-NH-$CH_2$-5-(3-am)-thioph<br>ESI-MS $[M+H]^+$ 560 |
| 266. | 3-(3-Benzyl-ureido)-benzoyl-D-Ala-Pyr-NH-$CH_2$-5-(3-am)-thioph<br>ESI-MS $[M+H]^+$ 574 |
| 267. | 3-(3-Phenyl-ureido)-benzoyl-D-Ala-Pyr-NH-$CH_2$-5-(3-am)-thioph<br>ESI-MS $[M+H]^+$ 560 |

(fortgesetzt)

| | |
|---|---|
| 268. | 4-(3-Phenyl-ureido)-benzoyl-Gly-Pyr-NH-$CH_2$-5-(3-am)-thioph<br>ESI-MS $[M+H]^+$ 546 |
| 269. | 3-(3-Benzyl-ureido)-benzoyl-Gly-Pyr-NH-$CH_2$-5-(3-am)-thioph<br>ESI-MS $[M+H]^+$ 560 |
| 270. | 3-(3-Phenyl-ureido)-benzoyl-Gly-Pyr-NH-$CH_2$-5-(3-am)-thioph<br>ESI-MS $[M+H]^+$ 546 |
| 271. | 3-(3-Benzoyl-ureido)-benzoyl-Gly-Pyr-NH-$CH_2$-5-(3-am)-thioph<br>ESI-MS $[M+H]^+$ 574 |
| 272. | 4-(3-Phenyl-ureido)-benzoyl-D-Val-Pyr-NH-$CH_2$-5-(3-am)-thioph<br>ESI-MS $[M+H]^+$ 588 |
| 273. | 3-(3-Phenyl-ureido)-benzoyl-D-Val-Pyr-NH-$CH_2$-5-(3-am)-thioph<br>ESI-MS $[M+H]^+$ 588 |
| 274. | 3-[3-(3-Acetyl-Phenyl)-ureido)-benzoyl-D-Val-Pyr-NH-$CH_2$-5-(3-am)-thioph<br>ESI-MS $[M+H]^+$ 630 |
| 275. | 4-Benzyloxy-benzoyl-D-Ala-Pyr-NH-$CH_2$-5-(3-am)-thioph<br>ESI-MS $[M+H]^+$ 532 |
| 276. | 4-(4-Chlor-Benzyloxy)-benzoyl-D-Ala-Pyr-NH-$CH_2$-5-(3-am)-thioph<br>ESI-MS $[M+H]^+$ 566 |
| 277. | 3-(4-Benzyloxy-phenyl)-propionyl-D-Ala-Pyr-NH-$CH_2$-5-(3-am)-thioph<br>ESI-MS $[M+H]^+$ 560 |
| 278. | 3-[4-(4-Chlor-Benzyloxy)-phenyl]-propionyl-D-Ala-Pyr-NH-$CH_2$-5-(3-am)-thioph<br>ESI-MS $[M+H]^+$ 594 |
| 279. | 4-Benzyloxy-benzoyl-Gly-Pyr-NH-$CH_2$-5-(3-am)-thioph<br>ESI-MS $[M+H]^+$ 518 |
| 280. | 4-(4-Chlor-Benzyloxy)-benzoyl-Gly-Pyr-NH-$CH_2$-5-(3-am)-thioph<br>ESI-MS $[M+H]^+$ 552 |
| 281. | 3-(4-Benzyloxy-phenyl)-propionyl-Gly-Pyr-NH-$CH_2$-5-(3-am)-thioph<br>ESI-MS $[M+H]^+$ 546 |
| 282. | 3-[4-(4-Chlor-Benzyloxy)-phenyl]-propionyl-Gly-Pyr-NH-$CH_2$-5-(3-am)-thioph<br>ESI-MS $[M+H]^+$ 580 |
| 283. | 4-Benzyloxy-benzoyl-D-Val-Pyr-NH-$CH_2$-5-(3-am)-thioph<br>ESI-MS $[M+H]^+$ 560 |
| 284. | 4-(4-Chlor-Benzyloxy)-benzoyl-D-Val-Pyr-NH-$CH_2$-5-(3-am)-thioph<br>ESI-MS $[M+H]^+$ 594 |
| 285. | 3-(4-Benzyloxy-phenyl)-propionyl-D-Val-Pyr-NH-$CH_2$-5-(3-am)-thioph<br>ESI-MS $[M+H]^+$ 588 |
| 286. | 3-[4-(4-Chlor-Benzyloxy)-phenyl]-propionyl-D-Val-Pyr-NH-$CH_2$-5-(3-am)-thioph<br>ESI-MS $[M+H]^+$ 622 |
| 287. | phenyl-C≡C-CO-D-Chg-Pyr-NH-$CH_2$-5-(3-am)-thioph<br>MS $[M+H]^+$ 518 |
| 288. | phenyl-C≡C-CO-D-Abu-Pyr-NH-$CH_2$-5- (3-am)-thioph<br>MS $[M+H]^+$ 466 |
| 289. | 4-benzoylbenzoyl-D-Abu-Pro-NH-$CH_2$-5-(3-am)-thioph<br>MS $[M+H]^+$ 546 |
| 290. | 4-benzoylbenzoyl-D-Chg-Pyr-NH-$CH_2$-5-(3-am)-thioph<br>MS $[M+H]^+$ 598 |

(fortgesetzt)

| | |
|---|---|
| 291. | HOOC-p-$C_6H_4$-$CH_2$-D-Pro-Pyr-NH-$CH_2$-5-(3-am)-thioph<br>MS $[M+H]^+$ 482 |
| 292. | HOOC-p-$C_6H_4$-$CH_2$-D,L-thienyl (3)glycin-Pyr-NH-$CH_2$-5-(3-am)-thioph<br>MS $[M+H]^+$ 524 |
| 293. | p-COOH-benzyl-D-Abu-Pyr-NH-$CH_2$-5-(3-am)thioph<br>MS $[M+H]^+$ 470 |
| 294. | 4-benzoyl-benzoyl-Acpc-Pyr-NH-$CH_2$-5- (3-am)-thioph<br>MS $[M+H]^+$ 570 |
| 295. | 4-benzoyl-benzoyl-N-Me-D-Val-Pyr-NH-$CH_2$-5-(3-am)-thioph<br>MS $[M+H]^+$ 572 |
| 296. | p-carboxy-benzyl-D-Ile-Pyr-NH-$CH_2$-5-(3-am)-thioph<br>MS $[M+H]^+$ 498 |
| 297. | HOOC-p-$C_6H_4$-$CH_2$-D-Nva-Pyr-NH-$CH_2$-5-(3-am)-thioph<br>MS $[M+H]^+$ 484 |
| 298. | HOOC-p-$C_6H_4$-$CH_2$-D-Leu-Pyr-NH-$CH_2$-5-(3-am)-thioph<br>MS $[M+H]^+$ 498 |
| 299. | 4-benzoylbenzoyl-D-Nva-Pyr-NH-$CH_2$-5-(3-am)-thioph<br>MS $[M+H]^+$ 558 |
| 300. | p-carboxy-benzyl-D-Ala-Pyr-NH-$CH_2$-5-(3-am)-thioph<br>MS $[M+H]^+$ 456 |
| 301. | p-carboxy-benzyl-Acpc-Pyr-NH-$CH_2$-5-(3-am)-thioph<br>MS $[M+H]^+$ 496 |
| 302. | HOOC-p-$C_6H_4$-$CH_2$-N-Me-D-Val-Pyr-NH-$CH_2$-5-(3-am)-thioph<br>MS $[M+H]^+$ 498 |
| 303. | p-benzoyl-benzyl-D-Abu-Pyr-NH-$CH_2$-5-(3-am)-thioph<br>MS $[M+H]^+$ 530 |
| 304. | 2-carboxy-benzyl-D-Abu-Pyr-NH-$CH_2$-5-(3-am)-thioph<br>MS $[M+H]^+$ 470 |
| 305. | (4-COOH-CH=CH) -benzyl-D-Abu-Pyr-NH-$CH_2$-5-(3-am)-thioph<br>MS $[M+H]^+$ 496 |
| 306. | 4-carboxy-benzyl-D-Abu-3-Me-Pro-NH-$CH_2$-5-(3-am)-thioph<br>MS $[M+H]^+$ 486 |
| 307. | HOOC-p-$C_6H_4$-$CH_2$-D-Abu-5-Me-Pro-NH-$CH_2$-5-(3-am)-thioph<br>MS $[M+H]^+$ 486 |
| 308. | 2-(carboxymethyloxy)-benzyl-D-Abu-Pyr-NH-$CH_2$-5-(3-am)-thioph<br>MS $[M+H]^+$ 500 |
| 309. | benzyl-D-Abu-Pyr-NH-$CH_2$-5-(3-am)-thioph<br>MS $[M+H]^+$ 426 |
| 310. | 4-(carboxymethyloxy)-benzyl-D-Abu-Pyr-NH-$CH_2$-5-(3-am)-thioph<br>MS $[M+H]^+$ 500 |
| 311. | benzylsulfonyl-D-Abu-Pyr-NH-$CH_2$-5-(3-am)-thioph<br>MS $[M+H]^+$ 490 |
| 312. | HOOC-p-$C_6H_4$-$CH_2$-D-Abu-Pyr-NH-$CH_2$-5-(2-am)-thioph<br>MS $[M+H]^+$ 470 |
| 313. | 4-benzoyl-benzoyl-D-Pro-Pyr-NH-$CH_2$-5-(2-am)-thioph<br>MS $[M+H]^+$ 556 |

(fortgesetzt)

| | |
|---|---|
| 314. | HOOC-p-$C_6H_4$-$CH_2$-D-Pro-Pyr-NH-$CH_2$-5-(2-am)-thioph<br>MS $[M+H]^+$ 482 |
| 315. | HOOC-p-$C_6H_4$-$CH_2$-D-Pip-Pyr-NH-$CH_2$-5-(3-am)-thioph<br>MS $[M+H]^+$ 473 |
| 316. | HOOC-p-$C_6H_4$-$CH_2$-D-Abu-Pro-NH-$CH_2$-5-(3-am)-thioph<br>MS $[M+H]^+$ 472 |
| 317. | 4-carboxy-benzyl-D-allo-Ile-Pyr-NH-$CH_2$-5-(3-am)-thioph<br>MS $[M+H]^+$ 498 |
| 318. | 2-HOOC-thienyl (5)-$CH_2$-D-Abu-Pyr-NH-$CH_2$-5-(3-am)-thioph<br>MS $[M+H]^+$ 476 |
| 319. | 2-COOH-furanyl (5) -CO-D-Abu-Pyr-NH-$CH_2$-5-(3-am)-thioph<br>MS $[M+H]^+$ 460 |
| 320. | HOOC-p-$C_6H_4$-$CH_2$-D-Nle-Pyr-NH-$CH_2$-5-(3-am)-thioph<br>MS $[M+H]^+$ 498 |
| 321. | benzoyl-D-Abu-Pyr-NH-$CH_2$-5-(3-am)-thioph<br>MS $[M+H]^+$ 440 |
| 322. | 4-$MeSO_2$-$C_6H_4$-$CH_2$-D-Abu-Pyr-NH-$CH_2$-5-(3-am)-thioph<br>MS $[M+H]^+$ 504 |
| 323. | phenylsulfonyl-D-Chg-Pyr-NH-$CH_2$-5-(3-am)-thioph<br>MS $[M+H]^+$ 530 |
| 324. | phenylacetyl-D-Abu-Pyr-NH-$CH_2$-5-(3-am)-thioph<br>MS $[M+H]^+$ 454 |
| 325. | phenylsulfonyl-D-Abu-Pyr-NH-$CH_2$-5-(3-am)-thioph<br>MS $[M+H]^+$ 476 |
| 326. | naphthyl(1)-$CH_2$CO-D-Abu-Pyr-NH-$CH_2$-5-(3-am)-thioph<br>MS $[M+H]^+$ 504 |
| 327. | naphthyl-(2)-$CH_2$CO-D-Abu-Pyr-NH-$CH_2$-5-(3-am)-thioph<br>MS $[M+H]^+$ 504 |
| 328. | indanyl(1)-CO-D-Abu-Pyr-NH-$CH_2$-5-(3-am)-thioph<br>MS $[M+H]^+$ 480 |
| 329. | benzhydryl-CO-D-Abu-Pyr-NH-$CH_2$-5-(3-am)-thioph<br>MS $[M+H]^+$ 530 |
| 330. | 2-Cl-phenyl-$CH_2$CO-D-Abu-Pyr-NH-$CH_2$-5-(3-am)-thioph<br>MS $[M+H]^+$ 488 |
| 331. | 2,6-dichlorophenyl-$CH_2$CO-D-Abu-Pyr-NH-$CH_2$-5-(3-am)-thioph<br>MS $[M+H]^+$ 524 |
| 332. | 2-methyl-phenyl-$CH_2$CO-D-Abu-Pyr-NH-$CH_2$-5-(3-am)-thioph<br>MS $[M+H]^+$ 468 |
| 333. | biphenyl-$CH_2$CO-D-Abu-Pyr-NH-$CH_2$-5-(3-am)-thioph<br>MS $[M+H]^+$ 530 |
| 334. | p-methyl-phenyl-$CH_2$CO-D-Abu-Pyr-NH-$CH_2$-5-(3-am)-thioph<br>MS $[M+H]^+$ 468 |
| 335. | 3-methyl-phenyl-$CH_2$CO-D-Abu-Pyr-NH-$CH_2$-5-(3-am)-thioph<br>MS $[M+H]^+$ 468 |
| 336. | 2-nitro-phenyl-$CH_2$CO-D-Abu-Pyr-NH-$CH_2$-5-(3-am)-thioph<br>MS $[M+H]^+$ 499 |

(fortgesetzt)

| | |
|---|---|
| 337. | fluorenyl(1)-$CH_2$CO-D-Abu-Pyr-NH-$CH_2$-5-(3-am)-thioph<br>MS $[M+H]^+$ 542 |
| 338. | 2-Br-phenyl-$CH_2$CO-D-Abu-Pyr-NH-$CH_2$-5-(3-am)-thioph<br>MS $[M+H]^+$ 534 |
| 339. | 2-fluoro-phenyl-$CH_2$CO-D-Abu-Pyr-NH-$CH_2$-5-(3-am)-thioph<br>MS $[M+H]^+$ 472 |
| 340. | 2-phenyl-isobutyryl-D-Abu-Pyr-NH-$CH_2$-5-(3-am)-thioph<br>MS $[M+H]^+$ 482 |
| 341. | p-benzyloxy-benzoyl-D-Val-Pyr-NH-$CH_2$-5-(3-am)-thioph<br>MS $[M+H]^+$ 560 |
| 342. | 2,6-dichlorophenyl-$CH_2$CO-D-Abu-Pyr-NH-$CH_2$-5-(2-am)-thioph<br>MS $[M+H]^+$ 524 |
| 343. | 2,6-dichlorophenyl-$CH_2$CO-D-Val-Pyr-NH-$CH_2$-5-(3-am)-thioph<br>MS $[M+H]^+$ 538 |
| 344. | 2,6-dichloro-phenyl-$CH_2$CO-D-Chg-Pyr-NH-$CH_2$-5-(3-am)-thioph<br>MS $[M+H]^+$ 578 |
| 345. | naphthyl(1)-CO-D-Abu-Pyr-NH-$CH_2$-5-(3-am)-thioph<br>MS $[M+H]^+$ 490 |
| 346. | cyclopentyl-$CH_2$CO-D-Abu-Pyr-NH-$CH_2$-5-(3-am)-thioph<br>MS $[M+H]^+$ 446 |
| 347. | adamantyl(1)-CO-D-Abu-Pyr-NH-$CH_2$-5-(3-am)-thioph<br>MS $[M+H]^+$ 498 |
| 348. | cyclohexyl-$CH_2$CO-D-Abu-Pyr-NH-$CH_2$-5-(3-am)-thioph<br>MS $[M+H]^+$ 460 |
| 349. | thienyl(2)-$CH_2$CO-D-Abu-Pyr-NH-$CH_2$-5-(3-am)-thioph<br>MS $[M+H]^+$ 460 |
| 350. | naphthyl(2)-CO-D-Abu-Pyr-NH-$CH_2$-5-(3-am)-thioph<br>MS $[M+H]^+$ 490 |
| 351. | naphthyl(1)-$CH_2$-D-Abu-Pyr-NH-$CH_2$-5-(3-am)-thioph<br>MS $[M+H]^+$ 476 |
| 352. | naphthyl (2)-$CH_2$-D-Abu-Pyr-NH-$CH_2$-5-(3-am)-thioph<br>MS $[M+H]^+$ 476 |
| 353. | Benzyloxycarbonyl-D-Abu-Pyr-NH-$CH_2$-5-(3-am)-thioph<br>MS $[M+H]^+$ 470 |
| 354. | 4-MeOOC-benzyl-D-Val-Pyr-NH-$CH_2$-5-(3-ham) -thioph<br>MS $[M+H]^+$ 514 |
| 355. | 2-phenyl-2-hydroxy-acetyl-D-Abu-Pyr-NH-$CH_2$-5-(3-am)-thioph<br>MS $[M+H]^+$ 470 |
| 356. | 2-phenyl-2-methoxy-acetyl-D-Abu-Pyr-NH-$CH_2$-5-(3-am)-thioph<br>MS $[M+H]^+$ 484 |
| 357. | 2-(p-isobutyl-phenyl)propionyl-D-Abu-Pyr-NH-$CH_2$-5-(3-am)-thioph<br>MS $[M+H]^+$ 524 |
| 358. | (S)-2-phenyl-propionyl-D-Abu-Pyr-NH-$CH_2$-5-(3-am)-thioph<br>MS $[M+H]^+$ 468 |
| 359. | (R)-2-phenyl-propionyl-D-Abu-Pyr-NH-$CH_2$-5-(3-am)-thioph<br>MS $[M+H]^+$ 468 |

(fortgesetzt)

| | |
|---|---|
| 360. | 3-pyridyl-$CH_2$CO-D-Abu-Pyr-NH-$CH_2$-5-(3-am)-thioph<br>MS $[M+H]^+$ 455 |
| 361. | phenyl-O-$CH_2$CO-D-Abu-Pyr-NH-$CH_2$-5-(3-am)-thioph<br>MS $[M+H]^+$ 470 |
| 362. | adamantyl(1)-$CH_2$CO-D-Abu-Pyr-NH-$CF_2$-5-(3-am)-thioph<br>MS $[M+H]^+$ 512 |
| 363. | 2,4,6-trimethylphenyl-$CH_2$CO-D-Abu-Pyr-NH-$CH_2$-5-(3-am)-thioph<br>MS $[M+H]^+$ 496 |
| 364. | p-pentoxy-benzoyl-D-Val-Pyr-NH-$CH_2$-5-(3-am)-thioph<br>MS $[M+H]^+$ 540 |
| 365. | p-benzyloxy-phenyl-$CH_2$CO-D-Val-Pyr-NH-$CH_2$-5-(3-am)-thioph<br>MS $[M+H]^+$ 574 |
| 366. | indanyl(1)-CO-D-Val-Pyr-NH-$CH_2$-5-(3-am)-thioph<br>MS $[M+H]^+$ 494 |
| 367. | 2, 6-dichlorophenyl-$CH_2$CO-D-Val-Pyr-NH-$CH_2$-5-(2-am)-thioph<br>MS $[M+H]^+$ 538 |
| 368. | benzthienyl(2)-CO-D-Abu-Pyr-NH-$CH_2$-5-(3-am)-thioph<br>MS $[M+H]^+$ 496 |
| 369. | HOOC-p-$C_6H_4$-$CH_2$-D-Nva-Pyr-NH-3-(6-am)-pico<br>MS $[M+H]^+$ 465 |
| 370. | Tetrahydronaphthyl(2)-CO-D-Abu-Pyr-NH-$CH_2$-5-(3-am)-thioph<br>MS $[M+H]^+$ 494 |
| 371. | indanyl(1)-CO-D-Ile-Pyr-NH-$CH_2$-5-(3-am)-thioph<br>MS $[M+H]^+$ 508 |
| 372. | Benzocyclobutan(1)-CO-D-Abu-Pyr-NH-CH2-5-(3-am)-thioph<br>MS $[M+H]^+$ 466 |
| 373. | Benzocyclobutan(1)-CO-D-Val-Pyr-NH-$CH_2$-5-(3-am)-thioph<br>MS $[M+H]^+$ 480 |
| 374. | 2,4,6-trimethylphenyl-$CH_2$CO-D-Val-Pyr-NH-$CH_2$-5-(3-am)-thioph<br>MS $[M+H]^+$ 510 |
| 375. | indanyl(1)-CO-D-Chg-Pyr-NH-$CH_2$-5-(3-am)-thioph<br>MS $[M+H]^+$ 534 |
| 376. | indanyl(1)-CO-D-Leu-Pyr-NH-$CH_2$-5-(3-am)-thioph<br>MS $[M+H]^+$ 508 |
| 377. | indanyl(1)-CO-D-Phe-Pyr-NH-$CH_2$-5-(3-am)-thioph<br>MS $[M+H]^+$ 542 |
| 378. | anthracenyl(1)-CO-D-Abu-Pyr-NH-$CH_2$-5-(3-am)-thioph<br>MS $[M+H]^+$ 540 |
| 379. | benzylsulfonyl-D-Cha-Pyr-NH-$CH_2$-5-(3-am)-thioph<br>MS $[M+H]^+$ 558 |
| 380. | p-hexyloxy-benzoyl-D-Val-Pyr-NH-$CH_2$-5-(3-am)-thioph MS $[M+H]^+$ 554 |
| 381. | 2-(p-(phenyloxy)phenyl)-acetyl-D-Val-Pyr-NH-$CH_2$-5-(3-am)-thioph<br>MS $[M+H]^+$ 560 |
| 382. | (R)-indanyl(1)-CO-D-Abu-Pyr-NH-$CH_2$-5-(3-am)-thioph<br>MS $[M+H]^+$ 480 |

(fortgesetzt)

| | |
|---|---|
| 383. | indanyl(1)-CO-D-Val-Pyr-NH-$CH_2$-5-(2-am)-thioph<br>MS $[M+H]^+$ 494 |
| 384. | (S)-indanyl(1)-CO-D-Val-Pyr-NH-$CH_2$-5-(3-am)-thioph<br>MS $[M+H]^+$ 494 |
| 385. | butylsulfonyl-D-Phe-Pyr-NH-$CH_2$-5-(3-am)-thioph<br>MS $[M+H]^+$ 518 |
| 386. | (3,5-bistrifluormethyl)phenyl(1)-$CH_2$CO-D-Val-Pyr-NH-$CH_2$-5-(3-am)-thioph<br>MS $[M+H]^+$ 604 |
| 387. | (3-trifluormethyl)phenyl(1)-$CH_2$CO-D-Val-Pyr-NH-$CH_2$-5-(3-am)-thioph<br>MS $[M+H]^+$ 536 |
| 388. | 1-phenyl-cyclopropyl(1)-CO-D-Val-Pyr-NH-$CH_2$-5-(3-am)-thioph<br>MS $[M+H]^+$ 494 |
| 389. | (S)-indanyl(1)-CO-D-Val-Pyr-NH-$CH_2$-5-(3-am)-thioph<br>MS $[M+H]^+$ 494 |
| 390. | p-isopropyl-phenyl-$CH_2$CO-D-Val-Pyr-NH-$CH_2$-5-(3-am)-thioph<br>MS $[M+H]^+$ 510 |
| 391. | p-butoxyphenyl-$CH_2$CO-D-Val-Pyr-NH-$CH_2$-5-(3-am)-thioph<br>MS $[M+H]^+$ 540 |
| 392. | phenyl-CH(iPr)-CO-D-Val-Pyr-NH-$CH_2$-5-(3-am)-thioph<br>MS $[M+H]^+$ 510 |
| 393. | 1-(4-Cl-phenyl)-cyclobutyl(1)CO-D-Val-Pyr-NH-$CH_2$-5-(3-am)-thioph<br>MS $[M+H]^+$ 542 |
| 394. | 2-carboxy-thienyl(5)-$CH_2$-D-Val-Pyr-NH-$CH_2$-5-(3-am)-thioph<br>MS $[M+H]^+$ 490 |
| 395. | 1-phenyl-cyclopentyl(1)-CO-D-Val-Pyr-NH-$CH_2$-5-(3-am)-thioph<br>MS $[M+H]^+$ 522 |
| 396. | adamantyl(1)-$CH_2$CO-D-Val-Pyr-NH-$CH_2$-5-(3-am)-thioph<br>MS $[M+H]^+$ 526 |
| 397. | fluorenyl(1)-CO-D-Val-Pyr-NH-$CH_2$-5-(3-am)-thioph<br>MS $[M+H]^+$ 542 |
| 398. | benzhydryl-CO-D-Val-Pyr-NH-$CH_2$-5-(3-am)-thioph<br>MS $[M+H]^+$ 544 |
| 399. | (R)-indanyl(1)-CO-D-Val-Pyr-NH-$CH_2$-5-(2-am)-thioph<br>MS $[M+H]^+$ 494 |
| 400. | (S)-indanyl(1)-CO-D-Val-Pyr-NH-$CH_2$-5-(2-am)-thioph<br>MS $[M+H]^+$ 494 |
| 401. | p-COOH-benzoyl-D-Val-Pyr-NH-$CH_2$-5-(3-am)-thioph<br>MS $[M+H]^+$ 498 |
| 402. | 2-carboxy-furyl(5)-$CH_2$-D-Val-Pyr-NH-$CH_2$-5-(3-am)-thioph<br>MS $[M+H]^+$ 474 |
| 403. | p-COOMe-benzoyl-D-Val-Pyr-NH-$CH_2$-5-(3-am)-thioph<br>MS $[M+H]^+$ 512 |
| 404. | m-COOH-phenyl-$SO_2$-D-Chg-Pyr-NH-$CH_2$-5-(3-am)-thioph<br>MS $[M+H]^+$ 574 |
| 405. | p-COOH-phenyl-$SO_2$-D-Chg-Pyr-NH-$CH_2$-5-(3-am)-thioph<br>MS $[M+H]^+$ 574 |

In der folgenden Tabelle werden die $C_{1S}$ und $C_{1R}$-Inhibitionswerte für einige erfindungsgemäße Verbindungen aufgeführt.

| Beispiel Nr. | $C_{1S}$ $IC_{50}$ [µmol/l] gemäß Beispiel B | $C_{1R}$ $IC_{50}$ [µmol/l] gemäß Beispiel A |
|---|---|---|
| 29 | 0,6 | 0,9 |
| 22 | 0,6 | 0,9 |
| 23 | 0,8 | 0,5 |
| 24 | 0,8 | >100 |
| 42 | 1 | 0, 7 |
| 49 | 1 | 1 |
| 21 | 1 | 4 |
| 20 | 2 | 0,6 |
| 35 | 2 | 2 |
| 41 | 2 | 2 |
| 15 | 2 | 3 |
| 26 | 2 | >100 |
| 50 | 3 | 20 |
| 4 | 3 | 30 |
| 44 | 3 | 40 |
| 51 | 3 | 40 |
| 52 | 4 | 10 |
| 17 | 4 | 40 |
| 7 | 4 | >100 |
| 38 | 5 | 10 |
| 30 | 5 | >100 |
| 6 | 6 | |
| 25 | 6 | 50 |
| 1 | 6 | >100 |
| 8 | 6 | >100 |
| 18 | 7 | 10 |
| 54 | 8 | |
| 5 | 10 | |
| 39 | 10 | 2 |
| 31 | 10 | 3 |
| 43 | 10 | 6 |
| 13 | 10 | 30 |
| 45 | 20 | 6 |
| 53 | 20 | 8 |
| 27 | 20 | 10 |
| 46 | 20 | 40 |
| 2 | 20 | 50 |

(fortgesetzt)

| Beispiel Nr. | $C_{1S}$ $IC_{50}$ [µmol/l] gemäß Beispiel B | $C_{1R}$ $IC_{50}$ [µmol/l] gemäß Beispiel A |
|---|---|---|
| 34 | 20 | 70 |
| 9 | 20 | >100 |
| 28 | 20 | >100 |
| 16 | 20 | >100 |
| 10 | 20 | >100 |
| 14 | 20 | >100 |
| 32 | 30 | 10 |
| 19 | 30 | 30 |
| 48 | 30 | 50 |
| 3 | 30 | >100 |
| 11 | 30 | >100 |
| 12 | 30 | >100 |
| 35 | 40 | 20 |
| 33 | 40 | 40 |
| 47 | 50 | 10 |

## Patentansprüche

**1.** Verbindungen der allgemeinen Formel I,

$$A—B—D—E—G—NH—CH_2—Q^K—L \qquad (I)$$

deren Tautomere, pharmakologisch verträglichen Salze und Prodrugs, wobei gilt:

A steht für
$H$, $C_{1-6}$-Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{3-8}$-Cycloalkyl-$C_{1-3}$-Alkyl, $C_{1-3}$-Alkylaryl), $R^{A2}R^{A3}NCO$ ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^{A2}$ ($R^{A4}$ gleich $C_{1-6}$-Alkyl, $C_{1-3}$-Alkylaryl), $R^{A4}CONR^{A2}$, $R^{A1}O$, $R^{A2}R^{A3}N$, HO-$SO_2$-, Phenoxy, $R^{A2}R^{A3}N$-$SO_2$, Cl, Br, F, Tetrazolyl, $H_2O_3PNO_2$, $R^{A1}N(OH)$-CO-, $R^{A1}R^{A2}NCONR^{A3}$. wobei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B} -L^B -(CH_2)_{m^B} -$$

mit

$l^B$ = 0, 1, 2;
$m^B$ = 0, 1, 2;
$L^B$ gleich

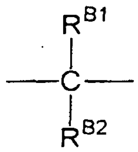, 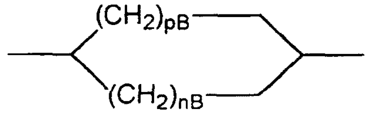, 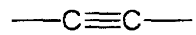, 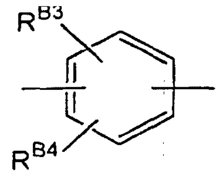

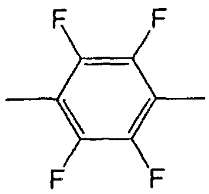, 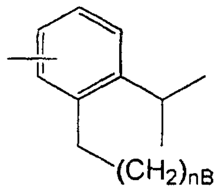

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

| | |
|---|---|
| $n^B$ | = 0, 1; |
| $p^B$ | = 0, 1; |
| $R^{B1}$ | gleich $C_{0-3}$-Alkylaryl, $C_{0-3}$-Alkylheteroaryl, $C_{0-3}$-Alkyl-$C_{3-8}$-Cycloalkyl; |
| $R^{B2}$ | gleich H, $C_{1-6}$-Alkyl, $C_{0-3}$-Alkylaryl, $C_{0-3}$-Alkylheteroaryl; |
| $R^{B3}$ | gleich H, $C_{1-6}$-Alkyl, $R^{B5}OCO$ ($R^{B5}$ gleich H, $C_{1-6}$-Alkyl), $R^{B6}$-O ($R^{B6}$ gleich H, $C_{1-6}$-Alkyl), F, Cl, Br, $NO_2$, $CF_3$; |
| $R^{B4}$ | gleich H, $C_{1-6}$-Alkyl, $R^{B6}$-O, Cl, Br, F, $CF_3$; |
| $R^{B1}$ und $R^{B2}$ | können auch miteinander verbunden sein; |
| B | steht weiterhin für |

$$-(CH_2)_{l^B} - L^B - M^B - L^B - (CH_2)_{m^B}-,$$

wobei

$l^B$ und $m^B$ oben angegebene Bedeutung besitzen und die beiden Gruppen $L^B$ unabhängig voneinander für die unter $L^B$ genannten Reste stehen;

$M^B$ bedeutet Einfach-Bindung, O, S, $CH_2$, $CH_2$-$CH_2$, $CH_2$-O, O-$CH_2$, $CH_2$-S, S-$CH_2$, CH=CH, C≡C;
B steht weiterhin für
-adamantyl(1)-$CH_2$-, -adamantyl (2)-$CH_2$-,

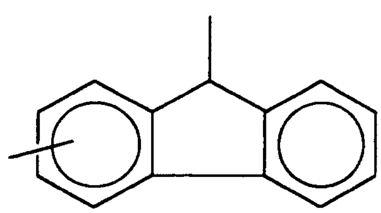, 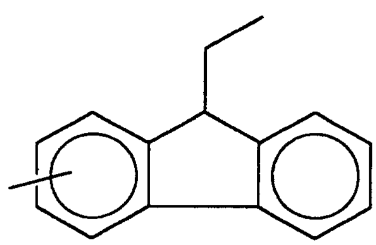,

B kann weiterhin stehen für

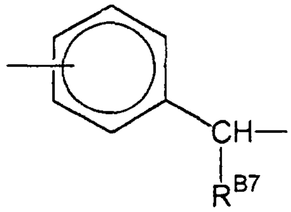 , 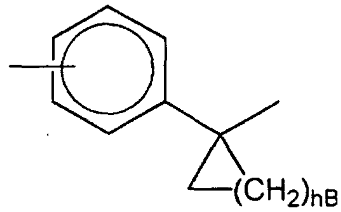

$h^B$ gleich 1, 2, 3, 4;

$R^{B7}$ gleich $C_{1-6}$-Alkyl, $C_{3-8}$-Cycloalkyl;

B kann weiterhin stehen für

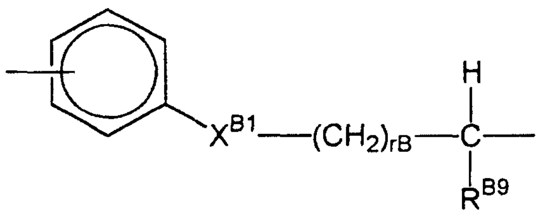

mit $X^{B1}$ gleich einer Bindung, O, S, oder

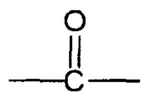 ;

mit $r^B$ gleich 0, 1, 2, 3;
mit $R^{B9}$ gleich H, $C_{1-3}$-Alkyl;
A-B kann stehen für

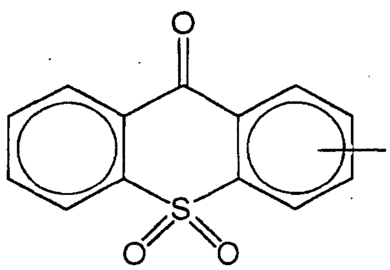 , 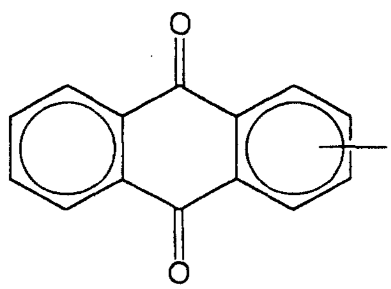

D steht für eine Einfach Bindung bzw. für CO, OCO, $NR^{D1}$-CO-($R^{D1}$ gleich H, $D_{1-4}$-Alkyl, $C_{0-3}$-Alkylaryl), $SO_2$, $NR^{D1}SO_2$;

E steht für

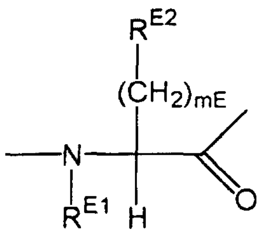

mit

$m^E$ = 0, 1, 2, 3;
$R^{E1}$ bedeutet H, $C_{1-6}$-Alkyl;
$R^{E2}$ bedeutet H, $C_{1-6}$-Alkyl, $C_{3-8}$-Cycloalkyl, wobei die vorgenannten Reste bis zu drei Substituenten der Gruppe $C_{1-6}$-Alkyl, F tragen können, $CH(CH_3)OH$, $CH(CF_3)_2$;

die unter $R^{E1}$ und $R^{E2}$ genannten Gruppen können über eine Bindung miteinander verknüpft sein

E kann auch stehen für D-Asp, D-Glu, D-Lys, D-Orn, D-His, D-Dab, D-Dap, D-Arg;
G bedeutet

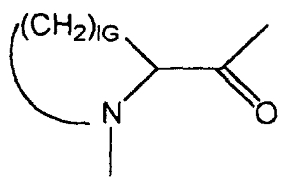 ,

mit $l^G$ = 2, 3, wobei eine $CH_2$-Gruppe des Rings durch S, $CHCH_3$ ersetzt sein kann;

G bedeutet weiterhin

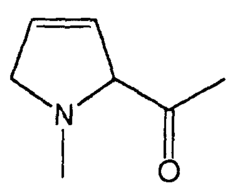 ,

$Q^K$ gleich

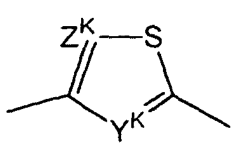 , 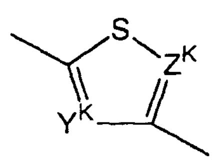

$Y^K$ gleich =CH-, =N-;
$Z^K$ gleich=CH-, =N-;

L:

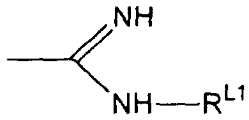

bzw.

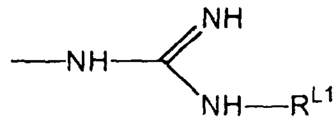

mit

$R^{L1}$ gleich H, OH, CO-$C_{1-6}$-Alkyl, $CO_2$-$C_{1-6}$-Alkyl, $CO_2$-$C_{1-3}$-Alkylaryl.

**2.** Verbindungen der allgemeinen Formel I,

$$A—B—D—E—G—NH—CH_2—Q^K—L \qquad (I)$$

deren Tautomere, pharmakologisch verträglichen Salze und Prodrugs, wobei gilt:

A steht für
H, $C_{1-6}$-Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{3-8}$-Cycloalkyl-$C_{1-3}$-Alkyl, $C_{1-3}$-Alkylaryl), $R^{A2}R^{A3}NCO$ ($R^{A2}$ gleich H-, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^2$ ($R^{A4}$ gleich $C_{1-6}$-Alky, $C_{1-3}$-Alkylaryl), $R^{A4}CONR^{A2}$, $R^{A1}O$, $R^{A2}R^{A3}N$, HO-$SO_2$-, Phenoxy, $R^{A2}R^{A3}N$-$SO_2$, Cl, Br, F, Tetrazolyl, $H_2O_3P$-, $NO_2$, $R^{A1}$-N(OH)-CO-, $R^{A1}R^{A2}NCONR^{A3}$, wobei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B} - L^B -(CH_2)_{m^B} -$$

mit
$l^B$ = 0, 1;
$m^B$ = 0, 1, 2;
$L^B$ gleich

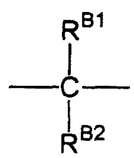

,

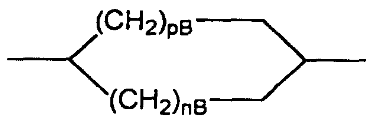

,

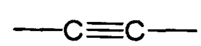

,

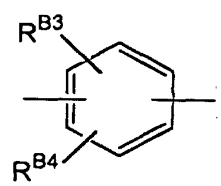

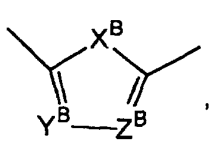

,

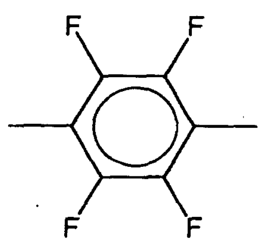

,

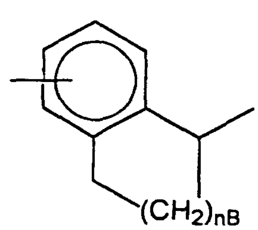

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

$n^B$ = 0, 1;
$p^B$ = 0, 1;
$R^{B1}$ gleich $C_{0-3}$-Alkylaryl, $C_{0-3}$-Alkylheteroaryl, $C_{0-3}$-Alkyl-$C_{3-8}$-Cycloalkyl;
$R^{B2}$ gleich H, $C_{1-6}$-Alkyl, $C_{0-3}$-Alkylaryl, $C_{0-3}$-Alkylheteroaryl;
$R^{B3}$ gleich H, $C_{1-6}$-Alkyl, $R^{B5}OCO$ ($R^{B5}$ gleich H, $C_{1-6}$-Alkyl), $R^{B6}$-O ($R^{B6}$ gleich H, $C_{1-6}$-Alkyl), F, Cl, Br, $NO_2$, $CF_3$;
$R^{B4}$ gleich H, $C_{1-6}$-Alkyl, $R^{B6}$-O, Cl, Br, F, $CF_3$;
$R^{B1}$ und $R^{B2}$ können auch miteinander verbunden sein;
$X^B$ gleich O, S;
$Y^B$ gleich =CH-, =N-;
$Z^B$ gleich =CH-, =N-;
B steht weiterhin für

$$-(CH_2)_{l^B}-L^B-M^B-L^B-(CH_2)_{m^B}-$$

wobei

$l^B$ und $m^B$ oben angegebene Bedeutung besitzen und die beiden Gruppen $L^B$ unabhängig voneinander für die unter $L^B$ genannten Reste -C≡C-,

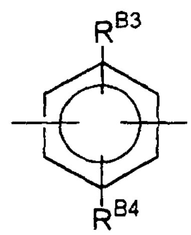 , 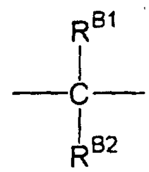

stehen

$M^B$ bedeutet Einfach-Bindung, O, $CH_2$-S, S-$CH_2$, CO, $SO_2$, $CH_2$-O;
B kann weiterhin stehen für

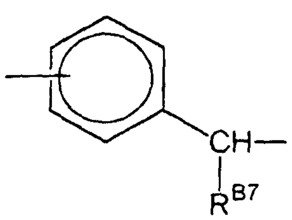 , 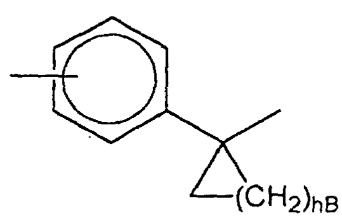

$h^B$ gleich 1, 2, 3, 4;
$R^{B7}$ gleich $C_{1-6}$-Alkyl, $C_{3-8}$-Cycloalkyl
B kann weiterhin stehen für Fluorenyl(1)-, Adamantyl(1)-, Adamantyl(1)-$CH_2$-
A-B kann stehen für Pyndy)(2)-$CH_2$-, Benzthienyl(2)-, Benzthienyl(3)-,

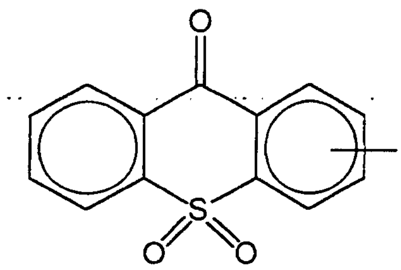 , 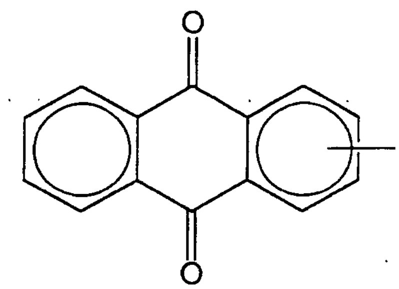

D steht für eine Einfach-Bindung bzw. für CO, $SO_2$;
E steht für

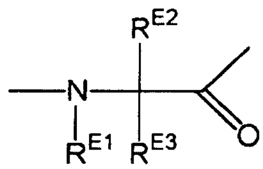

mit

$R^{E1}$ bedeutet H, $CH_3$;
$R^{E2}$ bedeutet H, $C_{1-6}$-Alkyl, $C_{3-8}$-Cycloalkyl, Thienyl, $CH(CH_3)OH$, $CH(CF_3)_2$;
$R^{E3}$ bedeutet H;

die unter $R^{E1}$ und $R^{E2}$ genannten Gruppen können über eine Bindung miteinander verknüpft sein; auch die unter $R^{E2}$ und $R^{E3}$ genannten Gruppen können über eine Bindung miteinander verbunden sein;

E kann auch stehen für D-Lys, D-Orn, D-Dab, D-Dap, D-Arg;
G bedeutet

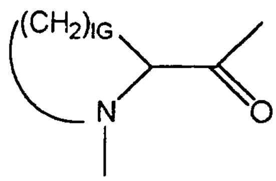 ,

mit $l^G$ = 2, 3, wobei eine $CH_2$-Gruppe des Rings durch $CHCH_3$ ersetzt sein kann;

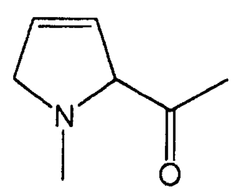

$Q^K$ gleich

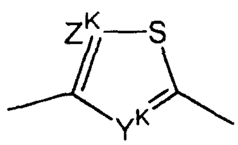, 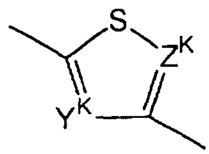, 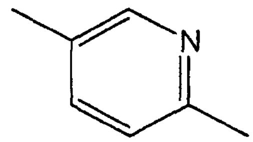

$Y^K$ gleich =CH-, =N-;
$Z^K$ gleich =CH-, =N-;
L:

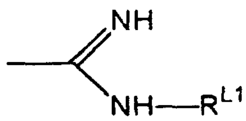 bzw. 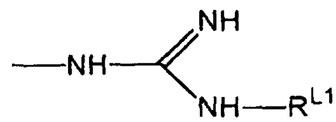

$R^{L1}$ gleich H, OH.

**3.** Verbindungen der allgemeinen Formel I,

$$A—B—D—E—G—NH—CH_2—Q^K—L \qquad (I)$$

deren Tautomere, pharmakologisch verträglichen Salze und Prodrugs, wobei gilt:

A steht für
H, $C_{1-6}$-Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl), $R^{A2}R^{A3}NCO$ ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^{A2}$, $R^{A4}CONR^{A2}$ ($R^{A4}$ gleich $C_{1-6}$-Alkyl, $C_{1-3}$-Alkylaryl), $R^{A1}O$, Phenoxy, $R^{A2}R^{A3}N$, HO-$SO_2$, $R^{A2}R^{A3}N$-$SO_2$, Cl, Br, F, Tetrazolyl, $H_2O_3P$, $NO_2$, $R^{A1}$-N(OH)-CO, $R^{A1}R^{A2}NCONR^{A3}$, wobei Aryl jeweils mit bis zu 2 gleichen oder verschiedenen Resten aus der Gruppe F, Cl, Br, $OCH_3$, $CH_3$, $CF_3$, $NO_2$ substituiert sein kann;
B steht für

$$-(CH_2)_{l^B}-L^B-(CH_2)_{m^B}-$$

mit
$l^B$ = 0, 1, 2;
$m^B$ =0,1,2;
$L^B$ gleich

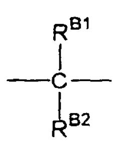, 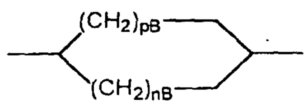, 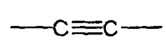, 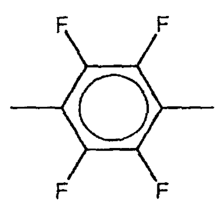, 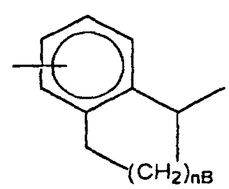

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$ ($R^8$ gleich H, $C_{1-3}$-Alkyl) substituiert sein kann;
mit

$n^B$ = 0, 1, 2;
$p^B$ = 0, 1, 2;
$R^{B1}$ gleich $C_{0-3}$-Alkylaryl, $C_{0-3}$-Alkylheteroaryl, $C_{0-3}$-Alkyl-$C_{3-8}$-Cycloalkyl;

| | |
|---|---|
| $R^{B2}$ | gleich H, $C_{1-6}$-Alkyl, $C_{0-3}$-Alkylaryl, $C_{0-3}$-Alkylheteroaryl, |
| $R^{B1}$ und $R^{B2}$ | können auch miteinander verbunden sein; |
| B | steht weiterhin für -adamantyl(1)-$CH_2$-, -adamantyl(2)-$CH_2$-, |

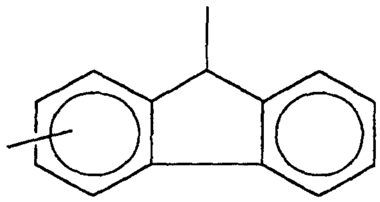
,
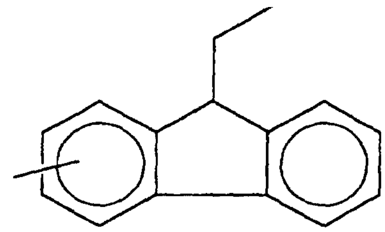
,

B steht weiterhin

$$-(CH_2)_{l^B} - L^{B1} - M^B - L^{B2} - (CH_2)_{m^B} -$$

wobei

$l^B$ und $m^B$ oben angegebene Bedeutung besitzen und die beiden Gruppen $L^{B1}$ und $L^{B2}$ unabhängig voneinander für folgende Reste stehen:

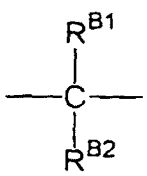
,
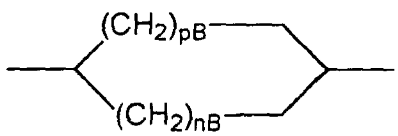
,
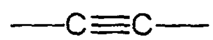
,
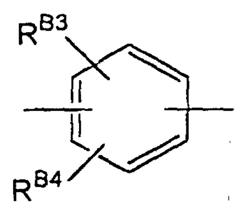

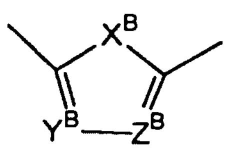
,
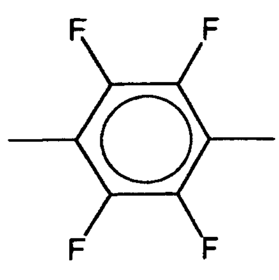
,
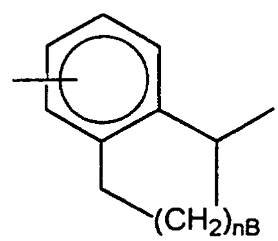

wobei in den vorgenannten Ringsystemen jeweils ein Phenylring ankondensiert sein kann; mit

| | |
|---|---|
| $n^B$ | = 0, 1, 2; |
| $p^B$ | =0,1,2; |
| $R^{B1}$ | gleich H (nur für $L^{B2}$), $C_{1-6}$-Alkyl (nur für $L^{B2}$), $C_{0-3}$-Alkylaryl, $C_{0-3}$-Alkylheteroaryl, $C_{0-3}$-Alkyl-$C_{3-8}$-Cycloalkyl; |
| $R^{B2}$ | gleich H, $C_{1-6}$-Alkyl, $C_{0-3}$-Alkylaryl, $C_{0-3}$-Alkylheteroaryl; |
| $R^{B3}$ | gleich H, $C_{1-6}$-Alkyl, Aryl, Heteroaryl, $R^{B5}$OCO ($R^{B5}$ gleich H, $C_{1-6}$-Alkyl), $R^{B6}$-O ($R^{B6}$ gleich H, $C_{1-6}$-Alkyl), F, Cl, Br, $NO_2$ $CF_3$; |
| $R^{B4}$ | gleich H, $C_{1-6}$-Alkyl, $R^{B6}$-O, Cl, Br, F, $CF_3$; |
| $X^B$ | gleich O, S; |
| $Y^B$ | gleich =CH-, =N-; |
| $Z^B$ | gleich =CH-, =N-; |
| $R^{B1}$ und $R^{B2}$ | können auch miteinander verbunden sein; |

$M^B$ bedeutet Einfach-Bindung, O, S, $CH_2$, $CH_2$-$CH_2$, $CH_2$-O, O-$CH_2$, $CH_2$-S, S-$CH_2$, CO, $SO_2$, CH=CH, C≡C;

B kann weiterhin stehen für

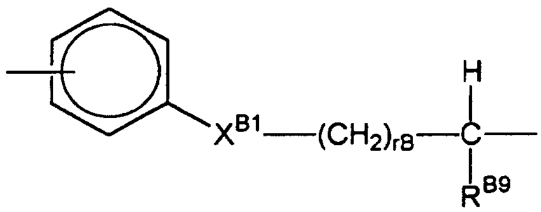

mit $X^{B1}$ gleich eine Bindung O, S oder

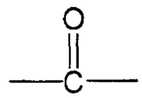 ;

mit $r^B$ gleich 0, 1, 2, 3;
mit $R^{B9}$ gleich H, $C_{1-3}$-Alkyl;
A-B kann stehen für

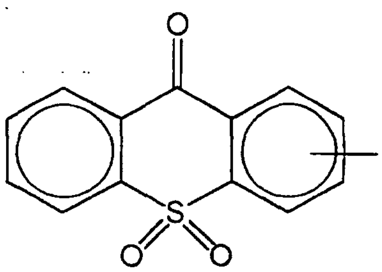 , 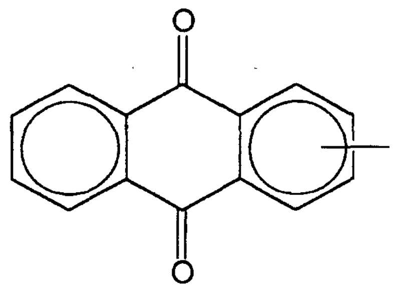

D steht für eine Einfach-Bindung bzw.
für CO, OCO, $NR^{D1}$-CO ($R^{D1}$ gleich H, $C_{1-4}$-Alkyl, $C_{0-3}$-Alkyl-aryl), $SO_2$, $NR^{D1}SO_2$;

B-D kann stehen für

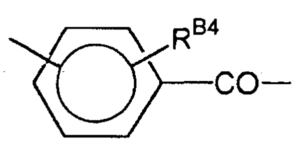

E steht für

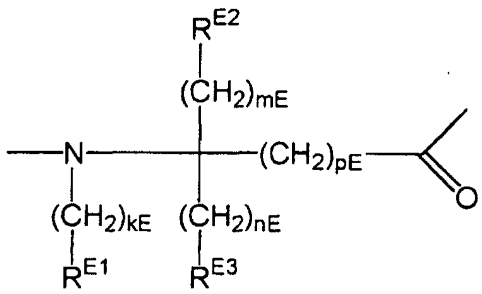

$k^E$ = 0, 1;
$m^E$ = 0,1;
$n^E$ = 0, 1;
$p^E$ = 0, 1;
$R^{E1}$ bedeutet H, $C_{1-6}$-Alkyl, $C_{3-8}$-Cycloalkyl, Phenyl, Naphthyl, Pyridyl, Thienyl, $C_{3-8}$-Cycloalkyl mit ankondensiertem Phenylring;
$R^{E2}$ bedeutet H, $C_{1-6}$-Alkyl, $C_{3-8}$-Cycloalkyl, Phenyl, Pyridyl, Thienyl, Furyl, Imidazolyl, Tetrahydropyranyl, Tetrahydrothiopyranyl, $CH(CH_3)OH$, $CH(CF_3)_2$;
$R^{E3}$ bedeutet H, $C_{1-6}$-Alkyl, $C_{3-8}$-Cycloalkyl, Phenyl;

die unter $R^{E1}$ und $R^{E2}$ genannten Gruppen können über eine Bindung miteinander verknüpft sein; auch die unter $R^{E2}$ und $R^{E3}$ genannten Gruppen können über eine Bindung miteinander verbunden sein;

E kann auch stehen für D-Asp, D-Glu, D-Lys, D-Orn, D-His, D-Dab, D-Dap, D-Arg;

G bedeutet

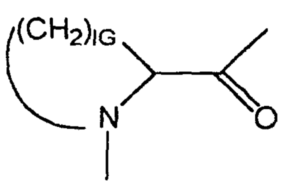 ,

mit $l^G$ = 2, 3, 4, wobei eine $CH_2$-Gruppe des Rings durch $CHCH_3$ ersetzt sein kann

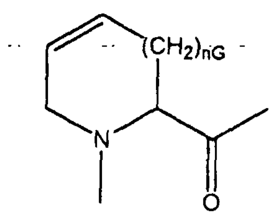

mit

$n^G$ = 0, 1;

weiterhin steht G für

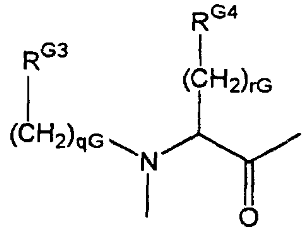

$q^G$ 0, 1;
$r^G$ 0, 1;
$R^{G3}$ H, $C_{1-6}$-Alkyl, $C_{3-8}$-Cycloalkyl;
$R^{G4}$ H, $C_{1-6}$-Alkyl, $C_{3-8}$-Cycloalkyl, Phenyl;
$Q^K$ gleich

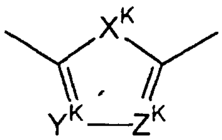

$X^K$ gleich O, S;
$Y^K$ gleich =CH-, =N-;
$Z^K$ gleich =CH-, =N-;
L:

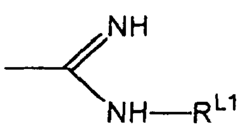

bzw.

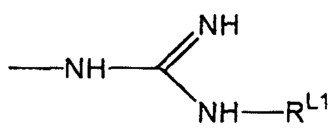

mir
$R^{L1}$ gleich H, OH, CO-$C_{1-6}$-Alkyl, $CO_2$-$C_{1-6}$-Alkyl, $CO_2$-$C_{1-5}$-Alkylaryl.

**4.** Verbindungen der allgemeinen Formel 1,

$$A—B—D—E—G—NH—CH_2—Q^K—L \quad (I)$$

deren Tautomere, pharmakologisch verträglichen Salze und Prodrugs, wobei gilt:

A steht für
H, $C_{1-6}$-Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl), $R^{A2}R^{A3}NCO$ ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^{A2}$, $R^{A4}CONR^{A2}$ ($R^{A4}$ gleich $C_{1-6}$-Alkyl, $C_{1-3}$-Alkylaryl), $R^{A1}O$, Phenoxy, $R^{A2}R^{A3}N$, HO-$SO_2$, $R^{A2}R^{A3}N$-$SO_2$, Cl, Br, F, Tetrazolyl, $H_2O_3P$, $NO_2$, $R^{A1}$-N(OH)-CO, $R^{A1}R^{A2}NCONR^{A3}$, wobei Aryl jeweils mit bis zu 2 gleichen oder verschiedenen Resten aus der Gruppe F, Cl, Br, $OCH_3$, $CH_3$, $CF_3$, $NO_2$ substituiert sein kann;

B steht für

$$-(CH_2)_{l^B}-L^B-(CH_2)_{m^B}-$$

mit

$l^B$ = 0, 1;

$m^B$ = 0, 1, 2;

$L^B$ gleich

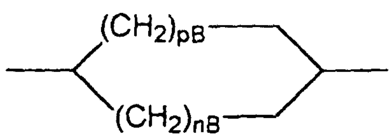 , 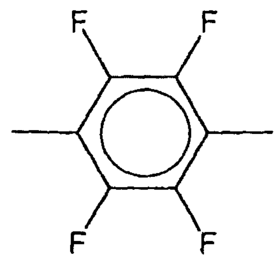 , 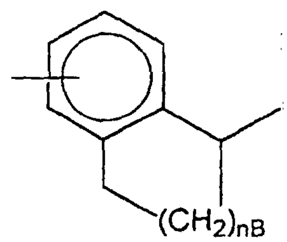

wobei in den vorgenannten Ringsystemen jeweils ein Phenylring ankondensiert sein kann, der mit bis zu 2 gleichen oder verschiedenen Resten aus der Gruppe $CH_3$, $CF_3$, Br, Cl, F substituiert sein kann, oder mit $R^8OOC$- ($R^8$ gleich H, $C_{1-3}$-Alkyl) substituiert sein kann;
mit

$n^B$ = 0,1;
$p^B$ = 0, 1;
B steht weiterhin für adamantyl(1)-$CH_2$-, -adamantyl(2)-$CH_2$-,

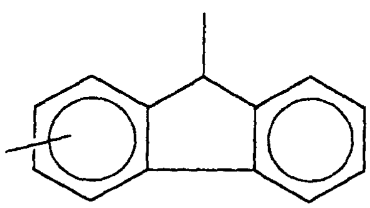

B steht weiterhin für

$$-(CH_2)_{l^B}-L^{B1}-M^B-L^{B2}-(CH)_{m^B}-$$

wobei

$l^B$ und $m^B$ die oben angegebene Bedeutung besitzen und die beiden Gruppen $L^{B1}$ und $L^{B2}$ unabhängig voneinander für folgende Reste stehen:

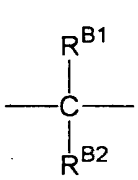 , 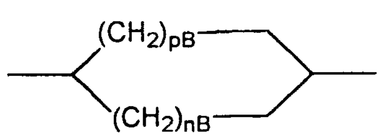 , 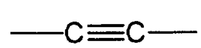 , 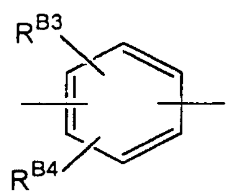 ,

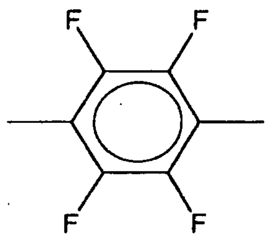
,
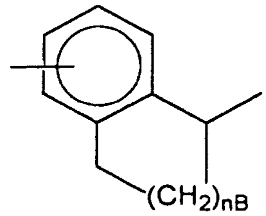

wobei in den vorgenannten Ringsystemen jeweils ein Phenylring ankondensiert sein kann;
mit

| | |
|---|---|
| $n^B$ | = 1; |
| $p^B$ | = 0, 1; |
| $R^{B1}$ | gleich H (nur für $L^{B2}$), $C_{1-6}$-Alkyl (nur für $L^{B2}$), $C_{0-3}$-Alkylaryl, $C_{0-3}$-Alkylheteroaryl, $C_{0-3}$-Alkyl-$C_{3-8}$-Cycloalkyl; |
| $R^{B2}$ | gleich H, $C_{1-6}$-Alkyl, $C_{0-3}$-Alkylaryl, $C_{0-3}$-Alkylheteroaryl; |
| $R^{B3}$ | gleich H, $C_{1-6}$-Alkyl, $R^{B6}$-O ($R^{B6}$ gleich H, $C_{1-6}$-Alkyl) , F, Cl, Br, $NO_2$, $CF_3$; |
| $R^{B4}$ | gleich H, $C_{1-6}$-Alkyl, $R^{B6}$-O, Cl, Br, F, $CF_3$; |
| $R^{B1}$ und $R^{B2}$ | können auch miteinander verbunden sein; |
| $M^B$ | bedeutet Einfach-Bindung, O, S, $CH_2$, $CH_2$-$CH_2$, $CH_2$-O, O-$CH_2$, $CH_2$-S, S-$CH_2$, CO, $SO_2$; |
| A-B | kann stehen für Pyridyl(2)-$CH_2$-, Benzthienyl(2)-, |

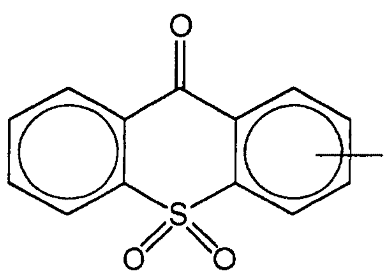
,
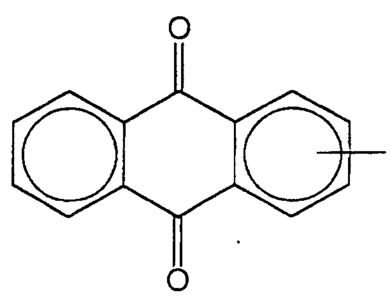

| | |
|---|---|
| D | steht für eine Einfach-Bindung bzw. für CO, $SO_2$; |
| B-D | kann stehen für |

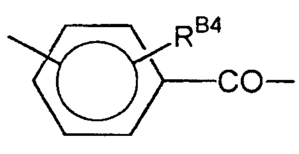

E steht für

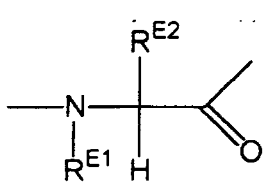

| | |
|---|---|
| $R^{E1}$ | bedeutet H; |
| $R^{E2}$ | bedeutet H, $C_{1-6}$-Alkyl, $C_{3-8}$-Cycloalkyl, Phenyl, Pyridyl, Thienyl, Furyl, Imidazolyl, Tetrahydropy- |

ranyl, Tetrahydrothiopyranyl, $CH(CH_3)OH$, $CH(CF_3)_2$;

die unter $R^{E1}$ und $R^{E2}$ genannten Gruppen können über eine Bindung miteinander verknüpft sein;

E kann auch stehen für D-Lys, D-Orn, D-Dab, D-Dap, D-Arg;

G bedeutet

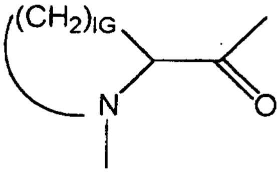

mit $l^G$ = 2, 3, wobei eine $CH_2$-Gruppe des Rings durch $CHCH_3$ ersetzt sein kann

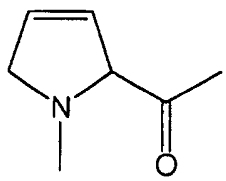

,

$Q^K$ gleich

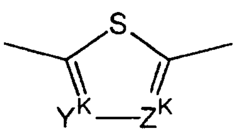

$Y^K$ gleich =CH-, =N-;
$Z^K$ gleich =CH-, =N-;

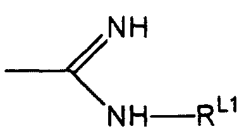

bzw.

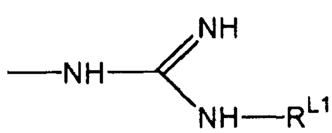

L:
mit

$R^{L1}$ gleich H, OH.

**5.** Verbindungen der allgemeinen Formel I,

$$A—B—D—E—G—NH—CH_2—Q^K—L \quad (I)$$

deren Tautomere, pharmakologisch verträglichen Salze und Prodrugs, wobei gilt:

A steht für
H, $C_{1-6}$ Alkyl, $C_{1-6}$-Alkyl-$SO_2$, $R^{A1}OCO$ ($R^{A1}$ gleich H, $C_{1-12}$-Alkyl, $C_{3-8}$-Cycloalkyl, $C_{1-3}$-Alkyl-$C_{3-8}$-Cycloalkyl, $C_{1-3}$-Alkylaryl) , $R^{A2}R^{A3}NCO$ ($R^{A2}$ gleich H, $C_{1-6}$-Alkyl, $C_{0-3}$-Alkylaryl, $C_{0-3}$-Alkylheteroaryl, $R^{A3}$ gleich H, $C_{1-6}$-Alkyl, $C_{0-3}$-Alkylaryl), $R^{A4}OCONR^{A2}$ ($R^{A4}$ gleich $C_{1-6}$-Alkyl, $C_{1-3}$-Alkylaryl), $R^{A4}CONR^{A2}$, $R^{A1}O$, $R^{A2}R^{A3}N$, HO-$SO_2$-, Phenoxy, $R^{A2}R^{A3}N$-$SO_2$, Cl, Br, F, Tetrazolyl, $H_2O_3P$-, $NO_2$, $R^{A1}$-N(OH)-CO-, $R^{A1}R^{A2}NCONR^{A3}$, wobei Aryl jeweils mit bis zu 2 gleichen oder verschiedenen Resten aus der Gruppe F, Cl, Br, $CF_3$, $CH_3$, $OCH_3$, $NO_2$ substituiert sein kann;

B steht für

$$-(CH_2)_{l^B}-L^B-(CH_2)_{m^B}-$$

mit
$l^B$ =0,1;
$m^B$ = 0, 1, 2;
$L^B$ gleich

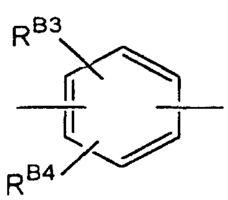 , 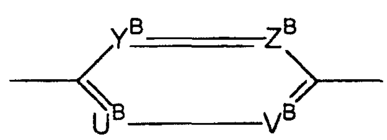 , 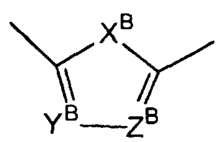 , 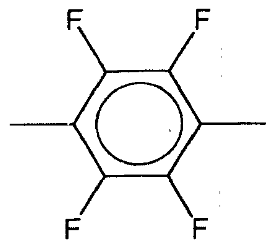

wobei in den vorgenannten Ringsystemen jeweils ein Phenylring ankondensiert sein kann;

$R^{B3}$ gleich H, $C_{1-6}$-Alkyl, Aryl, $R^{B5}OCO$ ($R^{B5}$ gleich H, $C_{1-6}$-Alkyl, $C_{1-3}$-Alkylaryl), $R^{B6}$-O ($R^{B6}$ gleich H, $C_{1-6}$-Alkyl), F, Cl, Br, $NO_2$, $CF_3$;
$R^{B4}$ gleich H, $C_{1-6}$-Alkyl, $R^{B6}$-O, Cl, Br, F, $CF_3$;
$X^B$ gleich O, S;
$Y^B$ gleich =CH-, =N-;
$Z^B$ gleich =CH-, =N-;
$U^B$ gleich =CH-, =N-;
$V^B$ gleich =CH-, =N-;
B kann weiterhin stehen für

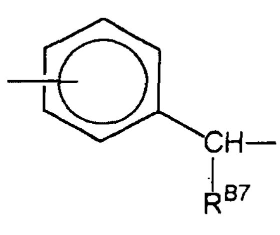 , 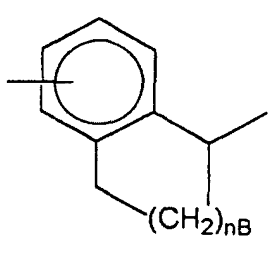

$q^B$ gleich 0, 1, 2;

($R^{B7}$ gleich $C_{1-6}$-Alkyl, $C_{3-8}$-Cycloalkyl)

A-B kann stehen für

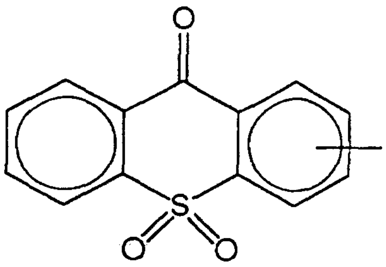,
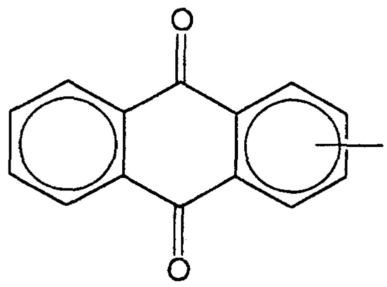

D steht für eine Einfach-Bindung

E steht für

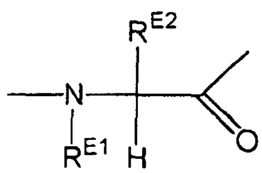

mit

$R^{E1}$ bedeutet H;

$R^{E2}$ bedeutet H, $C_{1-6}$-Alkyl, $C_{3-8}$-Cycloalkyl, Phenyl, Pyridyl, Furyl, Thienyl, Imidazolyl, Tetrahydropyranyl, Tetrahydrothiopyranyl, wobei die vorgenannten Reste bis zu drei gleiche oder verschiedene Substituenten der Gruppe O-$C_{1-6}$-Alkyl, F tragen können, $CH(CH_3)OH$, $CH(CF_3)_2$;

die unter $R^{E1}$ und $R^{E2}$ genannten Gruppen können über eine Bindung miteinander verknüpft sein;

E kann auch stehen für D-Lys, D-Orn, D-Dab, D-Dap, D-Arg;

G bedeutet

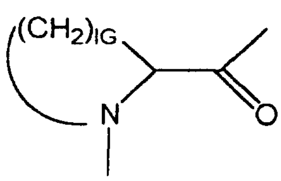

mit $l^G$ = 2, 3, wobei eine $CH_2$-Gruppe des Rings durch $CHCH_3$ ersetzt sein kann;

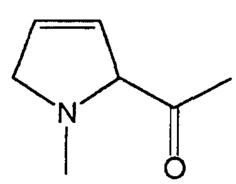,

$Q^K$ gleich

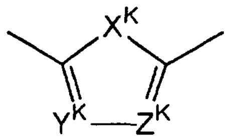

$X^K$ gleich O, S;

$Y^K$ gleich=CH-, =N-;

$Z^K$ gleich =CH-, =N-;

L:

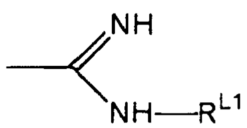

bzw.

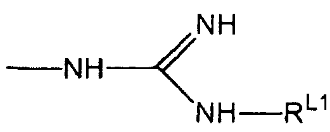

$R^{L1}$ gleich -H, -OH.

**6.** Arzneimittel enthaltend neben üblichen Trägern und Hilfsstoffen Verbindungen der allgemeinen Formel I nach einem der Ansprüche 1 bis 5.

**7.** Verwendung von Verbindungen der allgemeinen Formel I nach einem der Ansprüche 1 bis 5 zur Herstellung von Arzneimitteln zur Behandlung von Krankheiten, die durch teilweise oder vollständige Inhibition von $C_{1s}$ oder $C_{1r}$ gelindert oder geheilt werden.

**8.** Verwendung von Verbindungen der allgemeinen Formel I nach einem der Ansprüche 1 bis 5 zur Herstellung von Arzneimitteln zur Behandlung oder Prophylaxe von

- Reperfusionsschäden nach Ischämien; Ischämische Zustände treten ein während z.B. Operationen unter Zuhilfenahme von Herz-Lungenmaschinen; Operationen, in denen Blutgefäße generell zur Vermeidung großer Blutungen abgeklemmt werden; Myokardinfarkt; thromboembolischer Hirnschlag; Lungenthrombosen etc.;
- Hyperakute Organabstoßung; speziell bei Xenotransplantationen;
- Organversagen wie z.B. multiples Organversagen oder ARDS (adult respiratory distress syndrome);
- Krankheiten, die auf Trauma (Schädeltrauma) oder Polytrauma beruhen, wie z.B. Thermotrauma (Verbrennungen) und "thermal injury";
- Anaphylaktischer Schock;
- Sepsis; "vascular leak syndrom": bei Sepsis und nach Behandlung mit biologischen Agenzien, wie Interleukin 2 bzw. nach Transplantation;
- Alzheimer Krankheit sowie andere entzündliche neurologische Krankheiten wie Myastenia graevis, multiple Sklerose, zerebraler Lupus, Guillain-Barre Syndrome; Meningitiden; Encaphilitiden;
- Systemischer Lupus erythematosus (SLE),
- Rheumatoide Arthritis und andere entzündliche Krankheiten des rheumatoiden Krankheitskreises, wie z.B. Behcet's Syndrom; Juvenile rheumatoide Arthritis;
- Nierenentzündungen unterschiedlicher Genese, wie z.B. Glomerulonephritis, Lupus nephriti;
- Pankreatitis;
- Asthma; chronische Bronchitis;
- Komplikationen während Dialyse bei Nierenversagen;
- Vasculitis; Thyroiditis;
- Ulcerative Colitis sowie andere entzündliche Erkrankungen des Magen-Darmtraktes
- Autoimmunerkrankungen;
- spontanen Frühgeburten.

**9.** Verbindungen der allgemeinen Formel I nach einem der Ansprüche 1 bis 5, wobei L -CN, $-C(=O)NH_2$ oder $-C(=S)NH_2$ entspricht.

## Claims

**1.** Compounds of the general formula I,

$$A—B—D—E—G—NH—CH_2—Q^K—L \qquad (I)$$

the tautomers, pharmacologically acceptable salts and prodrugs thereof, wherein:

A denotes
H, $C_{1-6}$-alkyl, $C_{1-6}$-alkyl-$SO_2$, $R^{A1}OCO$ ($R^{A1}$ being H, $C_{1-12}$-alkyl, $C_{3-8}$-cycloalkyl, $C_{3-8}$-cycloalkyl-$C_{1-3}$-alkyl, $C_{1-3}$-alkylaryl), $R^{A2}R^{A3}NCO$ ($R^{A2}$ being H, $C_{1-6}$-alkyl, $C_{0-3}$-alkylaryl, $C_{0-3}$-alkylheteroaryl, $R^{A3}$ being H, $C_{1-6}$-alkyl, $C_{0-3}$-alkylaryl), $R^{A4}OCONR^{A2}$ ($R^{A4}$ being $C_{1-6}$-alkyl, $C_{1-3}$-alkylaryl), $R^{A4}CONR^{A2}$, $R^{A1}O$, $R^{A2}R^{A3}N$, HO—$SO_2$-, phenoxy, $R^{A2}R^{A3}N$-$SO_2$, Cl, Br, F, tetrazolyl, $H_2O_3P$-, $NO_2$, $R^{A1}$-N(OH)-CO-, $R^{A1}R^{A2}NCONR^{A3}$, wherein aryl may in each case be substituted with up to 2 identical or different residues from the group F, Cl, Br, $CF_3$, $CH_3$, -$OCH_3$, $NO_2$;

B denotes

$$-(CH_2)_{l^B}-L^B-(CH_2)_{m^B}-$$

with

$l^B$ = 0, 1, 2;
$m^B$ = 0, 1, 2;
$L^B$ being

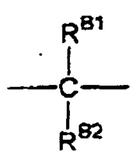 , 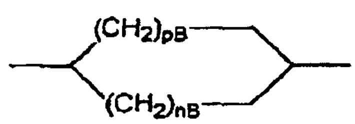 , 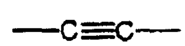 , 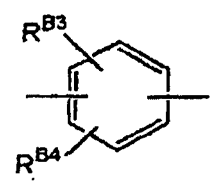

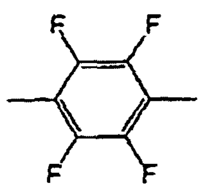 , 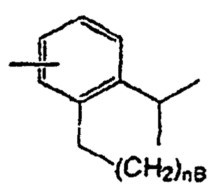

wherein, in the above stated ring systems, a phenyl ring may in each case be fused thereon, which phenyl ring may be substituted with up to 2 identical or different residues from the group $CH_3$, $CF_3$, Br, Cl, F, or may be substituted with $R^8OOC$- ($R^8$ being H, $C_{1-3}$-alkyl);
with

$n^B$ = 0, 1;
$p^B$ = 0, 1;
$R^{B1}$ being $C_{0-3}$-alkylaryl, $C_{0-3}$-alkylheteroaryl, $C_{0-3}$-alkyl-$C_{3-8}$-cycloalkyl;
$R^{B2}$ being H, $C_{1-6}$-alkyl, $C_{0-3}$-alkylaryl, $C_{0-3}$-alkylheteroaryl;
$R^{B3}$ being H, $C_{1-6}$-alkyl, $R^{B5}OCO$ ($R^{B5}$ being H, $C_{1-6}$-alkyl), $R^{B6}$-O ($R^{B6}$ being H, $C_{1-6}$-alkyl) , F, Cl, Br, $NO_2$, $CF_3$;
$R^{B4}$ being H, $C_{1-6}$-alkyl, $R^{B6}$-O; Cl, Br, F, $CF_3$;
$R^{B1}$ and $R^{B2}$ may also be joined to one another;

B furthermore denotes

$$-(CH_2)_{l^B}-L^B-M^B-L^B-(CH_2)_{m^B}-$$

wherein
$l^B$ and $m^B$ have the above-stated meaning and the two groups $L^B$ mutually independently denote the residues stated under $L^B$;

$M^B$ means a single bond, O, S, $CH_2$, $CH_2$-$CH_2$, $CH_2$-O, O-$CH_2$, $CH_2$-S, S-$CH_2$, CH=CH, C≡C;
B furthermore denotes
-1-adamantyl-$CH_2$-, -2-adamantyl-$CH_2$-,

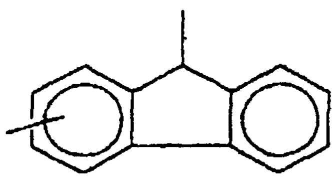 , 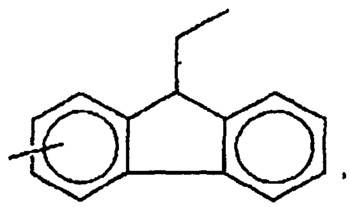

B may furthermore denote

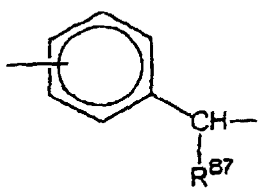 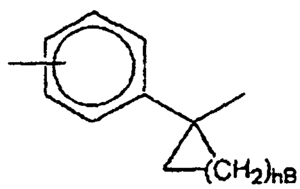

$h^B$ being 1, 2, 3, 4;
$R^{B7}$ being $C_{1-6}$-alkyl, $C_{3-8}$-cycloalkyl;
B may furthermore denote

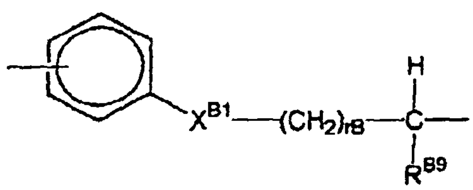

with $X^{B1}$ being a bond, O, S, or

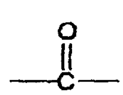

with $r^B$ being 0, 1, 2, or 3;
with $R^{B9}$ being H, $C_{1-3}$-alkyl;

A-B may denote

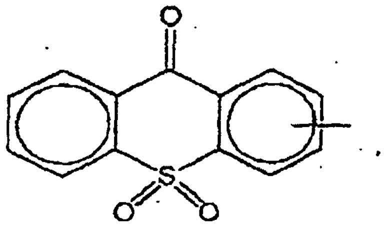

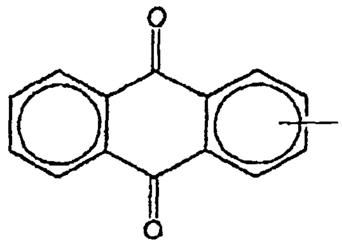

D denotes a single bond or CO, OCO, $NR^{D1}$-CO ($R^{D1}$ being H, $C_{1-4}$-alkyl, $C_{0-3}$-alkylaryl), $SO_2$, $NR^{D1}SO_2$;

E denotes

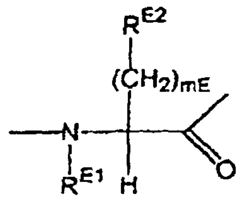

with

$m^E$ = 0, 1, 2, or 3;
$R^{E1}$ means H, $C_{1-6}$-alkyl;
$R^{E2}$ means H, $C_{1-6}$-alkyl, $C_{3-8}$-cycloalkyl, wherein the above-stated residues may bear up to three substituents from the group $C_{1-6}$-alkyl, F, or means $CH(CH_3)OH$, $CH(CF_3)_2$;

the groups stated under $R^{E1}$ and $R^{E2}$ may be linked to one another via a bond

E may also denote D-Asp, D-Glu, D-Lys, D-Orn, D-His, D-Dab, D-Dap, D-Arg;
G means

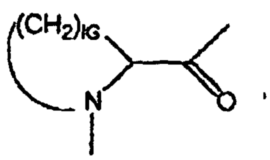

with $l^G$ = 2, 3, wherein a $CH_2$ group of the ring may be replaced by S, $CHCH_3$;

G furthermore means

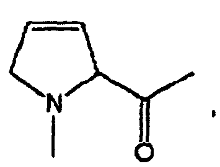

$Q^K$ being

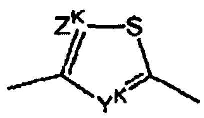
,
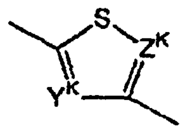

$Y^K$ being =CH-, =N-;
$Z^K$ being =CH-, =N-;
L:

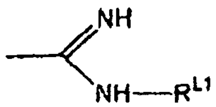
or
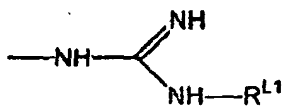

with

$R^{L1}$ being H, OH, CO-$C_{1-6}$-alkyl, $CO_2$-$C_{1-6}$-alkyl, $CO_2$-$C_{1-3}$-alkylaryl.

**2.** Compounds of the general formula I,

$$A—B—D—E—G—NH—CH_2—Q^K—L \qquad (I)$$

the tautomers, pharmacologically acceptable salts and prodrugs thereof, wherein:

A denotes
H, $C_{1-6}$-alkyl, $C_{1-6}$-alkyl-$SO_2$, $R^{A1}$OCO ($R^{A1}$ being H, $C_{1-12}$-alkyl, $C_{3-8}$-cycloalkyl, $C_{3-8}$-cycloalkyl-$C_{1-3}$-alkyl, $C_{1-3}$-alkylaryl), $R^{A2}R^{A3}$NCO ($R^{A2}$ being H-, $C_{1-6}$-alkyl, $C_{0-3}$-alkylaryl, $C_{0-3}$-alkylheteroaryl, $R^{A3}$ being H, $C_{1-6}$-alkyl, $C_{0-3}$-alkylaryl), $R^{A4}$OCONR$^2$ ($R^{A4}$ being $C_{1-6}$-alkyl, $C_{1-3}$-alkylaryl), $R^{A4}$CONR$^{A2}$, $R^{A1}$O, $R^{A2}R^{A3}$N, HO-$SO_2$-, phenoxy, $R^{A2}R^{A3}$N-$SO_2$, Cl, Br, F, tetrazolyl, $H_2O_3P$-, $NO_2$, $R^{A1}$-N(OH)-CO-, $R^{A1}R^{A2}$NCONR$^{A3}$, wherein aryl may in each case be substituted with up to 2 identical or different residues from the group F, Cl, Br, $CF_3$, $CH_3$, $OCH_3$, $NO_2$;

B denotes

$$-(CH_2)_{l^B}-L^B-(CH_2)_{m^B}-$$

with
$l^B$ =0, 1,
$m^B$ = 0, 1, 2;
$L^B$ being

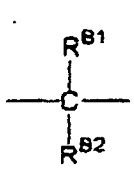
,
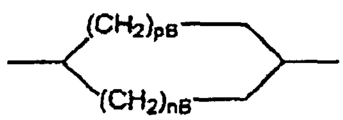
,
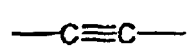
,
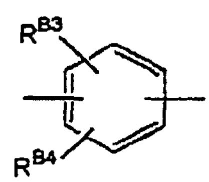

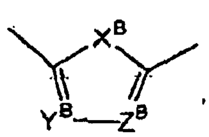
,
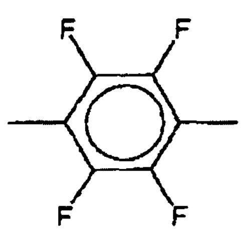
,
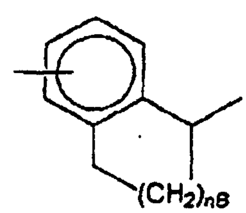

wherein, in the above stated ring systems, a phenyl ring may in each case be fused thereon, which phenyl ring may be substituted with up to 2 identical or different residues from the group $CH_3$, $CF_3$, Br, Cl, F, or may be substituted with $R^8OOC$- ($R^8$ being H, $C_{1-3}$-alkyl);
with

| | |
|---|---|
| $n^B$ | = 0, 1; |
| $p^B$ | = 0, 1; |
| $R^{B1}$ | being $C_{0-3}$-alkylaryl, $C_{0-3}$-alkylheteroaryl, $C_{0-3}$-alkyl-$C_{3-8}$-cycloalkyl; |
| $R^{B2}$ | being H, $C_{1-6}$-alkyl, $C_{0-3}$-alkylaryl, $C_{0-3}$-alkylheteroaryl; |
| $R^{B3}$ | being H, $C_{1-6}$-alkyl, $R^{B5}OCO$ ($R^{B5}$ being H, $C_{1-6}$-alkyl), $R^{B6}$-O ($R^{B6}$ being H, $C_{1-6}$-alkyl), F, Cl, Br, $NO_2$, $CF_3$; |
| $R^{B4}$ | being H, $C_{1-6}$-alkyl, $R^{B6}$-O, Cl, Br, F, $CF_3$; |
| $R^{B1}$ and $R^{B2}$ | may also be joined to one another; |
| $X^B$ | being O, S; |
| $Y^B$ | being =CH-, =N-; |
| $Z^B$ | being =CH-, =N-; |
| B | furthermore denotes |

$$-(CH_2)_{l^B}-L^B-M^B-L^B-(CH_2)_{m^B}-$$

wherein

$l^B$ and $m^B$ have the above-stated meaning and the two groups $L^B$ mutually independently denote the residues stated under $L^B$ -C≡C-,

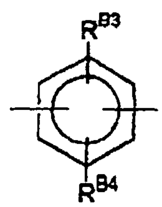
,
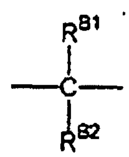
.

| | |
|---|---|
| $M^B$ | means a single bond, O, $CH_2$-S, S-$CH_2$, CO, $SO_2$, $CH_2$-O; |
| B | may furthermore denote |

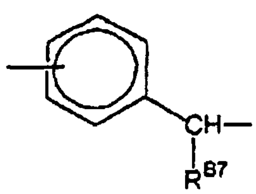
,
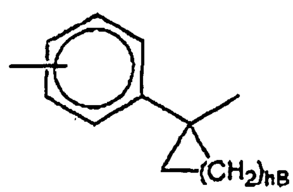

| | |
|---|---|
| $h^B$ | being 1, 2, 3, 4; |
| $R^{B7}$ | being $C_{1-6}$-alkyl, $C_{3-8}$-cycloalkyl |
| B | may furthermore denote -1-fluorenyl-, -1-adamantyl-, -1-adamantyl-$CH_2$- |
| A-B | may denote -2-pyridyl-$CH_2$-, -2-benzothienyl-, -3-benzothienyl-, |

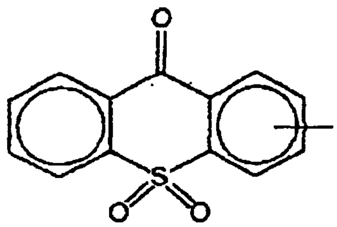 , 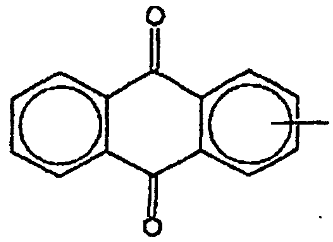 .

D denotes a single bond or CO, $SO_2$;
E denotes

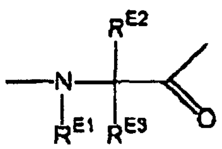

where

$R^{E1}$ means H, $CH_3$;
$R^{E2}$ means H, $C_{1-6}$-alkyl, $C_{3-8}$-cycloalkyl, thienyl, CH ($CH_3$) OH, CH ($CF_3$)$_2$;
$R^{E3}$ means H;

the groups stated under $R^{E1}$ and $R^{E2}$ may be linked to one another via a bond; the groups stated under $R^{E2}$ and $R^{E3}$ may also be joined to one another via a bond;

E may also denote D-Lys, D-Orn, D-Dab, D-Dap, D-Arg;
G means

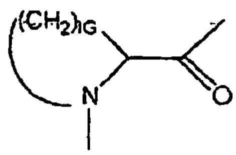 ,

with $l^G$ = 2, 3, wherein a $CH_2$ group of the ring may be replaced by $CHCH_3$;

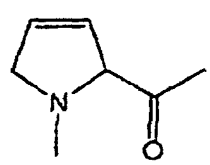

$Q^K$ being

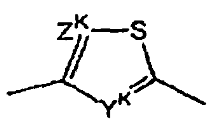 , 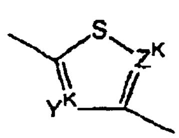 , 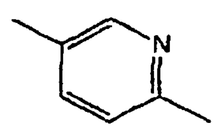

$Y^K$ being =CH-, =N-;
$Z^K$ being =CH-, =N-;

L:

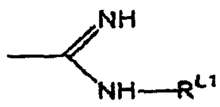 or 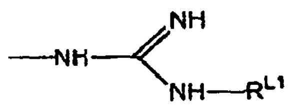

$R^{L1}$ being H, OH.

**3.** Compounds of the general formula I,

$$A—B—D—E—G—NH—CH_2—Q^K—L \qquad (I)$$

the tautomers, pharmacologically acceptable salts and prodrugs thereof, wherein:

A denotes
H, $C_{1-6}$-alkyl, $C_{1-6}$-alkyl-$SO_2$, $R^{A1}OCO$ ($R^{A1}$ being H, $C_{1-12}$-alkyl, $C_{3-8}$-cycloalkyl, $C_{1-3}$-alkyl-$C_{3-8}$-cycloalkyl, $C_{1-3}$-alkylaryl), $R^{A2}R^{A3}NCO$ ($R^{A2}$ being H, $C_{1-6}$-alkyl, $C_{0-3}$-alkylaryl, $C_{0-3}$-alkylheteroaryl, $R^{A3}$ being H, $C_{1-6}$-alkyl, $C_{0-3}$-alkylaryl), $R^{A4}OCONR^{A2}$, $R^{A4}CONR^{A2}$ ($R^{A4}$ being $C_{1-6}$-alkyl, $C_{1-3}$-alkylaryl), $R^{A1}O$, phenoxy, $R^{A2}R^{A3}N$, HO-$SO_2$, $R^{A2}R^{A3}N$-$SO_2$, Cl, Br, F, tetrazolyl, $H_2O_3P$, $NO_2$, $R^{A1}$-N(OH)-CO, $R^{A1}R^{A2}NCONR^{A3}$, wherein aryl may in each case be substituted with up to 2 identical or different residues from the group F, Cl, Br, $OCH_3$, $CH_3$, $CF_3$, $NO_2$;

B denotes

$$-(CH_2)_{l^B}-L^B-(CH_2)_{m^B}-$$

with
$l^B$ = 0, 1, 2;
$m^B$ = 0, 1, 2;
$L^B$ being

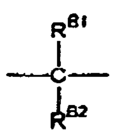 , 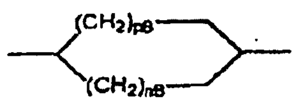 , 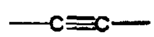 , 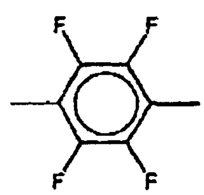 , 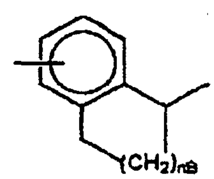

wherein, in the above stated ring systems, a phenyl ring may in each case be fused thereon, which phenyl ring may be substituted with up to 2 identical or different residues from the group $CH_3$, $CF_3$, Br, Cl, F, or may be substituted with $R^8OOC$- ($R^8$ being H, $C_{1-3}$-alkyl) ;
with

$n^B$ = 0, 1, 2;
$p^B$ = 0, 1, 2;
$R^{B1}$ being $C_{0-3}$-alkylaryl, $C_{0-3}$-alkylheteroaryl, $C_{0-3}$-alkyl-$C_{3-8}$-cycloalkyl;
$R^{B2}$ being H, $C_{1-6}$-alkyl, $C_{0-3}$-alkylaryl, $C_{0-3}$-alkylheteroaryl,
$R^{B1}$ and $R^{B2}$ may also be joined to one another;
B furthermore denotes -1-adamantyl-$CH_2$-, -2-adamantyl-$CH_2$-,

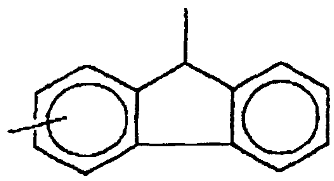, 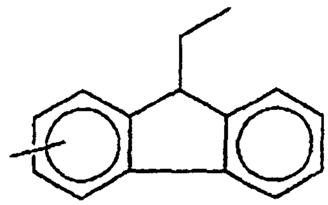,

B furthermore denotes

$$-(CH_2)_{l^B}-L^{B1}-M^B-L^{B2}-(CH_2)_{m^B}-$$

wherein

$l^B$ and $m^B$ have the above-stated meaning and the two groups $L^{B1}$ and $L^{B2}$ mutually independently denote the following residues:

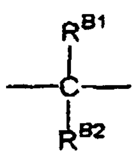, 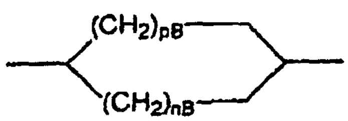, 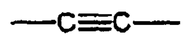, 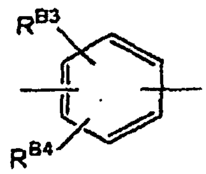

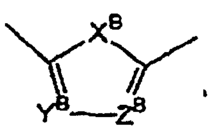, 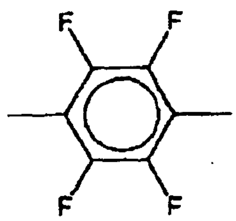, 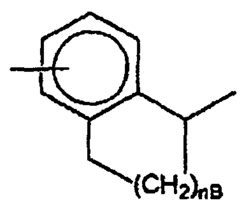

wherein, in the above-stated ring systems, a phenyl residue may in each case be fused thereon;
with

| | |
|---|---|
| $n^B$ | = 0, 1, 2; |
| $p^B$ | = 0, 1, 2; |
| $R^{B1}$ | being H (only for $L^{B2}$), $C_{1-6}$-alkyl (only for $L^{B2}$), $C_{0-3}$-alkylaryl, $C_{0-3}$-alkylheteroaryl, $C_{0-3}$-alkyl-$C_{3-8}$-cycloalkyl; |
| $R^{B2}$ | being H, $C_{1-6}$-alkyl, $C_{0-3}$-alkylaryl, $C_{0-3}$-alkylheteroaryl; |
| $R^{B3}$ | being H, $C_{1-6}$-alkyl, aryl, heteroaryl, $R^{B5}$OCO ($R^{B5}$ being H, $C_{1-6}$-alkyl) , $R^{B6}$-O ($R^{B6}$ being H, $C_{1-6}$-alkyl) , F, Cl, Br, $NO_2$, $CF_3$; |
| $R^{B4}$ | being H, $C_{1-6}$-alkyl, $R^{B6}$-O, Cl, Br, F, $CF_3$; |
| $X^B$ | being O, S; |
| $Y^B$ | being =CH-, =N-; |
| $Z^B$ | being =CH-, =N-; |
| $R^{B1}$ and $R^{B2}$ | may also be joined to one another; |
| $M^B$ | means a single bond, O, S, $CH_2$, $CH_2$-$CH_2$, $CH_2$-O, O-$CH_2$, $CH_2$-S, S-$CH_2$, CO, $SO_2$, CH=CH, C≡C; |
| B | may furthermore denote |

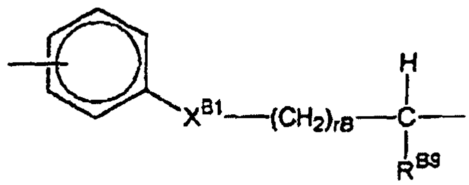

with $X^{B1}$ being a bond, O, S or

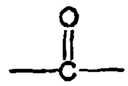

with $r^B$ being 0, 1, 2, 3;
with $R^{B9}$ being H, $C_{1-3}$-alkyl;
A-B may denote

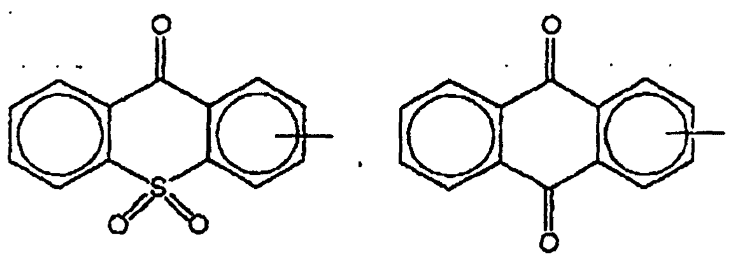

D denotes a single bond or CO, OCO, $NR^{D1}$-CO ($R^{D1}$ being H, $C_{1-4}$-alkyl, $C_{0-3}$-alkylaryl), $SO_2$, $NR^{D1}SO_2$;
B-D may denote

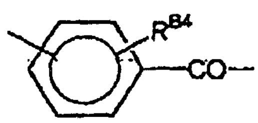

E denotes

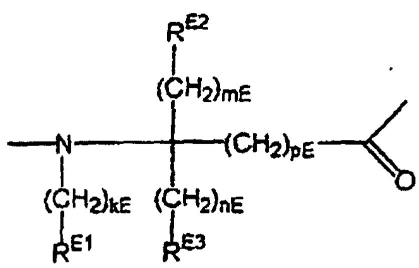

$k^E$ = 0, 1;
$m^E$ = 0, 1;
$n^E$ = 0, 1;
$p^E$ = 0, 1;
$R^{E1}$ means H, $C_{1-6}$-alkyl, $C_{3-8}$-cycloalkyl, phenyl, naphthyl, pyridyl, thienyl, $C_{3-8}$-cycloalkyl with a fused phenyl residue;
$R^{E2}$ means H, $C_{1-6}$-alkyl, $C_{3-8}$-cycloalkyl, phenyl, pyridyl, thienyl, furyl, imidazolyl, tetrahydropyranyl, tetrahy-

drothiopyranyl, $CH(CH_3)OH$, $CH(CF_3)_2$;
$R^{E3}$ means H, $C_{1-6}$-alkyl, $C_{3-8}$-cycloalkyl, phenyl;

the groups stated under $R^{E1}$ and $R^{E2}$ may be linked to one another via a bond; the groups stated under $R^{E2}$ and $R^{E3}$ may also be joined to one another via a bond;

E may also denote D-Asp, D-Glu, D-Lys, D-Orn, D-His, D-Dab, D-Dap, D-Arg;
G means

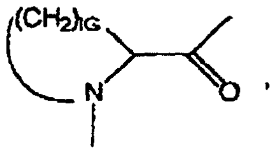

with $l^G$ = 2, 3, 4, wherein a $CH_2$ group of the ring may be replaced by $CHCH_3$

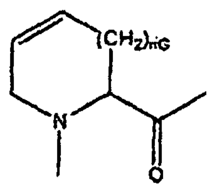

with

$n^G$ = 0, 1;
G furthermore denotes

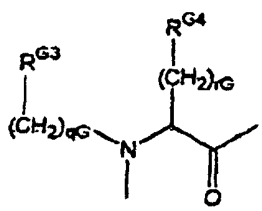

$q^G$ 0, 1;
$r^G$ 0, 1;
$R^{B3}$ being $C_{1-6}$-alkyl, $C_{3-8}$-cycloalkyl;
$R^{G4}$ H, $C_{1-6}$-alkyl, $C_{3-8}$-cycloalkyl, phenyl;
$Q^K$ being

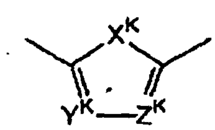

$X^K$ being O, S;
$Y^K$ being =CH-, =N-;
$Z^K$ being =CH-, =N-;

L:

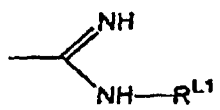 or 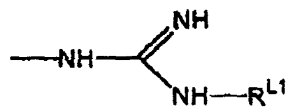

with

$R^{L1}$ being H, OH, CO-$C_{1-6}$-alkyl, $CO_2$-$C_{1-6}$-alkyl, $CO_2$-$C_{1-5}$-alkylaryl.

**4.** Compounds of the general formula I,

$$A—B—D—E—G—NH—CH_2—Q^K—L \qquad (I)$$

the tautomers, pharmacologically acceptable salts and prodrugs thereof, wherein:

A denotes
H, $C_{1-6}$-alkyl, $C_{1-6}$-alkyl-$SO_2$, $R^{A1}OCO$ ($R^{A1}$ being H, $C_{1-12}$-alkyl, $C_{3-8}$-cycloalkyl, $C_{1-3}$-alkyl-$C_{3-8}$-cycloalkyl, $C_{1-3}$-alkylaryl), $R^{A2}R^{A3}NCO$ ($R^{A2}$ being H, $C_{1-6}$-alkyl, $C_{0-3}$-alkylaryl, $C_{0-3}$-alkylheteroaryl, $R^{A3}$ being H, $C_{1-6}$-alkyl, $C_{0-3}$-alkylaryl), $R^{A4}OCONR^{A2}$, $R^{A4}CONR^{A2}$ ($R^{A4}$ being $C_{1-6}$-alkyl, $C_{1-3}$-alkylaryl), $R^{A1}O$, phenoxy, $R^{A2}R^{A3}N$, HO-$SO_2$, $R^{A2}R^{A3}N$-$SO_2$, Cl, Br, F, tetrazolyl, $H_2O_3P$, $NO_2$, $R^{A1}$-N(OH)-CO, $R^{A1}R^{A2}NCONR^{A3}$, wherein aryl may in each case be substituted with up to 2 identical or different residues from the group F, Cl, Br, $OCH_3$, $CH_3$, $CF_3$, $NO_2$;

B denotes

$$-(CH_2)_{l^B}-L^B-(CH_2)_{m^B}-$$

with

$l^B$ = 0, 1;

$m^B$ = 0, 1, 2;

$L^B$ being

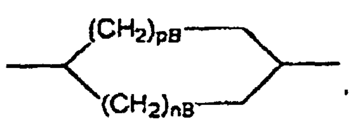 , 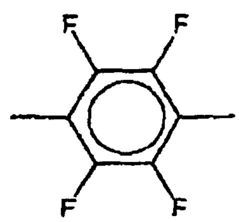 , 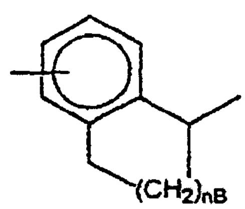

wherein, in the above stated ring systems, a phenyl ring may in each case be fused thereon, which phenyl ring may be substituted with up to 2 identical or different residues from the group $CH_3$, $CF_3$, Br, Cl, F, or may be substituted with $R^8OOC$- ($R^8$ being H, $C_{1-3}$-alkyl);

with

$n^B$ = 0, 1;

$p^B$ = 0, 1;

B furthermore denotes -1-adamantyl-$CH_2$-, -2-adamantyl-$CH_2$- ,

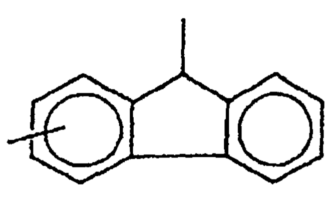

B furthermore denotes

$$-(CH_2)_{l^B}-L^{B1}-M^B-L^{B2}-(CH_2)_{m^B}-$$

$l^B$ and $m^B$ have the above-stated meaning and both the groups $L^{B1}$ and $L^{B2}$ mutually independently denote the following residues:

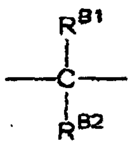 , 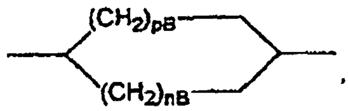 , 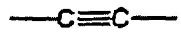 , 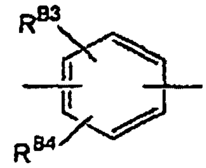 ,

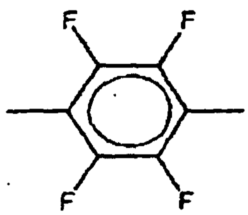 , 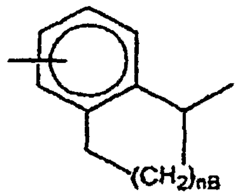

wherein, in the above-stated ring systems, a phenyl residue may in each case be fused thereon;
with

| | |
|---|---|
| $n^B$ | = 1; |
| $p^B$ | = 0, 1; |
| $R^{B1}$ | being H (only for $L^{B2}$), $C_{1-6}$-alkyl (only for $L^{B2}$) , $C_{0-3}$-alkylaryl, $C_{0-3}$-alkylheteroaryl, $C_{0-3}$-alkyl-$C_{3-8}$-cycloalkyl; |
| $R^{B2}$ | being H, $C_{1-6}$-alkyl, $C_{0-3}$-alkylaryl, $C_{0-3}$-alkylheteroaryl; |
| $R^{B3}$ | being H, $C_{1-6}$-alkyl, $R^{B6}$-O ($R^{B6}$ being H, $C_{1-6}$-alkyl), F, Cl, Br, $NO_2$, $CF_3$; |
| $R^{B4}$ | being H, $C_{1-6}$-alkyl, $R^{B6}$-O, Cl, Br, F, $CF_3$; |
| $R^{B1}$ and $R^{B2}$ | may also be joined to one another; |
| $M^B$ | means a single bond, O, S, $CH_2$, $CH_2$-$CH_2$, $CH_2$-O, O-$CH_2$, $CH_2$-S, S-$CH_2$, CO, $SO_2$; |
| A-B | may denote -2-pyridyl-$CH_2$-, -2-benzothienyl- |

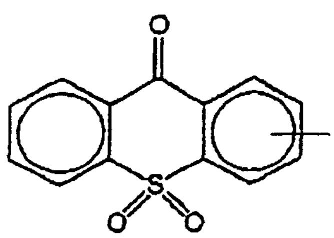 , 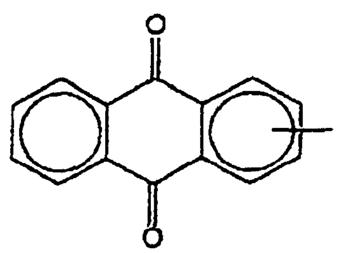

| | |
|---|---|
| D | denotes a single bond or CO, $SO_2$; |
| B-D | may denote |

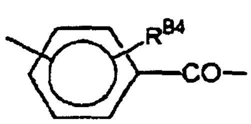

E denotes

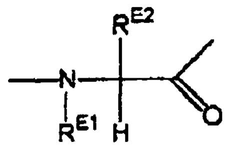

$R^{E1}$ means H;

$R^{E2}$ means H, $C_{1-6}$-alkyl, $C_{3-8}$-cycloalkyl, phenyl, pyridyl, thienyl, furyl, imidazolyl, tetrahydropyranyl, tetrahydrothiopyranyl, $CH(CH_3)OH$, $CH(CF_3)_2$;

the groups stated under $R^{E1}$ and $R^{E2}$ may be linked to one another via a bond

E may also denote D-Lys, D-Orn, D-Dab, D-Dap, D-Arg;

G means

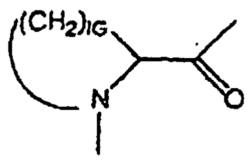

with $l^G$ = 2, 3, wherein a $CH_2$ group of the ring may be replaced by $CHCH_3$

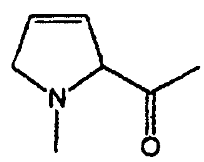
,

$Q^K$ being

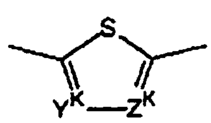

$Y^K$ being =CH-, =N-;

$Z^K$ being =CH-, =N-;

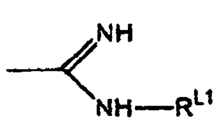

or

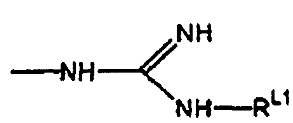

L:

with

$R^{L1}$ being H, OH.

**5.** Compounds of the general formula I,

$$A—B—D—E—G—NH—CH_2—Q^K—L \quad (I)$$

the tautomers, pharmacologically acceptable salts and prodrugs thereof, wherein:

A denotes
H, $C_{1-6}$-alkyl; $C_{1-6}$-alkyl-$SO_2$, $R^{A1}OCO$ ($R^{A1}$ being H, $C_{1-12}$-alkyl, $C_{3-8}$-cycloalkyl, $C_{1-3}$-alkyl-$C_{3-8}$-cycloalkyl, $C_{1-3}$-alkylaryl), $R^{A2}R^{A3}NCO$ ($R^{A2}$ being H, $C_{1-6}$-alkyl, $C_{0-3}$-alkylaryl, $C_{0-3}$-alkylheteroaryl, $R^{A3}$ being H, $C_{1-6}$-alkyl, $C_{0-3}$-alkylaryl), $R^{A4}OCONR^{A2}$ ($R^{A4}$ being $C_{1-6}$-alkyl, $C_{1-3}$-alkylaryl), $R^{A4}{}_{CONR}{}^{A2}$, $R^{A1}O$, $R^{A2}R^{A3}N$, HO-$SO_2$-, phenoxy, $R^{A2}R^{A3}N$-$SO_2$, Cl, Br, F, tetrazolyl, $H_2O_3P$-, $NO_2$, $R^{A1}$-N(OH)-CO-, $R^{A1}R^{A2}NCONR^{A3}$, wherein aryl may in each case be substituted with up to 2 identical or different residues from the group F, Cl, Br, $CF_3$, $CH_3$, $OCH_3$, $NO_2$ ;

B denotes

$$-(CH_2)_{l^B}-L^B-(CH_2)_{m^B}-$$

with
$l^B$ = 0, 1;
$m^B$ = 0, 1, 2;
$L^B$ being

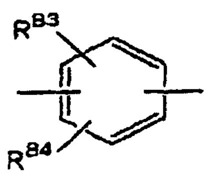 , 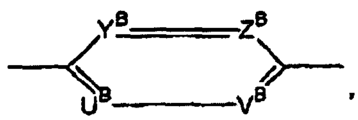 , 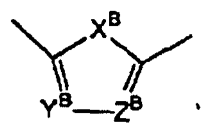 , 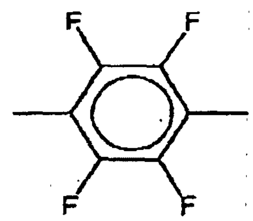

wherein, in the above-stated ring systems, a phenyl residue may in each case be fused thereon;

$R^{B3}$ being H, $C_{1-6}$-alkyl, aryl, $R^{B5}OCO$ ($R^{B5}$ being H, $C_{1-6}$-alkyl, $C_{1-3}$-alkylaryl), $R^{B6}$-O ($R^{B6}$ being H, $C_{1-6}$-alkyl), F, Cl, Br, $NO_2$, $CF_3$;
$R^{B4}$ being H, $C_{1-6}$-alkyl, $R^{B6}$-O, Cl, Br, F, $CF_3$;
$X^B$ being O, S;
$Y^B$ being =CH-, =N-;
$Z^B$ being =CH-, =N-;
$U^B$ being =CH-, =N-;
$V^B$ being =CH-, =N-;
B may furthermore denote

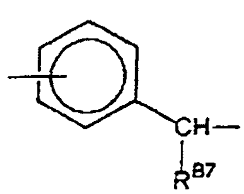 , 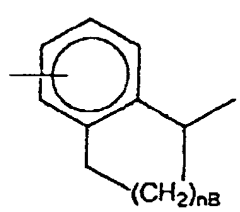

$q^B$ being 0, 1, 2;

($R^{B7}$ being $C_{1-6}$-alkyl, $C_{3-8}$-cycloalkyl)

A-B may denote

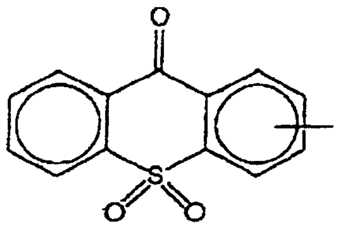 , 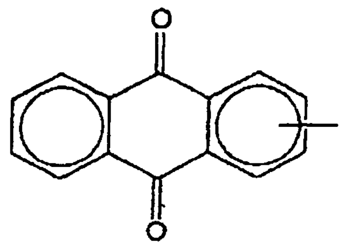

D denotes a single bond

E denotes

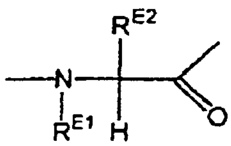

where

$R^{E1}$ means H;

$R^{E2}$ means H, $C_{1-6}$-alkyl, $C_{3-8}$-cycloalkyl, phenyl, pyridyl, furyl, thienyl, imidazolyl, tetrahydropyranyl, tetrahydrothiopyranyl, wherein the above-stated residues may bear up to three identical or different substituents from the group O-$C_{1-6}$-alkyl, F, or means $CH(CH_3)OH$, $CH(CF_3)_2$;

the groups stated under $R^{E1}$ and $R^{E2}$ may be linked to one another via a bond;
E may also denote D-Lys, D-Orn, D-Dab, D-Dap, D-Arg;

G means

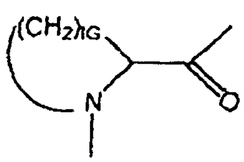

with $l^G$ = 2, 3, wherein a $CH_2$ group of the ring may be replaced by $CHCH_3$;

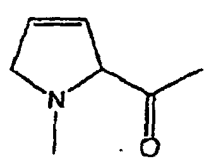 .

$Q^K$ being

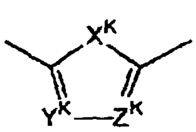

$x^K$ being O, S;

$Y^K$ being =CH-, =N-;

$Z^K$ being =CH-, =N-;

L:

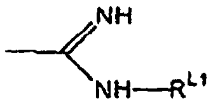

or

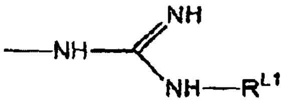

$R^{L1}$ being -H, -OH.

**6.** A pharmaceutical preparation containing compounds of the general formula I according to any one of claims 1 to 5, in addition to conventional excipients and auxiliary substances.

**7.** Use of compounds of the general formula I according to any one of claims 1 to 5 for the production of pharmaceutical preparations for the treatment of diseases which are alleviated or cured by partial or complete inhibition of $C_{1s}$ or $C_{1r}$.

**8.** Use of compounds of the general formula I according to any one of claims 1 to 5 for the production of pharmaceutical preparations for the treatment or prevention of

- reperfusion injuries after ischaemic episodes; ischaemic states occur, for example, during operations with the aid of heart-lung machines; operations in which blood vessels are in general clamped off to avoid major haemorrhage; myocardial infarction; thromboembolic cerebral infarction; pulmonary thrombosis etc.;
- hyperacute organ rejection, especially in xenotransplantation;
- organ failure, such as for example multiple organ failure or ARDS (adult respiratory distress syndrome);
- disorders due to trauma (cranial trauma) or multiple injury, such as for example burns and "thermal injury";
- anaphylactic shock;
- sepsis; "vascular leak syndrome": in the case of sepsis and after treatment with biological agents such as interleukin 2 or after transplantation;
- Alzheimer's disease and other inflammatory neurological disorders such as myaesthenia gravis, multiple sclerosis, cerebral lupus, Guillain-Barré syndrome; meningitis; encephalitis;
- systemic lupus erythematosus (SLE);
- rheumatoid arthritis and other inflammatory diseases of the rheumatoid disorder group, such as for example Behçet's syndrome; juvenile rheumatoid arthritis;
- renal inflammation of various origins, such as for example glomerulonephritis, lupus nephritis;
- pancreatitis;
- asthma; chronic bronchitis;
- complications during dialysis in the case of renal failure;
- vasculitis; thyroiditis;
- ulcerative colitis and other inflammatory diseases of the gastrointestinal tract;
- autoimmune diseases;
- spontaneous preterm birth.

**9.** Compounds of the general formula I according to any one of claims 1 to 5, wherein L corresponds to -CN, -C(=O)$NH_2$ or -C(=S)$NH_2$.

## Revendications

**1.** Composés de la formule générale I,

$$A - B - D - E - G - NH - CH_2 - Q^K - L \qquad (I)$$

leurs tautomères, sels pharmacologiquement compatibles et promédicaments, où:

A représente H, alkyle en $C_{1-6}$, (alkyl en $C_{1-6}$) -$SO_2$, $R^{A1}OCO$ ($R^{A1}$ est H, alkyle en $C_{1-12}$, cycloalkyle en $C_{3-8}$, (cycloalkyl en $C_{3-8}$) alkyle en $C_{1-3}$, (alkyl en $C_{1-3}$)aryle), $R^{A2}R^{A3}NCO$ ($R^{A2}$ est H, alkyle en $C_{1-6}$, (alkyl en $C_{0-3}$) aryle, (alkyl en $C_{0-3}$)hétéroaryle, $R^{A3}$ est H, alkyle en $C_{1-6}$, (alkyl en $C_{0-3}$)aryle), $R^{A4}OCONR^{A2}$ ($R^{A4}$ est alkyle

en $C_{1-6}$, (alkyl en $C_{1-3}$)aryle), $R^{A4}CONR^{A2}$, $R^{A1}O$, $R^{A2}R^{A3}N$, HO-$SO_2$-, phénoxy, $R^{A2}R^{A3}N$-$SO_2$-, Cl, Br, F, tétrazolyle, $H_2O_3P$-, $NO_2$-, $R^{A1}N(OH)$-CO-, $R^{A1}R^{A2}NCONR^{A3}$, où aryle peut être chaque fois substitué avec jusqu'à 2 restes identiques ou différents parmi le groupe F, Cl, Br, $CF_3$, $CH_3$, $OCH_3$, $NO_2$ ;

B représente

$$-(CH_2)_l{}^B - L^B - (CH_2)_m{}^B -$$

avec

$l^B$ = 0, 1, 2;

$m^B$ = 0, 1, 2;

$L^B$ est :

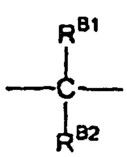
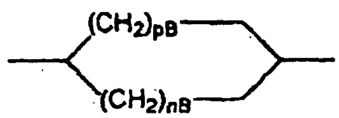
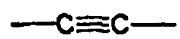
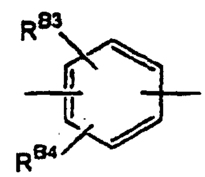

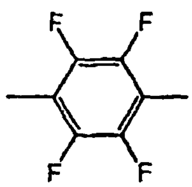
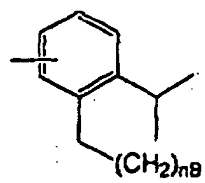

où dans les systèmes cycliques précédents, un cycle phényle peut être chaque fois condensé, qui peut être substitué avec jusqu'à 2 restes identiques ou différents parmi le groupe $CH_3$, $CF_3$, Br, Cl, F, ou qui peut être substitué par $R^8OOC$- ($R^8$ est H, alkyle $C_{1-3}$) ;

avec

$n^B$ = 0, 1 ;

$p^B$ = 0, 1 ;

$R^{B1}$ est (alkyl en $C_{0-3}$)aryle, (alkyl en $C_{0-3}$)hétéroaryle, (alkyl en $C_{0-3}$)cycloalkyle $C_{3-8}$ ;

$R^{B2}$ est H, alkyle en $C_{1-6}$, (alkyl en $C_{0-3}$)aryle, (alkyl en $C_{0-3}$) hétéroaryle ;

$R^{B3}$ est H, alkyle en $C_{1-6}$, $R^{B5}OCO$ ($R^{B4}$ est H, alkyle $C_{1-6}$), $R^{B6}$-O ($R^{B6}$ est H, alkyle $C_{1-6}$), F, Cl, Br, $NO_2$, $CF_3$ ;

$R^{B4}$ est H, alkyle en $C_{1-6}$, $R^{B6}O$, Cl, Br, F, $CF_3$ ;

$R^{B1}$ et $R^{B2}$ peuvent également être reliés l'un à l'autre ;

B représente d'autre part

$$-(CH_2)_l{}^B - L^B - M^B - L^B - (CH_2)_m{}^B -$$

où

$l^B$ et $m^B$ possèdent la signification donnée ci-dessus et les deux radicaux $L^B$ représentent indépendamment l'un de l'autre, les restes indiqués sous $L^B$;

$M^B$ représente une simple liaison, O, S, $CH_2$, $CH_2$-$CH_2$, $CH_2$-O, O-$CH_2$, $CH_2$-S, S-$CH_2$, CH=CH, C≡C;

B représente encore

-adamantyl(1)-$CH_2$-, -adamantyl(2)-$CH_2$-,

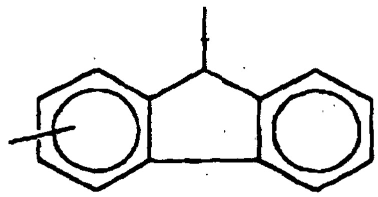

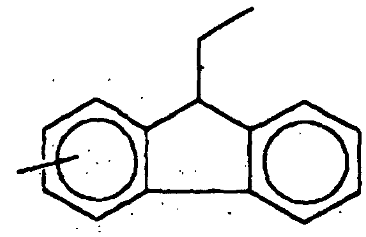

B peut représenter également

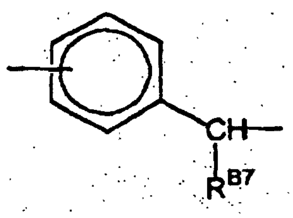

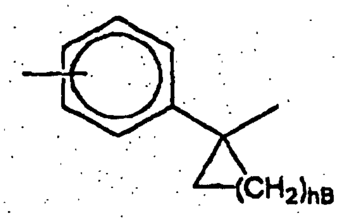

$h^B$ est 1, 2, 3, 4 ;
$R^{B7}$ est alkyle en $C_{1-6}$, cycloalkyle en $C_{3-8}$ ;
B peut représenter également

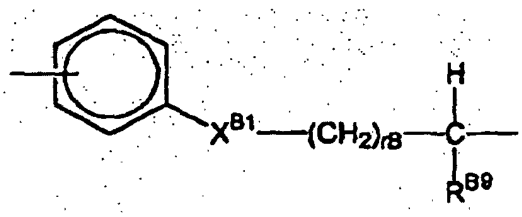

avec $X^{B1}$ est une liaison, O, S, ou C=O ;
avec $r^B$ est 0, 1, 2, 3 ;
avec $R^{B9}$ est H, alkyle en $C_{1-3}$ ;
A - B peut représenter

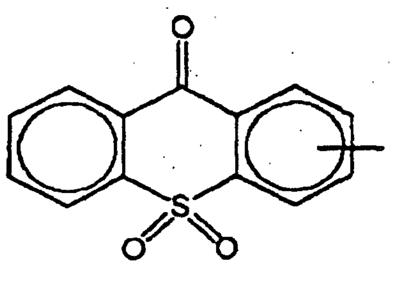

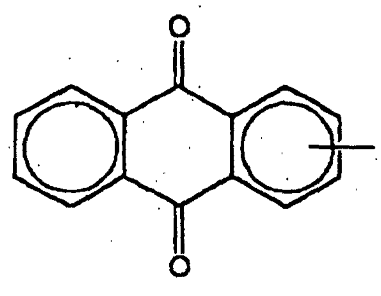

D représente une simple liaison ou représente CO, OCO, $NR^{D1}$-CO ($R^{D1}$ est H, alkyle en $C_{1-4}$, (alkyl en $C_{0-3}$) aryle), $SO_2$, $NR^{D1}SO_2$;
E représente

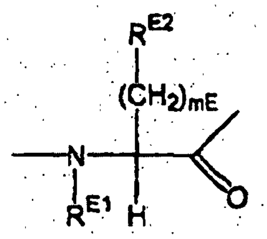

avec

$m^B$ = 0, 1, 2, 3;

$R^{E1}$ représente H, alkyle en $C_{1-6}$;

$R^{E2}$ représente H, alkyle en $C_{1-6}$, cycloalkyle $C_{3-8}$, où les restes indiqués peuvent porter jusqu'à trois substituants du groupe alkyle en $C_{1-6}$, F, représente $CH(CH_3)OH$, $CH(CF_3)_2$;

les radicaux donnés sous $R^{B1}$ et $R^{B2}$ peuvent être reliés les uns aux autres par une liaison;

E peut également représenter D-Asp, D-Glu, D-Lys, D-Orn, D-His, D-Dab, D-Dap, D-Arg;

G représente

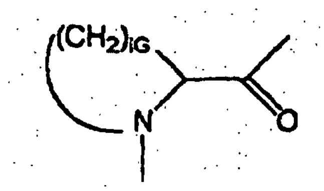

avec $1^G$ = 2, 3, où un radical $CH_2$ du cycle peut être remplacé par S, $CHCH_3$;

G représente également

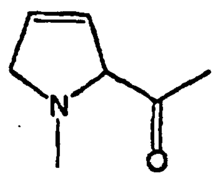

$Q^K$ est

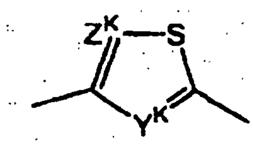

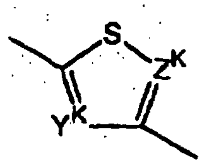

$Y^K$ est =CH, =N- ;

$Z^K$ est =CH, =N- ;

L

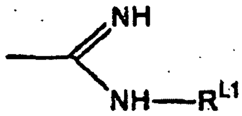

ou

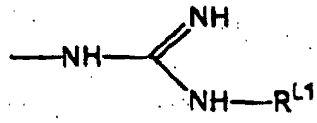

avec $R^{L1}$ est H, OH, CO- (alkyle en $C_{1-6}$), $CO_2$- (alkyle $C_{1-6}$), $CO_2$- (alkyl en $C_{1-3}$)aryle.

**2.** Composés de la formule générale I,

$$A - B - D - E - G - NH - CH_2 - Q^K - L \qquad (I)$$

leurs tautomères, sels pharmacologiquement compatibles et promédicaments, où:

A représente H, alkyle en $C_{1-6}$, (alkyl en $C_{1-6}$)-$SO_2$, $R^{A1}$OCO ($R^{A1}$ est H, alkyle en $C_{1-12}$, cycloalkyle en $C_{3-8}$, (cycloalkyl en $C_{3-8}$)alkyle en $C_{1-3}$, (alkyl en $C_{1-3}$)aryle), $R^{A2}R^{A3}$NCO ($R^{A2}$ est H, alkyle en $C_{1-6}$, (alkyl en $C_{0-3}$) aryle, (alkyl en $C_{0-3}$)hétéroaryle, $R^{A3}$ est H, alkyle en $C_{1-6}$, (alkyl en $C_{0-3}$)aryle), $R^{A4}$OCONR$^{A2}$ ($R^{A4}$ est alkyle en $C_{1-6}$, (alkyl en $C_{1-3}$) aryle), $R^{A4}$CONR$^{A2}$, $R^{A1}$O, $R^{A2}R^{A3}$N, HO-$SO_2$-, phénoxy, $R^{A2}R^{A3}$N-$SO_2$-, Cl, Br, F, tétrazolyle, $H_2O_3P$-, $NO_2$-, $R^{A1}$N(OH)-CO-, $R^{A1}R^{A2}$NCONR$^{A3}$, où aryle peut être chaque fois substitué avec jusqu'à 2 restes identiques ou différents parmi le groupe F, Cl, Br, $CF_3$, $CH_3$, $OCH_3$, $NO_2$ ;
B représente

$$- (CH_2)_{l^B} - L^B - (CH_2)_{m^B} -$$

avec
$l^B$ = 0, 1;
$m^B$ - 0, 1, 2;
$L^B$ est :

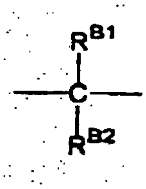

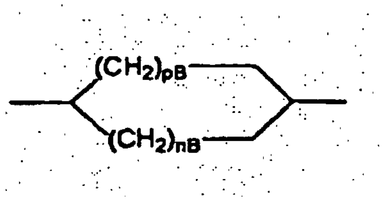

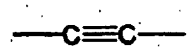

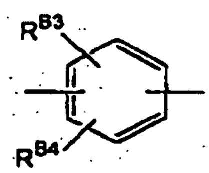

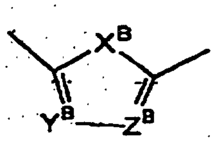

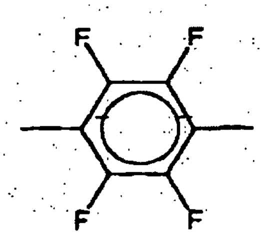

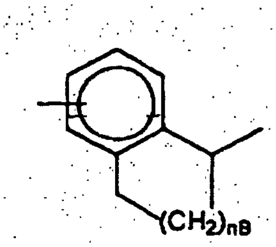

où dans les systèmes cycliques précédents, un cycle phényle peut être chaque fois condensé, qui peut être substitué avec jusqu'à 2 restes identiques ou différents parmi le groupe $CH_3$, $CF_3$, Br, Cl, F, ou qui peut être substitué par $R^8$OOC- ($R^8$ est H, alkyle en $C_{1-3}$) ;
avec
$n^B$ = 0, 1 ;
$p^B$ = 0, 1 ;

$R^{B1}$ est (alkyl en $C_{0-3}$)aryle, (alkyl en $C_{0-3}$) hétéroaryle, (alkyl en $C_{0-3}$)cycloalkyle en $C_{3-8}$ ;

$R^{B2}$ est H, alkyle en $C_{1-6}$, (alkyl en $C_{0-3}$)aryle, (alkyl en $C_{0-3}$)hétéroaryle ;

$R^{B3}$ est H, alkyle en $C_{1-6}$, $R^{B5}$OCO ($R^{B5}$ est H, alkyle en $C_{1-6}$), $R^{B6}$-O ($R^{B6}$ est H, alkyle en $C_{1-6}$), F, Cl, Br, $NO_2$, $CF_3$ ;

$R^{B4}$ est H, alkyle en $C_{1-6}$, $R^{B6}$O, Cl, Br, F, $CF_3$ ;

$R^{B1}$ et $R^{B2}$ peuvent également être reliés l'un à l'autre ;

$X^B$ est O, S ;

$Y^B$ est =CH, =N- ;

$Z^B$ est =CH, =N- ;

B représente d'autre part

$$- (CH_2)_{lB} - L^B - M^B - L^B - (CH_2)_{mB} -$$

où

$l^B$ et $m^B$ possèdent la signification donnée ci-dessus et les deux radicaux $L^B$ représentent indépendamment l'un de l'autre, les restes -C≡C-,

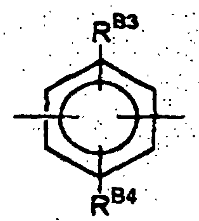

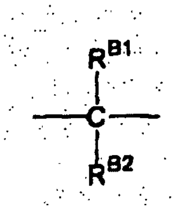

indiqués sous $L^B$ ;

$M^B$ représente une simple liaison, O, $CH_2$-S, S-$CH_2$, CO, $SO_2$, $CH_2$-O ;

B peut représenter également

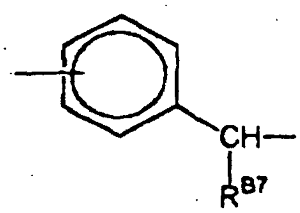

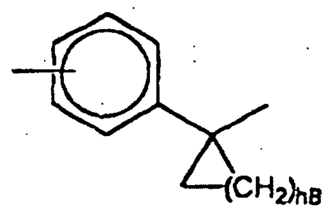

$h^B$ est 1, 2, 3, 4 ;

$R^{B7}$ est alkyle en $C_{1-6}$, cycloalkyle en $C_{3-8}$ ;

B peut représenter également fluorényle(1)-, adamantyle(1)-,-adamantyle(1)-$CH_2$-,

A - B peut représenter pyridyle(2)-$CH_2$-, benzothiényle (2)-, benzothiényle(3)-,

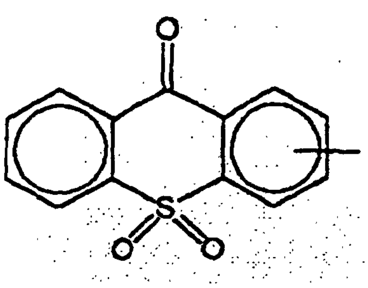

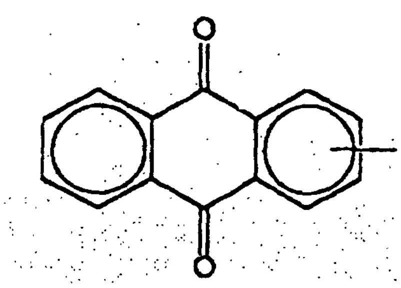

D représente une simple liaison ou représente CO, $SO_2$;

E représente

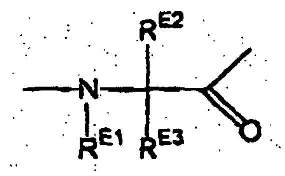

avec

$R^{B1}$ représente H, $CH_3$;

$R^{B2}$ représente H, alkyle en $C_{1-6}$, cycloalkyle en $C_{3-8}$, thiényle, $CH(CH_3)OH$, $CH(CF_3)_2$ ;

$R^{E3}$ représente H,

les radicaux donnés sous $R^{E1}$ et $R^{E2}$ peuvent être reliés les uns aux autres par une liaison ; également, les radicaux donnés sous $R^{B2}$ et $R^{E3}$ peuvent être reliés les uns aux autres par une liaison ;

E peut également représenter D-Lys, D-Orn, D-Dab, D-Dap, D-Arg ;

G représente

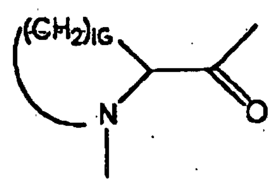

avec $1^G$ = 2, 3, où un radical $CH_2$ du cycle peut être remplacé par $CHCH_3$ ;

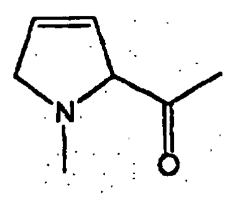

$Q^K$ est

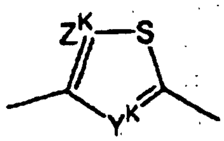
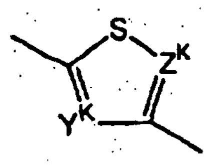
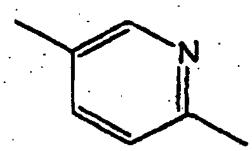

$Y^K$ est =CH, =N- ;

$Z^K$ est =CH, =N- ;

L

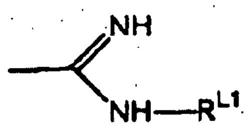

ou

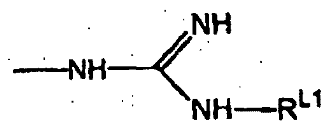

$R^{L1}$ est H, OH.

**3.** Composés de la formule générale I,

$$A - B - D - E - G - NH - CH_2 - Q^K - L \quad (I)$$

leurs tautomères, sels pharmacologiquement compatibles et promédicaments, où:

A représente H, alkyle en $C_{1-6}$, (alkyl en $C_{1-6}$) -$SO_2$, $R^{A1}OCO$ ($R^{A1}$ est H, alkyle en $C_{1-12}$, cycloalkyle en $C_{3-8}$, (alkyl en $C_{1-3}$) cycloalkyle en $C_{3-8}$, (alkyl en $C_{1-3}$)aryle), $R^{A2}R^{A3}NCO$ ($R^{A2}$ est H, alkyle en $C_{1-6}$, (alkyl en $C_{0-3}$) aryle, (alkyl en $C_{0-3}$)hétéroaryle, $R^{A3}$ est H, alkyle en $C_{1-8}$, (alkyl en $C_{0-3}$)aryle), $R^{A4}OCONR^{A2}$, $R^{A4}CONR^{A2}$ ($R^{A4}$ est alkyle en $C_{1-6}$, (alkyl en $C_{1-3}$)aryle), $R^{A1}O$, phénoxy, $R^{A2}R^{A3}N$, HO-$SO_2$-, $R^{A2}R^{A3}N$-$SO_2$-, Cl, Br, F, tétrazolyle, $H_2O_3P$-, $NO_2$-, $R^{A1}N(OH)$-CO-, $R^{A1}R^{A2}NCONR^{A3}$, où aryle peut être chaque fois substitué avec jusqu'à 2 restes identiques ou différents parmi le groupe F, Cl, Br, $OCH_3$, $CH_3$, $CF_3$, $NO_2$;
B représente

$$- (CH_2)_{lB} - L^B - (CH_2)_{mB} -$$

avec
$l^B$ = 0, 1, 2;
$m^B$ = 0, 1, 2;
$L^B$ est :

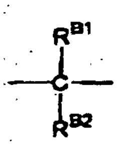

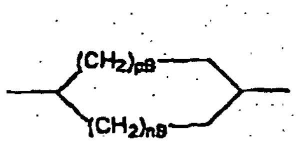

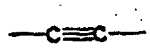

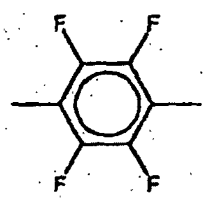

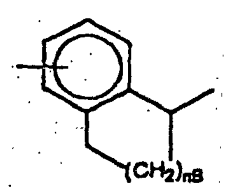

où dans les systèmes cycliques précédents, un cycle phényle peut être chaque fois condensé, qui peut être substitué avec jusqu'à 2 restes identiques ou différents parmi le groupe $CH_3$, $CF_3$, Br, Cl, F, ou qui peut être substitué par $R^8OOC$- ($R^8$ est H, alkyle $C_{1-3}$) ;
avec
$n^B$ = 0, 1, 2 ;
$p^B$ = 0, 1, 2 ;
$R^{B1}$ est (alkyl en $C_{0-3}$)aryle, (alkyl en $C_{0-3}$)hétéroaryle, (alkyl en $C_{0-3}$)cycloalkyle $C_{3-8}$ ;
$R^{B2}$ est H, alkyle en $C_{1-6}$, (alkyl en $C_{0-3}$)aryle, (alkyl en $C_{0-3}$) hétéroaryle ;
$R^{B1}$ et $R^{B2}$ peuvent également être reliés l'un à l'autre ;
B représente encore
-adamantyl (1) -$CH_2$-, -adamantyl (2) -$CH_2$-,

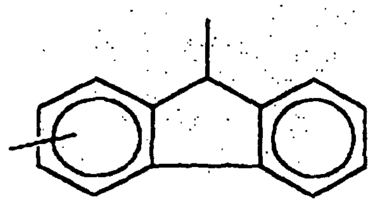 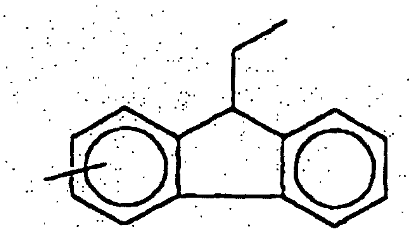

B représente d'autre part

$$-(CH_2)_{l^B}-L^{B1}-M^B-L^{B2}-(CH_2)_{m^B}-$$

où

$l^B$ et $m^B$ possèdent la signification donnée ci-dessus et les deux radicaux $L^{B1}$ et $L^{B2}$ représentent indépendamment l'un de l'autre, les restes suivants

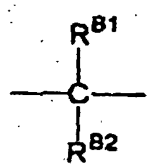 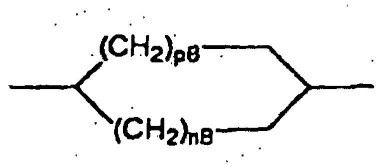 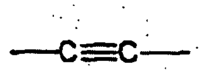 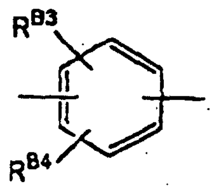

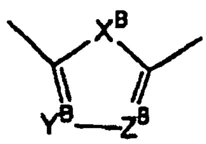 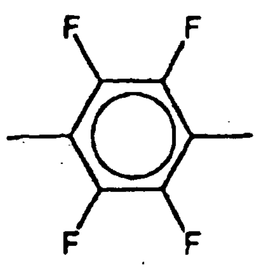 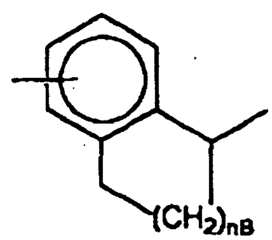

où dans les systèmes cycliques indiqués, un cycle phényle peut être condensé ;

avec

$n^B$ = 0, 1, 2 ;

$p^B$ = 0, 1, 2 ;

$R^{B1}$ est H (seulement pour $L^{B2}$), alkyle en $C_{1-6}$ (seulement pour $L^{B2}$) , (alkyl en $C_{0-3}$)aryle, (alkyl en $C_{0-3}$) hétéroaryle, (alkyl en $C_{0-3}$)cycloalkyle en $C_{3-8}$ ;

$R^{B2}$ est H, alkyle en $C_{1-6}$, (alkyl en $C_{0-3}$)aryle, (alkyl en $C_{0-3}$)hétéroaryle ;

$R^{B3}$ est H, alkyle en $C_{1-6}$, aryle, hétéroaryle, $R^{B5}{}_{OCO}$ ($R^{B5}$ est H, alkyle en $C_{1-6}$), $R^{B6}O$ ($R^{B6}$ est H, alkyle en $C_{1-6}$), F, Cl, Br, $NO_2$, $CF_3$ ;

$R^{B4}$ est H, alkyle en $C_{1-6}$, $R^{B6}O$, Cl, Br, F, $CF_3$ ;

$X^B$ est O, S ;

$Y^B$ est =CH, =N- ;

$Z^B$ est =CH, =N- ;

$R^{B1}$ et $R^{B2}$ peuvent également être reliés l'un à l'autre ;

$M^B$ représente une simple liaison, O, S, $CH_2$, $CH_2$-$CH_2$, $CH_2$-O, O-$CH_2$, $CH_2$-S, S-$CH_2$, CO, $SO_2$, CH=CH, C≡C;

B peut représenter également

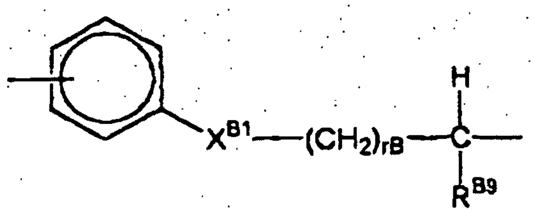

avec $X^{B1}$ est une liaison, O, S, ou

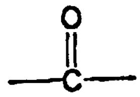

avec $r^B$ est 0, 1, 2, 3 ;
avec $R^{B9}$ est H, alkyle en $C_{1-3}$ ;
A - B peut représenter

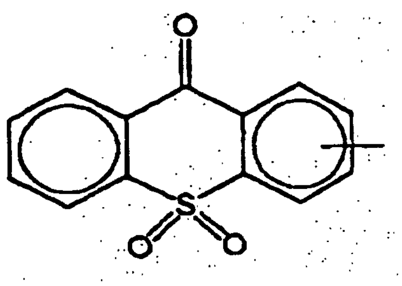 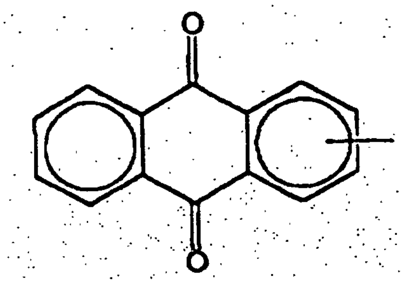

D représente une simple liaison ou représente CO, OCO, $NR^{D1}$-CO ($R^{D1}$ est H, alkyle en $C_{1-4}$, (alkyl en $C_{0-3}$) aryle), $SO_2$, $NR^{D1}SO_2$;
B-D peuvent représenter

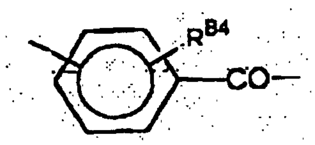

E représente

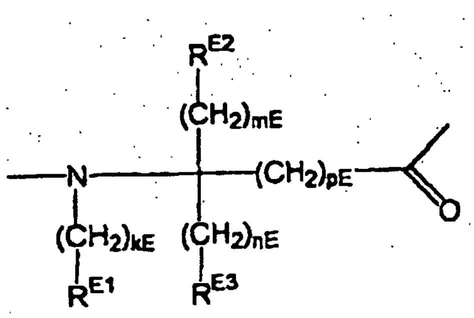

avec
$k^E = 0, 1$ ;
$m^E = 0, 1$;
$n^E = 0, 1$;
$p^E = 0, 1$;

$R^{E1}$ représente H, alkyle en $C_{1-6}$, cycloalkyle en $C_{3-8}$, phényle, naphtyle, pyridyle, thiényle, cycloalkyle en $C_{3-8}$ avec un cycle phényle condensé;

$R^{E2}$ représente H, alkyle en $C_{1-6}$, cycloalkyle en $C_{3-8}$, phényle, pyridyle, thiényle, furyle, imidazolyle, tétrahydropyrannyle, tétrahydrothiopyrannyle, $CH(CH_3)OH$, $CH(CF_3)_2$;

$R^{E3}$ représente H, alkyle en $C_{1-6}$, cycloalkyle en $C_{3-8}$, phényle;

les radicaux donnés sous $R^{E1}$ et $R^{E2}$ peuvent être reliés les uns aux autres par une liaison; également, les radicaux donnés sous $R^{E2}$ et $R^{E3}$ peuvent être reliés les uns aux autres par une liaison;

E peut également représenter D-Asp, D-Glu, D-Lys, D-Orn, D-His, D-Dab, D-Dap, D-Arg;

G représente

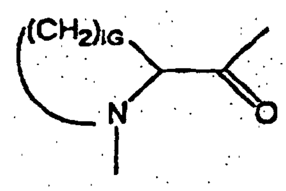

avec $1^G = 2, 3, 4$, où un radical $CH_2$ du cycle peut être remplacé par $CHCH_3$ ;

G représente également

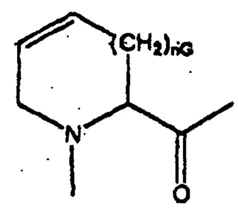

$n^G = 0, 1$;

d'autre part, G représente

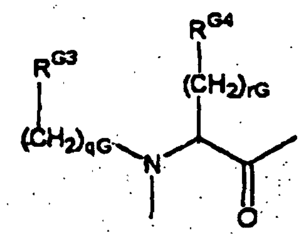

$q^G = 0, 1$;
$r^G = 0, 1$;

$R^{G3}$ représente H, alkyle en $C_{1-6}$, cycloalkyle en $C_{3-8}$ ;

$R^{G4}$ représente H, alkyle en $C_{1-6}$, cycloalkyle en $C_{3-8}$, phényle ;

$Q^K$ est

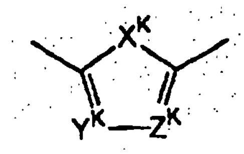

$X^K$ est O, S;
$Y^K$ est =CH, =N-;
$Z^K$ est =CH, =N-;
L

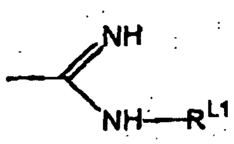

ou

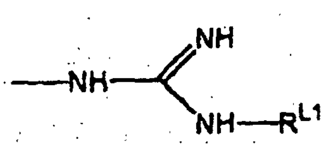

avec $R^{L1}$ est H, OH, CO- (alkyle en $C_{1-6}$) , $CO_2$- (alkyle en $C_1$-6), $CO_2$- (alkyl en $C_{1-5}$) aryle.

**4.** Composés de la formule générale I,

$$A - B - D - E - G - NH - CH_2 - Q^K - L \quad (I)$$

leurs tautomères, sels pharmacologiquement compatibles et promédicaments, où:

A représente H, alkyle en $C_{1-6}$, (alkyl en $C_{1-6}$) -$SO_2$, $R^{A1}OCO$ ($R^{A1}$ est H, alkyle en $C_{1-12}$, cycloalkyle en $C_{3-8}$, (alkyl en $C_{1-3}$)cycloalkyle en $C_{3-8}$, (alkyl en $C_{1-3}$)aryle), $R^{A2}R^{A3}NCO$ ($R^{A2}$ est H, alkyle en $C_{1-6}$, (alkyl en $C_{0-3}$) aryle, (alkyl en $C_{0-3}$)hétéroaryle, $R^{A3}$ est H, alkyle en $C_{1-6}$, (alkyl en $C_{0-3}$)aryle), $R^{A4}OCONR^{A2}$, $R^{A4}CONR^{A2}$ ($R^{A4}$ est alkyle en $C_{1-6}$, (alkyl en $C_{1-3}$)aryle), $R^{A1}O$, phénoxy, $R^{A2}R^{A3}N$, HO-$SO_2$-, $R^{A2}R^{A3}N$-$SO_2$-, Cl, Br, F, tétrazolyle, $H_2O_3P$-, $NO_2$-, $R^{A1}N(OH)$-CO-, $R^{A1}R^{A2}NCONR^{A3}$, où aryle peut être chaque fois substitué avec jusqu'à 2 restes identiques ou différents parmi le groupe F, Cl, Br, $OCH_3$, $CH_3$, $CF_3$, $NO_2$;
B représente

$$- (CH_2)_l^B - L^B - (CH_2)_m^B -$$

avec
$l^B$ = 0, 1;
$m^B$ = 0, 1, 2;
$L^B$ est :

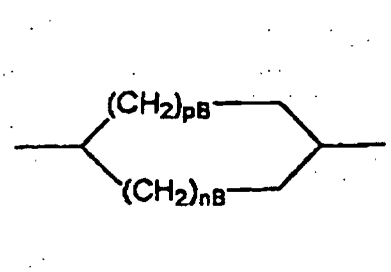

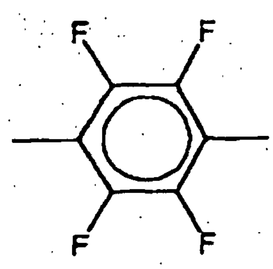

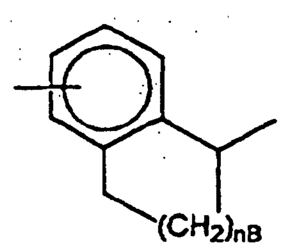

où dans les systèmes cycliques précédents, un cycle phényle peut être chaque fois condensé, qui peut être

substitué avec jusqu'à 2 restes identiques ou différents parmi le groupe $CH_3$, $CF_3$, Br, Cl, F, ou qui peut être substitué par $R^8OOC$- ($R^8$ est H, alkyle en $C_{1-3}$) ;

avec

$n^B = 0, 1$ ;

$p^B = 0, 1$ ;

B représente d'autre part, -adamantyl(1)-$CH_2$-,-adamantyl (2) -$CH_2$- ,

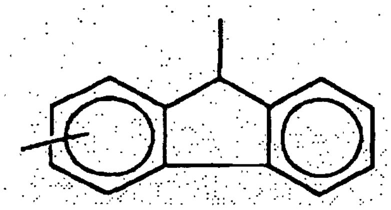

B représente d'autre part

$$- (CH_2)_{lB} - L^{B1} - M^B - L^{B2} - (CH_2)_{mB} -$$

où

$l^B$ et $m^B$ possèdent la signification donnée ci-dessus et les deux radicaux $L^{B1}$ et $L^{B2}$ représentent indépendamment l'un de l'autre, les restes suivants :

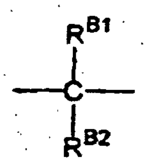
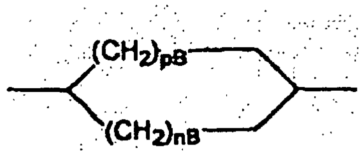
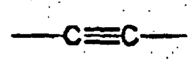
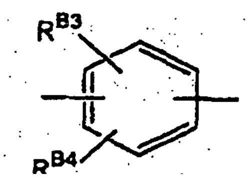

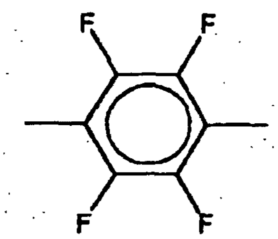
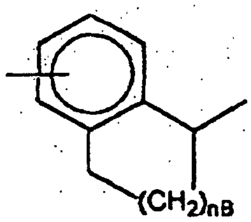

où dans les systèmes cycliques précédents, un cycle phényle peut être chaque fois condensé ;

avec

$n^B = 1$;

$p^B= 0, 1$;

$R^{B1}$ est H (seulement pour $L^{B2}$), alkyle en $C_{1-6}$ (seulement pour $L^{B2}$), (alkyl en $C_{0-3}$)aryle, (alkyl en $C_{0-3}$) hétéroaryle, (alkyl en $C_{0-3}$) cycloalkyle en $C_{3-8}$;

$R^{B2}$ est H, alkyle en $C_{1-6}$, (alkyl en $C_{0-3}$)aryle, (alkyl en $C_{0-3}$) hétéroaryle ;

$R^{B3}$ est H, alkyle en $C_{1-6}$, $R^{B6}$-O ($R^{B6}$ est H, alkyle en $C_{1-6}$), F, Cl, Br, $NO_2$, $CF_3$ ;

$R^{B4}$ est H, alkyle en $C_{1-6}$, $R^{B6}O$, Cl, Br, F, $CF_3$ ;

$R^{B1}$ et $R^{B2}$ peuvent également être reliés les uns aux autres ;

$M^B$ représente une simple liaison, O, S, $CH_2$, $CH_2$-$CH_2$, $CH_2$-O, O-$CH_2$, $CH_2$-S, S-$CH_2$, CO, $SO_2$ ;

A - B peut représenter pyridyle(2)-$CH_2$-, benzothiényle(2),

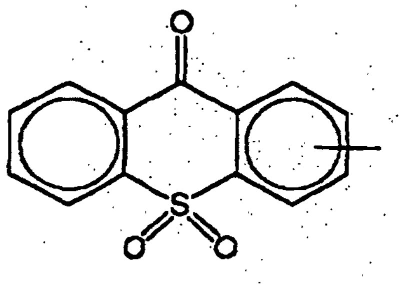 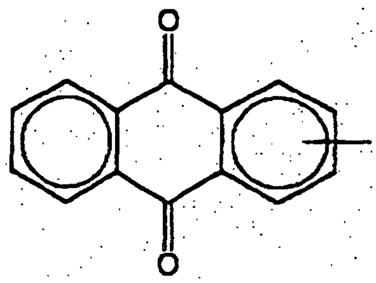

D représente une simple liaison ou représente CO, $SO_2$ ;
B-D peut représenter

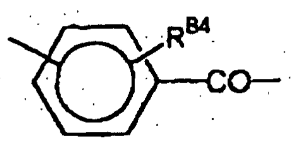

E représente

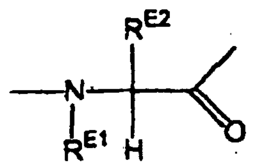

$R^{81}$ représente H ;
$R^{82}$ représente H, alkyle en $C_{1-6}$ , cycloalkyle en $C_{3-8}$, phényle, pyridyle, thiényle, furyle, imidazolyle, tétrahydropyrannyle, tétrahydrothiopyrannyle, $CH(CH_3)OH$, $CH(CF_3)_2$ ;
les radicaux donnés sous $R^{B1}$ et $R^{E2}$ peuvent être reliés les uns aux autres par une liaison ;
E peut également représenter D-Lys, D-Orn, D-Dab, D-Dap, D-Arg ;
G représente

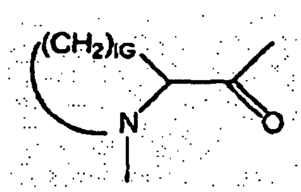

avec $1^G$ = 2, 3, où un radical $CH_2$ du cycle peut être remplacé par $CHCH_3$ ;
G représente également

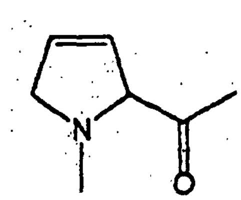

$Q^K$ est

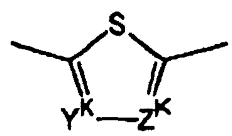

$Y^K$ est =CH, =N- ;
$Z^K$ est =CH, =N- ;
L

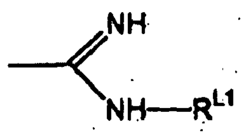

ou

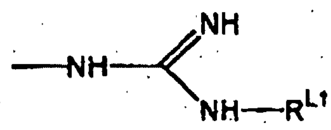

avec $R^{L1}$ est H, OH.

**5.** Composés de la formule générale I,

$$A - B - D - E - G - NH - CH_2 - Q^K - L \quad (I)$$

leurs tautomères, sels pharmacologiquement compatibles et promédicaments, où:

A représente H, alkyle en $C_{1-6}$, (alkyl en $C_{1-6}$) -$SO_2$, $R^{A1}OCO$ ($R^{A1}$ est H, alkyle en $C_{1-12}$, cycloalkyle en $C_{3-8}$, (alkyl en $C_1$-3) cycloalkyle en $C_{3-8}$, (alkyl en $C_{1-3}$)aryle), $R^{A2}R^{A3}NCO$ ($R^{A2}$ est H, alkyle en $C_{1-6}$, (alkyl en $C_{0-3}$) aryle, (alkyl en $C_{0-3}$) hétéroaryle, $R^{A3}$ est H, alkyle en $C_{1-6}$, (alkyl en $C_{0-3}$) aryle), $R^{A4}OCONR^{A2}$ ($R^{A4}$ est alkyle en $C_{1-6}$, (alkyl en $C_{1-3}$)aryle), $R^{A4}CONR^{A2}$, $R^{A1}O$, $R^{A2}R^{A3}N$, HO-$SO_2$-, phénoxy, $R^{A2}R^{A3}N$-$SO_2$-, Cl, Br, F, tétrazolyle, $H_2O_3$ $NO_2$- , $R^{A1}N(OH)$-CO-, $R^{A1}R^{A2}NCONR^{A3}$, où aryle peut être chaque fois substitué avec jusqu'à 2 restes identiques ou différents parmi le groupe F, Cl, Br, $CF_3$, $CH_3$, $OCH_3$, $NO_2$;
B représente

$$- (CH_2)_l{}^B - L^B - (CH_2)_m{}^B -$$

avec
$l^B$ = 0, 1 ;
$m^B$ = 0, 1, 2 ;
$L^B$ est :

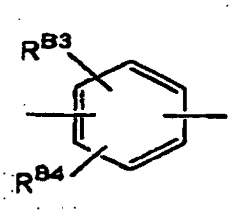

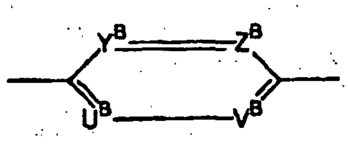

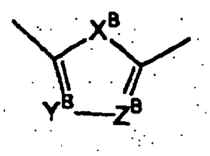

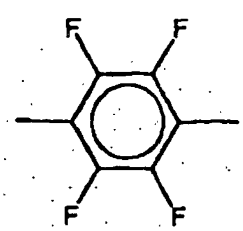

où dans les systèmes cycliques précédents, un cycle phényle peut être chaque fois condensé ;

$R^{B3}$ est H, alkyle en $C_{1-6}$, aryle, $R^{B5}$OCO($R^{B5}$ est H, alkyle en $C_{1-6}$, (alkyl en $C_{1-3}$)aryle), $R^{B6}$-O ($R^{B6}$ est H, alkyle en $C_{1-6}$), F, Cl, Br, $NO_2$, $CF_3$;

$_{R}$B4 est H, alkyle en $C_{1-6}$, $R^{B6}$O, Cl, Br, F, $CF_3$;

$X^B$ est O, S ;

$Y^B$ est =CH-, =N- ;

$Z^B$ est =CH-, =N- ;

$U^B$ est =CH-, =N- ;

$V^B$ est =CH-, =N- ;

B représente d'autre part,

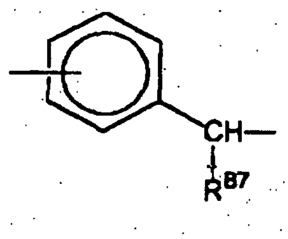

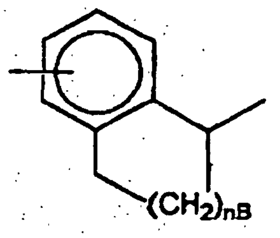

$q^B$ est 0, 1, 2 ;

($R^{B7}$ est alkyle en $C_{1-6}$, cycloalkyle en $C_{3-8}$);

A - B peut représenter

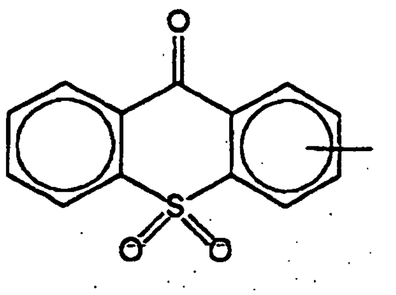

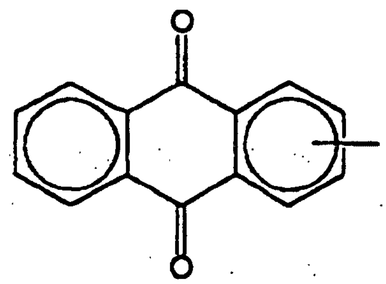

D représente une simple liaison ;

E représente

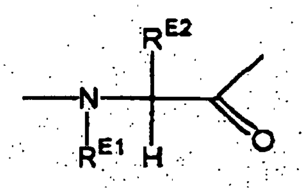

avec $R^{E1}$ représente H ;

$R^{E2}$ représente H, alkyle en $C_{1-6}$ , cycloalkyle en $C_{3-8}$, phényle, pyridyle, furyle, thiényle, imidazolyle, tétrahydropyrannyle, tétrahydrothiopyrannyle, où les restes indiqués peuvent porter jusqu'à trois substituants identiques ou différents du groupe O-alkyle en $C_{1-6}$, F, représente $CH(CH_3)OH$, $CH(CF_3)_2$ ;

les radicaux donnés sous $R^{E1}$ et $R^{E2}$ peuvent être reliés les uns aux autres par une liaison ;

E peut également représenter D-Lys, D-Orn, D-Dab, D-Dap, D-Arg ;

G représente

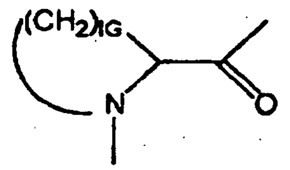

avec $1^G$ = 2, 3, où un radical $CH_2$ du cycle peut être remplacé par $CHCH_3$ ;
G représente également

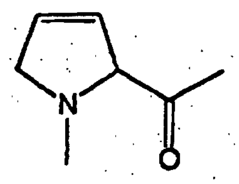

$Q^K$ est

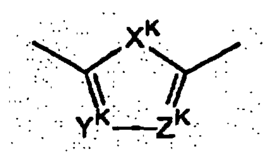

$X^K$ est O, S ;
$Y^K$ est =CH, =N- ;
$Z^K$ est =CH, =N- ;
L

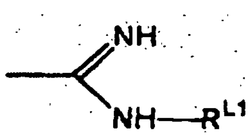 ou 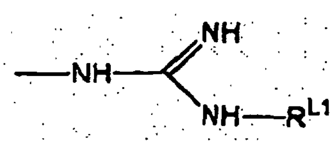

avec $R^{L1}$ est H, OH.

6. Médicament contenant en plus des excipients et auxiliaires usuels, des composés de la formule générale I selon l'une des revendications 1 à 5.

7. Utilisation des composés de la formule générale I selon l'une des revendications 1 à 5, pour la préparation d'agents pharmaceutiques pour le traitement de maladies, qui sont soulagées ou soignées par inhibition partielle ou complète des $C_{1s}$ ou $C_{1r}$.

8. Utilisation des composés de la formule générale I selon l'une des revendications 1 à 5, pour la préparation d'agents pharmaceutiques pour le traitement ou la prophylaxie de

- les troubles de reperfusion après ischémie ; les états ischémiques se développant par exemple pendant des opérations sous assistance de machines coeur-poumons ; des opérations dans lesquelles des vaisseaux sanguins sont clampés en général pour éviter de grandes hémorragies ; l'infarctus du myocarde ; les attaques cérébrales thrombo-emboliques ; les thromboses pulmonaires, etc. ;

- les arrêts organiques hypersévères, en particulier en cas de xénotransplantations ;
- les insuffisances organiques comme par exemple les insuffisances organiques multiples ou ARDS (adult respiratory distress syndrome) ;
- les maladies qui reposent sur un traumatisme (traumatisme crânien) ou un polytraumatisme, comme par exemple les thermotraumatismes (brûlures) et la « thermal injury »;
- le choc anaphylactique ;
- la septicémie, le « vascular leak syndrom » : lors de la septicémie et après traitement avec des agents biologiques, comme l'interleukine 2 ou après transplantation ;
- la maladie d'Alzheimer ainsi que d'autres maladies neurologiques inflammatoires comme la myasthénie grave, la sclérose en plaques, le lupus cérébral, le syndrome de Guillain-Barre ; les méningites ; les encéphalites ;
- le lupus érythémateux systémique (SLE) ;
- la polyarthrite rhumatoïde et d'autres maladies inflammatoires parmi les maladies rhumatoïdes, comme par exemple le syndrome de Behcet ; l'arthrite rhumatoïde juvénile ;
- les inflammations nerveuses de différentes genèses, comme par exemple la glomérulonéphrite, le lupus néphrétique ;
- la pancréatite ;
- l'asthme, la bronchite chronique ;
- les complications lors de la dialyse en cas d'insuffisance rénale ;
- la vasculite, la thyroïdite ;
- les coliques ulcéreuses ainsi que d'autres maladies inflammatoires du tractus gastro-intestinal ;
- les maladies auto-immunes ;
- les naissances prématurées spontanées.

**9.** Composés de la formule générale I selon l'une des revendications 1 à 5, où L correspond à -CN, $-C(=O)NH_2$ ou $-C(=S)NH_2$.